# EUROPEAN PATENT APPLICATION

(11) **EP 4 711 451 A1**
(43) Date of publication of application: **18.03.2026**
(21) Application number: 24803120.5
(22) Date of filing: 11.05.2024
(51) Int. Cl.: C12N 9/22, C12N 15/55, C12N 15/62, C12N 15/113, C12N 15/85, C07K 19/00, C12Q 1/6816, A61K 48/00

(54) **CAS PROTEIN, CORRESPONDING GENE EDITING SYSTEM THEREOF AND USE THEREOF**

(30) Priority: 11.05.2023 CN 202310532845; 22.08.2023 CN 202311060888; 10.11.2023 CN 202311497160
(71) Applicant: Yoltech Therapeutics Co., Ltd, Shanghai 201109 (CN)
(72) Inventor: ZHANG, Hongling, Shanghai 201109 (CN)
(74) Representative: CH Kilger Anwaltspartnerschaft mbB
(86) International application number: PCT/CN2024/092707
(87) International publication number: WO 2024/230842

(57) **Abstract**

The present disclosure provides a Cas protein, a composition comprising the Cas protein, and a CRISPR-Cas system. The present disclosure further provides applications and methods using the composition or the CRISPR-Cas system. The present disclosure further provides a cell comprising the Cas protein, the composition or the CRISPR-Cas system.

## Description

### TECHNICAL FIELD

This disclosure relates to the field of gene editing. Specifically, it relates to a Cas protein and its variant polypeptides, its corresponding gene-editing system and use thereof.

### BACKGROUND

The Clustered Regularly Interspaced Short Palindromic Repeats (CRISPR) system is developed by bacteria and archaea to defend against the DNA of invading bacteriophages. The most common one is the CRISPR/Cas9 system. The Cas9 protein, with the assistance of a trans-encoded small RNA (tracrRNA), can process pre-crRNA into mature crRNA that binds to the tracrRNA. Subsequently, it was discovered that an artificially constructed single-stranded chimeric guide RNA (gRNA) mimicking the crRNA-tracrRNA complex can effectively mediate the recognition and cleavage of a target site by the Cas9 protein. The three bases adjacent to the 3' end of the target site must be in the form of 5'-NGG-3', thereby forming the protospacer adjacent motif (PAM) structure required for the Cas/crRNA complex to recognize the target site.

However, the existing different CRISPR/Cas systems have different advantages and disadvantages. For example, there are differences in the guide RNAs, PAMs, and the size of Cas proteins.

Therefore, there is still a need to develop new Cas proteins and CRISPR-Cas systems to meet diverse application requirements.

### SUMMARY

The main object of the disclosure is to provide a novel Cas protein and its variant polypeptides, as well as a CRISPR-Cas system containing the same, in order to meet the above-mentioned application requirements.

In one aspect, the disclosure provides a Cas protein selected from the group consisting of:
(a) a polypeptide having the amino acid sequence represented by SEQ ID NO:1;
(b) a polypeptide having approximately at least 60% (e.g., 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5%) identity to the amino acid sequence represented by SEQ ID NO:1; and
(c) a polypeptide formed by substitution, deletion or addition of one or more amino acid residues in the amino acid sequence represented by SEQ ID NO:1.

In another aspect, the disclosure provides a fusion protein, which comprises the Cas protein according to the disclosure; and one or more functional domains.

In yet another aspect, the disclosure provides an isolated polynucleotide which encodes the Cas protein according to the disclosure or the fusion protein according to the disclosure;

Preferably, the polynucleotide has been codon-optimized for expression in eukaryotic cells.

Preferably, the polynucleotide has a nucleotide sequence with approximately at least 80% (e.g., approximately at least 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% or 100%) sequence identity to the nucleotide sequence represented by any one of SEQ ID NO: 2, 37, 83, 85, and 87.

Preferably, the polynucleotide has the nucleotide sequence represented by any one of SEQ ID NO: 2, 37, 83, 85, 87.

In yet another aspect, the disclosure provides a guide RNA (gRNA) comprising:
(1) a direct repeat (DR) sequence, which is capable of forming a complex with the Cas protein in the first aspect or the fusion protein in the second aspect;
(2) a spacer sequence, which is capable of hybridizing with a target sequence of a target DNA, thereby guiding the complex to the target DNA.

Optionally, the length of the spacer sequence is at least 15 nt. For example, the length of the spacer sequence is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70 nt.

Optionally, the length of the spacer sequence is 15-50 nt.

Optionally, the length of the spacer sequence is 18-41 nt.

Optionally, the length of the spacer sequence is 18-27 nt.

Optionally, the length of the spacer sequence is 18-24 nt.

Optionally, the length of the spacer sequence is 18-22 nt.

Optionally, the 5' end of the direct repeat sequence is linked to the spacer sequence.

Optionally, the spacer sequence contains at least 15 consecutive nucleotides in the nucleotide sequence of any one of SEQ ID NO: 6, 11, 53, 55, 57, 59, and 61.

In yet another aspect, the disclosure provides an isolated nucleic acid molecule that comprises, or consists of, a sequence selected from the following sequences:
(i) the sequence represented by SEQ ID NO: 5 or 71;
(ii) a sequence obtained by substitution, deletion, or addition of one or more bases (e.g., substitution, deletion, or addition of 1, 2, 3, 4, 5, 6, 7, 8, 9, or 10 bases) in the sequence represented by SEQ ID NO: 5 or 71;
(iii) a sequence having at least 20%, at least 25%, at least 30%, at least 35%, at least 40%, at least 50%, at least 60%, at least 70%, at least 80%, at least 90%, or at least 95% sequence identity to the sequence represented by SEQ ID NO: 5 or 71;
(iv) a sequence that hybridizes to the sequence according to any one of (i)-(iii) under a stringent condition; or
(v) a complementary sequence to the sequence according to any one of (i)-(iii);
and the sequence according to any one of (ii)-(v) substantially retains the biological function of the sequence from which it is derived.

For example, the isolated nucleic acid molecule is an RNA.

For example, the isolated nucleic acid molecule comprises a direct repeat (DR) sequence in a CRISPR/Cas system.

In yet another aspect, the disclosure provides a delivery composition, which comprises a delivery vehicle, and one or more selected from the group consisting of: the Cas protein according to the disclosure, the fusion protein according to the disclosure, the polynucleotide according to the disclosure, the complex according to the disclosure, the vector according to the disclosure, the CRISPR-Cas composition according to the disclosure, and the system according to the disclosure.

In yet another aspect, the disclosure provides a complex that comprises:
(i) a protein component selected from the group consisting of: the Cas protein according to the disclosure, the fusion protein according to the disclosure, or a combination thereof; and
(ii) the guide RNA according to the disclosure;
wherein, the protein component and the nucleic acid component bind to each other to form a complex.

In yet another aspect, the disclosure provides a CRISPR-Cas system that comprises:
(i) the Cas protein according to the disclosure or the fusion protein according to the disclosure, or a polynucleotide encoding the Cas protein or the fusion protein; and
(ii) a guide RNA, or a polynucleotide encoding the guide RNA;
   wherein the guide RNA comprises:
   (a) a direct repeat (DR) sequence capable of forming a complex with the Cas protein or the fusion protein, and
   (b) a spacer sequence capable of hybridizing with a target sequence of a target DNA, thereby guiding the complex to the target DNA.

In yet another aspect, the disclosure provides a CRISPR-Cas composition that comprises:
(i) a first component selected from the group consisting of: the Cas protein according to the disclosure, the fusion protein according to the disclosure, a nucleotide sequence encoding the Cas protein according to the disclosure or the fusion protein according to the disclosure, and any combination thereof; and
(ii) a second component, comprising one or more guide RNAs according to the disclosure, or a nucleotide sequence encoding the one or more guide RNAs according to the disclosure;
wherein the guide RNA is capable of forming a complex with the protein or fusion protein described in (i).

In yet another aspect, the disclosure provides a CRISPR-Cas system comprising one or more vectors, wherein the one or more vectors comprise:
(i) a first nucleic acid, which is a nucleotide sequence encoding the Cas protein according to the disclosure or the fusion protein according to the disclosure; optionally, the first nucleic acid is operably linked to a first regulatory element; and
(ii) a second nucleic acid, which encodes a nucleotide sequence comprising the guide RNA according to the disclosure; optionally, the second nucleic acid is operably linked to a second regulatory element;
wherein:
the first nucleic acid and the second nucleic acid are present in the same vector or in different vectors;
the guide RNA is capable of forming a complex with the protein or fusion protein described in (i).

The disclosure provides a kit comprising one or more components selected from: the Cas protein according to the disclosure, the fusion protein according to the disclosure, the polynucleotide according to the disclosure, the complex according to the disclosure, the vector according to the disclosure, the CRISPR-Cas composition according to the disclosure, and the system according to the disclosure.

In certain embodiments, the kit further includes a label or instructions.

In certain embodiments, the kit is used for one or more of: gene or genome editing, disease treatment, targeting a target gene, and cleaving a gene of interest or a non-target gene.

In yet another aspect, the disclosure provides a delivery composition comprising a delivery vehicle, and one or more selected from: the Cas protein according to the disclosure, the fusion protein according to the disclosure, the polynucleotide according to the disclosure, the complex according to the disclosure, the vector according to the disclosure, the CRISPR-Cas composition according to the disclosure, and the system according to the disclosure.

In yet another aspect, the disclosure provides a host cell comprising the Cas protein according to the disclosure, the fusion protein according to the disclosure, the polynucleotide according to the disclosure, the complex according to the disclosure, the vector according to the disclosure, the composition according to the disclosure, the system according to the disclosure, or the delivery composition according to the disclosure.

In yet another aspect, the disclosure provides an enzyme preparation, comprising the Cas protein according to the disclosure, the fusion protein according to the disclosure, the complex according to the disclosure, the CRISPR-Cas composition according to the disclosure, the system according to the disclosure, or the delivery composition according to the disclosure.

In certain embodiments, the enzyme preparation includes an injection and/or a freeze-dried preparation.

In yet another aspect, the disclosure provides a pharmaceutical kit, which includes:
a first container, and in the first container, the complex according to the disclosure, the composition according to the disclosure, or the system according to the disclosure, or a drug containing the complex according to the disclosure, the composition according to the disclosure, or the system according to the disclosure.

In yet another aspect, the disclosure provides a pharmaceutical kit, which includes:
(a1) a first container, and in the first container, the Cas protein according to the disclosure, the fusion protein according to the disclosure, an encoding gene or expression vector thereof, or a drug containing the Cas protein according to the disclosure, the fusion protein according to the disclosure, or an encoding gene or expression vector thereof; and
(b1) optionally a second container, and in the second container, the guide RNA according to the disclosure or its expression vector, or a drug containing the guide RNA according to the disclosure or its expression vector.

In yet another aspect, the disclosure provides a method for targeting and editing a target gene or cleaving a target gene. The method includes bringing the Cas protein according to the disclosure, the fusion protein according to the disclosure, the complex according to the disclosure, the composition according to the disclosure, the system according to the disclosure, the delivery composition according to the disclosure, the enzyme preparation according to the disclosure, or the pharmaceutical kit according to the disclosure into contact with the target gene; or delivering it into a cell containing the target gene, where the target sequence is present in the target gene.

In yet another aspect, the disclosure provides a method for inducing a change in the state of a cell, comprising: contacting a target gene in the cell with the Cas protein according to the disclosure, the fusion protein according to the disclosure, the complex according to the disclosure, the composition according to the disclosure, the system according to the disclosure, the delivery composition according to the disclosure, the enzyme preparation according to the disclosure, or the pharmaceutical kit according to the disclosure.

In yet another aspect, the disclosure provides a method for altering the expression of a gene product, comprising: bringing the Cas protein according to the disclosure, the fusion protein according to the disclosure, the complex according to the disclosure, the composition according to the disclosure, the system according to the disclosure, the delivery composition according to the disclosure, the enzyme preparation according to the disclosure, or the pharmaceutical kit according to the disclosure into contact with a nucleic acid molecule encoding the gene product; or delivering it into a cell containing the nucleic acid molecule, where the target sequence is present in the nucleic acid molecule.

In yet another aspect, the disclosure provides a cell or its progeny obtained by the method according to the disclosure, wherein the cell comprises a modification that is not present in its wild-type form.

In yet another aspect, the disclosure provides a cell product of the cell or its progeny according to the disclosure.

In yet another aspect, the disclosure provides an in-vitro, ex-vivo or in-vivo cell or cell line or their progeny. The cell or cell line or their progeny comprises: the Cas protein according to the disclosure, the fusion protein according to the disclosure, the polynucleotide according to the disclosure, the complex according to the disclosure, the vector according to the disclosure, the CRISPR-Cas composition according to the disclosure, the system according to the disclosure, or the delivery composition according to the disclosure.

In yet another aspect, the disclosure provides the use of the Cas protein according to the disclosure, the fusion protein according to the disclosure, the polynucleotide according to the disclosure, the complex according to the disclosure, the vector according to the disclosure, the CRISPR-Cas composition according to the disclosure, the system according to the disclosure, the kit according to the disclosure, the delivery composition according to the disclosure, the enzyme preparation according to the disclosure, or the pharmaceutical kit according to the disclosure in the manufacture of a medicament or formulation for nucleic acid editing (e.g. gene or genome editing).

In yet another aspect, the disclosure provides the use of the Cas protein according to the disclosure, the fusion protein according to the disclosure, the polynucleotide according to the disclosure, the complex according to the disclosure, the vector according to the disclosure, the CRISPR-Cas composition according to the disclosure, the system according to the disclosure, the kit according to the disclosure, the delivery composition according to the disclosure, the enzyme preparation according to the disclosure, or the pharmaceutical kit according to the disclosure in the manufacture of a medicament or formulation for one or more selected from the group consisting of:
(i) *ex-vivo* gene editing or genome editing;
(ii) *ex-vivo* detection of a single-stranded DNA;
(iii) modifying an organism or a non-human organism by editing a target sequence in a target locus;
(iv) treating a disorder caused by a defect in a target sequence in a target locus; and
(v) treating a disorder or disease of a subject in need.

In yet another aspect, the disclosure provides a method for detecting the presence of a target nucleic acid molecule in a sample, comprising: contacting the sample with the Cas protein according to the disclosure, the fusion protein according to the disclosure, the complex according to the disclosure, the CRISPR-Cas composition according to the disclosure, the system according to the disclosure, the kit according to the disclosure, the delivery composition according to the disclosure, or the enzyme preparation according to the disclosure, and a non-target sequence; and detecting a detectable signal generated by the cleavage of the non-target sequence, thereby detecting the target nucleic acid molecule, wherein the non-target sequence does not hybridize to the guide RNA.

In certain embodiments, if the non-target sequence is cleaved by the protein in the complex, the CRISPR-Cas composition, the system, or the delivery composition, it indicates the presence of the target nucleic acid molecule in the sample; and if the non-target sequence is not cleaved by the protein in the complex, the CRISPR-Cas composition, the system, or the delivery composition, it indicates the absence of the target nucleic acid molecule in the sample.

In yet another aspect, the disclosure provides a method for diagnosing, preventing, or treating a disease of a subject in need, comprising: administering to the subject the vector according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, the enzyme preparation according to the disclosure, or the pharmaceutical kit according to the disclosure, wherein the disease is associated with a target DNA, the spacer sequence is capable of hybridizing to a target sequence of the target DNA, and the target DNA is modified, thereby diagnosing, preventing or treating the disease.

In yet another aspect, the disclosure provides a pharmaceutical composition which comprises the vector according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, the host cell according to the disclosure, or the enzyme preparation according to the disclosure; and a pharmaceutically acceptable carrier or excipient.

According to WIPO Standard ST.26, the symbol "t" is used to represent T in DNA and U in RNA. Therefore, in the sequence listing prepared in accordance with ST.26, when the sequence is RNA, T in the sequence should be interpreted as U.

It should be understood that within the scope of the disclosure, the above-mentioned technical features of the disclosure and the technical features specifically described hereinafter (such as in the examples) can be combined with each other to formulate new or preferred technical solutions. Due to space limitations, they will not be repeated here one by one here.

### DESCRIPTION OF THE DRAWINGS

Understanding of certain features and advantages of the disclosure will be obtained with reference to the following detailed description and the drawings. The following detailed description sets forth illustrative embodiments in which the principles of the disclosure can be utilized, and in these drawings:
Figure 1 shows the maps of the CasY7 recombinant expression plasmid (Figure 1A) and the LbCpf1 recombinant expression plasmid (Figure 1B).
Figure 2 shows the predicted secondary structure of the RNA transcript of the direct repeat (DR) sequence corresponding to CasY7.
Figure 3 shows the map of the Target plasmid used to evaluate the cleavage activity of CasY7 in *Escherichia coli.*
Figure 4 shows a comparison of editing efficiencies between CasY7 and LbCpf1 in Escherichia coli.
Figure 5 shows the map of the PHK09T plasmid for expressing gRNA.
Figure 6 shows a comparison of editing efficiencies between CasY7 and LbCpf1 in HEK293T cells.
Figure 7 shows four mutants of CasY7 with different point mutations, which eliminate the catalytic activity (cleavage activity) compared to the WT CasY7 protein.
Figure 8 shows the activity test of a base editor containing dCasY7 in base editing (A>G). Figure 8A shows the single-base editing (A>G) efficiency at sites A2, A4, A13, A15-A17 of a target sequence in the TTR gene; Figure 8B shows the single-base editing (A>G) efficiency at sites A7, A13, A20 of another target sequence in the TTR gene.
Figure 9 shows a comparison of cleavage activity of different mutants of CasY7 on a target sequence in the TTR gene.
Figure 10 shows a comparison of cleavage activity of different mutants of CasY7 on a target sequence in the PD-1 gene, wherein WT represents the cleavage activity of wild-type CasY7.
Figure 11 shows a comparison of cleavage activity of different mutants of CasY7 on the Trac-1 target sequence in the TRAC gene.
Figure 12 shows a comparison of cleavage activity of different mutants of CasY7 on the Trac-2 target sequence in the TRAC gene.
Figure 13 shows a comparison of cleavage activity of different mutants of CasY7 on the Trac-3 target sequence in the TRAC gene.
Figure 14 shows a comparison of cleavage activity of different mutants of CasY7 on the Trac-4 target sequence in the TRAC gene.
Figure 15 shows the predicted secondary structure of the RNA transcript of the variant DR-1 of the DR sequence.
Figure 16 shows a comparison of cleavage activity of CasY7 targeting a target sequence in the TTR gene mediated by gRNAs containing different DR sequences.
Figure 17 shows a dual-luciferase reporter system. Figure 17A shows the constitution of the LU×UC sequence, and Figure 17B shows the map of the dual-luciferase reporter plasmid.
Figure 18 shows the editing activity of the fusion protein of CasY7 variant C05440 and T5 on the target sequence of the hHAO1 gene. The results show that the editing activity of C05440-T5 is the best, much higher than that of T5-C05440 and C05440.
Figure 19 shows the on-target cleavage activity on the Trac gene target mediated by CasY7, as well as the off-target cleavage at 28 OT (off-target) targets.

### DETAILED EMBODIMENTS

The following examples are only used to describe the disclosure, not to limit it. Unless otherwise specified, the experiments and methods described in the examples are basically carried out according to the conventional methods well-known in the art and described in various references.

In addition, for those examples where specific conditions are not indicated, they are carried out according to the conventional conditions or the conditions recommended by the manufacturer. Reagents or instruments for which the manufacturers are not specified are all conventional products commercially available. Those skilled in the art are aware that the examples illustrate the disclosure by way of example and are not intended to limit the scope of protection of the invention. All publications and other references mentioned herein are incorporated herein by reference in their entireties.

To provide a better understanding of the disclosure, certain terms are first defined. As used in this application, unless otherwise explicitly defined herein, each of the following terms shall have the meaning given below. Other definitions are set forth throughout the application.

### Terms

Unless otherwise described, all technical and scientific terms used herein have the same meaning as commonly understood by one of ordinary skill in the art. For the purposes of interpreting this specification, the following terms will be used for description, and where appropriate, terms used in the singular form also include the plural, and vice versa. The contents of all patents and other publications cited herein are incorporated herein by reference in their entirety. In the event of any conflict between the description and interpretation of terms herein and those in any document incorporated herein by reference, the following description and interpretation of terms shall prevail.

As used herein, the singular forms "a/an" and "the" include both singular and plural referents, unless the context clearly dictates otherwise.

As used herein, the term "about" may refer to a value or composition within an acceptable error range for a specific value or composition as determined by one of ordinary skill in the art, which partially depends on how the value or composition is measured or determined. For example, as used herein, the expression "about 100" includes all values between 99 and 101 (e.g., 99.1, 99.2, 99.3, 99.4, etc.).

As used herein, the terms "comprising" or "including" can be open-ended, semi-close-ended, or close-ended. In other words, these terms also include "consisting essentially of" or "consisting of".

As used herein, the term "optional" or "optionally" means that the subsequently described event, situation, or substituent may or may not occur, and this expression includes examples where the event or situation occurs and examples where the event or situation does not occur.

As used in the text, the term "substantially" or "essentially" refers to a degree, quantity, level, value, number, frequency, percentage, scale, size, amount, weight, or length that is approximately 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or higher as compared to a degree, reference quantity, level, value, number, frequency, percentage, scale, size, amount, weight, or length. For example, as used herein, a "substantially identical sequence" may refer to a sequence, polynucleotide, or polypeptide having a certain degree of identity to a reference sequence.

As used herein, the term "and/or" refers to either or both of the alternatives.

As used herein, the terms "nucleic acid", "nucleic acid molecule", and "polynucleotide" are used interchangeably and refer to a polymeric form of nucleotides of any length, deoxyribonucleotides or ribonucleotides or analogs thereof, in single-stranded, double-stranded, or multi-stranded form. A polynucleotide can be exogenous or endogenous to a cell. A polynucleotide can exist in a cell-free environment. A polynucleotide can be a gene or a fragment thereof. A polynucleotide can be DNA or RNA. A polynucleotide can include one or more analogs (e.g., modified backbones, sugars, or nucleobases). Modifications to the nucleotide structure, if present, can be introduced either before or after assembly of the polymer. Some non-limiting examples of analogs include: 5-bromouracil, peptide nucleic acids, xeno nucleic acids, morpholinos, locked nucleic acids, glycol nucleic acids, threose nucleic acids, dideoxynucleotides, cordycepin, 7-deaza-GTP, fluorophores (e.g., rhodamine or fluorescein attached to a sugar), thiol-containing nucleotides, biotin-linked nucleotides, fluorescent base analogs, CpG islands, methyl-7-guanosine, methylated nucleotides, inosine, thiouridine, pseudouridine, dihydrouridine, queuosine, and wyosine. Non-limiting examples of polynucleotides include coding or non-coding regions of a gene or gene fragment, multiple loci (or one locus) defined according to linkage analysis, exons, introns, messenger RNA (mRNA), transfer RNA (tRNA), ribosomal RNA (rRNA), small interfering RNA (siRNA), short hairpin RNA (shRNA), microRNA (miRNA), ribozymes, cDNA, recombinant polynucleotides, branched polynucleotides, plasmids, vectors, isolated DNA of any sequence, isolated RNA of any sequence, cell-free polynucleotides including cell-free DNA (cfDNA) and cell-free RNA (cfRNA), nucleic acid probes, and primers. A nucleotide sequence can be interspersed with non-nucleotide components. A polynucleotide may include a mixture of nucleotides found in the nature and nucleotide analogs (e.g., synthetic nucleotide analogs).

As used herein, the terms "peptide", "polypeptide", and "protein" are used interchangeably herein and generally refer to a polymer of at least two amino acid residues linked by peptide bonds. This term does not denote a specific length of the polymer, nor is it intended to imply or distinguish whether the peptide is produced by recombinant techniques, chemical or enzymatic synthesis, or is naturally occurring. The term applies to naturally occurring amino acid polymers as well as amino acid polymers that include at least one modified amino acid. In some cases, the polymer may be interspersed with non-amino acids. The term encompasses amino acid chains of any length, including full-length proteins as well as proteins with or without secondary and/or tertiary structures (e.g., domains). The term also encompasses amino acid polymers that have been modified; for example, modified by disulfide bond formation, glycosylation, lipidation, acetylation, phosphorylation, oxidation, and any other means, such as conjugation to a label.

As used herein, the term "identity" refers to the overall relatedness between polymer molecules, for example, between nucleic acid molecules (e.g., DNA molecules and/or RNA molecules) and/or between polypeptide molecules. Sequence identity (or homology) is determined by comparing two aligned sequences along a predefined comparison window, which can be 50%, 60%, 70%, 80%, 90%, 95%, or 100% of the length of a reference nucleotide sequence or protein, and determining the number of positions at which the identical residues occur. Commonly, it is expressed as a percentage. Taking polypeptide sequences as an example, if the mutation is one or more of substitution/replacement of one or more amino acids/nucleotides, insertion within a sequence, and deletion within a sequence, then the total number of residues is calculated based on the larger one of two molecules being compared. If the mutation also includes insertions (extensions) at either end or both ends of a sequence or deletions (truncations) at either end or both ends of a sequence, then the number of amino acids inserted or deleted at one or both ends (e.g., the number of insertions or deletions at both ends is less than 20) is not counted in the total number of residues. In calculation of the percentage of identity, the sequences being compared are aligned in a way allowing the maximum match between the sequences, and gaps in the alignment (if present) are resolved by a specific algorithm. The same applies to the calculation of nucleotide sequence identity.

As used herein, the terms "amino acid" and "amino acids" generally refer to natural and non-natural amino acids, including but not limited to modified amino acids and amino acid analogs. Modified amino acids may include natural and non-natural amino acids that have been chemically modified to contain groups or chemical moieties that do not naturally occur on amino acids. Amino acid analogs may refer to amino acid derivatives. The term "amino acid" encompasses D-amino acids and L-amino acids.

As used herein, the term "nuclease" refers to a polypeptide capable of cleaving the phosphodiester bond between nucleotide subunits of a nucleic acid; the term "endonuclease" refers to a polypeptide capable of catalyzing (e.g. cleaving) the phosphodiester bond within a polynucleotide (e.g. DNA or RNA) chain.

As used herein, the term "exonuclease" refers to a protein or polypeptide capable of digesting a nucleic acid (e.g., RNA or DNA) from a free end thereof.

As used herein, the term "non-natural" may generally refer to a nucleic acid or polypeptide sequence that is not found in a natural nucleic acid or protein. "Non-natural" may refer to an affinity tag. "Non-natural" may refer to a fused part. "Non-natural" may refer to a naturally occurring nucleic acid or polypeptide sequence that includes mutations, insertions, and/or deletions. A non-natural sequence can exhibit and/or encode an activity (e.g., enzymatic activity, methyltransferase activity, acetyltransferase activity, kinase activity, ubiquitination activity, etc.) that can also be exhibited by a nucleic acid and/or polypeptide sequence fused to the non-natural sequence. A non-natural nucleic acid or polypeptide sequence can be genetically engineered to be linked to a naturally occurring nucleic acid or polypeptide sequence (or a variant thereof) to produce a chimeric nucleic acid and/or a polypeptide sequence encoded by a chimeric nucleic acid and/or polypeptide.

As used herein, the term "fusion protein" refers to a hybrid polypeptide that contains protein domains from at least two different proteins. One protein may be located at the amino-terminal (N-terminal) or at the carboxyl-terminal (C-terminal) of the fusion protein, thus forming an amino-terminal fusion protein or a carboxyl-terminal fusion protein, respectively. The protein may contain different domains, for example, a nucleic acid-binding domain (e.g., the gRNA-binding domain of a Cas protein, which guides the binding of the protein to a target site) and a nucleic acid-cleaving domain, or the catalytic domain of a nucleic acid-editing protein. In some embodiments, there may be a linker between the proteins. In some embodiments, the protein contains a protein part (e.g., an amino acid sequence that constructs a nucleic acid-binding domain) and an organic compound (e.g., a compound that can act as a nucleic acid-cleaving agent). In some embodiments, the protein is complexed or associated with a nucleic acid (e.g., RNA or DNA).

As used herein, the term "linker" refers to any means, entity, or moiety used to join two or more entities. In some embodiments, the linker is a covalent linker. In some embodiments, the linker is a non-covalent linker. Examples of covalent linkers include a covalent bond, or a linker covalently attached to one or more proteins or domains to be linked. In some embodiments, the linker is a non-covalent bond, such as a metal-organic bond through a metal center (such as a platinum atom). The joining can be permanent or reversible. For covalent linkage, various functional groups can be used, such as amide groups, including carbonate derivatives, ethers, esters (including organic and inorganic esters), amines, carbamates, urea, etc. To provide linkage, a domain can be modified by oxidation, hydroxylation, substitution, reduction, or the like to provide a coupling site. Conjugation methods are well known to those skilled in the art and are included for use in the disclosure. A linker includes, but is not limited to, a chemical linker, or for example a peptide linker (a linker sequence). The length and type of a linker can be designed as needed. In some embodiments, a linker can be an artificially synthesized amino acid sequence or a naturally occurring polypeptide sequence. It should be understood that modifications that do not significantly reduce the functions of the RNA-binding domain and the effector domain are preferred.

As used herein, the term "complementary" refers to the ability of the nucleobases of a first polynucleotide sequence (e.g., a guide sequence) to base-pair with the nucleobases of a second polynucleotide sequence (such as a target sequence) through traditional Watson-Crick base-pairing. In some embodiments, a first polynucleotide may be substantially complementary to a second polynucleotide, that is, it has at least approximately 80%, 81%, 82%, 83%, 84%, 85%, 86%, 87%, 88%, 89%, 90%, 91%, 92%, 93%, 94%, 95%, 96%, 97%, 98%, or 99% complementarity to the second polynucleotide. In some embodiments, a first polynucleotide is completely complementary to a second polynucleotide, that is, having 100% complementarity to the second polynucleotide. "Substantially complementary" refers to a degree of complementarity of at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50 or more nucleotides, or refers to two nucleic acids that hybridize under a stringent condition. The term "stringent condition" related to hybridization refers to a condition under which a nucleic acid complementary to a target sequence hybridizes predominantly to the target sequence, and substantially does not hybridize to non-target sequences. Stringent conditions are usually sequence-dependent and depend on many factors. Generally, the longer the sequence, the higher the temperature at which the sequence specifically hybridizes to its target sequence. "Hybridization" refers to a reaction in which one or more polynucleotides react to form a complex that is stabilized by hydrogen-bonding between the bases of these nucleotide residues. The complex may include two strands forming a duplex, three or more strands forming a multi-chain complex, a self-hybridizing single strand, or any combination of these. The hybridization reaction can serve as a step in a larger process (such as the start of PCR, or the cleavage of a polynucleotide by an enzyme). A sequence capable of hybridizing to a given sequence is called the "complement" of the given sequence.

As used herein, the term "regulatory element" refers to a DNA sequence that controls or influences one or more aspects of transcription and/or expression, and is intended to include promoters, enhancers, silencers, transcription termination signals, internal ribosome entry sites (IRES), protein degradation signals, and other expression control elements (such as polyadenylation signals and poly-U sequences, and such regulatory elements are described in, for example, Goeddel, GENE EXPRESSION TECHNOLOGY: METHODS IN ENZYMOLOGY 185, Academic Press, San Diego, California (1990)). Regulatory elements include those that direct constitutive expression of a nucleotide sequence in many types of host cells, as well as those that direct expression of a nucleotide sequence only in certain host cells (e.g., tissue-specific regulatory sequences). Regulatory elements can also direct expression in a time-dependent manner, e.g., in a cell-cycle-dependent or developmental-stage-dependent manner, which may or may not be tissue-specific or cell-type-specific.

As used herein, the term "domain" or "protein domain" refers to a part of a protein sequence that can exist and function independently of the rest of the protein chain.

As used herein, the term "operably linked" refers to a functional connection between a regulatory sequence and a target nucleic acid sequence, resulting in the expression of the latter (e.g., in an in vitro transcription/translation system, or in a host cell when a vector is introduced into the host cell). For example, a first nucleic acid sequence is operably linked to a second nucleic acid sequence when the first nucleic acid sequence is placed in a functional relationship with the second nucleic acid sequence. For instance, a promoter is operably linked to a coding sequence if the promoter affects the transcription or expression of the coding sequence.

As used herein, the term "wild-type" is a term understood by those skilled in the art and refers to the typical form of an organism, strain, gene, or trait which naturally occurs and is distinct from a mutant or variant form. Therefore, as used herein, when an amino acid or nucleotide sequence refers to a wild-type sequence, a variant refers to a variant of said sequence, e.g., including substitutions, deletions, or insertions. For example, the "wild-type Cas protein" according to the disclosure refers to a naturally occurring Cas protein that has not been artificially modified. Its nucleotides can be obtained through genetic engineering techniques such as genome sequencing, polymerase chain reaction (PCR), etc., and its amino acid sequence can be deduced from the nucleotide sequence.

As used herein, the terms "parent", "parent polypeptide", and "parent sequence" refer to an original polypeptide (e.g., a reference polypeptide or starting polypeptide) to be altered to generate a variant polypeptide according to the disclosure.

As used herein, the term "variant polypeptide" refers to a polypeptide that contains changes (e.g., but not limited to, substitutions, insertions, deletions, additions, and/or fusions) at one or more residue positions as compared to a parent polypeptide. In some embodiments, a variant polypeptide is different from its reference. For example, a variant polypeptide may differ from a reference polypeptide by one or more differences in the amino acid sequence and/or one or more differences in chemical moieties (e.g., carbohydrates, lipids, etc.) covalently attached to the polypeptide backbone. In some embodiments, a variant polypeptide exhibits an overall sequence identity to a reference polypeptide (e.g., the Cas protein according to the disclosure), wherein the sequence identity is at least 60%, 65%, 70%, 75%, 80%, 85%, 86%, 87%, 88%, 89%, 90%, 91%, 92%, 93%, 94%, 95%, 96%, 97%, 98%, or 99%. Alternatively or additionally, in some embodiments, a variant polypeptide does not share at least one characteristic sequence element with a reference polypeptide. In some embodiments, a reference polypeptide has one or more biological activities. In some embodiments, a variant polypeptide shares one or more biological activities of the reference polypeptide, such as nuclease activity. In some embodiments, a variant polypeptide lacks one or more biological activities of the reference polypeptide. In some embodiments, compared to the reference polypeptide, a variant polypeptide exhibits a reduced level of one or more biological activities (e.g., nuclease activity, e.g., off-target nuclease activity). In some embodiments, if a polypeptide of interest has the same amino acid sequence as a parent except for a small number of sequence changes at specific positions, the polypeptide of interest is considered a "variant" of the parent or reference polypeptide. Typically, a variant has substitutions less than 20%, 15%, 10%, 9%, 8%, 7%, 6%, 5%, 4%, 3%, 2%, or 1% of the residues as compared to the parent or reference polypeptide. In some embodiments, a variant has 10, 9, 8, 7, 6, 5, 4, 3, 2, or 1 substituted residue as compared to the parent or reference polypeptide. A variant often has a very small number (e.g., less than 5, 4, 3, 2, or 1) of substituted functional residues (i.e., residues involved in a specific biological activity). In some embodiments, as compared to the parent or reference polypeptide, a variant has no more than 5, 4, 3, 2, or 1 addition or deletion, and often no addition or deletion. Furthermore, any addition or deletion involves typically less than about 40, about 35, about 30, about 25, about 20, about 19, about 18, about 17, about 16, about 15, about 14, about 13, about 12, about 11, about 10, about 9, about 8, about 7, about 6 residues, and usually less than about 5, about 4, about 3, or about 2 residues. In some embodiments, the parent or reference polypeptide is the wild type. A variant of a polynucleotide or polypeptide may be naturally occurring, such as an allelic variant, or may be a variant known to be non-naturally occurring. Non-naturally occurring variants of polynucleotides and polypeptides can be prepared by mutagenesis techniques, by direct synthesis, or by other recombinant methods known to those skilled in the art.

As used herein, the term "conservative amino acid substitution" refers to substitution of an amino acid with a chemically or functionally similar one without affecting the normal function of the protein. For example, amino acid residues having similar side chains have interchangeability within a protein: the group of amino acids with aliphatic side chains, consisting of glycine, alanine, valine, leucine, and isoleucine; the group with aliphatic-hydroxyl side chains, consisting of serine and threonine; the group with amide-containing side chains, consisting of asparagine and glutamine; the group with aromatic side chains, consisting of phenylalanine, tyrosine, and tryptophan; the group with basic side chains, consisting of lysine, arginine, and histidine; and the group with sulfur-containing side chains, consisting of cysteine and methionine. In some embodiments, amino acid substitutions that are considered conservative include: aspartic acid-glutamic acid, lysine-arginine-histidine, serine-threonine-asparagine-glutamine, glycine-alanine-valine-leucine-isoleucine, cysteine-methionine-proline, and phenylalanine-tyrosine-tryptophan. In some embodiments, amino acid substitutions that are considered conservative include: valine-leucine-methionine-isoleucine, and alanine-serine-threonine.

As used herein, the terms "clustered regularly interspaced short palindromic repeats (CRISPR) - associated (Cas) (CRISPR-Cas) system" and "CRISPR system" are used interchangeably and have the meaning commonly understood by those skilled in the art, which generally encompass transcriptional products or other elements related to the expression of CRISPR-associated ("Cas") genes, or transcriptional products or other elements capable of guiding the activity of said Cas genes.

As used herein, the term "Cas protein" is used interchangeably with Cas polypeptide in the disclosure and is used in the broadest sense, including the parent or reference Cas protein (e.g., the Cas12i protein comprising the sequence represented by SEQ ID NO:1), its derivatives or variants, and functional fragments, such as its nucleic-acid-binding fragment, including the endonuclease-defective (inactive) Cas polypeptide (dCas).

As used herein, the term "complex" refers to a combination of two or more molecules. In some embodiments, a complex includes a polypeptide and a nucleic acid molecule that interact with each other (e.g., binding, contacting, adhering). For example, the term "complex" may refer to a combination of a guide RNA and a polypeptide (e.g., a Cas protein). Alternatively, the term "complex" may refer to a combination of a guide RNA, a Cas protein, and a complementary region of a target nucleic acid.

As used herein, the term "cleavage" refers to breakage of the covalent backbone of a DNA molecule. The cleavage can be carried out by various methods, including but not limited to enzymatic or chemical hydrolysis of a phosphodiester bond. Both single-strand cleavage and double-strand cleavage are possible, and double-strand cleavage can occur as a result of two distinct single-strand cleavage events. Cleavage of DNA can result in blunt ends or sticky ends.

The term "cleave" or "cleavage" refers to the hydrolysis of at least one phosphodiester bond within the backbone of a target nucleotide sequence, which leads to breakage of a single strand or double strands of a target sequence. For example, the Cas protein according to the disclosure or its variant polypeptide can serve as an endonuclease, or an exonuclease (removing consecutive nucleotides from the end (5' end and/or 3' end) of a polynucleotide) to cleave nucleotides within a polynucleotide. In some embodiments, the cleavage of a target polynucleotide by the Cas protein according to the disclosure or its variant polypeptide can result in staggered breaks or blunt ends.

As used herein, the term "protospacer adjacent motif" or "PAM" refers to a short sequence (or motif) adjacent to the protospacer sequence on the non-target strand of a target nucleic acid recognized by a CRISPR complex. The target nucleic acid is a double-stranded DNA (dsDNA), of which one strand contains the target sequence adjacent to PAM and is called the "PAM strand" (e.g., the non-target strand or the non-spacer-complementary strand), and the other complementary strand is called the "non-PAM strand" (e.g., the target strand or the spacer-complementary strand). As used herein, the term "adjacent" includes the situation where the RNA guide of the complex specifically binds, interacts, or associates with the target sequence immediately adjacent to PAM. In such cases, there are no nucleotides between the target sequence and PAM. The term "adjacent" also includes the situation where a small number (e.g., 1, 2, 3, 4, or 5) of nucleotides exist between the target sequence bound to the targeting moiety and PAM.

As used herein, the terms "guide nucleic acid", "RNA guide", "RNA guide sequence", "guide RNA (gRNA)", and "single-guide RNA (sgRNA)" are used interchangeably and refer to a nucleic-acid-based molecule capable of forming a complex with a CRISPR-Cas protein (e.g., the Cas protein according to the disclosure), and includes a sequence (e.g., guide sequence) that is sufficiently complementary to the target nucleic acid to hybridize to the target nucleic acid and guide the complex to the target nucleic acid. The nucleic acid includes, but is not limited to, RNA-based molecules, such as guide RNA. The guide nucleic acid may contain a region that can be referred to as a "nucleic-acid-targeting segment" or "nucleic-acid-targeting sequence", which may contain a sub-region that can be referred to as a "protein-binding segment" or "protein-binding sequence" or "Cas-protein-binding segment". The guide nucleic acid may be a DNA molecule, an RNA molecule, or a DNA/RNA hybrid molecule. A "DNA/RNA hybrid molecule" refers to a nucleic acid containing one or more modified or unmodified ribonucleotides and one or more modified or unmodified deoxyribonucleotides, continuous or not. However, a "DNA molecule" or "RNA molecule" may also refer to a DNA molecule containing one or more modified or unmodified ribonucleotides, continuous or not, or an RNA molecule containing one or more modified or unmodified deoxyribonucleotides, continuous or not.

As used herein, the term "activity" refers to biological activity. In some embodiments, nuclease activity includes the enzymatic activity of a nuclease, such as catalytic ability. For example, nuclease activity can include endonuclease activity. In some embodiments, nuclease activity includes binding activity, such as the binding activity of a nuclease to an RNA guide and/or a target nucleic acid.

As used herein, the terms "upstream" and "downstream" refer to the relative positions within a single nucleic acid (e.g., DNA) sequence of a nucleic acid molecule. "Upstream" and "downstream" respectively refer to the 5'-to-3' direction in which RNA transcription proceeds. A first sequence is upstream of a second sequence when the 3' end of the first sequence is present before the 5' end of the second sequence. A first sequence is downstream of a second sequence when the 5' end of the first sequence is present after the 3' end of the second sequence.

As used herein, "regulatory elements" include promoters, enhancers, internal ribosome entry sites (IRES), and other expression control elements (such as transcription termination signals, like polyadenylation signals, poly-U sequences), and detailed descriptions can be seen in Goeddel, GENE EXPRESSION TECHNOLOGY: METHODS IN ENZYMOLOGY 185, Academic Press, San Diego, Calif (1990). In some cases, regulatory elements include those sequences that direct constitutive expression of a nucleotide sequence in many types of host cells, as well as those sequences that direct the expression of the nucleotide sequence only in certain host cells (e.g., tissue-specific regulatory sequences). Tissue-specific promoters can primarily direct expression in the desired tissue of interest, such as muscle, neurons, bone, skin, blood, specific organs (e.g., liver, pancreas), or specific cell types (e.g., lymphocytes). In other cases, regulatory elements can also direct expression in a time-dependent manner (such as in a cell-cycle-dependent or development-stage-dependent manner), which may or may not be tissue-specific or cell-type-specific. The term "promoter" refers to a non-coding nucleotide sequence that is located upstream of a gene and can initiate the expression of a downstream gene. A constitutive promoter is a nucleotide sequence that, when operably linked to a polynucleotide encoding or defining a gene product, causes production of the gene product in a cell under most or all physiological conditions of the cell. An inducible promoter is a promoter that selectively expresses a coding sequence or functional RNA in response to the presence of an endogenous or exogenous stimulus, such as in response to a chemical compound (chemical inducer), or in response to environmental, hormonal, chemical, and/or developmental signals. Inducible or regulatable promoters include, for example, those induced or regulated by light, heat, stress, waterlogging or drought, salt stress, osmotic stress, plant hormones, wounding, or chemicals (such as ethanol, abscisic acid (ABA), jasmonates, salicylic acid, or safeners).

As used herein, the term "on-target" refers to the binding, cleavage, and/or editing of a predetermined or intended region of DNA by the Cas protein according to the disclosure or its variant polypeptide.

As used herein, the term "off-target" refers to the binding, cleavage, and/or editing of a region of DNA that is not predetermined or intended, by for example the Cas protein according to the disclosure or its variant polypeptide. In some embodiments, a DNA region is an off-target region when it differs from the predetermined or intended DNA region to be bound, cleaved, and/or edited via 1, 2, 3, 4, 5, 6, 7, 8, 9, 10 or more nucleotides. In some embodiments, such sites are detected by targeted sequencing of off-target sites predicted by in-silico simulation, or other methods known in the art.

As used herein, the term "in vivo" refers to events that occur within a multicellular organism such as a human or non-human animal. In the case of a cell-based system, it can be used to refer to events that occur within living cells (as opposed to, for example, an in vitro system).

As used herein, the term "ex vivo/in vitro" refers to events that occur in cells or tissues that grow outside a multicellular organism rather than inside a multicellular organism.

As used herein, the term "cell" should be understood to refer not only to a specific single cell, but also to the progeny or potential progeny of the cell. Since certain modifications may occur in the progeny due to mutations or environmental influences, in fact, such progeny may be different from the parent cell, but are still included within the scope of this term.

As used herein, the terms "subject", "individual", and "patient" are used interchangeably herein and refer to a vertebrate, preferably a mammal, more preferably a human. Mammals include, but are not limited to, murine, simians, humans, farm animals, sport animals, and pets. Also encompassed are tissue, cells, and their progeny of biological entities obtained in vivo or cultured in vitro.

As used herein, the term "disease" refers to any disorder or condition that impairs or interferes with the normal functions of a cell, tissue, or organ, including but not limited to the specific diseases that have been medically or clinically defined.

As used herein, the term "treatment" refers to the administration of a therapeutic molecule (e.g., the CRISPR-Cas system described herein), which partially or completely alleviates, improves, relieves, or inhibits one or more symptoms or signs of a disease, disorder, and/or condition, delays the onset of one or more symptoms or signs of a disease, disorder, and/or condition, reduces the severity of one or more symptoms or signs of a disease, disorder, and/or condition, and/or reduces the incidence of one or more symptoms or signs of a disease, disorder, and/or condition. Such treatment can be applied to subjects who do not exhibit signs of a relevant disease, disorder, and/or condition and/or to subjects who exhibit only signs of an early stage of a disease, disorder, and/or condition. Alternatively or additionally, such treatment can be applied to subjects who exhibit one or more established signs of a relevant disease, disorder, and/or condition.

As used herein, a "biological sample" may contain whole cells and/or living cells and/or cell debris. A biological sample may include (or be derived from) a "body fluid". In some embodiments, the body fluid may be selected from amniotic fluid, aqueous humor, vitreous humor, bile, serum, milk, cerebrospinal fluid, cerumen (earwax), chyle, chyme, endolymph, perilymph, exudate, feces, female ejaculate, gastric acid, gastric juice, lymph, mucus (including nasal drainage and sputum), pericardial fluid, peritoneal fluid, pleural fluid, pus, rheum, saliva, sebum (skin oil), semen, sputum, synovial fluid, sweat, tears, urine, vaginal secretions, vomit, and a mixture of one or more thereof. Biological samples include cell cultures, body fluids, and cell cultures from body fluids. Body fluids may be obtained from a mammal, for example, by puncture or other collection or sampling procedures.

As used herein, the term "activity" refers to biological activity. In some embodiments, nuclease activity includes the enzymatic activity of a nuclease, such as catalytic ability. For example, nuclease activity can include endonuclease activity. In some embodiments, nuclease activity includes binding activity, such as the binding activity of a nuclease to an RNA guide and/or a target nucleic acid.

As used herein, "expression of a genomic locus" or "gene expression" is a process by which information from a gene is used to synthesize a functional gene product. The product of gene expression is usually a protein, but for non-protein-coding genes such as rRNA genes or tRNA genes, the product is a functional RNA. All known life forms, eukaryotes (including multicellular organisms), prokaryotes (bacteria and archaea), and viruses, use gene expression to produce functional products for survival. As used herein, the "expression" of a gene or nucleic acid encompasses not only cellular gene expression, but also the transcription and translation of nucleic acids in a cloning system and in any other context. As used herein, "expression" also refers to the process of transcription of a polynucleotide from a DNA template (such as transcription into mRNA or other RNA transcripts) and/or the subsequent translation of the transcribed mRNA into a peptide, polypeptide, or protein. The transcript and the encoded polypeptide can be collectively referred to as "gene products". If a polynucleotide is derived from genomic DNA, expression can include splicing of mRNA in a eukaryotic cell.

Any protein provided herein can be produced by any method known in the art. For example, the proteins provided herein can be produced via expression and purification of recombinant proteins, which is particularly suitable for fusion proteins containing peptide linkers. Methods for expression and purification of recombinant proteins are well-known and include those described in Molecular Cloning: A Laboratory Manual (Green and Sambrook, Molecular Cloning: A Laboratory Manual (4th ed., Cold Spring Harbor Laboratory Press, Cold Spring Harbor, N.Y. (2012))), the entire content of which is incorporated herein by reference.

Various embodiments are described hereinafter. It should be noted that a specific embodiment is not intended as an exhaustive description or as a limitation of broader aspects discussed herein. An aspect described in connection with a specific embodiment is not necessarily limited to that embodiment and can be practiced with any other embodiments. Throughout the specification, references to "one embodiment", "an embodiment", "some embodiments", "the embodiment", and "an exemplary embodiment" mean that a particular feature, structure, or characteristic described in connection with the embodiment is included in at least one embodiment of the disclosure. Therefore, the phrase "in one embodiment", "in an embodiment", or "an exemplary embodiment" that appears throughout the specification does not necessarily, although may, refer to the same embodiment. Furthermore, in one or more embodiments, particular features, structures, or characteristics can be combined in any suitable manner, which will be apparent to those skilled in the art in light of the present disclosure. Additionally, although some embodiments described herein include some but not other features included in other embodiments, combinations of features of different embodiments are intended to be included within the scope of the disclosure. For example, in the appended claims, any claimed embodiments can be used in any combination.

### Novel Cas Protein

The disclosure has discovered a novel Cas protein, named CasY7. It has single-stranded or double-stranded DNA cleavage activity. Its sequence has extremely low sequence identity with other known Cas proteins. Its protein molecule is relatively small, enabling better delivery.

The CasY7 protein according to the disclosure exhibits excellent cleavage activity on exogenous or endogenous genes both in vitro and at the cellular level. Its cleavage activity is comparable to, or even better than, that of LbCpf1. At the cellular level, its cleavage activity on a specific target sequence of an exogenous or endogenous gene can be greater than approximately 8%, 9%, 10%, 15%, 20%, 30%, 40%, 50%, or higher. Overall, its cleavage activity on specific target sequences of exogenous or endogenous genes at the cellular level is higher than that of LbCpf1. The above-mentioned novel Cas protein may also contain amino acid mutations that have no substantial impact on the catalytic activity (endonuclease cleavage activity) or nucleic acid-binding function.

### Parent or Reference Cas Protein

In some aspects, the parent or reference Cas protein is selected from:
(1) The Cas protein (CasY7) having an amino acid sequence represented by SEQ ID NO:1 according to the disclosure;
(2) Orthologs, homologs, variants, or functional fragments of the Cas protein defined in (1);
(3) A polypeptide having approximately 60% identity to any one of (1) or (2). Optionally, the parent or reference Cas protein may be a wild type or non-wild type.

In a preferred embodiment of the disclosure, the wild-type Cas protein is CasY7, having the sequence represented by SEQ ID NO:1.

It should be understood that the amino acid numbering for the mutant proteins according to the disclosure is based on the parent or reference Cas protein. When the homology between a specific mutant protein and the sequence of the parent or reference Cas protein reaches 80% or more, there may be a misalignment of the amino acid numbering of the mutant protein relative to that of the parent or reference Cas protein, such as a misalignment of 1-100 positions towards the N-terminus or C-terminus of the amino acid sequence. However, using conventional sequence alignment techniques in the art, those skilled in the art can generally understand that such a misalignment is within a reasonable range, and a mutant protein with a homology of 80% (such as 90%, 95%, 98%) and having gene-editing activity (such as cleavage activity) the same as, similar to, or higher than that of the wild-type Cas protein (SEQ ID NO:1) should not be excluded from the scope of the mutant proteins according to the disclosure because of the misalignment of amino acid numbering.

### Representative Variant Polypeptides (or Mutant Proteins) and Characterization of Variant Polypeptides

In one aspect of the disclosure, a variant polypeptide according to the disclosure has, retains, or improves the endonuclease activity (on-target) on a target DNA to allow for specific cleavage of the target DNA. In some embodiments, the variant polypeptide has double-stranded-DNA on-target cleavage activity. In some embodiments, the variant polypeptide substantially retains the double-stranded-DNA on-target cleavage activity of SEQ ID NO:1.

In another aspect of the disclosure, the variant polypeptide of the Cas protein according to the disclosure can be substantially deficient in endonuclease activity (on-target), but retains its ability to complex with a guide RNA, be guided to a target DNA, and thus guide the functional domain associated with the Cas protein variant polypeptide to the target DNA and exert its function. The characterization of the Cas protein variant polypeptide of the disclosure is not limited to its DNA on-target cleavage ability.

### Increased On-target Cleavage Activity

In some aspects, the disclosure provides a variant polypeptide of the Cas protein, which is engineered based on the parent or reference Cas protein. Exemplarily, this variant polypeptide has mutations at multiple amino acid sites of the polypeptide represented by SEQ ID NO:1. As compared with the parent or reference Cas protein, it has higher double-stranded-DNA on-target cleavage activity. For example, as compared with the parent or reference Cas protein (mediated by the same guide RNA), its double-stranded-DNA on-target cleavage activity increases by at least approximately 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 210%, 220%, 230%, 240%, 250%, 260%, 270%, 280%, 290%, 300% or more.

In some embodiments, the variant polypeptide is a polypeptide having at least approximately 60% identity, such as 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.5% identity to the amino acid sequence represented by SEQ ID NO:1.

In some embodiments, the variant polypeptide according to the disclosure has amino acid substitutions relative to the parent or reference Cas protein. In some embodiments, the substitutions are conservative amino acid substitutions.

In one aspect, the disclosure provides a variant polypeptide that, relative to SEQ ID NO:1, contains any amino acid substitutions at one or more of the following sites:
V7, R8, T11, S12, 5110, 1149, N150, H151, N152, L153, Q160, E161, Y162, N163, C164, Y165, S166, S167, F168, K195, S196, K197, S198, A199, C215, T216, A217, K219, S225, L227, L229, M231, D234, 5240, 5241, Q242, E243, 1244, 5250, F251, E252, K253, V254, K260, T261, E263, N276, Y282, D283, A285, N292, S303, I304, L307, Y308, S309, R315, E316, T317, I318, I319, V348, I349, E350, P351, L354, S355, N356, L357, K358, I373, G416, I417, E418, F419, D420, I455, R456, V458, H486, N487, S488, L501, R509, P510, V511, L512, G513, N514, R515, V516, L525, 1526, N527, K528, K529, C633, T634, T635, K636, N637, D638, R639, G640, E641, F642, E648, L650, A651, Y652, A661, T681, N682, E683, S684, G727, K728, N729, E730, S743, E748, G749, 5751, K752, K781, L782, G783, E784, C785, S787, K865, P866, Y867, N868, I872, D896, N936, M957, F958, Q960, W961, P1018, S1019, R1020, N1021, and S1022.

In some embodiments, the number of amino acid mutations in the variant polypeptide relative to SEQ ID NO:1 includes approximately 1-50, approximately 1-40, approximately 1-30, approximately 2-25, preferably 2-20, such as 1, 2, 3, 4, 5, 6, 7, 8, 9, 10, 11, 12, 13, 14, 15, 16, 17, 18, 19, or 20.

In some embodiments, the variant polypeptide contains the following mutations relative to SEQ ID NO:1:
(a) M231+D234;
   Preferably, selected from M231K+D234V;
(b) S240+S241+Q242+E243;
   Preferably, selected from S240A+S241R+Q242P+E243L, S240L+5241A+Q2425+E243H+I244L+L307R+Y308F+S309A;
(c) L227+L229;
   Preferably, selected from L227I+L229R, S225P+L227A+L229R;
(d) L307+Y308+S309;
   Preferably, selected from S240L+S241A+Q242S+E243H+1244L+L307R+Y308F+S309A, D2835+A285P+L307R+Y308F+S309A, L307R+Y308F+S309A+E648R+L650I+A651P+Y652F, D283S+A285P+L307R+Y308F+S309A+I373V+E748G+S751G+K752R+S787F, S303T+I304M+L307R+Y308A+S309I, S196N+K197F+S198N+A199T+I304A+L307R+Y308A+S309A, L307R+Y308F+S309A;
(e) S250+F251+E252+K253+V254;
   Preferably, selected from S250W+F251A+E252A+K253R+V254R;
(f) K260+T261+E263;
   Preferably, selected from K260R+T261P+E263H;
(g) R315+E316+T317+I318+I319;
   Preferably, selected from R315L+E316R+T317R+I318V+I319A, Y282F+D283Q+A285T+R315A+E316Q+T317R+I318L+I319Q, Y282F+D283Q+A285T+R315S+E316R+T317K+I318K+I319W+E648S+A651L;
(h) L354+S355+N356+L357+K358;
   Preferably, selected from L354F+S355G+N356D+L3571+K358R;
(i) E648+A651;
   Preferably, selected from E648S+A651L, E648S+A651T+Y652W, Y282F+D283Q+A285T+E648T+A6515+Y652F,
   Y282F+D283Q+A285T+E648L+A651G, N276S+D283N+E648S+A651L, Y282F+D283Q+A285T+R315S+E316R+T317K+I318K+I319W+E648S+A651L;
(j) T681+N682+E683;
   Preferably, selected from T681V+N682G+E683R+S684G, T681P+N682T+E683G+S684R, T681S+N682P+E683R+S684G, T681A+N682H+E683Y+S684R, Y282F+D283Q+A285T+T681P+N682P+E683Q+S684C, Y282F+D283Q+A285T+G416S+I417H+E418Q+F419T+D420V+L501P+T681N+N682P+E683G+S684A, Y165W+S166R+G416R+I417V+E418Q+F419V+D420T+T681D+N682A+E683R+S684D, Y16SW+S166R+S198N+G416R+I417V+E418Q+F419V+D420T+T681S+N682R+E683R, Y165W+S166R+G416R+I417V+E418Q+F419V+D420T+R509N+P510G+V511G+L512S+T681S+N682 R+E683R, G416R+I417V+E418Q+F419V+D420T+T681S+N682R+E683R+G727V+K728C+N729G+E730G, Y282F+D283Q+A285T+G416A+I417R+E418V+F419C+D420Q+R509V+P510R+V511L+L512A+T681S +N682R+E683R, Y165W+S166R+G416R+I417V+E418Q+F419V+D420T+R509Q+P510A+V511A+L512R+T681S+N682 R+E683R+A661V, S110G+Y165W+S166R+G416R+I417V+E418Q+F419V+D420T+R509A+P510A+V511R+L512Q+T681S +N682R+E683R, Y165W+S166R+G416R+I417V+E418Q+F419V+D420T+R509L+P510V+V511S+L512F+T681S+N682R +E683R, Y282F+D283Q+A285T+G416A+I417R+E418V+F419C+D420Q+R509G+P510K+L512C+T681S+N682R +E683R, Y16SW+S166R+G416R+1417V+E418Q+F419V+D420T+H486A+S488W+T634V+T63SY+K636S+N637 R+T681S+N682R+E683R;
(k) Y16S+S166;
   Preferably, selected from Y165W+S166R, Y165W+S166V+S167I+F168I, Y165W+S166R+G416R+I417V+E418Q+F419V+D420T, Y165W+S166R+G416R+I417E+E418Q+F419V+D420A, Y165W+S166R+G416R+I417V+E418Q+F419V+D420T+T681D+N682A+E683R+S684D, Y165W+S166R+S198N+G416R+I417V+E418Q+F419V+D420T+T681S+N682R+E683R, Y165W+S166R+G416R+I417V+E418Q+F419V+D420T+N682G+E683A+D896N, Y165W+S166R+G416R+I417V+E418Q+F419V+D420T+R509N+P510G+V511G+L512S+T681S+N682 R+E683R, Y165W+S166R+G416R+I417V+E418Q+F419V+D420T+G513L+N514A+R515V+V516M+N682G+E68 3A+S684R+D896N, Y165W+S166R+G416R+I417V+E418Q+F419V+D420T+G513C+R515Y+V516M+N682G+E683A+S684 R+D896N, Y165W+S166R+G416A+I417R+E418V+F419C+D420Q+H486S+N487N+S488W+N682G+E683A+S68 4R+D896N, Y165W+S166R+G416R+I417V+E418Q+F419V+D420T+R509Q+P510A+V511A+L512R+T681S+N682 R+E683R+A661V, S110G+Y165W+S166R+G416R+I417V+E418Q+F419V+D420T+R509A+P510A+V511R+L512Q+T681S +N682R+E683R, Y165W+S166R+G416R+I417V+E418Q+F419V+D420T+R509L+P510V+V511S+L512F+T681S+N682R +E683R, Y165W+S166R+G416R+I417V+E418Q+F419V+D420T+G513C+R515Y+V516M+T635S+K636G+N637 L+N682G+E683A+S684R+D896N, Y16SW+S166R+G416R+1417V+E418Q+F419V+D420T+H486A+S488W+T634V+T63SY+K636S+N637 R+T681S+N682R+E683R, Y165W+S166R+G416A+I417R+E418V+F419C+D420Q+H486S+S488W+K781T+G783S+E784F+C785L +D896N, Y165W+S166R+G416A+I417R+E418V+F419C+D420Q+H486S+S488W+D638F+R639P+G640N+E641 Y+F642L, Y165W+S166R+G416R+I417V+E418Q+F419V+D420T+H486A+S488W+N682G+E683A+S684R+P101 8L+51019R+R1020P+N1021R+51022V;
(l) Q160+E161+Y162+N163+C164;
   Preferably, selected from Q160V+E1611+Y162T+N163S+C164V, Q160S+E161S+Y162L+N163C+C164V, Q160P+E161V+Y162H+N163S+C164T, Q160L+E161S+Y162F+N163S+C164A;
(m) V7+T11+S12;
   Preferably, selected from V7H+T11R+S12M, V7F+R8L+T11R+S12V, V71+T11V+S12M+Y282F+D283Q+A28ST;
(n) I149+N150+H151+N152+L153;
   Preferably, selected from I149M+N150S+H151C+N152T+L153Y;
(o) K19S+K197+S198+A199;
   Preferably, selected from K195R+K197A+S198A+A199G;
(p) T216+A217+K219;
   Preferably, selected from T216L+A217T+K219R, C215V+T216S+A217S+K219R;
(q) D283+A285;
   Preferably, selected from Y282F+D283Q+A285T, D283I+A285R+N292L, Y282F+D283Q+A285T+K865S+P866S+Y867H+N868F+1872L, Y282F+D283Q+A285T+M957V+F958L+Q960V+W961V, Y282F+D283Q+A285T+V348I+I349V+E350L+P351T, Y282F+D283Q+A285T+T681P+N682P+E683Q+S684C, Y282F+D283Q+A285T+E748A+G749S+S751G, Y282F+D283Q+A285T+G416S+I417H+E418Q+F419T+D420V, Y282F+D283Q+A285T+G416L+I417Q+E418M+F419R+D420A, Y282F+D283Q+A285T+G416A+I417R+E418V+F419C+D420Q, Y282F+D283Q+A285T+G4165+I417H+E418Q+F419T+D420V+L501P+N936S, Y282F+D283Q+A285T+G416S+I417H+E418Q+F419T+D420V+L501P+T681N+N682P+E683G+S684A, Y282F+D283Q+A285T+G416A+I417R+E418V+F419C+D420Q, Y282F+D283Q+A285T+G416L+I417Q+E418M+F419R+D420A+I455G+R456E+V458E, Y282F+D283Q+A285T+G416A+I417R+E418V+F419C+D420Q+R509C+P510S+L512F+L525R+1526V+ N527D+K528P+K529G+N682G+E683A+S684R+D896N, Y282F+D283Q+A285T+G416A+I417R+E418V+F419C+D420Q+R509V+P510R+V511L+L512A+T681S +N682R+E683R, Y282F+D283Q+A285T+G416A+I417R+E418V+F419C+D420Q+P510D+V511H+L512V, Y282F+D283Q+A285T+G416A+I417R+E418V+F419C+D420Q+R509G+P510K+L512C+T681S+N682R +E683R, Y282F+D283Q+A285T+G416A+I417R+E418V+F419C+D420Q+R509C+P510S+L512F+N682G+E683A +S684R+G727E+K728H+N729Q+E730R, Y282F+D283Q+A285T+G416A+I417R+E418V+F419C+D420Q+R509C+P510S+L512F+N682G+E683A +S684R+S743T, Y282F+D283Q+A285T+G416A+I417R+E418V+F419C+D420Q+P510D+V511H+L512V+K781H+L782l +G783F+E784R+C785S, Y282F+D283Q+A285T+G416A+I417R+E418V+F419C+D420Q+P510D+V511H+L512V+D638P+E641 M+F642V, Y282F+D283Q+A285T+G416A+I417R+E418V+F419C+D420Q+P510D+V511H+L512V+P1018S+S101 9R+R1020V+N1021M+S1022P, Y282F+D283Q+A285T+G416A+I417R+E418V+F419C+D420Q+R509C+P510S+L512F+C633N+T634E +T635E+K636G+N637L+N682G+E683A+S684R+S743T, Y282F+D283Q+A285T+G416A+I417R+E418V+F419C+D420Q+R509C+P510S+L512F+C633F+T634P+ T635F+K636P+N637C+N682G+E683A+S684R+S743T, Y282F+D283Q+A285T+G416A+I417R+E418V+F419C+D420Q+R509C+P510S+L512F+C633S+T634C+ T635G+K636H+N637F+N682G+E683A+S684R+S743T, Y282F+D283Q+A285T+G416A+I417R+E418V+F419C+D420Q+R509C+P510S+L512F+C633R+T634Y+ T635L+K636V+NE637D+N682G+E683A+S684R+5743T;
(r) G416+1417+E418+F419+D420;
   Preferably, selected from G416R+1417W+E418T+F419R+D420V, Y282F+D283Q+A285T+G416S+I417H+E418Q+F419T+D420V, Y16SW+S166R+G416R+I417V+E418Q+F419V+D420T, Y282F+D283Q+A285T+G416L+I417Q+E418M+F419R+D420A, Y282F+D283Q+A285T+G416A+I417R+E418V+F419C+D420Q, Y165W+S166R+G416R+I417E+E418Q+F419V+D420A, Y282F+D283Q+A285T+G416S+I417H+E418Q+F419T+D420V+L501P+N936S, Y282F+D283Q+A285T+G416S+I417H+E418Q+F419T+D420V+L501P+T681N+N682P+E683G+S684A, Y165W+S166R+G416R+I417V+E418Q+F419V+D420T+T681D+N682A+E683R+S684D, Y165W+S166R+S198N+G416R+I417V+E418Q+F419V+D420T+T681S+N682R+E683R, Y165W+5166R+G416R+I417V+E418Q+F419V+D420T+N682G+E683A+D896N, Y282F+D283Q+A285T+G416A+I417R+E418V+F419C+D420Q, R509C+P510S+L512F+G416R+I417V+E418Q+F419V+D420T+H486A+S488W+N682G+E683A+S684R +D896N, Y165W+S166R+G416R+I417V+E418Q+F419V+D420T+R509N+P510G+V511G+L512S+T681S+N682 R+E683R, Y165W+S166R+G416R+I417V+E418Q+F419V+D420T+G513L+N514A+R515V+V516M+N682G+E68 3A+S684R+D896N, Y165W+S166R+G416R+I417V+E418Q+F419V+D420T+G513C+R515Y+V516M+N682G+E683A+S684 R+D896N, G416R+I417V+E418Q+F419V+D420T+T681S+N682R+E683R+G727V+K728C+N729G+E730G, Y282F+D283Q+A285T+G416L+I417Q+E418M+F419R+D420A+1455G+R456E+V458E, Y165W+S166R+G416A+I417R+E418V+F419C+D420Q+H486S+N487N+S488W+N682G+E683A+S68 4R+D896N, Y282F+D283Q+A285T+G416A+I417R+E418V+F419C+D420Q+R509C+P510S+L512F+L52SR+I526V+ N527D+K528P+K529G+N682G+E683A+S684R+D896N, Y282F+D283Q+A285T+G416A+I417R+E418V+F419C+D420Q+R509V+P510R+V511L+L512A+T681S +N682R+E683R, Y165W+S166R+G416R+I417V+E418Q+F419V+D420T+R509Q+P510A+V511A+L512R+T681S+N682 R+E683R+A661V, Y282F+D283Q+A285T+G416A+1417R+E418V+F419C+D420Q+P510D+V511H+L512V, S110G+Y165W+S166R+G416R+l417V+E418Q+F419V+D420T+R509A+P510A+V511R+L512Q+T681S +N682R+E683R, Y165W+S166R+G416R+I417V+E418Q+F419V+D420T+R509L+P510V+V511S+L512F+T681S+N682R +E683R, Y282F+D283Q+A285T+G416A+I417R+E418V+F419C+D420Q+R509G+P510K+L512C+T6815+N682R +E683R, Y282F+D283Q+A285T+G416A+I417R+E418V+F419C+D420Q+R509C+P5105+L512F+N682G+E683A +S684R+G727E+K728H+N729Q+E730R, Y165W+S166R+G416R+I417V+E418Q+F419V+D420T+G513C+R515Y+V516M+T635S+K636G+N637 L+N682G+E683A+S684R+D896N, Y165W+S166R+G416R+I417V+E418Q+F419V+D420T+H486A+S488W+T634V+T635Y+K636S+N637 R+T681S+N682R+E683R, Y282F+D283Q+A285T+G416A+I417R+E418V+F419C+D420Q+R509C+P5105+L512F+N682G+E683A +S684R+S743T, Y282F+D283Q+A285T+G416A+I417R+E418V+F419C+D420Q+P510D+V511H+L512V+K781H+L7821 +G783F+E784R+C785S, Y165W+S166R+G416A+I417R+E418V+F419C+D420Q+H486S+S488W+K781T+G783S+E784F+C785L +D896N, Y282F+D283Q+A285T+G416A+I417R+E418V+F419C+D420Q+P510D+V511H+L512V+D638P+E641 M+F642V, Y165W+S166R+G416A+I417R+E418V+F419C+D420Q+H486S+S488W+D638F+R639P+G640N+E641 Y+F642L, Y165W+S166R+G416R+I417V+E418Q+F419V+D420T+H486A+S488W+N682G+E683A+S684R+P101 8L+51019R+R1020P+N1021R+51022V, Y282F+D283Q+A285T+G416A+I417R+E418V+F419C+D420Q+P510D+V511H+L512V+P10185+S101 9R+R1020V+N1021M+S1022P, Y282F+D283Q+A285T+G416A+I417R+E418V+F419C+D420Q+R509C+P510S+L512F+C633N+T634E +T635E+K636G+N637L+N682G+E683A+S684R+S743T, Y282F+D283Q+A285T+G416A+I417R+E418V+F419C+D420Q+R509C+P510S+L512F+C633F+T634P+ T635F+K636P+N637C+N682G+E683A+S684R+S743T, Y282F+D283Q+A285T+G416A+1417R+E418V+F419C+D420Q+R509C+P5105+L512F+C633S+T634C+ T635G+K636H+N637F+N682G+E683A+S684R+S743T, Y282F+D283Q+A285T+G416A+I417R+E418V+F419C+D420Q+R509C+P510S+L512F+C633R+T634Y+ T635L+K636V+N637D+N682G+E683A+S684R+S743T.

### Endonuclease-deficient Variant Polypeptide (dCas)

In some aspects, the present disclosure provides an endonuclease-deficient variant polypeptide.

Exemplarily, relative to the parent or reference Cas protein, this variant polypeptide retains only a portion (e.g., less than 50%, 45%, 40%, 35%, 30%, 25%, 20%, 15%, 10%, 5%, 4%, 3%, 2%, 1% or less) of the spacer-specific endonuclease cleavage activity for a target sequence of a target DNA complementary to a guide RNA.

In some embodiments, the variant polypeptide is substantially devoid of spacer-sequence-specific (on-target) double-stranded-DNA cleavage activity.

In some embodiments, the variant polypeptide is substantially devoid of the spacer-sequence-specific (on-target) double-stranded-DNA cleavage activity of SEQ ID NO:1.

In some embodiments, when used in combination with the same guide RNA, as compared to SEQ ID NO:1, the variant polypeptide has reduced spacer-sequence-specific (on-target) double-stranded-DNA cleavage activity, for example, reduced by at least about 5%, 10%, 15%, 20%, 25%, 30%, 35%, 40%, 45%, 50%, 55%, 60%, 65%, 70%, 75%, 80%, 85%, 90%, 95%, or 100%.

In some embodiments, relative to SEQ ID NO:1, the variant polypeptide contains one or more amino acid substitutions at the following positions: D592, D643, E820, and D992.

In some embodiments, relative to the amino acid sequence represented by SEQ ID NO:1, the variant polypeptide contains one or more amino acid substitutions selected from DS92A, D643A, E820A, and D992A.

In some embodiments, relative to the amino acid sequence represented by SEQ ID NO:1, the variant polypeptide comprises one amino acid substitution selected from D592A, D643A, E820A, and D992A.

In some embodiments, the variant polypeptide contains an amino acid sequence represented by any one of SEQ ID NO:47-50, or an amino acid sequence having at least about 80% (e.g., at least about 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% or 100%) sequence identity to the amino acid sequence represented by any one of SEQ ID NO:47-50.

### Fusion Protein

In one aspect, the present disclosure provides a fusion protein. The fusion protein includes the Cas protein or variant polypeptide according to the present disclosure, and one or more functional domains associated with the Cas protein or variant polypeptide. In one embodiment, the functional domain is a heterologous functional domain.

As used herein, the term "functional domain" is interpreted in its broadest sense and includes a protein such as an enzyme or a factor itself or a fragment/domain thereof having a specific function.

When a fusion protein includes more than one functional domain, the functional domains can be the same or different.

In one embodiment, the functional domain is selected from a nuclear localization signal (NLS), a nuclear export signal (NES), a reporter protein (e.g., fluorescent protein), a Cas protein targeting moiety, a DNA-binding domain (e.g., Lex A DBD, Gal4 DBD, Sp1 DBD), an epitope tag (e.g., His, myc, V5, FLAG, HA, VSV-G, etc.), a transcriptional activation domain (e.g., VP64, VPR, p65, Rta), a transcriptional repression domain (e.g., KRAB domain, SID domain, NuE domain, NcoR domain or SID4X domain), a nuclease, a deaminase (e.g., adenosine deaminase or cytidine deaminase), a methylase (e.g., DNA methylase DNMT), demethylase, a transcriptional release factor, HDAC, a cleavage-active polypeptide, a ligase, an integrase, a transposase, a recombinase, a polymerase, an exonuclease (e.g., T5E), and a base-excision repair inhibitor (such as uracil-DNA glycosylase inhibitor (UGI)).

In some embodiments, the functional domain includes one or more of the following enzymatic activities on a target sequence: methylase activity, demethylase activity, acetyltransferase activity, deacetylase activity, kinase activity, phosphatase activity, ubiquitin-ligase activity, deubiquitination activity, adenylation activity, deadenylation activity, SUMOylation activity, deSUMOylation activity, ribosylation activity, deribosylation activity, myristoylation activity, demyristoylation activity, glycosylation activity (e.g., from O-GlcNAc transferase), and deglycosylation activity.

In some embodiments, the functional domain is selected from deaminases.

As used herein, "adenosine deaminase" or "adenosine deaminase protein" refers to a protein, polypeptide, or one or more functional domains of a protein or polypeptide, that is capable of catalyzing the hydrolytic deamination reaction that converts adenine (or an adenine moiety of a molecule) to hypoxanthine (or a hypoxanthine moiety of a molecule). In some embodiments, the functional domain is selected from adenosine deaminases. In some embodiments, the adenosine deaminase is capable of deaminating adenine (A) to produce hypoxanthine (I). In some embodiments, deamination of adenine into hypoxanthine converts adenosine (A) or deoxyadenosine (dA) containing adenine to guanosine (G) or deoxyguanosine (dG).

In some embodiments, the adenosine deaminase includes, but not limited to, members of the enzyme family called adenosine deaminases acting on RNA (ADAR), members of the enzyme family called adenosine deaminases acting on tRNA (ADAT), and other members of the family containing adenosine deaminase domains (ADAD). In some embodiments, the adenosine deaminase is capable of targeting adenine in RNA/DNA and RNA duplexes. In some embodiments, the adenosine deaminase has been modified to increase its ability to edit DNA in an RNA/DNA heteroduplex of RNA duplexes.

In some embodiments, the adenosine deaminase is derived from one or more metazoan species, including but not limited to mammals, birds, frogs, squids, fish, flies, and worms. In some embodiments, the adenosine deaminase is from a human, squid, or Drosophila.

In some embodiments, the adenosine deaminase is a human ADAR, including hADAR1, hADAR2, and hADAR3. In some embodiments, the adenosine deaminase is a Caenorhabditis elegans ADAR protein, including ADR-1 and ADR-2. In some embodiments, the adenosine deaminase is a Drosophila ADAR protein, including dAdar. In some embodiments, the adenosine deaminase is a squid (Loligo pealeii) ADAR protein, including sqADAR2a and sqADAR2b. In some embodiments, the adenosine deaminase is a human ADAT protein. In some embodiments, the adenosine deaminase is a Drosophila ADAT protein. In some embodiments, the adenosine deaminase is a human ADAD protein, including TENR (hADAD1) and TENRL (hADAD2).

In some embodiments, the adenosine deaminase is a TadA protein, such as Escherichia coli TadA. See Kim et al., Biochemistry 45:6407-6416 (2006); Wolf et al., EMBO J. 21:3841-3851 (2002). In some embodiments, the adenosine deaminase is mouse ADA. See Grunebaum et al., Curr. Opin. Allergy Clin. Immunol. 13:630-638 (2013). In some embodiments, the adenosine deaminase is human ADAT2. See Fukui et al., J. Nucleic Acids 2010:260512 (2010). In some embodiments, the deaminase (such as adenosine deaminase or cytidine deaminase) is one or more described in the following references: Cox et al., Science. November 24, 2017; 358(6366):1019-1027; Komore et al., Nature. May 19, 2016; 533(7603):420-4; and Gaudelli et al., Nature. November 23, 2017; 551(7681):464-471.

In some embodiments, the adenosine deaminase protein recognizes one or more target adenosine residues in a double-stranded nucleic acid substrate and converts them to inosine residues. In some embodiments, the double-stranded nucleic acid substrate is an RNA-DNA hybrid duplex. In some embodiments, the adenosine deaminase protein recognizes a binding window on the double-stranded substrate. In some embodiments, the binding window contains at least one target adenosine residue. In some embodiments, the binding window is in the range of about 3 bp to about 100 bp. In some embodiments, the binding window is in the range of about 5 bp to about 50 bp. In some embodiments, the binding window is in the range of about 10 bp to about 30 bp. In some embodiments, the binding window is about 1 bp, 2 bp, 3 bp, 5 bp, 7 bp, 10 bp, 15 bp, 20 bp, 25 bp, 30 bp, 40 bp, 45 bp, 50 bp, 55 bp, 60 bp, 65 bp, 70 bp, 75 bp, 80 bp, 85 bp, 90 bp, 95 bp, or 100 bp.

In some embodiments, the adenosine deaminase protein contains one or more deaminase domains. Without being bound to a particular theory, it is expected that the deaminase domain is used to recognize one or more target adenosine (A) residues contained in a double-stranded nucleic acid substrate and convert them to inosine (I) residues. In some embodiments, the deaminase domain contains an active center. In some embodiments, the active center contains a zinc ion. In some embodiments, during the A-to-I editing process, the base-pairing at the target adenosine residue is disrupted, and the target adenosine residue is "flipped" out of the double helix to become accessible to the adenosine deaminase. In some embodiments, the amino acid residues in or near the active center interact with one or more nucleotides 5' upstream of the target adenosine residue. In some embodiments, the amino acid residues in or near the active center interact with one or more nucleotides 3' downstream of the target adenosine residue. In some embodiments, the amino acid residues in or near the active center further interact with the nucleotide complementary to the target adenosine residue in the opposite strand. In some embodiments, the amino acid residues form a hydrogen bond with the 2' hydroxyl group of the nucleotide.

In some embodiments, the adenosine deaminase contains the full-length human ADAR2 protein (hADAR2) or its deaminase domain (hADAR2-D). In some embodiments, the adenosine deaminase is an ADAR family member homologous to hADAR2 or hADAR2-D.

In some embodiments, the adenosine deaminase contains the wild-type amino acid sequence of hADAR2-D. In some embodiments, the adenosine deaminase contains one or more mutations in the hADAR2-D sequence such that the editing efficiency and/or substrate editing preference of hADAR2-D is altered according to specific needs.

In some embodiments, the adenosine deaminase is tRNA adenosine deaminase (TadA) or its deaminase domain or a functional variant or fragment thereof. Exemplarily, the adenosine deaminase is selected from TadA8e (SEQ ID NO:79), TadA8.17, TadA8.20, TadA9, TadA8EV106W, TadA8EV106W+D108Q, TadA-CDa, TadA-CDb, TadA-CDc, TadA-CDd, TadA-CDe, TadA-dual, TADAC-1.2, TADAC-1.14, TADAC-1.17, TADAC-1.19, TADAC-2.5, TADAC-2.6, TADAC-2.9, TADAC-2.19, TADAC-2.23, TadA8e-N46L, and TadA8e-N46P.

In some embodiments, the adenosine deaminase contains an amino acid sequence having at least 80%, 82%, 85%, 87%, 90%, 92%, 95%, 96%, 97%, 98% or 99% identity to the amino acid sequence represented by SEQ ID NO:31 (the deaminase 005V1 from CN114634923A, SEQ ID NO:2 in that application) or SEQ ID NO:70 (the deaminase 004V1 from CN114634923A, SEQ ID NO:1 in that application), and retains the deamination activity of the amino acid sequence represented by SEQ ID NO:31.

In some embodiments, the amino acid sequence of the adenosine deaminase has amino acid additions, insertions, deletions, and substitutions relative to the amino acid sequence represented by SEQ ID NO:31. In some embodiments, the catalytic domain of the adenosine deaminase includes a mutant of the amino acid sequence represented by SEQ ID NO:31. In some embodiments, the adenosine deaminase contains the amino acid sequence represented by SEQ ID NO:32.

In some embodiments, the adenosine deaminase contains an amino acid sequence having at least 80%, 82%, 85%, 87%, 90%, 92%, 95%, 96%, 97%, 98% or 99% identity to the amino acid sequence represented by SEQ ID NO:32, and retains the deamination activity of the amino acid sequence represented by SEQ ID NO:32.

In some embodiments, the amino acid sequence of the adenosine deaminase has amino acid additions, insertions, deletions, and substitutions relative to the amino acid sequence represented by SEQ ID NO:32. In some embodiments, the catalytic domain of the adenosine deaminase includes a mutant of the amino acid sequence represented by SEQ ID NO:32. In some embodiments, the adenosine deaminase contains the amino acid sequence represented by SEQ ID NO:32.

In some embodiments, the amino acid sequence of the adenosine deaminase contains an amino acid sequence having at least 80%, 82%, 85%, 87%, 90%, 92%, 95%, 96%, 97%, 98% or 99% identity to the amino acid sequence represented by SEQ ID NO:70 (the deaminase 004V1 from CN114634923A, SEQ ID NO:1 in that application), and retains the deamination activity of the amino acid sequence represented by SEQ ID NO:70.

In some embodiments, the functional domain is selected from cytidine deaminases.

As used herein, the term "cytidine deaminase" or "cytidine deaminase protein" refers to a protein, polypeptide, or one or more functional domains of a protein or polypeptide that are capable of catalyzing the hydrolytic deamination reaction that converts cytosine (or a cytosine moiety of a molecule) into uracil (or a uracil moiety of a molecule). In some embodiments, the cytosine-containing molecule is cytidine (C), and the uracil-containing molecule is uridine (U). The cytosine-containing molecule may be deoxyribonucleic acid (DNA) or ribonucleic acid (RNA).

In some embodiments, the cytidine deaminase is selected from APOBEC (e.g., APOBEC3, such as APOBEC3A, APOBEC3B, and APOBEC3C).

In some embodiments, the cytidine deaminase is selected from hAPOBEC3-W104A (SEQ ID NO:95).

In some embodiments, the cytidine deaminase contains the wild-type amino acid sequence of cytosine deaminase. In some embodiments, the cytidine deaminase contains one or more mutations in the cytosine deaminase sequence such that the editing efficiency and/or substrate editing preference of cytosine deaminase is altered according to specific needs.

In some embodiments, the functional domain is a base-excision repair inhibitor. Exemplarily, the base-excision repair inhibitor is uracil-DNA glycosylase inhibitor (UGI).

In some embodiments, the uracil-DNA glycosylase inhibitor (UGI) is derived from the human UGI domain (exemplarily, the UGI domain is represented by SEQ ID NO:96).

In one embodiment, the functional domain is a methylase, such as Hhal DNA m5c-methyltransferase (M.Hhal), DNA methyltransferase 1 (DNMT1), DNA methyltransferase 3a (DNMT3a), DNA methyltransferase 3b (DNMT3b), METI, DRM3, ZMET2, CMT1, CMT2, etc.

In one embodiment, the functional domain is a demethylase, such as TET1 (ten-eleven translocation 1), ten-eleven translocation (TET) dioxygenase 1 (TET1CD), DME, DML1, DML2, ROS1, etc.

In one embodiment, the functional domain is a transcriptional release factor, such as eukaryotic release factor 1 (ERF1), eukaryotic release factor 3 (ERF3).

In one embodiment, the nuclear export signal includes human protein tyrosine kinase 2.

In one embodiment, the reporter protein includes one or more of glutathione-S-transferase, horseradish peroxidase, chloramphenicol acetyltransferase, β-galactosidase, β-glucuronidase, or autofluorescent proteins.

In one embodiment, the autofluorescent protein includes one or more of green fluorescent protein, HcRed, DsRed, cyan fluorescent protein, yellow fluorescent protein, or blue fluorescent protein.

In one embodiment, the DNA-binding domain includes one or more of methyl-binding proteins, LexA DBD, Sp1 DBD, or Gal4 DBD.

In one embodiment, the epitope tag includes one or more of His (histidine tag), V5 tag, FLAG tag, influenza virus hemagglutinin tag, Myc tag, VSV-G tag, or thioredoxin tag.

In one embodiment, the transcriptional activation domain includes one or more of VP64, p65, Rta, and VPR.

In one embodiment, the transcriptional repression domain includes KRAB and/or SID.

In one embodiment, the nuclease includes Fokl.

In one embodiment, the cleavage-active polypeptide includes a polypeptide with single-stranded RNA cleavage activity, a polypeptide with double-stranded RNA cleavage activity, a polypeptide with single-stranded DNA cleavage activity, or a polypeptide with double-stranded DNA cleavage activity.

In one embodiment, the ligase includes DNA ligase and/or RNA ligase.

In some embodiments, the functional domain is an exonuclease. According to the disclosure herein, there are various exonucleases or programmable exonucleases that meet the criteria. Some examples of exonucleases suitable for use as part of the fusion protein in the present application include MRE11, EXO1, EXO III, EXO VII, EXOT, DNA2, CtIP, TREX1, TREX2, Apollo, RecE, Red, T5, Lexo, RecBCD, and mung bean exonuclease. Other suitable exonucleases are also contemplated.

In some embodiments, the exonuclease is selected from T5 exonuclease or T7 exonuclease.

In some embodiments, the T5 exonuclease contains an amino acid sequence having at least 60%, 65%, 70%, 75%, 80%, 85%, 90%, 91%, 92%, 93%, 94%, 95%, 96%, 97%, 98%, 99% or higher sequence identity to the amino acid sequence represented by SEQ ID NO:81.

In some embodiments, the functional domain is a nuclear localization signal. As used herein, "NLS", "nuclear localization sequence", or "nuclear localization signal" are used interchangeably herein and refer to an amino acid sequence that promotes the entry of a protein into the cell nucleus. Nuclear localization sequences are known in the art (for example, as described in the PCT application PCT/EP2000/011690 filed by Plank et al. on November 23, 2000 and published as WO/2001/038547 on May 31, 2001), and this patent application is incorporated herein by reference to its disclosure of exemplary nuclear localization sequences. In other embodiments, the NLS is an optimized NLS, for example, as described in Koblan et al., Nature Biotech. 2018 doi: 10.1038/nbt.4172. In some embodiments, the NLS contains the following amino acid sequences: KRTADGSEFESPKKKRKV (SEQ ID NO:38), AVKRPAATKKAGQAKKKKLD (SEQ ID NO:39), KRPAATKKAGQAKKKK (SEQ ID NO:40), KKTELQTTNAENKTKKL (SEQ ID NO:41), KRGINDRNFWRGENGRKTR (SEQ ID NO:42), RKSGKIAAIVVKRPRK (SEQ ID NO:43), PKKKRKV (SEQ ID NO:44), or MDSLLMNRRKFLYQFKNVRWAKGRRETYLC (SEQ ID NO:45).

### Polynucleotide

In one aspect, the disclosure provides a polynucleotide, which is a polynucleotide sequence encoding the Cas protein or its variant polypeptide according to the disclosure, or a polynucleotide sequence encoding the fusion protein according to the disclosure.

In one embodiment, the polynucleotide is a DNA molecule with codons optimized according to the codon preference of a host cell.

The optimization described in the present disclosure may require mutation of the nucleotide sequence encoding a protein (such as the Cas protein or its variant polypeptide of the present disclosure) to mimic the codon preference of the intended host organism or cell when it encodes the same protein. Thus, the codons can be altered, but the encoded protein remains unchanged. For example, if the intended target cell is a human cell, a nucleotide sequence encoding the protein optimized with human codons can be used. As another non-limiting example, if the intended host cell is an animal cell (such as a mouse cell or an insect cell), a nucleotide sequence encoding the protein optimized with the codons of that animal can be generated. As yet another non-limiting example, if the intended host cell is a plant cell, a nucleotide sequence encoding the protein optimized with plant codons can be generated.

Choices of codons are readily available. For example, the "Codon Usage Database" is available at www.kazusa.or.jp/codon. In some cases, the nucleic acid of the present disclosure contains a nucleotide sequence encoding CasY7 or its variant polypeptide or its fusion protein, and the codons of the nucleotide sequence are optimized for expression in eukaryotic cells. In some cases, the nucleic acid of the present disclosure contains a nucleotide sequence encoding CasY7 or its variant polypeptide or its fusion protein, and the codons of the nucleotide sequence are optimized for expression in animal cells. In some cases, the nucleic acid of the present disclosure contains a nucleotide sequence encoding CasY7 or its variant polypeptide or its fusion protein, and the codons of the nucleotide sequence are optimized for expression in fungal cells. In some cases, the nucleic acid of the present disclosure contains a nucleotide sequence encoding CasY7 or its variant polypeptide or its fusion protein, and the codons of the nucleotide sequence are optimized for expression in plant cells.

In some embodiments, the nucleic acid molecule of the disclosure contains a nucleotide sequence having at least about 80% (e.g., at least about 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% or 100%) sequence identity to the nucleotide sequence represented by any one of SEQ ID NO:2, 37, 83, 85, and 87.

In some embodiments, the polynucleotide is the nucleotide sequence represented by any one of SEQ ID NO:2, 37, 83, 85, 87.

In one embodiment, the host cell includes a prokaryotic cell or a eukaryotic cell.

In some embodiments, the disclosure provides a polynucleotide encoding a guide RNA. In some embodiments, the polynucleotide encoding a guide RNA includes DNA, RNA, or a DNA/RNA mixture. A "DNA/RNA mixture" refers to a nucleic acid containing one or more modified or unmodified ribonucleotides and one or more modified or unmodified deoxyribonucleotides, continuous or not. However, "DNA" or "RNA" may also refer to DNA containing one or more modified or unmodified ribonucleotides, continuous or not, or RNA containing one or more modified or unmodified deoxyribonucleotides, continuous or not. In some embodiments, the polynucleotide encoding a guide RNA is operably linked to a promoter or under the regulation of a promoter.

In some embodiments, the polynucleotide encoding a Cas protein is DNA, RNA, or a DNA/RNA mixture. A "DNA/RNA mixture" refers to a nucleic acid containing one or more modified or unmodified ribonucleotides and one or more modified or unmodified deoxyribonucleotides, continuous or not. However, "DNA" or "RNA" may also refer to DNA containing one or more modified or unmodified ribonucleotides, continuous or not, or RNA containing one or more modified or unmodified deoxyribonucleotides, continuous or not.

In some embodiments, the polynucleotide encoding a Cas protein is operably linked to a promoter or under the regulation of a promoter. In some embodiments, the promoter is a broad-spectrum promoter, a tissue-specific promoter, a cell-type-specific promoter, a constitutive promoter, or an inducible promoter.

In some embodiments, the polynucleotide encoding a guide RNA and/or a Cas protein is functionally or operably linked to a regulatory element and thus regulated. Optionally, the promoter may be selected from the group consisting of pol I promoter, pol II promoter, pol III promoter, T7 promoter, U6 promoter, H1 promoter, retroviral Rous sarcoma virus (RSV) LTR promoter, cytomegalovirus (CMV) promoter, SV40 promoter, dihydrofolate reductase promoter, β-actin promoter, phosphoglycerate kinase (PGK) promoter, and EF1α promoter. In some embodiments, the promoter is the U6 promoter.

### CRISPR System

The Cas protein and its variant polypeptides according to the disclosure may be used in combination with a guide RNA and be guided by the guide RNA to a target DNA to exert effects on the target DNA.

In one aspect, the disclosure provides a CRISPR-Cas system, comprising:
(i) A first component selected from the group consisting of a Cas protein or variant polypeptide according to the disclosure, a fusion protein according to the disclosure, a nucleotide sequence encoding a Cas protein or variant polypeptide or a fusion protein according to the disclosure, and any combination thereof; and
(ii) A second component, which is a guide RNA according to the disclosure, or a nucleotide sequence encoding a guide RNA according to the disclosure.

In some embodiments, the guide RNA comprises:
(i) A direct repeat (DR) sequence capable of forming a complex with the Cas protein or fusion protein; and
(ii) A spacer sequence capable of hybridizing to a target sequence of a target DNA, thereby guiding the complex to the target DNA.

In some embodiments, the CRISPR-Cas system is non-naturally occurring or engineered.

In some embodiments, the system is a complex comprising a Cas protein complexed with a guide RNA.

In some embodiments, the complex further comprises a target DNA hybridized to the target sequence.

In one aspect, the disclosure provides a complex, which comprises:
(i) a protein component selected from the group consisting of a Cas protein according to the disclosure, a fusion protein according to the disclosure, or a combination thereof; and
(ii) a guide RNA according to the disclosure.

In one aspect, the present invention also provides a CRISPR-Cas composition, comprising:
(1) a protein component: a Cas protein or a fusion protein according to the disclosure; or a nucleic acid molecule encoding the Cas protein or fusion protein according to the disclosure;
(2) an RNA component: a guide RNA according to the disclosure, or one or more nucleic acids encoding the guide RNA, or a precursor RNA of the guide RNA, or a nucleic acid encoding the precursor RNA of the guide RNA;
wherein the protein component and the nucleic acid component bind to each other to form a complex.

In one embodiment, the composition is an activated CRISPR complex which further comprises a target sequence of a target nucleic acid bound to the guide RNA.

### Guide RNA

In another aspect, the disclosure provides a guide RNA (gRNA), which comprises:
(1) a direct repeat (DR) sequence capable of forming a complex with a Cas protein according to the disclosure, and
(2) a spacer sequence capable of hybridizing to a target sequence of a target DNA, thereby guiding the complex to the target DNA.

In some embodiments, the guide RNA does not contain a tracrRNA.

In some embodiments, the direct repeat (DR) sequence is at the 5' end of the spacer sequence and is capable of forming a complex with a Cas protein or its variant polypeptide according to the disclosure, or with a fusion protein according to the disclosure.

In some embodiments, the guide RNA comprises, consists essentially of, or consists of a direct repeat (DR) sequence and a spacer sequence. In some embodiments, the guide RNA is a single-molecule nucleic acid, and the direct repeat (DR) sequence is linked to the spacer sequence.

In some embodiments, the guide RNA comprises multiple spacer sequences arranged in tandem, optionally separated by a nucleotide sequence such as a direct repeat (DR) sequence as defined herein. The positions of different spacer sequences are in tandem without affecting the activity.

In some embodiments, the spacer sequence comprises a series of 2, 3, 4, 5, 6, 7, 8, 9, 10, 15, 25, 25, 30 or more sequences, each capable of specifically hybridizing to a target sequence in a target genomic locus in a cell. In some embodiments, a functional CRISPR-Cas system or complex can edit multiple target sequences. For example, the target sequences can comprise genomic loci, and in some embodiments, there can be an alteration in gene expression. In some embodiments, a functional CRISPR-Cas system or complex can comprise other functional domains. In some embodiments, the disclosure provides a method for altering or modifying the expression of multiple gene products. The method may include introduction into a cell containing the target nucleic acid, such as a DNA molecule, or a cell containing and expressing the target nucleic acid, such as a DNA molecule; for example, the target nucleic acid may encode a gene product or provide for the expression of a gene product (such as a regulatory sequence). In some embodiments, the guide RNA comprises a direct repeat (DR) sequence, a spacer sequence, and a direct repeat (DR) sequence (DR-spacer-DR). This is a typical configuration of pre-crRNA. In some embodiments, the guide RNA comprises a DR-spacer-DR-spacer structure. In some embodiments, the guide RNA comprises two or more DRs and two or more spacer sequences. In some embodiments, the crRNA comprises a truncated direct repeat (DR) sequence and a spacer sequence. This is a typical processed or mature guide RNA. In some embodiments, the Cas protein or its variant polypeptide according to the disclosure forms a complex with the guide RNA, and the spacer sequence guides the complex to a target nucleic acid complementary to the spacer sequence to achieve sequence-specific binding.

### Multiple Guide RNAs

In one aspect, the CRISPR-Cas system according to the disclosure comprises multiple (exemplarily, 2, 3, 4, 5, or more) guide RNAs. For example, each of the multiple guide RNAs specifically targets a DNA molecule encoding a gene product in a cell, so that each of the multiple guide RNAs targets its specific DNA molecule encoding the gene product, and the Cas protein cleaves the target DNA molecule encoding the gene product, thereby altering the expression of the gene product; and wherein the CRISPR protein and the guide RNA do not occur together in nature.

In some embodiments, multiple guide nucleic acids are operably linked to the same regulatory element (e.g., a promoter) or to separate regulatory elements (e.g., promoters) or are under the regulation thereof.

In some embodiments, the guide RNA comprises non-naturally occurring nucleic acids and/or non-naturally occurring nucleotides and/or nucleotide analogs and/or chemical modifications. Preferably, these non-naturally occurring nucleic acids and non-naturally occurring nucleotides are outside the guide RNA. The non-naturally occurring nucleic acids may include, for example, a mixture of naturally and non-naturally occurring nucleotides. The non-naturally occurring nucleotides and/or nucleotide analogs may be modified at the ribose, phosphate, and/or base moieties. In one embodiment, the guide RNA comprises ribonucleotides and non-ribonucleotides.

In one embodiment, the guide RNA comprises one or more non-naturally occurring nucleotides or nucleotide analogs, such as nucleotides with phosphorothioate linkages, locked nucleic acids (LNAs) or bridged nucleic acids (BNAs) containing a methylene bridge between the 2' and 4' carbon atoms of a ribose ring. Other examples of modified nucleotides include 2'-O-methyl analogs, 2'-deoxy analogs, or 2'-fluoro analogs. Other examples of modified bases include but are not limited to 2-aminopurine, 5-bromo-uridine, pseudouridine, inosine, and 7-methylguanosine. Examples of chemical modifications of the guide RNA include but are not limited to the incorporation of 2'-O-methyl (M), 2'-O-methyl 3' phosphorothioate (MS), S-constrained ethyl (cEt), or 2'-O-methyl 3' thioPACE (MSP) at one or more terminal nucleotides.

In one embodiment, the 5' and/or 3' ends of the guide RNA are modified with various functional moieties including fluorescent dyes, polyethylene glycol, cholesterol, proteins, or detection tags (see Kelly et al., 2016, J. Biotech. 233:74-83). In one embodiment, the guide RNA contains ribonucleotides in the region binding to a target sequence, and contains one or more deoxyribonucleotides and/or nucleotide analogs in the region binding to a nucleic acid-guided nuclease. In one embodiment, 3 to 5 nucleotides at the 3' or 5' end of the guide RNA are chemically modified. In one embodiment, only minor modifications, such as 2'-F modification, are introduced in the seed region. In one embodiment, the guide RNA is modified to contain a chemical moiety at its 3' and/or 5' end. Such moieties include but are not limited to amines, azide, alkynes, thiols, dibenzocyclooctyne (DBCO), or rhodamine. In some embodiments, the chemical moiety is conjugated to the guide RNA through a linker such as an alkyl chain. In one embodiment, the chemical moiety of a modified guide RNA can be used to attach the guide RNA to another molecule, such as DNA, RNA, a protein, or nanoparticles. Such chemically modified guide RNAs can be used to identify or enrich cells generally edited by a nucleic acid-guided nuclease and a related system (see Lee et al., eLife, 2017, 6:e25312, DOI:10.7554).

### Direct Repeat (DR) Sequence

The disclosure provides a DR sequence corresponding to a Cas protein and its variant polypeptide, which is capable of binding to the Cas protein or its variant polypeptide to form a complex. In some embodiments, the DR comprises the sequence represented by SEQ ID NO:5 or SEQ ID NO:71, or a nucleotide sequence having at least about 50% (e.g., at least 55%, 60%, 65%, 70%, 75%, 80%, 85%, 90%, 91%, 92%, 93%, 94%, 95%, 96%, 97%, 98%, 99%, or 100%) identity to the nucleotide sequence represented by any one of SEQ ID NO: 5 and 71. In some embodiments, the DR is a "functional variant" (such as a "functionally truncated version", a "functionally extended version", or a "functionally substituted version") of the RNA sequence represented by SEQ ID NO:5 or 71, but still has the DR function and retains at least a part (e.g., at least about 10%, 20%, 30%, 40%, 50%, 60%, 70%, 80%, 90%, 95% or more) of the function of the reference DR (the parent DR).

In some embodiments, the DR sequence contains a stem-loop structure near the 3' end (adjacent to the spacer sequence). A "stem-loop structure" refers to a nucleic acid with a secondary structure, including a region of nucleotides known or predicted to form a double-stranded (stem) portion, and is connected at one end via a region of essentially single-stranded nucleotides as a connecting region (loop). The term "hairpin" structure is also used herein to refer to a stem-loop structure. These structures are well known in the art, and these terms are used in the meaning commonly known in the art. The stem-loop structure does not require precise base-pairing. Thus, the stem may contain one or more base mismatches. Alternatively, the base-pairing may be precise, i.e., without any mismatches.

In one embodiment, the guide RNA according to the disclosure contains one DR, and this DR contains a stem-loop structure near the 3' end of the DR sequence. In some embodiments, the stem contained in the DR consists of 5 pairs of complementary bases that hybridize to each other, and the loop has a length of 6, 7, 8, or 9 nucleotides. In some embodiments, the loop has a length of 7 nucleotides. In some embodiments, the stem may contain at least 2, at least 3, at least 4, or at least 5 base pairs. In some embodiments, the DR contains two complementary nucleotide segments of approximately 5 nucleotides in length, separated by approximately 7 nucleotides. In some embodiments, the stem-loop structure contains a first stem nucleotide strand with a length of 5 nucleotides; a second stem nucleotide strand with a length of 5 nucleotides, wherein the first and second stem nucleotide strands may hybridize to each other; and a loop nucleotide strand arranged between the first and second stem nucleotide strands, wherein the loop nucleotide strand contains 6, 7, or 8 nucleotides.

As used herein, the secondary structures of two or more guide RNAs being substantially the same or having no substantial difference means that the stems and/or loops contained in these crRNAs differ in length by no more than 1, 2, or 3 nucleotides; in terms of nucleotide types (A, U, G, or C), when comparing the nucleotide sequences of these guide RNAs through sequence alignment, they differ by no more than 1, 2, 3, 4, 5, 6, 7, or 8 nucleotides. In some embodiments, the secondary structures of two or more guide RNAs being substantially the same or having no substantial difference means that the stems contained in the crRNAs differ by at most one pair of complementary bases, and/or the loops differ by at most one nucleotide in length, and/or they contain stems of the same length but with base mismatches.

In some embodiments, the stem-loop structure contains 5'-X1X2X3X4X5NNNNNNNX6X7X8X9X10-3'; wherein X1, X2, X3, X4, X5, X6, X7, X8, X9, and X10 are any bases of A, T, C, or G, and N is any base of A, T, C, or G; wherein X1, X2, X3, X4, X5 can hybridize to X6, X7, X8, X9, X10 to form a stem and cause the NNNNNNN to form a loop; more preferably, wherein the DR sequence contains, near the 3' end of the DR sequence, a stem-loop structure of any of the following:
5'-CCGTCNNNNNNNGACGG-3' (SEQ ID NO:80); wherein N is any base of A, T, C, or G.

In some embodiments, the DR sequence that can direct any Cas protein or variant polypeptide of the disclosure to a target site contains one or more nucleotide changes selected from nucleotide additions, insertions, deletions, and substitutions, and these changes do not result in a substantial difference in the secondary structure as compared to the DR sequences listed in SEQ ID NO: 5, 70, or their functionally truncated versions.

### Spacer Sequence

In certain embodiments, the length of the spacer sequence is at least about 15 nucleotides, preferably from about 15 to about 100 nucleotides, more preferably from about 15 to about 50 nucleotides (e.g., any one of about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides). In certain embodiments, the spacer sequence is from about 16 to about 27 nucleotides, such as from about 17 to about 24 nucleotides, from about 18 to about 24 nucleotides, or from about 18 to about 22 nucleotides.

In certain embodiments, the complementarity of the spacer sequence to the target sequence is at least about 70% (e.g., at least about 75%, 80%, 85%, 90%, 91%, 92%, 93%, 94%, 95%, 96%, 97%, 98%, 99% or 100%). In certain embodiments, there are at least about 15 (e.g., at least about 16, 17, 18, 19, 20, 21, 22, 23, 24, 25, 26, 27, 28, 29, 30, 35, 40, 45, 50 or more) nucleotide matches between the spacer sequence and the target sequence of a target nucleic acid (e.g., DNA). For the spacer sequence, complete complementarity is not required, as long as there is sufficient complementarity for the guide RNA to exert its function (i.e., to guide the Cas protein to the target site). In some embodiments, the cleavage efficiency mediated by the Cas protein can be adjusted by introducing one or more mismatches between the spacer sequence and the target sequence (e.g., 1 or 2 mismatches between the spacer sequence and the target sequence, including the positions of the mismatches along the spacer/target sequence). When the mismatches are located closer to the center of the spacer sequence (i.e., not at the 3' or 5' end of the spacer sequence), the mismatches (such as double mismatches) have a greater impact on the cleavage efficiency. Thus, by choosing the positions of the mismatches along the spacer sequence, the cleavage efficiency of the Cas protein can be regulated. For example, if a cleavage rate of less than 100% of the target sequence is desired (e.g., in a cell population), 1 or 2 mismatches between the spacer sequence and the target sequence can be introduced into the spacer sequence.

In some embodiments, the spacer sequence comprises at least 15 consecutive nucleotides in the nucleotide sequence represented by any one of SEQ ID NO:6, 11, 53, 55, 57, 59, 61, and 101.

In some embodiments, the spacer sequence comprises the nucleotide sequence represented by any one of SEQ ID NO:6, 11, 53, 55, 57, 59, 61, and 101.

### PAM; Target Sequence

The PAM identification experiment may be carried out according to any example in Karvelis et al., Methods. May 15, 2017; 121-122:3-8 (the entire content of which is incorporated herein by reference) to identify the PAM sequence specificity to allow optimal synthetic sequence targeting. In one embodiment, cells carrying a plasmid encoding any of the enzymes described herein and a pre-spacer targeting guide RNA are co-transformed with a plasmid library containing an antibiotic-resistance gene and a pre-spacer sequence flanked by randomized PAM sequences. Plasmids containing a functional PAM are cleaved by the enzyme, leading to cell death. Deep sequencing of a pool of plasmids resistant to the enzymatic cleavage isolated from the surviving cells reveals the depleted plasmids, which contain functional PAMs that allow cleavage, thereby identifying the PAM preference of the Cas protein.

In certain embodiments, the Cas protein of the present disclosure can recognize a PAM (protospacer adjacent motif) to act on the target DNA. In certain embodiments, the PAM comprises or consists of 5'-TTN-3' (where N is A, T, G, or C). In certain embodiments, the PAM comprises or consists of 5'-TTC-3', 5'-TTA-3', 5'-TTT-3', or 5'-TTG-3'.

As used herein, the terms "target nucleic acid", "target sequence", "target nucleic acid sequence", and "target nucleic acid molecule" are used interchangeably and refer to a specific nucleic acid that contains a nucleic acid sequence that is completely or partially complementary to the spacer sequence in the guide RNA. It is a polynucleotide targeted by the spacer sequence in the guide RNA, for example, a sequence complementary to the spacer sequence. Hybridization between the target sequence and the spacer sequence promotes the formation of a CRISPR-Cas complex (including the Cas protein and the guide RNA), such that the target DNA is specifically modified by the Cas protein. Complete complementarity is not required, as long as there is sufficient complementarity to cause hybridization and promote the formation of a CRISPR-Cas complex. In some embodiments, the target sequence contains a non-coding region (e.g., a promoter or a terminator). In some embodiments, the target nucleic acid is single-stranded or double-stranded. The target sequence can comprise any polynucleotide, such as DNA. In certain cases, the target sequence is located inside or outside a cell. In certain cases, the target sequence is located within the nucleus, cytoplasm, or organelles (such as mitochondria or chloroplasts) of a cell. The target nucleic acid may be a sequence encoding a gene product (e.g., a protein) or a non-coding sequence (e.g., a regulatory polynucleotide or junk DNA). In certain cases, the target sequence should be associated with a protospacer adjacent motif (PAM).

In some embodiments, the protospacer sequence is a segment of about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more consecutive nucleotides of a target DNA, or a segment of consecutive nucleotides within a numerical range between any two of the foregoing values, for example, a segment of about 15 to about 50 or about 17 to about 22 consecutive nucleotides of a target DNA. In some embodiments, the protospacer sequence is a segment of about 20 consecutive nucleotides of a target DNA.

In some embodiments, the protospacer sequence comprises about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more consecutive nucleotides of a target DNA, or a segment of consecutive nucleotides within a numerical range between any two of the foregoing values, for example, about 15 to about 50 or about 16 to about 23 consecutive nucleotides of a target DNA. In some embodiments, the protospacer sequence comprises about 20 consecutive nucleotides of a target DNA.

In some embodiments, the target sequence is a segment of consecutive nucleotides identified from a target strand of a target DNA. The target sequence is the reverse-complementary sequence to the protospacer sequence. In some embodiments, the target sequence is a segment of about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more consecutive nucleotides in the target strand of the target DNA, or a segment of consecutive nucleotides within a numerical range between any two of the foregoing values, for example, a segment of about 15 to about 50 or about 16 to about 22 consecutive nucleotides in the target strand of the target DNA. In some embodiments, the target sequence is a segment of about 20 consecutive nucleotides in the target strand of the target DNA.

In some embodiments, the target sequence comprises about or at least about 15 consecutive nucleotides of a target DNA, for example, about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more consecutive nucleotides of the target DNA, or within a numerical range between any two of the foregoing values, such as about 16 to about 50 or about 17 to about 22 consecutive nucleotides of the target DNA. In some embodiments, the target sequence comprises about 20 consecutive nucleotides of the target DNA.

In some embodiments, the target sequence comprises about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more consecutive nucleotides in the target strand of a target DNA, or a segment of consecutive nucleotides within a numerical range between any two of the foregoing values, such as about 15 to about 50 or about 17 to about 23 consecutive nucleotides in the target strand of the target DNA. In some embodiments, the target sequence comprises about 20 consecutive nucleotides in the target strand of the target DNA.

In some embodiments, the reverse-complementary sequence to the target sequence is adjacent to the 3' end of the protospacer adjacent motif (PAM).

In some embodiments, the non-target strand is the sense strand of the target DNA. In some embodiments, the non-target strand is the antisense strand of the target DNA. In some embodiments, the target strand is the sense strand of the target DNA. In some embodiments, the target strand is the antisense strand of the target DNA.

In some embodiments, the protospacer sequence or the target sequence is located within an exon of a target DNA. In some embodiments, the protospacer sequence or the target sequence is located within an intron of a target DNA. In some embodiments, the target DNA includes DNA derived from a eukaryote or DNA derived from a prokaryote. In some embodiments, the target DNA is eukaryotic DNA. In some embodiments, the target nucleic acid includes non-human mammalian DNA, human DNA, insect DNA, avian DNA, reptilian DNA, amphibian DNA, rodent DNA, fish DNA, worm DNA, nematode DNA, or yeast DNA.

In one embodiment, the non-human mammalian DNA includes non-human primate DNA. In some embodiments, the target DNA is associated with a disease-related mutation.

In some embodiments, the length of the spacer sequence is about or at least about 15 nucleotides, for example, the length is about or at least about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 or more nucleotides, or within a numerical range between any two of the foregoing values, such as about 15 to about 50 nucleotides, about 17 to about 22 nucleotides, or about 18 to about 20 nucleotides. In some embodiments, the length of the spacer sequence is about 20 nucleotides.

In some embodiments, (1) the spacer sequence is at least about 50%, 55%, 60%, 65%, 70%, 75%, 80%, 85%, 90%, 95% or 100% (completely), optionally about 100% (completely) reverse-complementary to the target sequence; (2) the spacer sequence contains no more than 5, 4, 3, 2 or 1 mismatch with the target sequence or no mismatch; or (3) the spacer sequence contains no mismatch with the target sequence within the first 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 nucleotides at the 5' end of the guide RNA sequence. In some embodiments, the spacer sequence is about 100% (completely) reverse-complementary to the target sequence.

In some embodiments, various strategies and methods can be employed to use the system according to the present disclosure to modify a target DNA in a cell. As used herein, "modification" includes but is not limited to cleavage, nicking, editing, deletion, knockout, knockdown, mutation, correction, exon skipping, etc. In some embodiments, the modification results in correction of or compensation for a mutation in a cell, thereby generating an edited cell such that the expression of a target DNA is altered, for example, the level of the transcript (mRNA) of the target DNA is decreased or increased, or the level of the expression product (e.g. protein) of the target DNA is decreased or increased. In some embodiments, the modification includes suppressing or eliminating the expression of a gene product (e.g., lowering the expression by at least about 10%, about 20%, about 30%, about 40%, about 50%, about 60%, about 70%, about 80%, about 85%, about 90%, or more) through gene knockdown or knockout. In some embodiments, the modification increases the level of the expression product (e.g., protein) of the target DNA (e.g., increasing by at least about 10%, about 20%, about 30%, about 40%, about 50%, about 60%, about 70%, about 80%, about 85%, about 90%, or more). In some embodiments, the modification decreases the level of the expression product (e.g., protein) of the target DNA (e.g., decreasing by at least about 10%, about 20%, about 30%, about 40%, about 50%, about 60%, about 70%, about 80%, about 85%, about 90%, or more).

### Kit

In one aspect, the disclosure provides a kit comprising one or more components selected from: the Cas protein according to the disclosure, the fusion protein according to the disclosure, the polynucleotide according to the disclosure, the complex according to the disclosure, the vector according to the disclosure, the CRISPR-Cas composition according to the disclosure, or the system according to the disclosure.

In some embodiments, the kit further includes one or more labels or instructions, and/or an instruction for combination with one or more additional components that can be obtained elsewhere or are necessary. In some embodiments, the kit further includes instructions for using the kit, for example, instructions in more than one language.

In one aspect, the disclosure also provides a container that contains the aforementioned kit.

In one embodiment, the container includes a sterile container.

In one embodiment, the container includes a syringe.

In some embodiments, the kit further includes one or more buffers that can be used to dissolve any of one or more components contained therein and/or provide suitable reaction conditions for one or more of the components. Such buffers may include one or more of: PBS, HEPES, Tris, MOPS, Na2CO3, NaHCO3, NaB, or combinations thereof. In some embodiments, the reaction conditions include an appropriate pH, such as an alkaline pH. In some embodiments, the pH is between 7 and 10. In some embodiments, any one or more of the components in the kit may be stored in a suitable container or at a suitable temperature, for example, at 4 degrees Celsius. In certain embodiments, the kit is used for one or more of: gene or genome editing, disease treatment, targeting a target gene, and cleaving a gene of interest or a non-gene of interest.

### Delivery

The components of the CRISPR-Cas system/composition according to the disclosure can be delivered in various forms. For example, the Cas protein can be delivered as a polynucleotide encoding DNA, a polynucleotide encoding RNA, or as a protein. The guide can be delivered as a DNA-encoding polynucleotide or as RNA. All possible combinations are contemplated, including mixed delivery forms.

In one aspect, the disclosure provides a delivery composition, which comprises a delivery vehicle, and one or more selected from: the Cas protein according to the disclosure, the fusion protein according to the disclosure, the polynucleotide according to the disclosure, the vector according to the disclosure, the complex according to the disclosure, the CRISPR-Cas system according to the disclosure, the CRISPR-Cas composition according to the disclosure, and the system according to the disclosure.

As used herein, methods for introducing nucleic acids into a host cell are known in the art, and any convenient method can be used to introduce polynucleotides (e.g., an expression construct/vector for a Cas protein) into a target cell (e.g., a prokaryotic cell, a eukaryotic cell, a plant cell, an animal cell, a mammalian cell, a human cell, etc.). Suitable methods include, for example, viral infection, transfection, conjugation, protoplast fusion, liposome transfection, electroporation, calcium phosphate precipitation, polyethyleneimine (PEI)-mediated transfection, DEAE-dextran-mediated transfection, liposome-mediated transfection, particle gun technology, calcium phosphate precipitation, direct microinjection, nanoparticle-mediated nucleic acid delivery (see, for example, Panyam et al., Adv Drug Deliv Rev. September 13, 2012. pii: S0169-409X(12)00283-9. doi: 10.1016/j.addr.2012.09.023), etc.

In some embodiments, the Cas protein according to the disclosure can be provided as a polynucleotide encoding the Cas protein (e.g., mRNA, DNA, plasmid, expression vector, viral vector, etc.). In some embodiments, the Cas protein according to the disclosure is provided directly as a protein (e.g., together with an associated guide RNA (i.e. as a ribonucleoprotein complex (RNP)) or not). The Cas protein according to the disclosure can be introduced into a cell (provided to a cell) by any convenient methods; and such methods are known to those of ordinary skill in the art. Exemplarily, for instance, the Cas protein according to the disclosure or a fusion protein containing it may be directly injected into a cell (e.g., with or without a guide RNA for the Cas protein or a nucleic acid encoding a guide RNA for the Cas protein, and with or without a donor polynucleotide). As another example, a pre-formed complex (RNP) of the Cas protein and a guide RNA for the Cas protein according to the disclosure may be introduced into a cell (e.g., a eukaryotic cell) (e.g., by injection, by nucleofection; by conjugation to a protein transduction domain (PTD) of one or more components, such as conjugation to the Cas protein, conjugation to a guide RNA, conjugation to the Cas protein and the guide RNA according to the disclosure, etc.).

In some embodiments, a fusion protein containing a Cas protein (e.g., dCas fused to a functional domain, etc.) is provided as a nucleic acid encoding the fusion protein (e.g., mRNA, DNA, plasmid, expression vector, viral vector, etc.). In some embodiments, the fusion protein according to the disclosure is provided directly as a protein (e.g., together with an associated guide RNA (i.e. as a ribonucleoprotein complex (RNP)) or not). The fusion protein comprising the Cas protein according to the disclosure can be introduced into a cell by any convenient methods; and such methods are known to those of ordinary skill in the art. As an illustrative example, the fusion protein according to the disclosure may be directly injected into a cell (e.g., with or without a nucleic acid encoding the guide RNA, and with or without a donor polynucleotide). As another example, a pre-formed complex of the fusion protein and a guide RNA for the Cas protein (RNP) according to the disclosure can be introduced into a cell (e.g., by injection; by nucleofection; by conjugation to a protein transduction domain (PTD) of one or more components, such as conjugation to the fusion protein, conjugation to a guide RNA, conjugation to the fusion protein and the guide RNA according to the disclosure, etc.).

In some embodiments, a polynucleotide (e.g., a guide RNA for a Cas protein; a polynucleotide containing the nucleotide sequence encoding the Cas protein according to the disclosure, etc.) is delivered to a cell (e.g., a target host cell) and/or a protein in or bound to a particle (e.g., Cas protein; a fusion protein containing a Cas protein). In some embodiments, the CRISPR-Cas system according to the disclosure is delivered to a cell in or associated with the particle. The terms "particle" and "nanoparticle" can be used interchangeably as appropriate. A recombinant expression vector containing the nucleotide sequence encoding the Cas protein according to the disclosure and/or a guide RNA for the Cas protein, an mRNA containing the nucleotide sequence encoding the Cas protein according to the disclosure, and a guide RNA can be co-delivered using particles or a lipid envelope; for example, the Cas protein and a guide RNA, for example as a complex (e.g., a ribonucleoprotein (RNP) complex), can be delivered by particles, for example, by delivery particles containing lipids or lipid-like substances and hydrophilic polymers (e.g., cationic lipids and hydrophilic polymers).

The Cas protein according to the disclosure (or an mRNA containing the nucleotide sequence encoding the Cas protein according to the disclosure; or the recombinant expression vector containing the nucleotide sequence encoding the Cas protein according to the disclosure) and/or a guide RNA for the Cas protein (or a nucleic acid, such as one or more expression vectors encoding a guide RNA for the Cas protein) can be co-delivered using particles or a lipid envelope. For example, nanoparticles with a biodegradable core-shell structure having a poly(β-amino ester) (PBAE) core encapsulated by a phospholipid bilayer shell can be used. In some embodiments, poly(β-amino alcohol) (PBAA) can be used to deliver the Cas protein according to the disclosure, the fusion protein according to the disclosure, the ribonucleoprotein (RNP) complex according to the disclosure, the polynucleotide according to the disclosure, or the CRISPR-Cas system according to the disclosure to a target cell. U.S. Patent Publication No. 20130302401 relates to a class of poly(β-amino alcohol) (PBAA) prepared using combinatorial polymerization.

In some embodiments, lipid nanoparticles (LNPs) are used to deliver the Cas protein according to the disclosure, the fusion polypeptide of the disclosure, the RNP of the disclosure, the polynucleotide according to the disclosure, or the CRISPR-Cas system according to the disclosure to a target cell. Negatively charged polymers (such as RNA) can be loaded into LNPs at a low pH (e.g., pH 4), where the ionizable lipids exhibit a positive charge. However, at physiological pH, LNPs exhibit a low surface charge compatible with a longer circulation time.

In some embodiments, LNPs can be used to deliver DNA molecules (e.g., containing the coding sequences of the CasY7 protein and/or guide RNA) and/or RNA molecules (e.g., CasY7 protein, guide RNA). In some embodiments, LNPs can be used to deliver the RNP complex of CasY7/guide RNA. Cationic lipids form a complex with mRNA to form a lipoplex, which is then endocytosed by a cell. In an exemplary embodiment, the LNP contains a cationic lipid, a helper lipid, cholesterol, and polyethylene glycol (PEG). In some embodiments, nanoparticles can be developed according to selective organ targeting (SORT), in which multiple classes of lipid nanoparticles are systematically engineered to specifically edit extra-hepatic tissue via the addition of supplemental SORT molecules. In one embodiment, the lipid nanoparticle contains a cationic lipid, a neutral lipid, cholesterol, a PEG lipid, or a combination thereof, wherein a plurality of lipid nanoparticles optionally have an average particle size between 80 nm and 160 nm; and wherein the lipid nanoparticle contains one or more polynucleotides encoding at least one polypeptide of the disclosure (e.g., the CasY7 polypeptide).

As used herein, a "nanoparticle" refers to any particle having a diameter of less than 1000 nm. In some embodiments, the nanoparticles suitable for delivering the Cas protein according to the disclosure, the fusion protein according to the disclosure, the RNP according to the disclosure, the polynucleotide according to the disclosure, or the CRISPR-Cas system according to the disclosure to a target cell have a diameter of 500 nm or less, for example, 25 nm to 35 nm, 35 nm to 50 nm, 50 nm to 75 nm, 75 nm to 100 nm, 100 nm to 150 nm, 150 nm to 200 nm, 200 nm to 300 nm, 300 nm to 400 nm, or 400 nm to 500 nm. In some embodiments, the nanoparticles suitable for delivering the Cas protein according to the disclosure, the fusion protein according to the disclosure, the RNP according to the disclosure, the polynucleotide according to the disclosure, or the CRISPR-Cas system according to the disclosure to a target cell have a diameter of 25 nm to 200 nm. In some embodiments, the nanoparticles suitable for delivering the Cas protein according to the disclosure, the fusion protein according to the disclosure, the RNP according to the disclosure, the polynucleotide according to the disclosure, or the CRISPR-Cas system according to the disclosure to a target cell have a diameter of 100 nm or less. In some cases, the nanoparticles suitable for delivering the Cas protein according to the disclosure, the fusion protein according to the disclosure, the RNP according to the disclosure, the polynucleotide according to the disclosure, or the CRISPR-Cas system according to the disclosure to a target cell have a diameter of 35 nm to 60 nm.

Nanoparticles suitable for delivering the Cas protein, the fusion protein, the RNP, the polynucleotide, or the CRISPR-Cas system according to the disclosure to a target cell can be provided in different forms, for example, as solid nanoparticles (e.g., metals such as silver, gold, iron, titanium, non-metals, lipid-based solids, polymers), a suspension of nanoparticles, or combinations thereof. Metal, dielectric, and semiconductor nanoparticles, as well as hybrid structures (e.g., core-shell nanoparticles), can be prepared. If nanoparticles made of a semiconductor material are small enough (usually less than 10 nm) so that quantization of electronic energy levels occurs, they can also be labeled as quantum dots. Such nanoscale particles are used as drug carriers or imaging agents in biomedical applications and can be suitable for similar purposes in the present disclosure.

In some embodiments, semi-solid and soft nanoparticles are also suitable for delivering the Cas protein, the fusion protein, the RNP, the polynucleotide, or the CRISPR-Cas system according to the disclosure to a target cell. A prototype nanoparticle with semi-solid properties is a liposome.

In some embodiments, exosomes can be used to deliver the Cas protein, the fusion protein, the RNP, the nucleic acid, or the CRISPR-Cas system according to the disclosure to a target cell. Exosomes are endogenous nanovesicles that transport RNA and proteins and can deliver RNA to the brain and other target organs.

In some embodiments, liposomes can be used to deliver the Cas protein, the fusion protein, the RNP, the polynucleotide, or the CRISPR-Cas system according to the disclosure to a target cell. Liposomes are spherical vesicular structures composed of a single or multiple lipid bilayers surrounding an internal aqueous compartment and a relatively impermeable outer lipophilic phospholipid bilayer. Liposomes can be made from several different types of lipids; however, phospholipids are most commonly used to generate liposomes. Although liposome formation is spontaneous when a lipid membrane is mixed with an aqueous solution, the formation of liposomes can also be accelerated by applying force by shaking using a homogenizer, sonicator, or an extrusion device. Several other additives can be added to liposomes to alter their structure and properties. For example, cholesterol or sphingomyelin may be added to the liposome mixture to help stabilize the liposome structure and prevent leakage of the inner cargo. Liposome formulations can consist primarily of natural phospholipids and lipids such as 1,2-distearoyl-sn-glycero-3-phosphocholine (DSPC), sphingomyelin, lecithin phosphatidylcholine, and monosialic acid ganglioside.

In some embodiments, lipids can be formulated with the CRISPR-Cas system according to the disclosure or one or more of its components or nucleic acids encoding them to form lipid nanoparticles (LNPs).

In some embodiments, the CRISPR-Cas system according to the disclosure or its components can be encapsulated in PLGA microspheres and delivered, such as the microspheres further described in U.S. Published Applications 20130252281, 20130245107, and 20130244279.

In some embodiments, supercharged proteins can be used to deliver the Cas protein, the fusion protein, the RNP, the polynucleotide, or the CRISPR-Cas system according to the disclosure to a target cell. Supercharged proteins are a class of engineered or naturally occurring proteins that have an unusually high level of positive or negative net theoretical charge. Both super-negatively charged proteins and super-positively charged proteins exhibit the ability to withstand heat- or chemically-induced aggregation. Super-positively charged proteins are also capable of penetrating mammalian cells. Associating cargo with these proteins (such as plasmid DNA, RNA, or other proteins) enables the functional delivery of these macromolecules to mammalian cells *in vitro* and *in vivo.*

In some embodiments, when the delivery target is a plant cell, cell-penetrating peptides (CPPs) can be used to deliver the Cas protein, the fusion protein, the RNP, the polynucleotide, or the CRISPR-Cas system according to the disclosure to the target cell. CPPs typically have an amino acid composition that contains relatively abundant positively charged amino acids (such as lysine or arginine), or have a sequence with a pattern of alternating polar/charged amino acids and nonpolar hydrophobic amino acids.

In some embodiments, an implantable device can be used to deliver the Cas protein, the fusion protein, the RNP, the nucleic acid (e.g., a guide RNA for the Cas protein, a nucleic acid encoding a guide RNA for the Cas protein, a polynucleotide encoding the Cas protein, a donor template, etc.) or the CRISPR-Cas system according to the disclosure to a target cell (e.g., an in vivo target cell, wherein the target cell is a target cell in circulation, in tissue, or in an organ, etc.). An implantable device suitable for delivering the Cas protein, the fusion protein, the RNP, the polynucleotide, or the CRISPR-Cas system according to the disclosure to a target cell (e.g., an in vivo target cell, wherein the target cell is a target cell in circulation, in tissue, or in an organ, etc.) may include a container (e.g., a reservoir, a matrix, etc.) that contains the Cas protein, the fusion protein, the RNP, or the CRISPR-Cas system (or its components, e.g., the nucleic acid according to the disclosure).

Suitable implantable devices may include, for example, a polymer substrate (such as a matrix) that serves as the main body of the device, and in some cases, additional scaffold materials (such as metal or other polymers), as well as materials that enhance visibility and imaging. Implantable delivery devices can advantageously provide localized and long-term release, where the polypeptide and/or nucleic acid to be delivered is released directly to a target site, such as the extracellular matrix (ECM), the vasculature surrounding a tumor, diseased tissue, etc. Suitable implantable delivery devices include those applicable for delivery to cavities such as the abdominal cavity and/or any other type of administration where the drug delivery system is not anchored or attached, including bio-stable and/or degradable and/or bio-absorbable polymer matrices, which can be, for example, optionally a matrix. In some cases, a suitable implantable drug delivery device contains a degradable polymer, where the main release mechanism is bulk erosion. In some cases, a suitable implantable drug delivery device contains a non-degradable polymer or a slowly degrading polymer, where the main release mechanism is diffusion rather than bulk erosion, such that the outer part serves as a membrane and its inner part serves as a drug reservoir. In fact, the drug reservoir is not affected by the surrounding environment over a long period (e.g. from about one week to about several months). A combination of different polymers with different release mechanisms may also be optionally used. During the effective period of the total release period, the concentration gradient can remain effectively constant, and thus the diffusion rate is effectively constant (referred to as "zero-mode" diffusion). As used herein, the term "constant" means that the diffusion rate is maintained above the lower threshold of therapeutic effectiveness, but it is still optionally characterized by an initial burst and/or can fluctuate, for example, increase and decrease to a certain extent. The diffusion rate can be maintained in this way for a long time, and the diffusion rate can be considered constant to a certain level to optimize the therapeutic effective period, such as an effective silencing period.

In some embodiments, the implantable delivery system is designed to protect nucleotide-based therapeutic agents from degradation, no matter whether the degradation is of a chemical nature or due to the attack of enzymes and other factors in the subject's body. The implantation site or target site of the device can be selected to obtain maximum therapeutic efficacy. For example, the delivery device can be implanted within or near a tumor environment, or within or near the blood supply associated with a tumor. Insertion methods (such as implantation) may optionally have been used for other types of tissue implantation and/or for insertion and/or for tissue sampling, optionally without modification, or alternatively with only non-major modifications optionally made in such methods. Such methods optionally include, but are not limited to, brachytherapy methods, biopsy, endoscopy with and/or without ultrasound (such as stereotactic methods in brain tissue), and laparoscopy (including implantation in joints, abdominal organs, bladder walls, and body cavities using a laparoscope). In some embodiments, the delivery device may be selected from a gene gun, a pre-filled syringe, an auto-injector, an aerosol spray, a spray can, or a patch injector.

In some embodiments, the delivery vehicle is a nanoparticle, a liposome, an exosome, a microvesicle, or a gene gun.

### Vector

As used herein, a vector is a nucleic acid molecule capable of transporting another nucleic acid molecule linked thereto. Vectors include, but are not limited to, single-stranded, double-stranded, or partially double-stranded nucleic acid molecules; nucleic acid molecules with one or more free ends or without free ends (such as circular); nucleic acid molecules including DNA, RNA, or both; and various other polynucleotides known in the art. A vector may be introduced into a host cell through transformation, transduction, or transfection, enabling the genetic elements it carries to be expressed in the host cell. A vector can be introduced into a host cell to generate transcripts, proteins, or peptides, including protein variants, fusion proteins, isolated nucleic acid molecules, etc. as described herein (for example, CRISPR transcripts, such as nucleic acid transcripts, proteins, or enzymes). A vector can contain various elements controlling expression, including but not limited to promoter sequences, transcription initiation sequences, enhancer sequences, selection elements, and reporter genes. A vector can also contain an origin of replication.

Vectors include plasmids and viral vectors. A plasmid refers to a circular double-stranded DNA loop into which additional DNA fragments can be inserted by, for example, standard molecular cloning techniques. For viral vectors, virus-derived DNA or RNA sequences are present in a vector used for virus packaging. Viruses include, for example, retroviruses, replication-defective retroviruses, adenoviruses, replication-defective adenoviruses, and adeno-associated viruses. A viral vector also contains a polynucleotide carried by a virus for transfection into a host cell. Some vectors (such as bacterial vectors with a bacterial origin of replication and episomal mammalian vectors) can replicate autonomously in the host cells into which they are introduced.

Other vectors (such as non-episomal mammalian vectors) are integrated into the genome of a host cell after being introduced into the host cell and thus replicate together with the host genome. Moreover, certain vectors can direct the expression of genes to which they are operably linked. Such vectors are called "expression vectors".

In some embodiments, vectors (such as viral vectors or non-viral vectors, such as lentiviral vectors or plasmids) can be delivered to target tissue by, for example, intramuscular injection, intravenous administration, transdermal administration, intranasal administration, oral administration, or mucosal administration. The above-mentioned delivery can be carried out at a single dose or multiple doses. Those skilled in the art should understand that the actual dose to be delivered herein may vary to a large extent depending on various factors, including but not limited to vector selection, target cells, organisms, tissues, the general condition of the subject to be treated, the degree of transformation/modification sought, the administration route, the mode of administration, and the type of transformation/modification sought.

In some aspects, the disclosure provides a vector comprising the polynucleotide according to the disclosure or a polynucleotide encoding a guide RNA according to the disclosure.

In some embodiments, the vector includes a plasmid or a viral vector.

In some embodiments, the viral vector is selected from the group consisting of adeno-associated virus (AAV), adenovirus, lentivirus, retrovirus, herpesvirus, SV40, poxvirus, or combinations thereof.

In some embodiments, the vector includes a cloning vector, a transformation vector, an expression vector, a shuttle vector, an integration vector, or a multifunctional vector.

In some embodiments, the vector comprises:
(1) a first regulatory element, which is operably linked to a nucleotide sequence encoding the Cas protein according to the disclosure or a nucleotide sequence encoding the fusion protein according to the disclosure; and
(2) a second regulatory element, which is operably linked to a nucleotide sequence encoding a guide RNA, wherein the guide RNA comprises:
   (a) a direct repeat (DR) sequence capable of forming a complex with the Cas protein or fusion protein according to the disclosure, and
   (b) a spacer sequence capable of hybridizing to a target sequence of a target DNA, thereby guiding the complex to the target DNA.

In some embodiments, the first regulatory element and the second regulatory element are located in the same vector or in different vectors.

In some embodiments, the first regulatory element and/or the second regulatory element is a promoter, such as an inducible promoter.

In some embodiments, the vector contains one or more promoters, and the promoter is operably linked to the nucleic acid sequence, an enhancer, a transcription termination signal, a polyadenylation sequence, an origin of replication, a selectable marker, a nucleic acid restriction site, and/or a homologous recombination site.

In one aspect, the disclosure provides a CRISPR-Cas system, including one or more vectors which comprise:
(1) a first regulatory element, which is operably linked to a nucleotide sequence encoding the Cas protein or a nucleotide sequence encoding the fusion protein according to the disclosure; and
(2) a second regulatory element, which is operably linked to a nucleotide sequence encoding the guide RNA, wherein the guide RNA comprises:
   (a) a spacer sequence capable of hybridizing to a target sequence of a target nucleic acid, and
   (b) a direct repeat (DR) sequence linked to the spacer sequence and capable of guiding the Cas protein or its variants to bind to the guide RNA to form a CRISPR-Cas complex targeting the target sequence;
wherein the first regulatory element and the second regulatory element are located in the same vector or in different vectors of the CRISPR-Cas vector system.

In one embodiment, the first regulatory element or the second regulatory element includes a promoter, and the promoter includes one or more of an inducible promoter, a constitutive promoter, or a tissue-specific promoter.

In one embodiment, the promoter includes one or more of T7, SP6, T3, CMV, EF1a, SV40, PGK1, human β-actin, CAG, U6, H1, T7, T7lac, araBAD, trp, lac, or Ptac.

In one embodiment, the first regulatory element and the second regulatory element are located in the same vector or in different vectors.

In one embodiment, the vector includes a retroviral vector, a lentiviral vector, an adenoviral vector, an adeno-associated viral vector, a herpes simplex vector, or a phagemid vector. In one embodiment, the vector includes a plasmid vector.

In some aspects, the disclosure provides a delivery composition, comprising a delivery vehicle, and one or more selected from: the Cas protein according to the disclosure, the fusion protein according to the disclosure, the polynucleotide according to the disclosure, the vector according to the disclosure, the complex according to the disclosure, the CRISPR-Cas system according to the disclosure, the CRISPR-Cas composition according to the disclosure, or the system according to the disclosure.

### Host Cell

The system according to the disclosure can be introduced into a host cell and cause the host cell to alter the production of one or more cellular products (such as antibodies, starch, ethanol, or any other desired product). Such host cells and their progeny are within the scope of the disclosure. As used herein, a "host cell" refers to a eukaryotic cell (e.g., an animal cell, a plant cell, a fungal cell, etc.), a prokaryotic cell (e.g., some microbial cells, *Escherichia coli, Bacillus subtilis,* etc.), or a cell from a multicellular organism cultured as a single-cell entity (e.g., a cell line). These cells serve as recipients of nucleic acids (e.g. expression vectors), and include the progeny of the original cells that have been genetically modified by nucleic acids.

It should be understood that the progeny of a single cell may not necessarily have exactly the same morphology, genome, or the like as the original parent cell due to natural, accidental, or intentional mutations. A "recombinant host cell" (also known as a "genetically modified host cell") is a host cell into which a heterologous nucleic acid, such as an expression vector, has been introduced. Those skilled in the art will understand that the design of an expression vector can depend on factors such as the choice of host cell to be transformed and the desired level of expression.

In one aspect, the disclosure provides a host cell or its progeny, which comprises the Cas protein according to the disclosure, or the fusion protein according to the disclosure, or the polynucleotide according to the disclosure, or the vector system according to the disclosure, or the CRISPR-Cas system according to the disclosure, or the composition according to the disclosure.

In some embodiments, the cell is a stem cell. In some embodiments, the cell is not a human embryonic stem cell. In some embodiments, the cell is not a human germ cell.

In some embodiments, the cell is a prokaryotic cell.

In some embodiments, the cell is a eukaryotic cell (e.g., an animal cell, a vertebrate cell, a mammalian cell, a non-human mammalian cell, a non-human primate cell, a rodent (e.g., mouse or rat) cell, a human cell, a plant cell, or a yeast cell) or a prokaryotic cell (e.g., a bacterial cell).

In some embodiments, the cell is from a plant or an animal.

In some embodiments, the plant is a dicotyledon. In some embodiments, the dicotyledon is selected from soybean, cabbage (e.g., Chinese cabbage), rapeseed, Brassica, watermelon, melon, potato, tomato, tobacco, eggplant, pepper, cucumber, cotton, alfalfa, eggplant, and grape. In some embodiments, the plant is a monocotyledon. In some embodiments, the monocotyledon is selected from rice, corn, wheat, barley, oats, sorghum, millet, grasses, Poaceae, Zizania, Avena, Coix, Hordeum, Oryza, Panicum (e.g., *Panicum miliaceum),* Secale, Setaria (e.g., *Setaria italica*), Sorghum, Triticum, Zea, Cymbopogon, Saccharum (e.g., *Saccharum officinarum*), Phyllostachys, Dendrocalamus, Bambusa, and Yushania.

In some embodiments, the animal is selected from swine, cattle, sheep, goats, mice, rats, alpacas, monkeys, rabbits, chickens, ducks, geese, and fish (e.g., zebrafish).

In some embodiments, the cell is a eukaryotic cell, such as a mammalian cell, including human cells (primary human cells or established human cell lines). In some embodiments, the cell is a non-human mammalian cell, such as cells from non-human primates (e.g., monkeys), cows/bulls/cattle, sheep, goats, swine, horses, dogs, cats, and rodents (such as rabbits, mice, rats, hamsters, etc.). In some embodiments, the cell is from fish (such as salmon), birds (such as poultry, including chickens, ducks, geese), reptiles, shellfish (e.g., oysters, clams, lobsters, shrimp), insects, worms, yeast, etc. In some embodiments, the cell is from a plant, such as a monocotyledon or a dicotyledon. In certain embodiments, the plant is a food crop, such as barley, cassava, cotton, groundnut or peanut, maize, millet, oil palm fruit, potato, dry bean, rapeseed or canola, rice, rye, sorghum, soybean, sugarcane, sugar beet, sunflower, and wheat. In certain embodiments, the plant is a cereal (barley, maize, millet, rice, rye, sorghum, and wheat). In certain embodiments, the plant is a tuber (cassava and potato). In certain embodiments, the plant is a sugar crop (sugar beet and sugarcane). In certain embodiments, the plant is an oil-bearing crop (soybean, groundnut or peanut, rapeseed or canola, sunflower, and oil palm fruit). In certain embodiments, the plant is a fiber crop (cotton). In certain embodiments, the plant is a tree (such as a peach or nectarine tree, an apple tree or a pear tree, a nut tree (such as an almond tree, a walnut tree, or a pistachio tree), or a citrus tree (e.g., an orange, grapefruit, or lemon tree)), grass, vegetables, fruits, or algae. In certain embodiments, the plant is a Solanum plant; a Brassica plant; a Lactuca plant; a Spinacia plant; a Capsicum plant; cotton, tobacco, asparagus, carrot, cabbage, broccoli, cauliflower, tomato, eggplant, pepper, lettuce, spinach, strawberry, blueberry, raspberry, blackberry, grape, coffee, cocoa, etc.

In one embodiment, the host cell includes a cell of a non-human mammalian, a human, an insect, an avian, a reptilian, an amphibian, a rodent, a fish, a worm, a nematode, or yeast.

In one aspect, the disclosure also provides a multicellular organism, which contains the aforementioned cells or their progeny.

In one embodiment, the multicellular organism is an animal model or a plant model for related diseases.

In yet another aspect, the disclosure provides a cell modified by the system or method according to the disclosure. In some embodiments, the cell is a eukaryote. In some embodiments, the cell is a human cell. In some embodiments, the cell is modified *in vitro, in vivo,* or *ex vivo.*

### Enzyme Preparation

In one aspect, the disclosure provides an enzyme preparation, which includes the Cas protein according to the disclosure, the fusion protein according to the disclosure, the complex according to the disclosure, the CRISPR-Cas system according to the disclosure, the CRISPR-Cas composition according to the disclosure, the system according to the disclosure, or the delivery composition according to the disclosure.

In some embodiments, the enzyme preparation includes an injection and/or a lyophilized preparation.

### Modification Methods

The CRISPR-Cas system according to the disclosure, which contains the Cas protein or fusion protein according to the disclosure, has a variety of uses, including modifying (e.g., cleaving, deleting, inserting, translocating, inactivating, or activating) a target DNA in various cell types. The method and/or system according to the disclosure can be used to modify a target DNA, for example, to modify the translation and/or transcription of one or more genes in a cell. For example, the modification can lead to an increase in the transcription/translation/expression of a gene. In other embodiments, the modification can lead to a decrease in the transcription/translation/expression of a gene.

As used herein, cleavage refers to a DNA break in a target nucleic acid generated by the Cas protein according to the disclosure. In some embodiments, the cleavage is breakage in a double-stranded DNA. In some embodiments, the cleavage is breakage in a single-stranded DNA. In some embodiments, the cleavage is to produce a nick in the non-spacer complementary strand of a double-stranded target nucleic acid. In some embodiments, the cleavage occurs at different sites on the two strands of a double-stranded nucleic acid, resulting in a staggered cleavage.

In one aspect, the disclosure provides a method for targeting and editing a target gene or cleaving a target gene, comprising bringing the Cas protein according to the disclosure, or the fusion protein according to the disclosure, or the complex according to the disclosure, or the CRISPR-Cas system according to the disclosure, or the composition according to the disclosure, or the system according to the disclosure, or the delivery composition according to the disclosure, or the enzyme preparation according to the disclosure, or the kit according to the disclosure, into contact with the target DNA, or delivering it into a cell containing the target DNA, wherein the target sequence is present in the target DNA.

In yet another aspect, the disclosure also provides a method for inducing a change in the state of a cell, comprising bringing the Cas protein according to the disclosure, or the fusion protein according to the disclosure, or the complex according to the disclosure, or the CRISPR-Cas system according to the disclosure, or the composition according to the disclosure, or the system according to the disclosure, or the delivery composition according to the disclosure, or the enzyme preparation according to the disclosure, or the kit according to the disclosure, into contact with a target gene in the cell. In some embodiments, the state of a cell includes apoptosis or dormancy.

In yet another aspect, the disclosure also provides a method for altering the expression of a gene product, comprising bringing the Cas protein according to the disclosure, or the fusion protein according to the disclosure, or the complex according to the disclosure, or the CRISPR-Cas system according to the disclosure, or the composition according to the disclosure, or the system according to the disclosure, or the delivery composition according to the disclosure, or the enzyme preparation according to the disclosure, or the kit according to the disclosure, into contact with a nucleic acid molecule encoding the gene product, or delivering it into a cell containing the nucleic acid molecule, wherein the target sequence is present in the nucleic acid molecule.

In yet another aspect, the disclosure provides a method for modifying a target DNA, the method comprising contacting the target DNA with the Cas protein according to the disclosure, or the fusion protein according to the disclosure, or the complex according to the disclosure, or the CRISPR-Cas system according to the disclosure, or the composition according to the disclosure, or the system according to the disclosure, or the delivery composition according to the disclosure, or the enzyme preparation according to the disclosure, or the kit according to the disclosure. In some embodiments, the target DNA is inside a cell. In some embodiments, the modification includes one or more of cleavage of a target DNA, base editing in a target DNA, repair of a target DNA, and insertion or integration of an exogenous sequence into a target DNA.

In some embodiments, the method for modifying a target DNA according to the disclosure includes contacting the target DNA with: the Cas protein according to the disclosure, the guide RNA according to the disclosure; a donor nucleic acid, e.g., a donor template. In some embodiments, the donor template is a double-stranded nucleic acid or a single-stranded nucleic acid. In some

examples, the donor template nucleic acid is linear or circular (e.g., a plasmid). In some examples, the donor template is an exogenous nucleic acid molecule. In some embodiments, the donor template is an endogenous nucleic acid molecule (e.g., a chromosome). In some embodiments, gene recombination can be achieved using the donor template, and this recombination is homologous recombination.

### Pharmaceutical Composition

In one aspect, the disclosure provides a pharmaceutical composition comprising: the complex according to the disclosure, the CRISPR-Cas system according to the disclosure, the composition according to the disclosure, the system according to the disclosure, the delivery composition according to the disclosure, or the host cell according to the disclosure; and a pharmaceutically acceptable excipient.

Suitable pharmaceutically acceptable excipients generally include inert substances that facilitate the administration of the pharmaceutical composition to a subject, facilitate the processing of the pharmaceutical composition into a deliverable formulation, or facilitate the storage of the pharmaceutical composition prior to administration. Pharmaceutically acceptable carriers may include agents that can stabilize, optimize, or otherwise modify the form, consistency, viscosity, pH, pharmacokinetics, or solubility of the formulation. Such agents include buffers, wetting agents, emulsifiers, diluents, encapsulating agents, and skin penetration enhancers. For example, the carrier may include, but is not limited to, saline, buffered saline, dextrose, arginine, sucrose, water, glycerol, ethanol, sorbitol, dextran, sodium carboxymethylcellulose, and combinations thereof.

Some non-limiting examples of substances that can be used as pharmaceutically acceptable carriers include: (1) sugar, such as lactose, glucose, and sucrose; (2) starch, such as corn starch and potato starch; (3) cellulose and derivatives thereof, such as sodium carboxymethylcellulose, methylcellulose, ethylcellulose, microcrystalline cellulose, and cellulose acetate; (4) powdered tragacanth; (5) malt; (6) gelatin; (7) lubricanting agents, such as magnesium stearate, sodium lauryl sulfate, and talc; (8) excipient, such as cocoa butter and suppository wax; (9) oil, such as peanut oil, cottonseed oil, safflower oil, sesame oil, olive oil, corn oil, and soybean oil; (10) glycol, such as propylene glycol; (11) polyol, such as glycerol, sorbitol, mannitol, and polyethylene glycol (PEG); (12) ester, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethanol; (20) pH-buffered solution; (21) polyester, polycarbonate, and/or polyanhydride; (22) filler, such as polypeptide and amino acid; (23) serum alcohol, such as ethanol; and (24) other non-toxic compatible substances used in pharmaceutical formulations. The formulation may further comprise wetting agents, coloring agents, release agents, coating agents, sweetening agents, flavoring agents, fragrant, preservatives, and antioxidants.

The pharmaceutical composition may contain one or more pH-buffering compounds to maintain the pH of the formulation at a predetermined level reflecting the physiological pH, such as in the range of about 5.0 to about 8.0. The pH-buffering compound for an aqueous liquid formulation may be an amino acid or a mixture of amino acids, such as histidine or a mixture of amino acids (such as histidine and glycine). Alternatively, the pH-buffering compound is preferably an agent that maintains the pH of the formulation at a predetermined level (such as in the range of about 5.0 to about 8.0) and does not chelate calcium ions. Illustrative examples of such pH-buffering compounds include, but are not limited to, imidazole and acetate ions. The pH-buffering compound may be present in any amount suitable for maintaining the pH of the formulation at a predetermined level.

The pharmaceutical composition may also contain one or more osmotic regulators, which adjust the osmotic properties (e.g., tonicity, osmolality, and/or osmotic pressure) of the formulation to a level acceptable to the bloodstream and blood cells of the recipient individual. The osmotic regulator may be an agent that does not chelate calcium ions. The osmotic regulator may be any compound known or available to those skilled in the art to regulate the osmotic properties of the formulation. Those skilled in the art can empirically determine the suitability of a given osmotic regulator in the formulation of the present invention. Illustrative examples of suitable types of osmotic regulators include, but are not limited to: salts, such as sodium chloride and sodium acetate; sugars, such as sucrose, dextrose, and mannitol; amino acids, such as glycine; and a mixture of one or more of these agents and/or dosage forms. One or more osmotic regulators may be present at any concentration sufficient to adjust the osmotic properties of the formulation.

### Therapeutic Methods

In one aspect, the disclosure provides a method for diagnosing, preventing, or treating a disease in a subject in need thereof. The method comprises administering to the subject (e.g., at a therapeutically effective dose) the Cas protein according to the disclosure, the fusion protein according to the disclosure, the polynucleotide according to the disclosure, the vector according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, the enzyme preparation according to the disclosure, the pharmaceutical kit according to the disclosure, or the pharmaceutical composition according to the disclosure; wherein the disease is associated with a target DNA, the spacer sequence is capable of hybridizing to a target sequence of the target DNA, the target DNA is modified by the complex, and the modification of the target DNA allows for diagnosis, prevention, or treatment of the disease.

As used herein, the term "treatment" refers to treating or curing a disorder in a subject, delaying the onset of symptoms of the disorder, and/or alleviating the severity of the disorder.

In some embodiments, suitable routes of administration include, but are not limited to: topical, subcutaneous, transdermal, intradermal, intralesional, intra-articular, intraperitoneal, intravesical, transmucosal, gingival, intradental, intracochlear, transtympanic, intra-organ, epidural, intrathecal, intramuscular, intravenous, intravascular, intraosseous, periorbital, intratumoral, intracerebral, and intraventricular administration. In some embodiments, administration to the subject is via injection, via a catheter, via a suppository, or via an implant, wherein the implant is a porous, nonporous, or gel-like material, including a membrane, such as a salivary membrane, or a fiber.

In some embodiments, a target DNA encodes mRNA, tRNA, ribosomal RNA, microRNA (miRNA), non-coding RNA, long non-coding (Inc) RNA, nuclear RNA, interfering RNA, small interfering RNA (siRNA), ribozyme, riboswitch, satellite RNA, microswitch, microzyme, or viral RNA.

In some embodiments, a target DNA is eukaryotic DNA. In some embodiments, the eukaryotic DNA is mammalian DNA (such as non-human mammalian DNA), non-human primate DNA, human DNA, plant DNA, insect DNA, avian DNA, reptilian DNA, rodent (e.g., mouse, rat) DNA, fish DNA, nematode DNA, or yeast DNA.

In some embodiments, the target DNA is inside a eukaryotic cell (e.g., inside a human cell, a non-human primate cell, or a mouse cell).

In some embodiments, administration includes topical administration or systemic administration. In some embodiments, administration is via injection or infusion. In some embodiments, the subject is a human, a non-human primate, or a mouse.

In some embodiments, as compared to the level of the transcript (e.g., mRNA) of a target DNA in a subject before administration, the level of the transcript (e.g., mRNA) of the target DNA in the subject is decreased or increased by at least about 10%, about 15%, about 20%, about 25%, about 30%, about 35%, about 40%, about 45%, about 50%, about 55%, about 60%, about 65%, about 70%, about 75%, about 80%, about 85% or more.

In some embodiments, as compared to the level of the expression product (e.g., protein) of a target DNA in a subject before administration, the level of the expression product (e.g., protein) of the target DNA in the subject is decreased or increased by at least about 10%, about 15%, about 20%, about 25%, about 30%, about 35%, about 40%, about 45%, about 50%, about 55%, about 60%, about 65%, about 70%, about 75%, about 80%, about 85% or more. In some embodiments, the expression product is a functional mutant of the expression product of the target DNA.

In some embodiments, the disease is a disease wherein non-dividing cells are to be treated. In one embodiment, the gene or transcript to be corrected is located in non-dividing cells. Exemplary non-dividing cells are muscle cells or neurons.

In some embodiments, the disease is selected from diseases affecting the eyes, such as glaucoma, or retinal degenerative diseases. In one embodiment, a lentiviral vector is selected for administration to the eyes.

In some embodiments, the disease is selected from muscle diseases and cardiovascular diseases. In some embodiments, the disease includes Duchenne muscular dystrophy (DMD).

In some embodiments, the disease is selected from liver and kidney diseases. In some embodiments, the disease may also be selected from epithelial diseases, lung diseases, skin diseases, and cancer.

In some embodiments, the disease is selected from familial hypercholesterolemia (FH), atherosclerotic cardiovascular disease (ASCVD), transthyretin amyloidosis (ATTR), alpha-1-antitrypsin deficiency (AATD), primary hyperoxaluria (PH1), hereditary angioedema (HAE), Angelman syndrome (AS), Alzheimer's disease (AD), transthyretin amyloid cardiomyopathy (ATTR-CM), cystic fibrosis (CF), diabetes, progressive pseudohypertrophic muscular dystrophy, Duchenne muscular dystrophy (DMD), Becker muscular dystrophy (BMD), spinal muscular atrophy (SMA), Pompe disease, myotonic dystrophy, Huntington's disease (HTT), Fragile X syndrome, Friedreich's ataxia, amyotrophic lateral sclerosis (ALS), frontotemporal dementia, hereditary chronic kidney disease, hyperlipidemia, Leber congenital amaurosis (LCA), sickle-cell disease, thalassemia (e.g., beta-thalassemia), Parkinson's disease (PD), myelodysplastic syndrome (MDS), retinitis pigmentosa (RP), age-related macular degeneration (AMD), Hepatitis B, non-alcoholic fatty liver disease (NAFLD), acquired immunodeficiency syndrome, corneal dystrophy (CD), hypercholesterolemia, heart diseases (e.g., hypertrophic cardiomyopathy (HCM)), and cancer.

In some embodiments, the median survival period of a subject with the disease who has received the administration is 5 days, 10 days, 20 days, 30 days, 2 months, 3 months, 4 months, 5 months, 6 months, 7 months, 8 months, 9 months, 10 months, 11 months, 12 months, 1.5 years, 2 years, 2.5 years, 3 years, 4 years, 5 years, 6 years, 7 years, 8 years, 9 years, 10 years or longer than that of a subject or a group of subjects with the disease who have not received the administration.

A therapeutically effective dose may be achieved via a single dose or multiple doses. Those skilled in the art should understand that the actual dose can vary significantly depending on various factors, such as the choice of vector, the target cell, the organism, tissue, the general condition of the subject to be treated, the degree of transformation/modification sought, the route of administration, the mode of administration, the type of transformation/modification sought, etc.

In one embodiment, the administration further includes administering the CRISPR-Cas composition to the subject or ex vivo cells of the subject.

### Detection Method

In yet another aspect, the disclosure provides a method for detecting the presence of a target nucleic acid molecule in a sample. The method comprises contacting the sample with the Cas protein according to the disclosure, the fusion protein according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, or the enzyme preparation according to the disclosure, and a non-target sequence; and detecting a detectable signal generated by the cleavage of the non-target sequence, thereby detecting the target nucleic acid molecule, wherein the non-target sequence does not hybridize to the guide RNA. In some embodiments, the modification generates a detectable signal, such as a fluorescent signal. In some embodiments, a reporter nucleic acid refers to a molecule that can be cleaved or otherwise inactivated by the activated CRISPR system proteins described herein. The reporter nucleic acid contains a nucleic acid element that can be cleaved by the CRISPR protein (for example, a single-stranded non-target nucleic acid molecule with different reporter groups or labeling molecules at both ends). Cleavage of the nucleic acid element generates a detectable signal. Before cleavage, or when the reporter nucleic acid is in an "active" state, the reporter nucleic acid prevents the generation or detection of a positive detectable signal. It will be understood that in some example embodiments, a minimal background signal may be generated in the presence of an active reporter nucleic acid. The positive detectable signal may be any signal that can be detected by an optical, fluorescent, chemiluminescent, or electrochemical method, or other detection methods known in the art. For example, in some embodiments, a first signal (i.e., a negative detectable signal) can be detected when the reporter nucleic acid is present, and then it is converted to a second signal (e.g., a positive detectable signal) after the detection of a target molecule and its cleavage or inactivation by the activated CRISPR protein. The reporter nucleic acid may be a single-stranded DNA molecule, a single-stranded RNA molecule, or a single-stranded DNA-RNA hybrid.

The detection method according to the disclosure may be used for the quantitative detection of a target nucleic acid to be detected. The quantitative detection index can be quantified according to the strength of the signal of the reporter group, such as according to the luminescence intensity of a fluorescent group, or according to the width of a color-developing band, etc.

### Uses

In one aspect, the Cas protein according to the disclosure, the fusion protein according to the disclosure, the polynucleotide according to the disclosure, the vector according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, the enzyme preparation according to the disclosure, or the pharmaceutical kit according to the disclosure are for use in the manufacture of a medicament or a formulation for nucleic acid editing (e.g., gene editing or genome editing).

In yet another aspect, the Cas protein according to the disclosure, the fusion protein according to the disclosure, the polynucleotide according to the disclosure, the vector according to the disclosure, the complex according to the disclosure, the CRISPR-Cas system according to the disclosure, the CRISPR-Cas composition according to the disclosure, the system according to the disclosure, the kit according to the disclosure, the delivery composition according to the disclosure, the enzyme preparation according to the disclosure, or the pharmaceutical kit according to the disclosure are for use in the manufacture of a medicament or a formulation for one or more selected from:
(i) *ex-vivo* gene editing or genome editing;
(ii) *ex-vivo* detection of a single-stranded DNA;
(iii) modifying an organism or a non-human organism by editing a target sequence in a target locus;
(iv) treating a disorder caused by a defect in a target sequence in a target locus; and
(v) treating a disorder or disease of a subject in need.

The disclosure will be further elaborated hereinafter in combination with specific examples. It should be understood that these examples are provided to only illustrate the disclosure and not to limit its scope. The experimental methods in the following examples without detailed conditions specified are usually carried out under conventional conditions, such as the conditions described in Sambrook et al., Molecular Cloning: A Laboratory Manual (New York: Cold Spring Harbor Laboratory Press, 1989), or according to the conditions recommended by the manufacturers. Unless otherwise stated, percentages and parts are percentages by weight and parts by weight.

Unless otherwise specified, the reagents and materials in the examples of the disclosure are all commercially available products.

### Example 1. Obtaining of Cas Protein

Through an analysis of the metagenome of an uncultured sample, and through analyses such as dereplication and protein clustering, a novel Cas protein was identified. The Blast analysis results showed that the sequence identity of this Cas protein to the reported Cas proteins was low. This Cas protein was named CasY7.

The amino acid sequence of the above-mentioned CasY7 protein is shown as SEQ ID NO:1, and its nucleotide coding sequence after human codon optimization is shown as SEQ ID NO:2.

The direct repeat (DR) sequence of the guide RNA corresponding to the above-mentioned CasY7 protein was analyzed by annotating the CRISPR locus. The results showed that:
The DNA sequence encoding the direct repeat (DR) sequence of the guide RNA corresponding to the CasY7 protein is: CAAGTTGAATCCGTCTATAACTGACGG (SEQ ID NO:5).

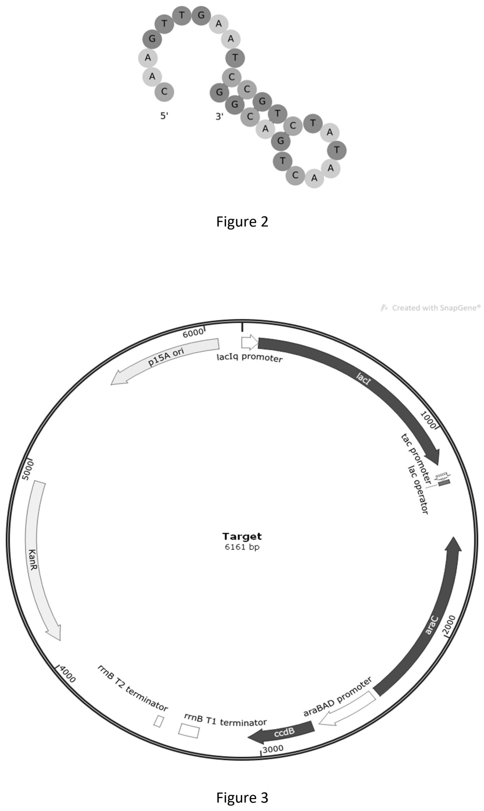

The RNA secondary structure of the DR sequence in the pre-crRNA was further analyzed by RNAfold. The analysis results are shown in Figure 2. In the sequence in the figure, any "T" represents "U" when referring to the DR sequence.

Through the PAM library depletion experiment (the experiment was carried out according to any embodiment of Karvelis et al., Methods. May 15, 2017; 121-122:3-8, the entire content of which is incorporated herein by reference) to identify the PAM sequence specificity of CasY7, and it was found through an analysis that the PAM corresponding to CasY7 is 5'-TTN, where N is A/T/C/G. The sgRNA (also known as crRNA) sequence of the CasY7 protein consists of a spacer sequence and a direct repeat (DR) sequence. The CasY7 of the disclosure was identified to belong to the Cas12 protein family.

### Example 2. Verification of the Cleavage Activity of Cas Protein

### 1. Plasmid Construction

(1) The TTR gene was used as the target, and according to the target sequence of the target gene TTR a spacer sequence was designed (Target-TTR-spacer1): GCATCTCCCCATTCCATGAG (SEQ ID NO: 6).

Based on the DR sequences of the CasY7 protein and the LbCpf1 protein, sgRNA sequences targeting the TTR target gene were designed, as shown in the following table.

| Sequence Name | Specific Sequence |
|---|---|
| CasY7-TTR-sgRNA1 sequence | CAAGTTGAATCCGTCTATAACTGACGGGCATCTCCCCATTCCATGAG (SEQ ID NO:7). The underlined part of the sequence is the DR sequence, and the remaining sequence is the spacer sequence targeting the TTR target sequence. |
| LbCpf1-TTR-sgRNA1 sequence | TAATTTCTACTAAGTGTAGATGCATCTCCCCATTCCATGAG (SEQ ID NO:8). The underlined part of the sequence is the DR sequence, and the remaining sequence is the spacer sequence targeting the TTR target sequence. |

According to the requirements of vector expression, a T7 promoter and an rrnB T2 terminator were added to the 5'-end and 3'-end of the sgRNA sequence of each of CasY7 and LbCpf1 mentioned above, obtaining the CasY7-sgRNA expression fragment sequence:
CAATTGTAATACGACTCACTATAGGCACCGCAAGTTGAATCCGTCTATAACTGACGGGCATCTCCCCATTCC ATGAGTTTTTTAAGCTTGGCGTagaaggccatcctgacggatggccttttACGCGT (SEQ ID NO:9), wherein the single-underlined sequence is the CasY7 DR sequence, the double-underlined sequence is the spacer sequence, the italic sequence is the T7 promoter, the wavy-underlined sequence is the rrnB T2 terminator sequence, the sequence between the spacer sequence and the rrnB T2 terminator sequence is a linker sequence, the dotted-line-underscored sequence is the Mfel restriction site, the sequence in bold is the Mlul restriction site, and the CACCG is a linker.

In the same way, the LbCpf1-sgRNA1 expression fragment sequence was synthesized:
CAATTGTAATACGACTCACTATAGGCACCGTAATTTCTACTAAGTGTAGATGCATCTCCCCATTCCATGAGTT TTTTAAGCTTGGCGTagaaggccatcctgacggatggccttttACGCGT (SEQ ID NO:10), wherein the single-underlined sequence is the LbCpf1 DR sequence, the double-underlined sequence is the spacer sequence, the italic sequence is the T7 promoter, the wavy-underlined sequence is the rrnB T2 terminator sequence, the sequence between the spacer sequence and the rrnB T2 terminator sequence is a linker sequence, the dotted-line-underscored sequence is the Mfel restriction site, the sequence in bold is the Mlul restriction site, and the CACCG is a linker.

To protect the integrity of the sequence, in the synthesis of the CasY7-sgRNA1 expression fragment sequence and the LbCpfl-sgRNA1 expression fragment sequence, AGC was introduced at the 5'-end and ATA was introduced at the 3'-end as protective bases.

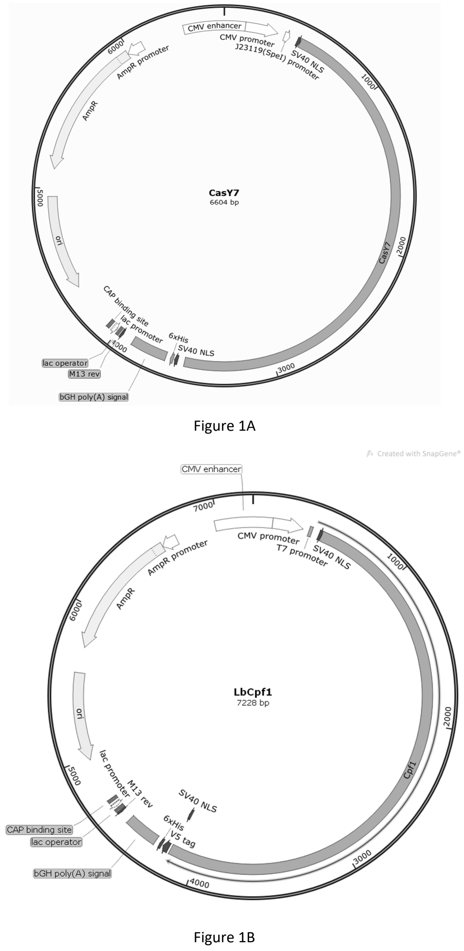

(2) A nucleotide sequence (as shown by SEQ ID NO: 2) fragment encoding the CasY7 protein was synthesized by Suzhou Synbio Technologies Co., Ltd. The synthesized nucleotide sequence fragment encoding the CasY7 protein was constructed into an ABE8e plasmid (Addgene, Plasmid #138489) at positions 466-5160, to construct a CasY7 recombinant expression plasmid (see Figure 1A in Figure 1 for the plasmid map,).

An optimized nucleotide sequence (SEQ ID NO: 20) encoding LbCpf1 (having the amino acid sequence of SEQ ID NO: 19) was synthesized by Suzhou Synbio Technologies Co., Ltd. and was constructed in the same way to obtain an LbCpf1 recombinant expression plasmid (see Figure 1B in Figure 1 for the plasmid map).

(3) The sgRNA expression sequence fragments (CasY7-sgRNA1 expression fragment sequence and LbCpf1-sgRNA1 expression fragment sequence) described in step (1) were synthesized by Suzhou Synbio Technologies Co., Ltd. The sgRNA expression fragment sequence was double-digested (Mfel/Mlul) and inserted into a CasY7 recombinant expression plasmid vector that was also double-digested (Mfel/Mlul), to obtain a recombinant CasY7+sgRNA1 expression plasmid expressing CasY7 and sgRNA. The LbCpf1+sgRNA1 expression plasmid was constructed in the same way.

(4) A Target plasmid with a target sequence was constructed as follows.

An araC-pBAD-CCDB fragment (SEQ ID NO: 18) with the TTR target sequence (SEQ ID NO: 6) was synthesized by Suzhou Synbio Technologies Co., Ltd, and was inserted into a pKESK22 plasmid (Addgene, Plasmid #64857) at positions 1284-1300 to obtain the Target plasmid. The sequence of the Target plasmid is of SEQ ID NO: 4, and the plasmid map is shown in Figure 3.

### 2. Preparation and Transformation of Escherichia coli Competent Cells

The Target plasmid was transferred into DH5α competent cells, and then streaked and inoculated on an LB solid medium containing 50 µg/ml kanamycin sulfate with an inoculating loop. The plate was placed in a biochemical incubator and cultured overnight at 37°C. The next day, a single colony was picked from the plate and inoculated into a test tube containing 4 ml of LB liquid medium with 50 µg/ml kanamycin sulfate (Sangon Biotech, A100408-0100), and cultured overnight under shaking at 37 °C and 200 rpm. The following day, 4 ml of the bacterial liquid culture was inoculated into a 2-L Erlenmeyer flask containing 400 ml of LB liquid medium with 50 µg/ml kanamycin sulfate, and cultured under shaking at 37 °C and 200 rpm for 2-3 hours.

When the OD600 nm value of the bacterial liquid culture reached 0.3-0.5, the Erlenmeyer flask was taken out and placed on ice for 10-15 min. Under a sterile condition, the bacterial liquid culture was poured into a pre-cooled 500-ml centrifuge bottle, centrifuged at 4 °C and 3000 rpm for 8 min. The supernatant was discarded, and approximately 200 ml of a pre-cooled CaCl2 solution was added. The mixture was pipetted to resuspend the bacterial cells, and then placed in an ice-bath for 30 min. Subsequently, the bacterial liquid culture was centrifuged at 4 °C and 3000 rpm for 8 min. The supernatant was discarded, and approximately 8 ml of a pre-cooled CaCl2 solution was added to resuspend the bacterial cells again. The resuspended bacterial cells were aliquoted into 1.5-ml EP tubes, 110 µl per tube, and stored in an ultra-low-temperature freezer at -80 °C for later use.

### 3. Determination of in vivo Editing Efficiency in Escherichia coli

The CasY7+sgRNA1 expression plasmid and the LbCpf1+sgRNA1 expression plasmid were respectively transferred into the competent cells prepared in Step 2. The detailed process was as follows.
(1) The competent cells were taken out of the -80 °C freezer and quickly buried in ice. After about 5 minutes, the cell mass thawed, and the CasY7+sgRNA1 expression plasmid was added. Then, they were gently mixed by tapping the bottom of the centrifuge tube by hand, let stand in ice for 25 minutes, heat-shocked in a 42 °C water-bath for 45 seconds, quickly put back on ice, and let stand for 2 minutes. 900 µl of sterile LB medium without antibiotics was added to the centrifuge tube, mixed well, and recovered at 37 °C and 220 rpm for 60 minutes. 100 µl of the bacterial liquid was spread onto each of an LB agar plate containing 30 µg/ml carbenicillin (Sangon Biotech, A100358-0001) (referred to as C-LB medium) and an LB agar plate containing both 30 µg/ml carbenicillin and 10 mM L-arabinose (Sangon Biotech, A610071-0100) (referred to as CL-LB medium). The two LB agar plates were placed upside-down in an incubator and cultured overnight at 37°C.
(2) Measurement of *in vivo* Editing Efficiency in *Escherichia coli*

As shown in Figure 3, the Target plasmid carried a PBAD promoter inducible by L-arabinose and a CCDB gene whose expression is regulated by the PBAD promoter. The CCDB gene can express the CCDB toxic protein. As a DNA gyrase inhibitor, the CCDB toxic protein can lock the DNA gyrase and the broken double-stranded DNA complex, preventing the DNA gyrase from functioning and ultimately leading to cell death.

Based on this, the applicant designed a method for measuring the in vivo editing efficiency in *Escherichia coli.*

When L-arabinose is present in the medium, if the CasY7 protein or the LbCpf1 protein under the guidance by the sgRNA can specifically target the target sequence (SEQ ID NO: 6) of the TTR gene on the Target plasmid and exert a cleavage effect, then the regulatory expression pathway of the PBAD promoter on the CCDB toxic protein is shut down, and the host cells survive because no ccdB toxic protein is produced. Conversely, if the CasY7 protein or the LbCpf1 protein cannot specifically target the TTR target sequence on the Target plasmid, the host *Escherichia coli* cells die because of the expression of the CCDB toxic protein regulated by the PBAD promoter induced by L-arabinose.

Therefore, the editing efficiency of the CasY7 protein in specifically targeting and cleaving the TTR target gene in *Escherichia coli* can be calculated as the ratio of the number of bacterial clones on the CL-LB medium to the number of bacterial clones on the C-LB medium in Step (2).

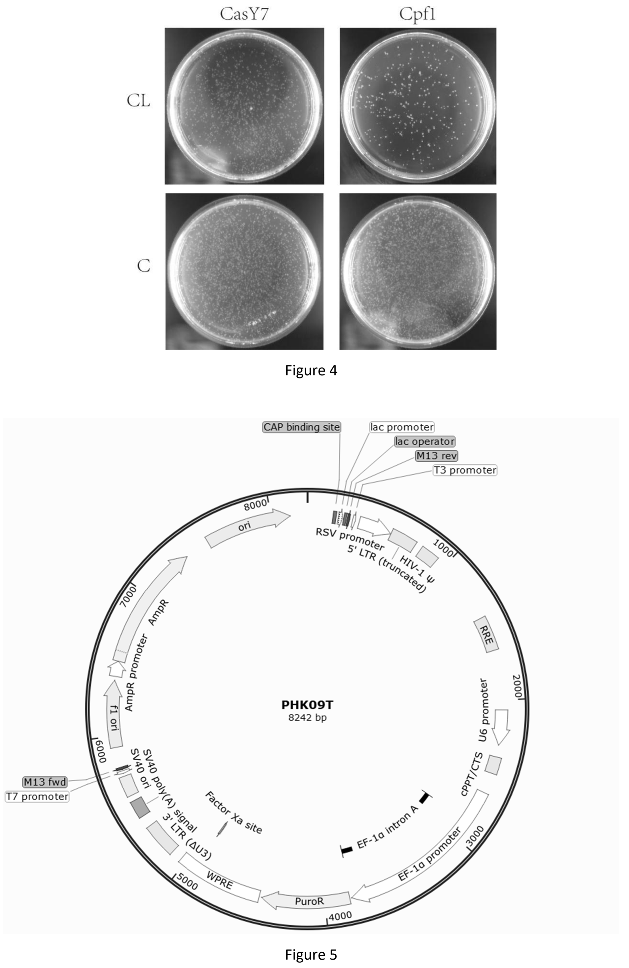

The results are shown in Figure 4. After counting the number of *Escherichia coli* clones and calculating the ratio, it was found that the editing efficiency of the CasY7 protein was 45.5%, and the editing efficiency of the LbCpf1 protein was 11.1%. The editing efficiency of the CasY7 protein was significantly higher than that of the LbCpf1 protein.

### Example 3. Measurement of Editing Efficiency in HEK293T Cells

### 1. Construction of TTR-sgRNA Expression Plasmid

(1) Based on the target sequence of the TTR gene (Target-TTR-spacer2) tagaagggatatacaaagtg (SEQ ID NO:11), TTR-sgRNA sequences were designed and oligonucleotides (oligos) were synthesized:
   CasY7-TTR-sgRNA2 sequence:
      CAAGTTGAATCCGTCTATAACTGACGGtagaagggatatacaaagtg (SEQ ID NO:12). The underlined part is the DR sequence, and the remaining sequence is the spacer sequence.
   LbCpf1-TTR-sgRNA2 sequence:
      TAATTTCTACTAAGTGTAGATtagaagggatatacaaagtg (SEQ ID NO:13). The underlined part is the DR sequence, and the remaining sequence is the spacer sequence.
(2) A sequence CACC was added to the 5'-end of the upstream sequence of the TTR-sgRNA, and a sequence AAAA was added to the 5'-end of the downstream sequence, and the oligos were synthesized. The specific sequences are as follows:

| sgRNA Name | Upstream and Downstream Primer Sequences |
|---|---|
| CasY7-TTR-sgRNA2 | CasY7-TTR-sgRNA2-Oligo-F: |
| | CACCCAAGTTGAATCCGTCTATAACTGACGGtagaagggatatacaaagtg (SEQ ID NO:14) |
| | CasY7-TTR-sgRNA-Oligo-R: |
| | AAAACACTTTGTATATCCCTTCTACCGTCAGTTATAGACGGATTCAACTTG (SEQ ID NO:15) |
| LbCpf1-TTR-sgRNA2 | LbCpf1-TTR-sgRNA2-Oligo-F: CACCTAATTTCTACTAAGTGTAGATtagaagggatatacaaagtg (SEQ ID NO:16) |
| | LbCpf1-TTR-sgRNA2-Oligo-R: |
| | AAAACACTTTGTATATCCCTTCTAATCTACACTTAGTAGAAATTA (SEQ ID NO:17) |

After the upstream and downstream primers of the aforementioned TTR-sgRNA were synthesized, annealing was carried out in a preset program (95 °C, 5 min; from 95 °C to 85 °C at a rate of -2 °C/s; from 85 °C to 25 °C at a rate of -0.1 °C/s; held at 4 °C). Then, the annealed product was ligated to a PHK09T vector linearized by BsmBI (NEB, #R0580L). The sequence of the PHK09T vector is SEQ ID NO: 3, and the plasmid map is shown in Figure 5.

The linearization of the PHK09T vector and its ligation with the annealed product of TTR-sgRNA were as follows.

First, the PHK09T vector was linearized in a linearization system: 3 µg of the PHK09T vector; 6 µL of a buffer (NEB: R0539L); 2 µL of BsmBl; and ddH₂O making up to 60 µL, which were digested at 50 °C overnight.

The ligation system of the annealed product of TTR-gRNA and the linearized vector:
1 µL of a T4 ligase buffer (NEB, #M0202L), 20 ng of the linearized vector, 5 µL of the annealed oligo fragment, 0.5 µL of T4 ligase (NEB, #M0202L), and ddH₂O making up to 10 µL, which were ligated at 16 °C overnight to obtain the CasY7-TTR-sgRNA2 expression plasmid and the LbCpf1-TTR-sgRNA2 expression plasmid.

(3) The CasY7-TTR-sgRNA2 expression plasmid and the LbCpf1-TTR-sgRNA2 expression plasmid obtained in step (2) were each transferred into Escherichia coli DH5α competent cells (Vazyme, DL1001). The specific steps were as follows.

The DH5α competent cells were taken out from a -80 °C refrigerator and quickly buried in ice. After 5 minutes, the cell mass thawed, and the ligation product was added. They were gently mixed by tapping the bottom of the centrifuge tube by hand, let stand in ice for 25 minutes, heat-shocked in a 42 °C water-bath for 45 seconds, quickly put back on ice, and let stand for 2 minutes. 700 µl of sterile LB medium without antibiotics was added to the centrifuge tube, mixed well, and recovered at 37 °C and 200 rpm for 60 minutes. The mixture was centrifuged at 3,000 rpm for one minute to collect the bacteria. With about 100 µl of the supernatant left in the tube, the cell mass was gently resuspended by pipetting, and spread onto an LB medium plate with Amp antibiotic. The plate was placed upside-down in a 37 °C incubator and cultured overnight. Single colonies were picked. After confirmation by sequencing, positive clones were cultured, the plasmids were extracted (with the Endo-Free Plasmid Mega Kit, TIANGEN: DP120-01), and the concentration was measured, followed by storage in a -20 °C refrigerator for later use.

### 2. Measurement of Editing Efficiency at the Cellular Level

### (1) HEK293T Cell Culture

HEK293T cells (purchased from ATCC) were inoculated into DMEM medium (Gibco, 11965092) supplemented with 10% FBS (v/v) and containing 1% Penicillin Streptomycin (v/v) (Gibco, 15140122), and cultured in a 37 °C cell incubator with 5% CO₂. On day one the cells to be transfected were inoculated into a 24-well cell-culturing plate and cultured. On day two, the cells were observed, and were transfected when the cell confluency reached approximately 80%.

(2) The CasY7 recombinant expression plasmid (see the plasmid map in Figure 1A of Figure 1), the CasY7-TTR-sgRNA2 expression plasmid, the LbCpf1 recombinant expression plasmid (see the plasmid map in Figure 1B of Figure 1), and the LbCpf1-TTR-sgRNA2 expression plasmid were each used together with the EGFP-C1 (Addgene, Plasmid, #54759) plasmid to co-transfect HEK293T cells.

The amounts of plasmids used to transfect the cells per well in the 24-well plate were 0.3 µg of the nuclease expression plasmid (CasY7 recombinant expression plasmid or LbCpf1 recombinant expression plasmid), 0.3 µg of the sgRNA expression plasmid (CasY7-TTR-sgRNA2 expression plasmid or LbCpf1-TTR-sgRNA2 expression plasmid), and 0.3 µg of the EGFP-C1 plasmid. The specific transfection procedure was as follows.

The CasY7 expression plasmid, the CasY7-TTR-sgRNA2 expression plasmid, and the EGFP-C1 plasmid were mixed and diluted with 25 µl of UltraFectin^{®} transfection-specific serum-free medium (Yuanpei Biology, L530KJ), then 2 µl of Lipofectamine 3000 (Invitrogen, L3000015) reagent was added, and the mixture was pipetted gently and mixed until uniform to obtain Reagent A, which was left to stand for 5 minutes. At the same time, 2 µl of Lipofectamine 3000 transfection reagent (Invitrogen, L3000015) was diluted and mixed with 25 µl of UltraFectin^{®} transfection-specific serum-free medium (Yuanpei Biology, L530KJ) to obtain Reagent B, which was left to stand for 5 minutes.

Reagent A and Reagent B were mixed and pipetted until uniform, and left to stand for 20 minutes. After the standing, the mixed reagent was added dropwise to the cells to be transfected in a 24-well plate, and then the plate was returned to a 37 °C, 5% CO₂ incubator for culturing. Six hours after transfection, the medium was replaced with DMEM medium containing 10% FBS.

In the same way, the LbCpf1 recombinant expression plasmid, the LbCpf1-TTR-sgRNA2 expression plasmid, and the EGFP-C1 plasmid were transfected into HEK293T cells.

### (3) Measurement of Editing Efficiency

Forty-eight hours after transfection, the expression of the EGFP fluorescent protein indicated successful cell transfection. EGFP-positive cells were sorted for the measurement of editing efficiency. Genomic DNA was extracted from these cells (using the Genomic DNA Extraction Kit, TIANGEN, DP304-03). Identification primers were designed according to experimental requirements, and the sequences of the identification primers used are shown in the following table:

| Primer Name | Specific Sequence |
|---|---|
| TTR-F | TCGTCGGCAGCGTCAGATGTGTATAAGAGACAGatccagactttcacacctta (SEQ ID NO:21) |
| TTR-R | GTCTCGTGGGCTCGGAGATGTGTATAAGAGACAGtcatggaatggggagatgcc(SEQ ID NO:22) |

With the genomic DNA as a template, the sequence near the target site was amplified by PCR using the primers shown in the above table. The PCR amplification system was as follows:
2× Taq Master Mix (Vazyme, P112-03) 25 µL; Primer-F (TTR-F) (10 pmol/µL) 1 µL; Primer-R (TTR-R) (10 pmol/µL) 1 µL; template 1 µL; ddH₂O making up to 50 µL.

The amplified PCR products were subjected to high-throughput deep sequencing (Tsingke Biotech Co., Ltd.) or Sanger sequencing (Biosune Biotechnology (Shanghai) Co., Ltd.) to determine the editing efficiency.

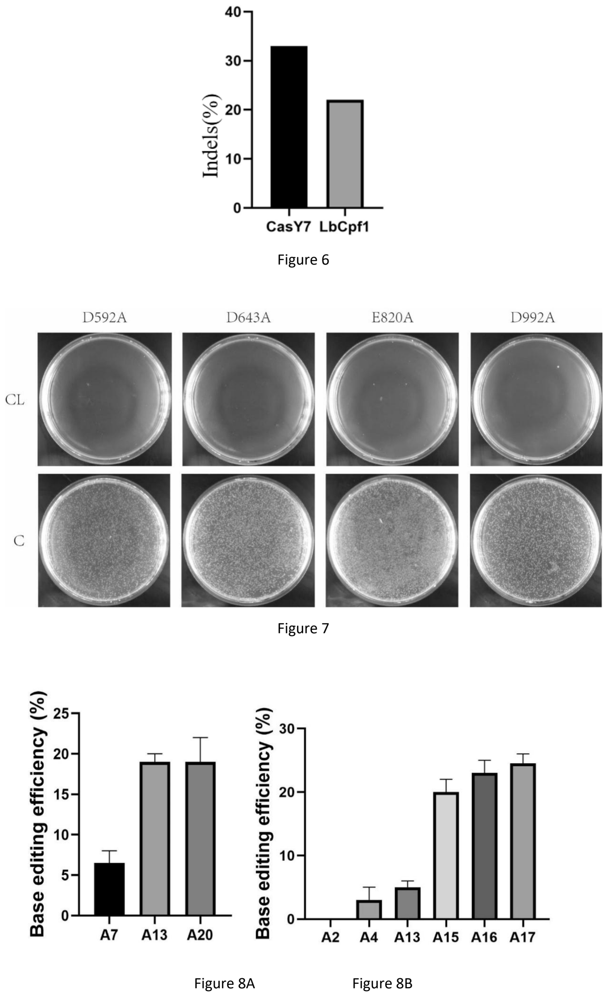

The results of measuring and determining the editing efficiency of CasY7 and LbCpf1 are shown in Figure 6. In 293T cells, the editing efficiency of CasY7 at the TTR gene was 33%, while that of LbCpf1 at the TTR gene was only 22%. The editing efficiency of the CasY7 protein was much higher than that of LbCpf1.

### Example 4. Application of CasY7 in Base Editing

### 1. Obtaining Catalytically Inactive CasY7

To obtain catalytically inactive (i.e., cleavage-inactive) dCasY7, the inventors constructed CasY7 mutants with single-point mutations D592A, D643A, E820A, and D992A, namely D592A-dCasY7, D643-dCasY7, E820A-dCasY7, and D992A-dCasY7. The amino acid sequences of the above-mentioned mutants are represented by SEQ ID NO:47-50 respectively. The specific construction methods therefor are as follows:
The CasY7+sgRNA1 expression plasmid obtained in Step 1 (3) of Example 2 was subjected to site-directed mutagenesis. The amino acids at four sites of CasY7 were modified, which were aspartic acid (Asp, D) at position 592, aspartic acid (Asp, D) at position 643, glutamic acid (Glu, E) at position 820, and aspartic acid (Asp, D) at position 992 of SEQ ID NO:1. The amino acids at the above-mentioned sites were mutated to alanine (Ala, A). The codons before and after the mutation of each of the above-mentioned amino acids are shown in the following table:

| Amino Acid before Mutation | Codon | Amino Acid after Mutation | Codon |
|---|---|---|---|
| Aspartic acid (Asp, D) | GAC | Alanine (Ala, A) | GCA |
| Aspartic acid (Asp, D) | GAC | Alanine (Ala, A) | GCA |
| Glutamic acid (Glu, E) | GAG | Alanine (Ala, A) | GCA |
| Aspartic acid (Asp, D) | GAC | Alanine (Ala, A) | GCA |

Forward and reverse primers were designed and synthesized for the amino acids and their codons shown in the above table, and PCR amplification was carried out with the CasY7+sgRNA1 expression plasmid as a template. After amplification, the Universal DNA Purification and Recovery Kit (TIANGEN Biotech (Beijing) Co., Ltd., DP214) was used to recover and purify the amplified products. The purified products were transformed into Escherichia coli Dh5α competent cells (Vazyme, DL1001) and cultured at 37 °C overnight. The next day, single colonies were picked and subjected to sequencing. After confirmation by sequencing, the positive clones were cultured, the plasmids were extracted (TIANGEN, DP120-01), and the concentration was measured, followed by storage in a -20 °C refrigerator for later use.

The obtained recombinant plasmids with point mutations were named as: D592A-dCasY7+sgRNA expression plasmid, D643A-dCasY7+sgRNA expression plasmid, E820A-dCasY7+sgRNA expression plasmid, and D992A-dCasY7+sgRNA expression plasmid.

Subsequently, the constructed D592A-dCasY7+sgRNA expression plasmid, D643A-dCasY7+sgRNA expression plasmid, E820A-dCasY7+sgRNA expression plasmid, and D992A-dCasY7+sgRNA expression plasmid were each measured for in vivo editing efficiency in Escherichia coli. The measurement method and calculation method were the same as those in Steps 2 and 3 of Example 2. The experimental results are shown in Figure 7. By the counting of the number of Escherichia coli clones and the calculation of the ratio, it was considered that D592A-dCasY7, D643A-dCasY7, E820A-dCasY7, and D992A-dCasY7 lost catalytic activity (cleavage activity). That is to say, the point mutations of D592A, D643A, E820A, and D992A caused the CasY7 protein to lose its cleavage activity.

### 2. Measurement of Base-Editing Efficiency at the Cellular Level

(1) Construction of CasY7-TTR-sgRNA3 and CasY7-TTR-sgRNA4 Plasmids
sgRNAs CasY7-TTR-sgRNA3 and CasY7-TTR-sgRNA4 sequences were designed based on the target sequence of the TTR gene, and oligonucleotides (oligos) were synthesized:
CasY7-TTR-sgRNA3: CAAGTTGAATCCGTCTATAACTGACGGtatatcccttctacaaattc (SEQ ID NO:23);
CasY7-TTR-sgRNA4: CAAGTTGAATCCGTCTATAACTGACGGgtgtctatttccactttgta (SEQ ID NO:24),
wherein the underlined part is the DR sequence, and the remaining sequence is the spacer sequence.

(2) A sequence CACC was added to the 5'-end of the upstream sequence of each sgRNA and a sequence AAAA was added to the 5'-end of the downstream sequence. The specific forms are as follows:

| sgRNA Name | Upstream and Downstream Sequences |
|---|---|
| CasY7-TTR-sgRNA3 | CasY7-TTR-sgRNA3-Oligo-F: |
| | CACCCAAGTTGAATCCGTCTATAACTGACGGtatatcccttctacaaattc (SEQ ID NO:25) |
| | CasY7-TTR-sgRNA3-Oligo-R: |
| | **AAAA**GAATTTGTAGAAGGGATATACCGTCAGTTATAGACGGATTCAACTTG (SEQ ID NO:26) |
| CasY7-TTR-sgRNA4 | CasY7-TTR-sgRNA4-Oligo-F: |
| | **CACC**CAAGTTGAATCCGTCTATAACTGACGGgtgtctatttccactttgta (SEQ ID NO:27) |
| | CasY7-TTR-sgRNA4-Oligo-R: |
| | **AAAA**TACAAAGTGGAAATAGACACCCGTCAGTTATAGACGGATTCAACTTG (SEQ ID NO:28) |

According to the method in Step 1 of Example 3, the upstream and downstream sequences of CasY7-TTR-sgRNA3 and CasY7-TTR-sgRNA4 were annealed and then ligated into PHK09T vectors to obtain the CasY7-TTR-sgRNA3 expression plasmid (named CasY7-TTR-sgRNA' expression plasmid) and the CasY7-TTR-sgRNA4 expression plasmid (named CasY7-TTR-sgRNA" expression plasmid). These plasmids were then transferred into *Escherichia coli* DH5α competent cells which were cultured for plasmid amplification. After sequencing to confirm correctness and measuring the concentration, they were stored for future use.

### (3) Construction of Base Editor Plasmid (taking deaminase 005V1-10-3 as an example)

The selected adenosine deaminase catalytic domain was a mutant of the amino-acid sequence of SEQ ID NO: 31 (named 005V1-10-3): Q148G+Q149M+P150R. The amino-acid sequence of this mutant is SEQ ID NO: 32, and the nucleotide sequence encoding the deaminase 005V1-10-3 is SEQ ID NO: 33. A base-editor fusion protein composed of the deaminase 005V10-3 and the CasY7 protein was constructed by homologous recombination. The specific operations were as follows.

First, the 005V1-10-3 nucleotide fragment with homologous-arm sequences and a linker was synthesized by Suzhou Synbio Technologies Co., Ltd.:
*ccaaagaagaagcggaaagtc***ATGAGTGAGCTGAATGATGCTTACTGGATGAAACAGGCACTCGCTTTAGCTC AGAAGGCCcggGAACAGGGAGAAGTTCCAGTGGGCGCTATTCTGGTGctgGATGATGAAGTGATAGGAC AGGGATGGAATAGAgccATCACCctgCACGACCCCACCGCCCACGCCGAGATCATGGCCCTGCAGCAGGG CGGCCAGATCGTGCAGAACTACCGGCTGCTGAACGCCACCCTGTACGTGACCttcGAGCCCTGCGTGATGT GCGCCGGCGCCATGGTGCACAGCAGGATCAAGAGACTGGTCTACGGCgtgAGCaactcaAAAagaGGCGCC GCCGGCAGCCTGCTGaacGTGCTGaactacCCCGGCATGAACCACCAGATCGAGATCACCGCCGGCGTGAT GGCCAACGAGTGCAGCGAGATGCTGtgcCAGTTCtatGGTATGCGCAGAGAAgtgttcaatGCTGAGCGTGA GGCTAGGCGGCTGAACCAACCTGATAGAGCTGAC**tccggaggatctagcggaggctcctctggctctgagacacctggca caagcgagaggcgcaacacctgaaagcaggcgcagcagcggggggtca*GCCACGAAGACCATCGTGAG* (SEQ ID NO:34), wherein the bold part is the 005V1-10-3 nucleotide sequence, the italic part is the left and right homologous-arm regions, and the wavy-line part is the linker sequence.

The D992A-dCasY7+sgRNA expression plasmid obtained in Step 1 was amplified by PCR for linearization to obtain a linearized expression vector. The primers used are shown in the following table:

| Primer Name | Detailed Sequence |
|---|---|
| D992A-dCasY7-F | GCCACGAAGACCATCGTGAG (SEQ ID NO:35) |
| D992A-dCasY7-R | Gactttccgcttcttctttgg (SEQ ID NO:36) |

The nucleotide fragment 005V1-10-3 with homologous-arm sequences and a linker (SEQ ID NO:34) and the linearized D992A-dCasY7+sgRNA expression vector were subjected to homologous recombination. The reaction was carried out with Gibson Assembly Master Mix (NEB, E2611S). After the reaction, the ligation product was transformed into *Escherichia coli* DH5α competent cells (Vazyme, DL1001). The specific process was as follows.

DH5α competent cells were taken out from a -80°C refrigerator and quickly buried in ice. After 5 minutes, the cell mass thawed, and the ligation product was added. They were gently mixed by tapping the bottom of the centrifuge tube by hand, let stand in ice for 25 minutes, heat-shocked in a 42°C water-bath for 45 seconds, quickly put back on ice, and let stand for 2 minutes. 700 µL of sterile LB medium was added to the centrifuge tube, mixed well, and recovered at 37 °C and 200 rpm for 60 minutes. The mixture was centrifuged at 5,000 rpm for one minute to collect the bacteria. With about 100 µl of the supernatant left in the tube, the cell mass was gently resuspended by pipetting, and spread on an LB medium plate with Amp antibiotic. The plate was placed upside-down in a 37°C incubator and cultured overnight. Single colonies were picked. After confirmation by sequencing, the positive clones were cultured, and the base-editor plasmids were extracted with the Endo-Free Plasmid Mega Kit (TIANGEN: DP120-01), measured for the concentration, and stored in a -20°C refrigerator for later use. The nucleotide sequence encoding the base-editor fusion protein 005V1-10-3-D992A-dCasY7 is SEQ ID NO:37, and the amino acid sequence is SEQ ID NO:46.

(4) According to the method in Step 2 of Example 3, the base-editor plasmid and the EGFP-C1 (Addgene, Plasmid #54759) plasmid were used together with the CasY7-TTR-sgRNA' or CasY7-TTR-sgRNA" expression plasmid to co-transfect 293T cells.

Forty-eight hours after transfection, the expression of EGFP fluorescent protein indicated successful cell transfection. EGFP-positive cells were sorted for the measurement of editing efficiency. The genome of the 293T cells was extracted with a kit (TIANGEN, DP304-03).

(5) The base-editing efficiency was measured according to the method in Step (3) of Example 3. Primers were designed according to experimental requirements. The sequences of the identification primers used are shown in the following table:

| Primer Name | Specific Sequence |
|---|---|
| TTR-F' | gggtgtattactttgccatg (SEQ ID NO:29) |
| TTR-R' | aacctttggtcattcatcaccttc (SEQ ID NO:30) |

The results are shown in Figures 8A and 8B of Figure 8. The base editor made from D992A-dCasY7 can achieve effective editing at multiple sites. As can be seen from Figure 8A, mediated by CasY7-TTR-sgRNA', there was effective editing at the +4, +13, +15, +16, and +17 sites. The editing efficiency at the +13, +15, +16, and +17 sites was about 5% to about 30%.

As can be seen from Figure 8B, mediated by CasY7-TTR-sgRNA", there was effective editing at the +7, +13, and +20 sites. The editing efficiency at site +7 was about 10%, the base-editing efficiency at site +13 was about 20%, and the editing efficiency at site +20 was 20% or more.

### Example 5. Screening for CasY7 Mutants

To screen for CasY7 mutants with high cleavage activity, the region from positions 7-684 of the CasY7 protein (SEQ ID NO:1) was mutated by engineering, and 31 mutants were constructed.

The method for obtaining the mutants was as follows. The CasY7 recombinant expression plasmid in Example 2 (see Figure 1A in Figure 1 for the plasmid map) was used as a template to design PCR primers with the mutation sites as the center, and the mutated nucleotide sequences were introduced into the PCR primers. Subsequently, each CasY7 mutant plasmid was obtained through site-directed mutagenesis by PCR.

The mutation methods for each mutant are shown in the following table:

| Mutant | Amino Acid Mutation Pattern (Corresponding to the Amino Acid Sequence of CasY7, SEQ ID NO:1) | Codon after Mutation (Corresponding to the Nucleotide Coding Sequence of CasY7, SEQ ID NO:2) |
|---|---|---|
| C02964 | M231K+D234V | AAG+GTA |
| C06342 | L227I+L229R | ATT+CGG |
| C06354 | S225P+L227A+L229R | CCC+GCC+CGG |
| C08361 | S240A+S241R+Q242P+E243L | GCA+AGG+CCA+CTT |
| C07864 | S250W+F251A+E252A+K253R+V254R | TGG+GCG+GCC+CGG+CGT |
| C05442 | K260R+T261P+E263H | CGC+CCG+CAC |
| C02644 | S303T+I304M+L307R+Y308A+S309I | ACT+ATG+CGG+GCG+ATA |
| C09389 | L307R+Y308F+S309A | CGG+TTT+GCA |
| C09484 | R315L+E316R+T317R+I318V+I319A | CTG+AGA+CGG+GTG+GCC |
| C06543 | G416R+I417W+E418T+F419R+D420V | CGG+TGG+ACA+AGG+GTA |
| C06887 | L354F+S355G+N356D+L357I+K358R | TTC+GGT+GAT+ATT+AGG |
| C04346 | E648S+A651L | TCT+CTT |
| C04870 | E648S+A651T+Y652W | TCT+ACT+TGG |
| C04122 | T681V+N682G+E683R+S684G | GTG+GGC+AGG+GGC |
| C03476 | T681P+N682T+E683G+S684R | CCG+ACC+GGC+CGG |
| C01959 | T681S+N682P+E683R+S684G | TCT+CCC+CGG+GGC |
| C02662 | T681A+N682H+E683Y+S684R | GCC+CAT+TAC+CGA |
| C06831 | Y165W+S166R | TGG+CGT |
| C02408 | Y165W+S166V+S167I+F168I | TGG+GTA+ATC+ATC |
| C03236 | Q160V+E161I+Y162T+N163S+C164V | GTC+ATC+ACT+TCC+GTC |
| C02085 | Q160S+E161S+Y162L+N163C+C164V | TCC+TCC+TTA+TGT+GTC |
| C07589 | Q160P+E161V+Y162H+N163S+C164T | CCG+GTT+CAC+AGC+ACC |
| C02074 | Q160L+E161S+Y162F+N163S+C164A | CTG+TCT+TTT+AGT+GCG |
| C08003 | V7H+T11R+S12M | CAT+AGA+ATG |
| C07848 | V7F+R8L+T11R+S12V | TTT+CTT+AGG+GTC |
| C04352 | I149M+N150S+H151C+N152T+L153Y | ATG+TCT+TGT+ACT+TAC |
| C03506 | K195R+K197A+S198A+A199G | CGG+GCG+GCA+GGA |
| C08768 | T216L+A217T+K219R | TTG+ACT+CGT |
| C09158 | C215V+T216S+A217S+K219R | GTG+TCC+TCA+AGA |
| C09822 | Y282F+D283Q+A285T | TTT+CAA+ACT |
| C05173 | D283I+A285R+N292L | ATT+AGG+CTC |

The gRNA targeting sequences were designed based on the target sequence of the TTR gene, PD-1 gene, and Trac gene. The expression plasmids of each targeted sgRNA were constructed using the same method as in Example 3. The CasY7 recombinant expression plasmid or its mutant recombinant expression plasmid was each co-transfected into HEK293 cells with the TTR-sgRNA2 expression plasmid by the PEI transfection method. A negative control group (using another targeted sgRNA, the NT-sgRNA targeting sequence is represented by SEQ ID NO:52, labeled as "NT") and a blank control group were also co-transfected in the same way, and identification primers were designed according to the targeting of each gRNA.

| Target | Spacer Sequence | sgRNA Sequence | Identification Primers |
|---|---|---|---|
| TTR-1 | SEQ ID NO:6 | SEQ ID NO:7 | TTR-F1 (SEQ ID NO:63); |
| | | | TTR-R1 (SEQ ID NO:64) |
| PD-1 | SEQ ID NO:53 | SEQ ID NO:54 | PD1-F (SEQ ID NO:65); |
| | | | PD1-R (SEQ ID NO:66) |
| Trac-1 | SEQ ID NO:55 | SEQ ID NO:56 | trac-F1 (SEQ ID NO:67); |
| | | | trac-R1 (SEQ ID NO:68) |
| Trac-2 | SEQ ID NO:57 | SEQ ID NO:58 | trac-F2 (SEQ ID NO:69); |
| | | | trac-R2 (SEQ ID NO:51) |
| Trac-3 | SEQ ID NO:59 | SEQ ID NO:60 | trac-F2 (SEQ ID NO:69); |
| | | | trac-R2 (SEQ ID NO:51) |
| Trac-4 | SEQ ID NO:61 | SEQ ID NO:62 | trac-F1 (SEQ ID NO:67); |
| | | | trac-R1 (SEQ ID NO:68) |

After culturing the cells for 48 hours, genomic DNA was extracted, and PCR amplification was carried out with the identification primers. The amplified PCR products were used for high-throughput deep sequencing (Tsingen Biotechnology Co., Ltd.) to identify the editing efficiency.

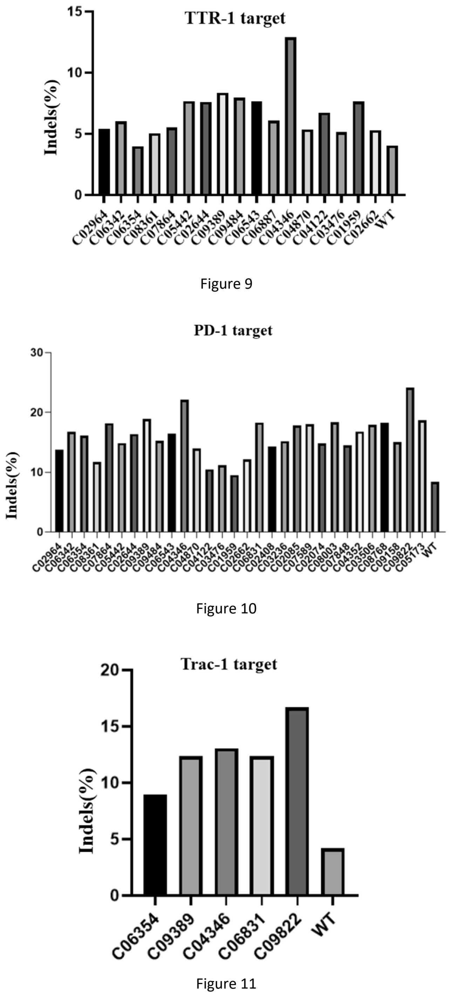

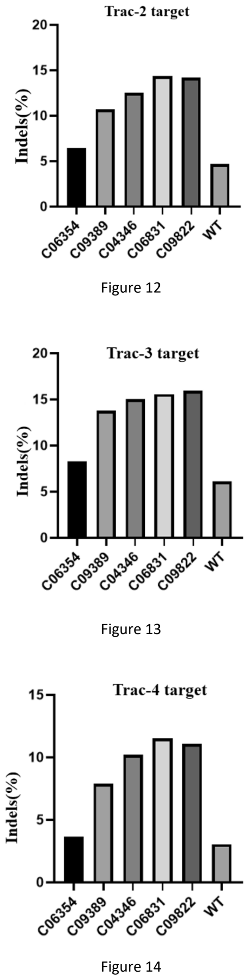

The cleavage efficiencies of wild-type CasY7 (WT) and each mutant on the TTR-1 target, PD-1 target, Trac-1 target, Trac-2 target, Trac-3 target, and Trac-4 target are shown in Figures 9 to 14, respectively. Through analysis, it can be seen that as compared with wild-type CasY7, the editing efficiency of each mutant of CasY7 on the respective target gene was close to or much higher than that of CasY7. For example, at the TTR gene locus, all mutants except for the C06354 showed higher cleavage activity than the wild-type (WT), especially C04346 which showed cleavage activity about 3 times or more than that of the parental protein. Similarly, at the PD-1 gene and Trac gene loci, as compared with the wild-type (WT), multiple mutants showed higher cleavage activity. The negative control (NT) group and the blank control group set for each target had no or only background-level cleavage activity.

### Example 6. Influence of Direct Repeat (DR) Sequences on the Cleavage Activity of CasY7

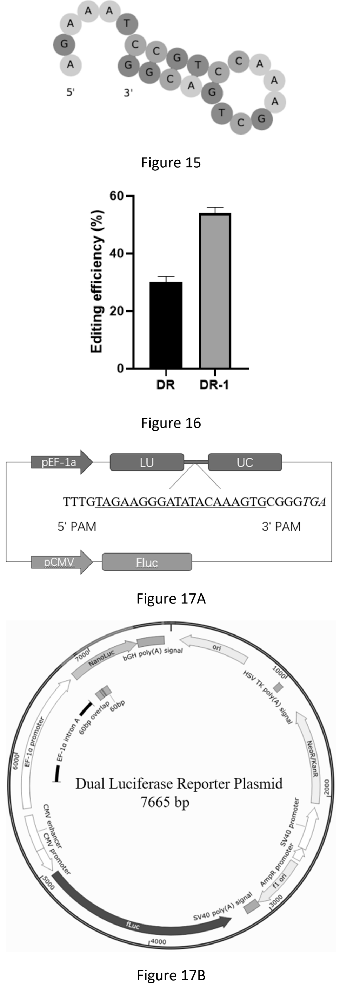

To test the effects of different DR sequences on the activity of CasY7, deletions and mismatches were designed at different positions in the DR sequence, and a new DR variant, DR-1 (SEQ ID NO:71), was designed. The secondary structure of DR-1 (Figure 15, any "T" in the sequence represents "U" when referring to the DR sequence) is basically the same as that of the wild-type DR sequence (SEQ ID NO:5) (Figure 2). The stem sequences of DR-1 and the wild-type DR are the same, both composed of 5'-CCGTC-3' and its complementary strand. There are certain differences in sequence in other regions.

With the hTTR gene as a target, a spacer sequence targeting a target sequence of the TTR gene (Target-TTR-spacer2, SEQ ID NO:11) and DR-1-TTR-sgRNA (SEQ ID NO:72) having the DR-1 sequence were designed. In the manner the same as in Example 3, the synthesized DR-1-TTR-sgRNA (SEQ ID NO:72) sequence fragment was constructed into a PHK09T vector to obtain the DR-1-CasY7-TTR-sgRNA expression plasmid.

Subsequently, in the manner of Steps 2 (2) and (3) of Example 3, the DR-1-CasY7-TTR-sgRNA expression plasmid and the CasY7 expression plasmid were used to transfect cells, and the upstream and downstream primers TTR-F (SEQ ID NO:21) and TTR-R (SEQ ID NO:22) were used to measure the editing efficiency. Through analysis, it was found that the DR-1 sequence can mediate higher editing activity of CasY7 (Figure 16). This also indicates that CasY7 has adaptability to DR sequences with different structures.

### Example 7. Screening for highly active mutants of CasY7

1. To screen for CasY7 mutants with high cleavage activity, directed mutagenesis and screening were carried out on the CasY7 protein (SEQ ID NO:1), and 17 mutants were constructed.

The method for obtaining the mutants was as follows. The CasY7 recombinant expression plasmid in Example 2 (see Figure 1A in Figure 1 for the plasmid map) was used as a template to design PCR primers with the mutation sites as the center, and the mutated nucleotide sequences were introduced into the PCR primers. Subsequently, each CasY7 mutant plasmid was obtained through site-directed mutagenesis by PCR.

| Mutant | Mutation Pattern | Codons after Mutation (Relative to the Coding Sequence of Wild-type CasY7) |
|---|---|---|
| C05440 | Y282F+D283Q+A285T+G416S+I417H+E418Q +F419T+D420V | TTT+CAA+ACT+TCC+CAT+CAG+ACT +GTC |
| C03897 | Y165W+S166R+G416R+I417V+E418Q+F419V +D420T | TGG+CGT+CGC+GTC+CAG+GTC+AC C |
| C06865 | Y282F+D283Q+A285T+V348I+I349V+E350L+ P351T | TTT+CAA+ACT+ATT+GTC+TTG+ACG |
| C04558 | D283S+A285P+L307R+Y308F+S309A | TCG+CCA+CGG+TTT+GCA |
| C08675 | L307R+Y308F+S309A+E648R+L650I+A651P+Y 652F | CGG+TTT+GCA+CGC+ATC+CCC+TTT |
| C01144 | Y282F+D283Q+A285T+E648T+A651S+Y652F | TTT+CAA+ACT+ACT+TCA+TTC |
| C06623 | Y282F+D283Q+A285T+E648L+A651G | TTT+CAA+ACT+CTT+GGT |
| C03023 | Y282F+D283Q+A285T+T681P+N682P+E683Q +S684C | TTT+CAA+ACT+CCC+CCG+CAG+TGC |
| C07879 | Y282F+D283Q+A285T+E748A+G749S+S751G | TTT+CAA+ACT+GCA+TCT+GGT |
| C03803 | N276S+D283N+E648S+A651L | AGC+AAT+TCT+CTT |
| C06467 | Y282F+D283Q+A285T+R315A+E316Q+T317R +I318L+I319Q | TTT+CAA+ACT+GCT+CAG+CGG+CTC +CAA |
| C08724 | Y282F+D283Q+A285T+R315S+E316R+T317K +I318K+I319W+E648S+A651L | TTT+CAA+ACT+AGC+CGT+AAG+AAA +TGG+TCT+CTT |
| C03563 | V7I+T11V+S12M+Y282F+D283Q+A285T | ATT+GTT+ATG+TTT+CAA+ACT |
| C05664 | S240L+S241A+Q242S+E243H+I244L+L307R+ Y308F+S309A | TTA+GCT+TCG+CAC+CTC+CGG+TTT +GCA |
| C02742 | S196N+K197F+S198N+A199T+I304A+L307R+ Y308A+S309A | AAC+TTC+AAT+ACA+GCC+CGG+GCT +GCG |
| C02009 | Y282F+D283Q+A285T+K865S+P866S+Y867H +N868F+I872L | TTT+CAA+ACT+AGC+AGT+CAT+TTT +CTA |
| C09752 | Y282F+D283Q+A285T+M957V+F958L+Q960 V+W961V | TTT+CAA+ACT+GTA+TTA+GTC+GTC |

To efficiently and sensitively measure the cleavage activity of each mutant, a dual-luciferase reporter system vector (Figure 17B) was constructed. It contains a Fluc (Firefly luciferase) coding sequence and a LUxUC coding sequence (Figure 17A). The LU and UC sequences in LUxUC are the 119-bp sequence at the 5' end and the 469-bp sequence at the 3' end of the NanoLuc (NanoLuc luciferase) coding sequence, respectively. The two sequences have an overlapping sequence of 60 bp. An insert sequence is designed in the middle of the LUxUC sequence, and contains the target sequence for measuring cleavage activity. Target sequences were designed according to the TTR and PD-1' targets, and insert sequences containing the target sequences were designed respectively. In this example, the insert sequences, target sequences, and the corresponding LUxUC sequences used are shown in the following table:

| Target | Insertion Sequence | Target Sequence | Corresponding LUxUC Sequence |
|---|---|---|---|
| TTR | SEQ ID NO:76 | SEQ ID NO:11 | SEQ ID NO:73 |
| PD-1' | SEQ ID NO:77 | SEQ ID NO:75 | SEQ ID NO:74 |

In this example, a dual-luciferase reporter system vector for detecting the targeted cleavage of the TTR target by each variant was constructed. Its nucleotide sequence is represented by SEQ ID NO.97, wherein positions 6759-7380 are the TTR-LUxUC sequence which is represented by SEQ ID NO.73 and contains a TTR-insertion sequence (SEQ ID NO.76). A target sequence (SEQ ID NO.11) is designed in the middle of the TTR-insertion sequence. Similarly, a dual-luciferase reporter system vector for detecting the targeted cleavage of the PD-1' target by each variant was also constructed (by replacing positions 6759-7380 in the reporter system plasmid represented by SEQ ID NO.97 with the PD-1'-LUxUC sequence), wherein the PD-1'-LUxUC sequence, PD-1' target sequence, and PD-1' insertion sequence are as shown in the above table.

There is a TAG premature stop codon in the middle of the target sequence. When it is cleaved, the LUxUC generates the correct NanoLuc coding frame through the recombination mechanism, and expresses NanoLuc luciferase which in turn catalyzes the substrate to emit fluorescence. The intensity of the fluorescence indicates the cleavage activity. In this case, Fluc luciferase serves as an internal reference, and the fluorescence it emits is used to indicate whether the host cell is successfully transfected with the reporter vector.

By the same method as in Example 1, a recombinant expression plasmid for each mutant was constructed. The sgRNA expression plasmids targeting TTR and PD-1' were constructed in the same manner as in Example 3, and by the transfection method in Example 3, the recombinant expression plasmids of CasY7 and its mutants were each co-transfected into a HEK293 cell line together with the corresponding dual-luciferase reporter system vector and sgRNA expression plasmid. After 24 hours of culturing, the expression intensities of Fluc and NanoLuc were measured on a microplate reader using a dual-luciferase reporter gene assay kit (Beyotime, RG028).

In addition, a negative control group was set for CasY7 and its mutants. The negative control group used an sgRNA expression plasmid with a non-target sequence (SEQ ID NO.78). It was found through analysis that the CasY7 and its mutants exhibited cleavage activities as shown in the following table, while no cleavage activity was detected in the negative control group, and the cleavage activity of each mutant was much higher than that of CasY7.

| Mutant | Targeted TTR Cleavage Activity (%) | Targeted PD-1' Cleavage Activity (%) |
|---|---|---|
| C05440 | 4.05 | 8.39 |
| C03897 | 33.92 | 31.64 |
| C06865 | 32.49 | 32.51 |
| C04558 | 30.10 | 27.15 |
| C08675 | 23.80 | 35.92 |
| C01144 | 29.49 | 31.38 |
| C06623 | 29.33 | 34.29 |
| C03023 | 29.75 | 39.67 |
| C07879 | 30.38 | 35.04 |
| C03803 | 25.31 | 38.54 |
| C06467 | 27.84 | 18.66 |
| C08724 | 26.15 | 36.45 |
| C03563 | 26.11 | 35.15 |
| C05664 | 28.69 | 40.12 |
| C02742 | 21.18 | 35.02 |
| C02009 | 27.09 | 32.39 |
| C09752 | 20.31 | 35.04 |
| CasY7 | 2.05 | 4.45 |

2. By the same method as in Step 1 of this example, expression plasmids for expressing 39 mutants (as shown in the following table) were constructed.

| Variant Name | Mutation Pattern | Codons after Mutation |
|---|---|---|
| C30725 | Y282F+D283Q+A285T+G416L+I417Q+E418M+F 419R+D420A | TTT+CAA+ACT+CTT+CAG+ATG+CGG+ GCA |
| C30381 | Y282F+D283Q+A285T+G416A+I417R+E418V+F 419C+D420Q | TTT+CAA+ACT+GCG+CGT+GTC+TGT+ CAG |
| C30924 | Y165W+S166R+G416R+I417E+E418Q+F419V+D 420A | TGG+CGT+AGG+GAA+CAG+GTG+GC G |
| C30301 | Y282F+D283Q+A285T+G416S+I417H+E418Q+F 419T+D420V+L501P+N936S | TTT+CAA+ACT+TCC+CAT+CAG+ACT+ GTC+CCG+AGC |
| C30486 | Y282F+D283Q+A285T+G416S+I417H+E418Q+F 419T+D420V+L501P+T681N+N682P+E683G+S6 84A | TTT+CAA+ACT+TCC+CAT+CAG+ACT+ GTC+CCG+AAC+CCG+GGA+GCT |
| C30591 | Y165W+S166R+G416R+I417V+E418Q+F419V+D 420T+T681D+N682A+E683R+S684D | TGG+CGT+CGC+GTC+CAG+GTC+ACC +GAT+GCG+CGA+GAT |
| C30725 | Y165W+S166R+S198N+G416R+I417V+E418Q+F 419V+D420T+T681S+N682R+E683R | TGG+CGT+AAC+CGC+GTC+CAG+GTC +ACC+TCG+CGG+CGA |
| C30316 | D283S+A285P+L307R+Y308F+S309A+I373V+E7 48G+S751G+K752R+S787F | TCG+CCA+CGG+TTT+GCA+GTT+GGG +GGG+CGT+TTT |
| C30834 | Y165W+S166R+G416R+I417V+E418Q+F419V+D 420T+N682G+E683A+D896N | TGG+CGT+CGC+GTC+CAG+GTC+ACC +GGG+GCC+AAC |
| C40492 | Y282F+D283Q+A285T+G416A+I417R+E418V+F 419C+D420Q | TTT+CAA+ACT+GCG+CGT+GTC+TGT+ CAG |
| C40780 | R509C+P510S+L512F+G416R+I417V+E418Q+F4 19V+D420T+H486A+S488W+N682G+E683A+S6 84R+D896N | TGT+AGT+TTC+CGC+GTC+CAG+GTC+ ACC+GCG+TGG+GGG+GCC+AGG+AA C |
| C40571 | Y165W+S166R+G416R+I417V+E418Q+F419V+D 420T+R509N+P510G+V511G+L512S+T681S+N6 82R+E683R | TGG+CGT+CGC+GTC+CAG+GTC+ACC +AAT+GGG+GGC+TCG+TCG+CGG+CG A |
| C40251 | Y165W+S166R+G416R+I417V+E418Q+F419V+D 420T+G513L+N514A+R515V+V516M+N682G+E 683A+S684R+D896N | TGG+CGT+CGC+GTC+CAG+GTC+ACC +CTT+GCC+GTA+ATG+GGG+GCC+AG G+AAC |
| C40460 | Y165W+S166R+G416R+I417V+E418Q+F419V+D 420T+G513C+R515Y+V516M+N682G+E683A+S 684R+D896N | TGG+CGT+CGC+GTC+CAG+GTC+ACC +TGT+TAT+ATG+GGG+GCC+AGG+AA C |
| C40107 | G416R+I417V+E418Q+F419V+D420T+T681S+N 682R+E683R+G727V+K728C+N729G+E730G | CGC+GTC+CAG+GTC+ACC+TCG+CGG +CGA+GTG+TGC+GGG+GGA |
| C40652 | Y282F+D283Q+A285T+G416L+I417Q+E418M+F 419R+D420A+I455G+R456E+V458E | TTT+CAA+ACT+CTT+CAG+ATG+CGG+ GCA+GGG+GAG+GAG |
| C50371 | Y165W+S166R+G416A+I417R+E418V+F419C+D 420Q+H486S+N487N+S488W+N682G+E683A+S 684R+D896N | TGG+CGT+GCG+CGT+GTC+TGT+CAG +AGC+AAC+TGG+GGG+GCC+AGG+A AC |
| C50725 | Y282F+D283Q+A285T+G416A+I417R+E418V+F 419C+D420Q+R509C+P510S+L512F+L525R+I52 6V+N527D+K528P+K529G+N682G+E683A+S68 4R+D896N | TTT+CAA+ACT+GCG+CGT+GTC+TGT+ CAG+TGT+AGT+TTC+CGT+GTT+GAT+ CCG+GGA+GGG+GCC+AGG+AAC |
| C50878 | Y282F+D283Q+A285T+G416A+I417R+E418V+F 419C+D420Q+R509V+P510R+V511L+L512A+T6 81S+N682R+E683R | TTT+CAA+ACT+GCG+CGT+GTC+TGT+ CAG+GTC+AGA+TTG+GCG+TCG+CGG +CGA |
| C50379 | Y165W+S166R+G416R+I417V+E418Q+F419V+D 420T+R509Q+P510A+V511A+L512R+T681S+N6 82R+E683R+A661V | TGG+CGT+CGC+GTC+CAG+GTC+ACC +CAG+GCT+GCG+AGG+TCG+CGG+CG A+GTG |
| C50576 | Y282F+D283Q+A285T+G416A+I417R+E418V+F 419C+D420Q+P510D+V511H+L512V | TTT+CAA+ACT+GCG+CGT+GTC+TGT+ CAG+GAC+CAT+GTT |
| C50501 | S110G+Y165W+S166R+G416R+I417V+E418Q+F 419V+D420T+R509A+P510A+V511R+L512Q+T6 81S+N682R+E683R | GGT+TGG+CGT+CGC+GTC+CAG+GTC +ACC+GCG+GCT+AGA+CAA+TCG+CG G+CGA |
| C50122 | Y165W+S166R+G416R+I417V+E418Q+F419V+D 420T+R509L+P510V+V511S+L512F+T681S+N68 2R+E683R | TGG+CGT+CGC+GTC+CAG+GTC+ACC +TTG+GTA+TCT+TTC+TCG+CGG+CGA |
| C50729 | Y282F+D283Q+A285T+G416A+I417R+E418V+F 419C+D420Q+R509G+P510K+L512C+T681S+N6 82R+E683R | TTT+CAA+ACT+GCG+CGT+GTC+TGT+ CAG+GGG+AAA+TGT+TCG+CGG+CG A |
| C50734 | Y282F+D283Q+A285T+G416A+I417R+E418V+F 419C+D420Q+R509C+P510S+L512F+N682G+E6 83A+S684R+G727E+K728H+N729Q+E730R | TTT+CAA+ACT+GCG+CGT+GTC+TGT+ CAG+TGT+AGT+TTC+GGG+GCC+AGG +GAG+CAT+CAG+AGA |
| C50132 | Y165W+S166R+G416R+I417V+E418Q+F419V+D 420T+G513C+R515Y+V516M+T635S+K636G+N 637L+N682G+E683A+S684R+D896N | TGG+CGT+CGC+GTC+CAG+GTC+ACC +TGT+TAT+ATG+TCT+GGT+TTA+GGG +GCC+AGG+AAC |
| C50337 | Y165W+S166R+G416R+I417V+E418Q+F419V+D 420T+H486A+S488W+T634V+T635Y+K636S+N6 37R+T681S+N682R+E683R | TGG+CGT+CGC+GTC+CAG+GTC+ACC +GCG+TGG+GTG+TAT+TCC+CGG+TC G+CGG+CGA |
| C50426 | Y282F+D283Q+A285T+G416A+I417R+E418V+F 419C+D420Q+R509C+P510S+L512F+N682G+E6 83A+S684R+S743T | TTT+CAA+ACT+GCG+CGT+GTC+TGT+ CAG+TGT+AGT+TTC+GGG+GCC+AGG +ACG |
| C62732 | Y282F+D283Q+A285T+G416A+I417R+E418V+F 419C+D420Q+P510D+V511H+L512V+K781H+L7 82I+G783F+E784R+C785S | TTT+CAA+ACT+GCG+CGT+GTC+TGT+ CAG+GAC+CAT+GTT+CAC+ATC+TTT+ AGA+AGT |
| C62072 | Y165W+S166R+G416A+I417R+E418V+F419C+D 420Q+H486S+S488W+K781T+G783S+E784F+C7 85L+D896N | TGG+CGT+GCG+CGT+GTC+TGT+CAG +AGC+TGG+ACC+AGT+TTT+CTT+AAC |
| C62302 | Y282F+D283Q+A285T+G416A+I417R+E418V+F 419C+D420Q+P510D+V511H+L512V+D638P+E 641M+F642V | TTT+CAA+ACT+GCG+CGT+GTC+TGT+ CAG+GAC+CAT+GTT+CCT+ATG+GTG |
| C68550 | Y165W+S166R+G416A+I417R+E418V+F419C+D 420Q+H486S+S488W+D638F+R639P+G640N+E 641Y+F642L | TGG+CGT+GCG+CGT+GTC+TGT+CAG +AGC+TGG+TTT+CCT+AAT+TAT+CTC |
| C65495 | Y165W+S166R+G416R+I417V+E418Q+F419V+D 420T+H486A+S488W+N682G+E683A+S684R+P 1018L+S1019R+R1020P+N1021R+S1022V | TGG+CGT+CGC+GTC+CAG+GTC+ACC +GCG+TGG+GGG+GCC+AGG+CTG+C GG+CCT+AGA+GTG |
| C69601 | Y282F+D283Q+A285T+G416A+I417R+E418V+F 419C+D420Q+P510D+V511H+L512V+P1018S+S 1019R+R1020V+N1021M+S1022P | TTT+CAA+ACT+GCG+CGT+GTC+TGT+ CAG+GAC+CAT+GTT+TCG+AGA+GTA +ATG+CCC |
| C62198 | Y282F+D283Q+A285T+G416A+I417R+E418V+F 419C+D420Q+R509C+P510S+L512F+C633N+T6 34E+T635E+K636G+N637L+N682G+E683A+S68 4R+S743T | TTT+CAA+ACT+GCG+CGT+GTC+TGT+ CAG+TGT+AGT+TTC+AAT+GAA+GAG +GGT+CTT+GGG+GCC+AGG+ACG |
| C65825 | Y282F+D283Q+A285T+G416A+I417R+E418V+F 419C+D420Q+R509C+P510S+L512F+C633F+T6 34P+T635F+K636P+N637C+N682G+E683A+S68 4R+S743T | TTT+CAA+ACT+GCG+CGT+GTC+TGT+ CAG+TGT+AGT+TTC+TTT+CCT+TTT+C CG+TGC+GGG+GCC+AGG+ACG |
| C61684 | Y282F+D283Q+A285T+G416A+I417R+E418V+F 419C+D420Q+R509C+P510S+L512F+C633S+T6 34C+T635G+K636H+N637F+N682G+E683A+S68 4R+S743T | TTT+CAA+ACT+GCG+CGT+GTC+TGT+ CAG+TGT+AGT+TTC+AGC+TGC+GGT +CAT+TTC+GGG+GCC+AGG+ACG |
| C61352 | Y282F+D283Q+A285T+G416A+I417R+E418V+F 419C+D420Q+R509C+P510S+L512F+C633R+T6 34Y+T635L+K636V+N637D+N682G+E683A+S68 4R+S743T | TTT+CAA+ACT+GCG+CGT+GTC+TGT+ CAG+TGT+AGT+TTC+CGT+TAT+CTC+ GTG+GAT+GGG+GCC+AGG+ACG |

According to the method in Example 3, expression plasmids for expressing sgRNAs targeting the TTR gene and Hao1 gene were synthesized respectively. The sgRNA sequences and targeting sequences are shown in the following table:

| Target Gene | sgRNA Sequence | Target Sequence |
|---|---|---|
| TTR | AGAAATCCGTCCAAAGCTGACGGtagaagggat atacaaagtg (SEQ ID NO:72) | tagaagggatatacaaagtg (SEQ ID NO:11) |
| Hao1 | AGAAATCCGTCCAAAGCTGACGGggacagaggg tcagcatgcc (SEQ ID NO:90) | ggacagagggtcagcatgcc (SEQ ID NO:101) |

The targets TTR and hHao1 were selected to measure the cleavage activity of each of the above-mentioned variants. A dual-luciferase reporter system vector (the reporter system vector for measuring the cleavage activity of the target TTR is represented by SEQ ID NO.97, and the reporter system vector for measuring the cleavage activity of the target hHao1 can be obtained by replacing positions 6759-7380 with the hHao1-LUxUC sequence) was used to measure its cleavage activity. The target sequences, insertion sequences, and LUxUC sequences are shown in the following table:

| Target | Insertion Sequence | Target Sequence | Corresponding LUxUC Sequence |
|---|---|---|---|
| TTR | SEQ ID NO.76 | SEQ ID NO.11 | SEQ ID NO.73 |
| hHao1 | SEQ ID NO.98 | SEQ ID NO.101 | SEQ ID NO.99 |

Analysis revealed that CasY7 and its mutants exhibited cleavage activities shown in the following table, while no cleavage activity was detected in the negative control (NT) group.

| Variant | TTR Target Cleavage Activity (%) | hHao1 Target Cleavage Activity (%) |
|---|---|---|
| C30725 | 35.65 | 13.22 |
| C30381 | 35.70 | 17.70 |
| C30924 | 36.44 | 15.25 |
| C30301 | 38.52 | 19.79 |
| C30486 | 38.42 | 16.28 |
| C30591 | 39.13 | 12.57 |
| C30725 | 44.20 | 16.88 |
| C30316 | 35.75 | 18.40 |
| C30834 | 40.73 | 13.51 |
| C40492 | 35.54 | 19.84 |
| C40780 | 40.96 | 15.76 |
| C40571 | 44.25 | 12.95 |
| C40251 | 43.21 | 14.12 |
| C40460 | 42.25 | 18.35 |
| C40107 | 47.74 | 22.17 |
| C40652 | 39.54 | 26.92 |
| C50371 | 37.73 | 20.56 |
| C50725 | 39.32 | 19.07 |
| C50878 | 35.59 | 23.10 |
| C50379 | 39.57 | 24.96 |
| C50576 | 36.5 | 26.94 |
| C50501 | 40.50 | 20.15 |
| C50122 | 39.24 | 18.08 |
| C50729 | 40.69 | 28.44 |
| C50734 | 37.95 | 22.30 |
| C50132 | 40.20 | 17.20 |
| C50337 | 37.65 | 20.76 |
| C50426 | 39.65 | 32.49 |
| C62732 | 37.12 | 23.75 |
| C62072 | 34.83 | 23.71 |
| C62302 | 34.11 | 20.28 |
| C68550 | 33.53 | 21.01 |
| C65495 | 38.86 | 26.50 |
| C69601 | 33.91 | 21.26 |
| C62198 | 33.34 | 19.17 |
| C65825 | 33.30 | 19.36 |
| C61684 | 34.14 | 20.36 |
| C61352 | 33.35 | 19.18 |
| CasY7 | 2.07 | 1.48 |

Through analysis, it can be seen that as compared with the wild-type CasY7 protein, each variant exhibited higher cleavage activity on the TTR gene and hHao1 gene targets. At the TTR gene, as compared with the wild-type CasY7 protein, the variants showed cleavage activity not less than 16 folds, up to approximately 23 folds. At the hHao1 gene, as compared with the wild-type CasY7 protein, the variants showed cleavage activity not less than 8.5 folds, up to approximately 22 folds.

### Example 8. Fusion with T5 Exonuclease to Improve Cleavage Activity

To enhance the cleavage activity of the CRISPR-Cas system, CasY7 variant C05440 (the mutating method was as described in Example 6, its amino acid sequence is represented by SEQ ID NO.82, and the nucleotide coding sequence is represented by SEQ ID NO.83) was fused with T5 exonuclease (its amino acid sequence is represented by SEQ ID NO.81, and the nucleotide coding sequence is represented by SEQ ID NO.100). T5 exonuclease may be linked to either the C-terminus or N-terminus of C05440:

| Fusion Protein | Fusion Structure |
|---|---|
| C05440-T5 | NH₂-[NLS]-[C05440]-[T5]-[NLS]-COOH, wherein "-" refers to an optional linker |
| T5-C05440 | NH₂-[NLS]-[T5]-[C05440]-[NLS]-COOH, wherein "-" refers to an optional linker |

In this example, the amino acid sequences and nucleotide coding sequences of the fusion proteins C05440-T5 and T5-C05440 are as follows:

| Fusion Protein | Amino Acid Sequence | Nucleotide Coding Sequence |
|---|---|---|
| C05440-T5 | SEQ ID NO:84 | SEQ ID NO:85 |
| T5-C05440 | SEQ ID NO:86 | SEQ ID NO:87 |

The C05440-T5 mRNA transcription template (SEQ ID NO.88) and T5-C05440 mRNA transcription template (SEQ ID NO.89) were synthesized by Nanjing Genscript Biotech, and subjected to an in-vitro transcription reaction by using a HiScribe^{®} T7 High Yield RNA Synthesis Kit (NEB, E2040S) to obtain C05440-T5 mRNA and T5-C05440 mRNA.

The hHAO1 gene was selected as the editing target. A spacer sequence was designed according to the target sequence, and hHAO1-crRNA was synthesized by Nanjing Genscript Biotech. The crRNA sequence is as follows:
AGAAATCCGTCCAAAGCTGACGGggacagagggtcagcatgcc (SEQ ID NO:90), wherein the underlined sequence is the DR-1 sequence, and the remaining sequence is the spacer sequence.

Delivery was carried out using a four-component LNP lipid (purchased from AVT (Shanghai) Pharmaceutical Tech Co., Ltd.). Specifically, Yoltech Lipid1 (Compound 10), DSPC, cholesterol, and PEG-DMG were dissolved in absolute ethanol in a molar ratio of 50:10:38.5:1.5. The C05440-T5 mRNA and T5-C05440 mRNA were each dissolved in a 100 mM enzyme-free citrate buffer at pH 4 (RNA concentration: 0.2 mg/mL) together with hHAO-crRNA targeting the hHAO1 gene (in a mass ratio of 1:1). The ethanol solution of the lipid carrier and the buffer solution of mRNA were mixed in a ratio of 1:3 (volume/volume) (wherein the mass ratio of total lipid to mRNA is 40:1), and passed through a microfluidic nanodrug manufacturing system (NanoAssemblr Ignite, Canada) at a flow rate of 12 mL/min to obtain nucleic acid-lipid nanoparticles. The obtained nucleic acid-lipid nanoparticles were immediately diluted 40-fold in a 1× DPBS buffer.

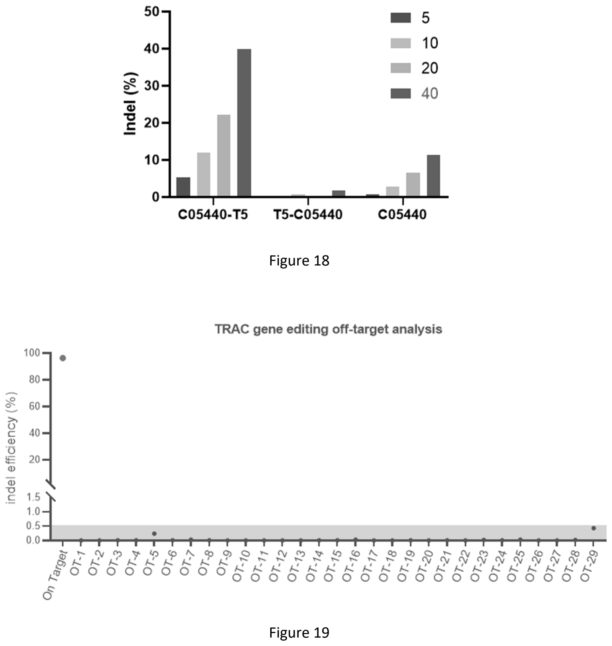

HepG2 cells (purchased from ATCC) were inoculated in a DMEM medium (Gibco, 11965092) supplemented with 10% FBS (v/v) and containing 1% Penicillin Streptomycin (v/v) (Gibco, 15140122), and cultured in a 37°C cell incubator with 5% CO₂. Cells for transfection were inoculated and cultured in a 96-well cell culture plate on day 1. On day 2, the cells were observed, and when the cell confluency reached about 80%, LNP transfection was carried out. LNP@mRNA (transfection doses were 5 ng/well, 10 ng/well, 20 ng/well, and 40 ng/well) was added to the HepG2 cells. After 48 hours of transfection, the cells were collected, and genomic DNA was extracted from the collected cells (TIANGEN, DP304-03) and measured for cleavage activity (Figure 18). The identification primers used were hHAO1-F (SEQ ID NO:91) and hHAO1-R (SEQ ID NO:92).

Analysis shows that at all dose levels, the cleavage activity of C05440-T5 was much higher than that of T5-C0544 and C05440, while the cleavage activity of T5-C0544 was the lowest at all dose levels. This indicates that the linking position of T5 exonuclease is associated with its cleavage activity.

The synthesis method for Yoltech Lipid1 (Compound 10) was as follows.

7-butyl-21-(10-butyl-3,9-dioxo-2,8-dioxahexadec-1-yl)-19-[3-(diethylamino)propyl]-8-oxo-19-aza-9-oxadocosan-22-yl 5-[(2-butyl-1-oxo-octyl)oxy]pentanoate.

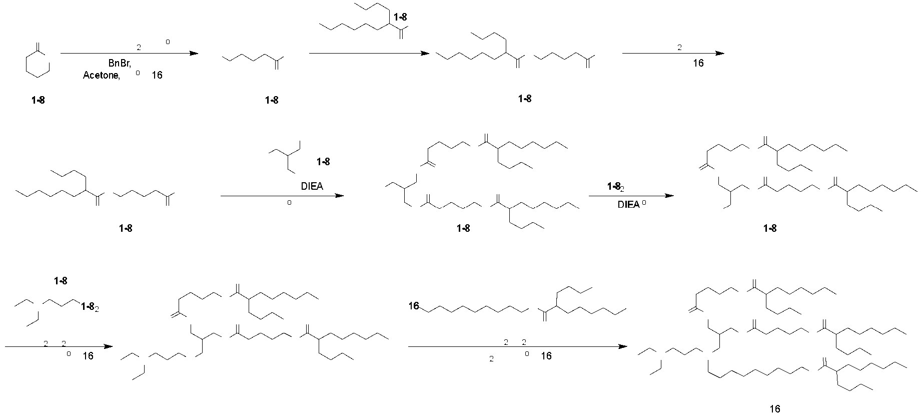

### Step 1: Synthesis of Compound 1-2

In a 500-mL round-bottom flask, δ-valerolactone (25.00 g, 249.70 mmol, 1.0 eq), distilled water (20 mL), ethanol (200 mL), and sodium hydroxide (10.99 g, 274.67 mmol, 1.1 eq) were added. After a reaction at 70°C for 3 hours, the solvent was removed by concentration under reduced pressure. Then 200 mL of acetone, tetrabutylammonium iodide (4.61 g, 12.48 mmol, 0.05 eq), and benzyl bromide (51.25 g, 299.64 mmol, 1.2 eq) were slowly added to the flask. Subsequently, a reaction was carried out at 70°C overnight, and quenched by adding 500 mL water. The mixture was extracted twice with 500 mL ethyl acetate each time. The organic phases were combined, washed with saturated brine, dried over anhydrous sodium sulfate, concentrated under reduced pressure, and purified by column chromatography to obtain benzyl 5-hydroxypentanoate (37.00 g, yield 71.2%).

### Step 2: Synthesis of Compound 1-4

In a 500-mL round-bottom flask, benzyl 5-hydroxypentanoate (37.00 g, 177.67 mmol, 1.0 eq), 2-butyloctanoic acid (35.59 g, 177.67 mmol, 1.0 eq), 250 mL dichloromethane, and 4-dimethylaminopyridine (21.70 g, 177.67 mmol, 1.0 eq) were added. Finally, 1-(3-dimethylaminopropyl)-3-ethylcarbodiimide hydrochloride (51.09 g, 266.50 mmol, 1.5 eq) was added. A reaction was carried out at room temperature for 4 hours. Then 500 mL water was added for dilution. The mixture was extracted twice with 500 mL dichloromethane each time. The organic phases were combined, washed with saturated brine, dried over anhydrous sodium sulfate, concentrated under reduced pressure, and purified by column chromatography to obtain 5-(benzyloxy)-5-oxopentyl 2-butyloctanoate (64.00 g, yield 92.2%).

### Step 3: Synthesis of Compound 1-5

In a 250-mL round-bottom flask, 5-(benzyloxy)-5-oxopentyl 2-butyloctanoate (64.00 g, 163.87 mmol, 1.0 eq), methanol (75 mL), tetrahydrofuran (75 mL), and finally Pd/C (3.49 g, 32.78 mmol, 0.2 eq, 10% purity) were added. A reaction was carried out at room temperature for 16 hours in a hydrogen atmosphere at 1 atm. After filtration and concentration, 5-[(2-butyl-1-oxooctyl)oxy]pentanoic acid (45.00 g, yield 91.4%) was obtained.

### Step 4: Synthesis of Compound 1-7

At room temperature, 5-[(2-butyl-1-oxooctyl)oxy]pentanoic acid (10.00 g, 33.29 mmol, 1.0 eq), 2-(hydroxymethyl)propane-1,3-diol (3.53 g, 33.29 mmol, 1.0 eq), 4-dimethylaminopyridine (0.81 g, 6.66 mmol, 0.2 eq), N-(3-dimethylaminopropyl)-N'-ethylcarbodiimide hydrochloride (9.57 g, 49.94 mmol, 1.5 eq), and N,N-diisopropylethylamine (8.60 g, 66.58 mmol, 2.0 eq) were added to a round-bottom flask containing 100 mL dichloromethane. The mixture was stirred at room temperature to allow a reaction to proceed for 4 hours. The reaction was quenched by adding 200 mL water. The mixture was extracted twice with 200 mL dichloromethane each time. The organic phases were combined, washed with brine, dried over anhydrous sodium sulfate, filtered, concentrated, and purified by column chromatography to obtain 18-butyl-8-(hydroxymethyl)-5,11,17-trioxo-6,10,16-trioxa-tetracosan-1-yl 2-butyloctanoate (7.80 g, yield 69.9%).

### Step 5: Synthesis of Compound 1-8

At room temperature, the compound 18-butyl-8-(hydroxymethyl)-5,11,17-trioxo-6,10,16-trioxa-tetracosan-1-yl 2-butyloctanoate (3.90 g, 5.81 mmol, 1.0 eq) and triethylamine (1.76 g, 17.43 mmol, 3.0 eq) were added to 30 mL dichloromethane. Methanesulfonic anhydride (2.02 g, 11.62 mmol, 2.0 eq) was slowly added at 0°C. The temperature was slowly restored to room temperature, and a reaction was carried out for 4 hours. The reaction mixture was quenched by adding 30 mL water. The mixture was extracted twice with 50 mL dichloromethane each time. The organic phases were combined, washed with brine, dried over anhydrous sodium sulfate, filtered, concentrated, and purified by column chromatography to obtain 12-butyl-2-(10-butyl-3,9-dioxo-2,8-dioxa-hexadec-1-yl)-5,11-dioxo-4,10-dioxa-octadecan-1-yl methanesulfonate (3.85 g, yield 88.4%).

### Step 6: Synthesis of Compound 1-10

At room temperature, Compound 1-8 (600.0 mg, 0.80 mmol, 1.0 eq), 3-amino-1-propanol (300.0 mg, 3.99 mmol, 5.0 eq), potassium carbonate (280.0 mg, 2.00 mmol, 2.5 eq), and potassium iodide (130.0 mg, 0.80 mmol, 1.0 eq) were added to 10 mL acetonitrile. Under nitrogen protection, the mixture was heated to 90°C and reacted for 16 hours. The reaction mixture was concentrated, diluted with water, and extracted three times with ethyl acetate. The organic phases were combined, washed with saturated brine, dried over anhydrous sodium sulfate, concentrated, and purified by column chromatography to obtain 12-butyl-2-{[(3-(diethylamino)propyl)amino]methyl}-5,11-dioxo-4,10-dioxa-octadecan-1-yl 5-[(2-butyl-1-oxooctyl)oxy]pentanoate (210.0 mg, 36.11%). MS: m/z [M+H]⁺ = 728.6.

### Step 7: Synthesis of Compound 10

At room temperature, the compound 8-(10-butyl-3,9-dioxo-2,8-dioxa-hexadec-1-yl)-14-ethyl-5-oxo-10,14-diaza-6-oxa-hexadecan-1-yl 2-butyloctanoate (500.0 mg, 0.64 mmol, 1.0 eq), 9-bromononyl 2-butyloctanoate (390.0 mg, 0.96 mmol, 1.5 eq), potassium carbonate (270.0 mg, 1.92 mmol, 3.0 eq), and potassium iodide (110.0 mg, 0.64 mmol, 1.0 eq) were added to 20 mL acetonitrile. Under nitrogen protection, the mixture was heated to 90°C and reacted overnight. The reaction mixture was concentrated, diluted with water, and extracted three times with dichloromethane. The organic phases were combined, washed with saturated brine, dried over anhydrous sodium sulfate, concentrated, and purified by column chromatography to obtain 7-butyl-21-(10-butyl-3,9-dioxo-2,8-dioxa-hexadec-1-yl)-19-[3-(diethylamino)propyl]-8-oxo-19-aza-9-oxa-docosan-22-yl 5-[(2-butyl-1-oxooctyl)oxy]pentanoate (132.8 mg, yield 18.8%). MS: m/z [M+H]⁺ = 1107.9. ¹H NMR (300 MHz, CDCl₃) δ 4.15-4.01 (m, 10 H), 3.44-3.20 (m, 4H), 2.71-2.50 (m, 6H), 2.39-2.23 (m, 12H), 2.02-1.40 (m, 28H), 1.38-1.22 (m, 48H), 0.92-0.75 (m, 18 H).

### Example 9. Verification of CasY7 Off-target Activity at the Trac Gene

To evaluate the application of CasY7 in human cells, based on the PAM sequence of CasY7, the spacer sequence Target-Trac-spacer3 (SEQ ID NO.59) targeting the Trac gene was designed. Additionally, 29 gene loci where off-target cleavage might occur and the off-target protospacer sequences were predicted, as shown in the following table:

| 5'-3'PAM | Protospacer Sequence / Spacer Sequence | Chromosome | Specific Location |
|---|---|---|---|
| TTT | gTGCTCCAGGtCACAGCtCT | chr2 | 237271881 |
| TTC | TTGCTCCAGGCCcCAGaAaT | chr4 | 121135280 |
| TTC | TTtCTCCtGGCCACAGCACT | chr4 | 145431515 |
| TTC | TTGaTCCAGGgaACAGCACT | chr6 | 104005064 |
| TTC | TaGCTCCAGGCCACAGaACT | chr10 | 81473452 |
| TTT | TaaCTgCAGGCCACAaCACT | chr15 | 43244023 |
| TTC | TTGgTCtAGGCCACtGtACT | chr15 | 81722418 |
| TTT | TTGCTCgAGGCCAtAGCctT | chr5 | 93592081 |
| TTC | cTcCTCCAGGCCtCAGCcCT | chr20 | 3866448 |
| TTT | gTGCTCCAGGaaAaAGCACT | chr1 | 54050493 |
| TTG | TTGCTgCAGGggACAGCACT | chr22 | 30822247 |
| TTG | TTGCTCaAGGtCACAGaACT | chr2 | 209032340 |
| TTT | TTtCTCCAGGgCtCAGCACa | chr12 | 32625916 |
| TTC | cTcCTCCAGtCCACAGCtCT | chr12 | 69453027 |
| TTT | TaGCTCCAGcCCACAGCtCa | chr12 | 86601659 |
| TTT | TTGCTgCAGcCCtCAcCACT | chr12 | 88218397 |
| TTC | TTGCTCaAGGaaACAGCtCT | chr12 | 114539273 |
| TTC | TTGCTCaAGGaaACAGCtCT | chr12 | 114569017 |
| TTT | TTttTtCAGGCCACAGCACT | chr4 | 133929247 |
| TTG | TTtCTCtAGGaCACAGCACT | chr4 | 144538441 |
| TTA | cTGCTtCAGGCCACtGCACT | chr17 | 76370058 |
| TTG | TTGCTCgAGGCCcCAGtcCT | chr9 | 133737920 |
| TTA | TTGCTtCtGGCCACAGCtCT | chrX | 1478102 |
| TTG | aTGCTCCAGGCtcCAGaACT | chrX | 76648084 |
| TTT | TTGCTCaAGaCtACAGaACT | chr14 | 57887175 |
| TTT | TTGCaCCAaGCtACAGaACT | chr6 | 169320105 |
| TTA | TTGCTtCtGGCCACAGCtCT | chrY | 1478102 |
| TTC | TcaCTCCAGtCCACAGaACT | chr13 | 79432519 |
| TTT | TTtCTCCAcGCCACAGaACT | chr3 | 192544218 |

In this example, the DR-1 sequence was used as the Direct Repeat sequence, and the DR-1-Trac sequence was as follows: AGAAATCCGTCCAAAGCTGACGGttgctccaggccacagcact (SEQ ID NO:93). The gRNA expression plasmid was constructed in the same manner as in Example 3. The CasY7 recombinant expression plasmid and the above-mentioned gRNA expression plasmid were co-introduced into HEK293 cells by PEI transfection. The on-target and off-target activities were measured by deep sequencing. Analysis shows that CasY7 basically did not show off-target cleavage activity at the predicted sites (Figure 19, "On Target" represents on-target cleavage activity, and "OT1-29" represent the predicted off-target gene loci). The CRISPR-Cas system of the present disclosure has a wide range of applications, high cleavage activity and low collateral activity, and can be used for drug screening, disease diagnosis and prognosis, as well as the treatment of various genetic diseases.

### Example 10. Cleavage Activity of CasY7 in Plant Cells

To evaluate the targeted cleavage of CasY7 in plant cells, the applicant selected the rice endogenous gene OsBEL as the target gene. Based on the PAM (5'-TTN-3') of CasY7, OsBEL-crRNA was designed. The DR sequence used was DR-1, and the OsBEL-crRNA sequence was as follows:
AGAAATCCGTCCAAAGCTGACGGATCTCCTTCTAGAAGCACAA (SEQ ID NO:94)

The purified CasY7 protein (60 µg) was combined with OsBEL-crRNA (120 µg) to form RNP at 37°C. The RNP was introduced into rice protoplast cells using PEG4000. After 24 hours of culturing, the editing activity was measured. Analysis revealed that the cleavage activity of CasY7 at the target site was approximately 75%. This indicates that the CRISPR-CasY7 system can be used in the field of plants and has a potential application value.

### Sequence information

| No. | Item | Sequence |
|---|---|---|
| 1 | CasY7 amino acid sequence | MATKTIVRPYTSNLSPNAAKKAMLDESFKFFDHAYTVFFSVFIKLWGGVKPTQVALVEN DTNKIDAICSILWFRLQTKTDSTNITLQSAEERIRRFKEYAQHDPSPLALSYLTGNLDPEKH |
| | | EWVDCRELYQNWCAELKCDLATDIETMINHNLLPISAKQEYNCYSSFSNLFGEAEKENK AEKVEWYRSVVAALKSKSAWNWDEYRKMLFEKIGCTAPKGGGRLSLLFLDMRSDTTGP ISSQEITKRITSFEKVAEEKSKTYELPHRLTVKQYICNQLAHPYDLAIWSAVANYAISSIRSK NSINMLYSDEKLNRETIIAKIKSQNPNIEQVQEILSQFRAGENNAFVIEPRHLSNLKSLFRF WSNSSMDEGITTLLQIPQSEYERSPIVDLLRHIHPHRNKFTAEQFIVAAELNGIEFDNAM KKIHPTVYGKTTVDYGPKSTIYGSITPPSKLIRGVHAGSHGMMWVTMTLLDNGKWIKH HIPFHNSRYYEEMYAYREGLPVGEKPRRPVLGNRVGNHIDNTGLINKKCRKASKAYLRTI QNMNHNVWFDPDTHFLVQRNGDNFAIQISSRIKESKPKSNVEVGDRILGMDQNQTAS NTYSVVEVVREGTENSHPYNNYFVKVIEDGNVTSCTTKNDRGEFDQLSYEGLAYSEFEIW RQARIAFLSAHDPELAQKMIDKTNESLYKWNNNYAYYLKVCMRNKMNGDNHALFRNE IKEFIEGMPVAGKNEKHKFFGSIRGSLSLESLEGLSKSRSLISCYFYLLEKKEIEQQKEFDSDL FKLGECLSEKRVNKREERANRIVSSVLQICSRLNVSRIVIENKLPTANHENKSSANRRATD WCPRKVQQKLLDAVKMVGIKVLAVKPYNTSHIDPFVNGESNRQALEARFMDVDVKDIT DRNIKQFKKMHRNQVGVLNSIYHNALRSFAANYGLNWNELPNMNLEQIKNALKDHVR VMFPQWGGRSFMSTHNVTRNSVRVSYNNRTRWLNFSDVIAALNIALRGSGNYEPKGD SQNTAPSRNS |
| 2 | Coding sequence of CasY7 protein | ATGGCCACGAAGACCATCGTGAGGCCATACACCAGCAACCTGTCCCCTAACGCCGCC AAGAAGGCCATGCTGGACGAGAGCTTCAAGTTCTTCGACCACGCCTACACCGTGTTC TTCAGCGTGTTCATCAAGCTGTGGGGCGGCGTGAAGCCCACCCAGGTGGCCCTGGT GGAGAACGACACCAACAAGATCGACGCCATCTGCAGCATCCTGTGGTTCAGGCTGC AGACCAAGACCGACAGCACCAACATCACCCTCCAGTCCGCCGAGGAGAGGATCCGC CGCTTCAAGGAGTACGCCCAGCACGACCCTAGCCCCCTGGCCCTCAGCTACCTGACG GGCAACCTGGACCCCGAGAAGCACGAGTGGGTCGACTGCCGCGAGCTGTACCAGA ACTGGTGCGCCGAGCTGAAGTGCGACCTCGCCACCGACATCGAAACCATGATCAAC CACAACCTGCTGCCTATCAGCGCGAAGCAGGAGTACAACTGCTACAGCTCGTTCAGC AACCTGTTCGGCGAGGCCGAGAAGGAGAACAAGGCCGAGAAGGTGGAGTGGTAC AGAAGCGTGGTGGCCGCGCTGAAGTCCAAGAGCGCCTGGAACTGGGACGAGTACA GGAAGATGCTGTTCGAGAAGATCGGCTGCACCGCCCCCAAGGGCGGCGGCAGACT GAGCCTCCTGTTCCTGGACATGAGGTCCGACACCACCGGCCCCATCAGCTCCCAGGA GATCACCAAGCGGATCACCAGCTTCGAGAAGGTGGCCGAGGAGAAGAGCAAGACC TACGAGCTCCCCCACAGGCTGACCGTGAAGCAGTACATCTGCAACCAGCTGGCCCAC CCCTACGACCTGGCCATCTGGAGCGCCGTGGCCAACTACGCCATCTCCAGCATCAGG AGCAAGAACAGCATCAATATGCTGTACAGCGACGAGAAGCTCAACAGGGAAACCAT CATCGCCAAGATCAAGAGCCAGAACCCCAACATCGAGCAGGTGCAGGAGATCCTGT |
| | | CCCAGTTCAGGGCCGGCGAGAACAACGCGTTCGTGATCGAGCCCAGACACCTGTCC AACCTGAAGAGCCTGTTCAGGTTCTGGTCCAACAGCAGCATGGACGAGGGCATCAC CACCCTGCTGCAGATCCCTCAGAGCGAGTACGAGCGCAGCCCCATCGTGGACCTGCT CCGGCACATCCACCCCCACAGGAACAAGTTCACCGCCGAGCAGTTCATCGTGGCCGC CGAGCTGAACGGCATCGAGTTCGACAACGCGATGAAGAAGATCCACCCCACCGTGT ACGGCAAGACCACCGTGGACTACGGCCCCAAGAGCACCATCTACGGCAGCATCACC CCACCAAGCAAGCTGATCCGCGGCGTGCACGCCGGCAGCCACGGCATGATGTGGGT GACCATGACCCTGCTGGACAACGGCAAGTGGATCAAGCACCACATCCCCTTCCACAA CAGCAGGTACTACGAGGAGATGTACGCCTACAGAGAGGGCCTGCCGGTCGGCGAG AAGCCCAGGAGGCCGGTGCTGGGCAACCGGGTGGGCAACCACATCGACAACACCG GCCTGATCAATAAGAAGTGCAGGAAGGCCAGCAAGGCCTACCTGAGGACCATCCAG AACATGAATCACAACGTGTGGTTCGACCCCGACACCCACTTCCTGGTGCAGAGGAAC GGCGACAACTTCGCCATCCAGATCTCCTCCAGGATCAAGGAGAGCAAGCCCAAGTC CAACGTGGAGGTGGGCGACCGCATCCTGGGCATGGACCAGAACCAGACGGCCAGC AACACCTACAGCGTGGTGGAGGTGGTGCGCGAGGGCACCGAGAATAGCCACCCCT ACAACAACTACTTTGTCAAGGTGATCGAGGACGGCAACGTGACCAGCTGCACCACC AAGAATGACAGGGGCGAGTTCGACCAGCTGAGCTACGAGGGCCTGGCCTACAGCG AGTTCGAGATCTGGCGGCAGGCCAGGATCGCCTTCCTGAGCGCCCACGACCCCGAG CTGGCCCAGAAGATGATCGACAAGACCAACGAGAGCCTGTACAAGTGGAACAACAA CTACGCCTACTACCTGAAGGTGTGCATGAGGAACAAGATGAACGGCGACAACCACG CCCTGTTCCGGAACGAGATCAAGGAGTTCATCGAGGGCATGCCCGTGGCGGGCAAG AACGAGAAGCACAAGTTCTTCGGCTCCATCAGGGGCAGCCTGTCCCTGGAGAGCCT GGAGGGCCTGAGCAAGTCCCGCAGCCTGATCAGCTGCTACTTCTACCTGCTCGAGA AGAAGGAGATCGAGCAGCAGAAGGAGTTCGACAGCGATCTGTTCAAGCTGGGCGA GTGCCTGAGCGAGAAGAGAGTGAACAAGCGGGAGGAGAGAGCCAACCGGATCGT GAGCTCGGTGCTGCAGATCTGCAGCCGGCTGAACGTGAGCAGGATCGTGATCGAG AACAAGCTGCCCACCGCCAACCACGAGAACAAGAGCAGCGCCAACCGCAGAGCCAC CGACTGGTGCCCCAGGAAGGTGCAGCAGAAGCTGCTGGACGCGGTGAAGATGGTG GGGATCAAGGTGCTGGCCGTGAAGCCCTACAACACCAGCCACATCGACCCCTTCGT GAACGGCGAGAGCAACAGACAGGCCCTGGAGGCCAGGTTCATGGACGTGGACGTG AAGGACATCACCGACAGAAACATCAAGCAGTTCAAGAAGATGCACAGAAACCAGGT GGGGGTGCTGAACTCCATCTACCACAACGCCCTGCGGTCCTTCGCCGCCAACTACGG CCTGAATTGGAACGAGCTGCCGAACATGAATCTGGAGCAGATCAAGAACGCCCTGA AGGACCACGTGAGGGTGATGTTCCCTCAGTGGGGCGGCCGGAGCTTCATGAGCACC |
| | | CACAACGTGACCCGGAACAGCGTGAGAGTGAGCTACAACAACAGGACCCGCTGGCT GAACTTCAGCGACGTGATCGCCGCCCTCAACATCGCCCTCAGGGGCAGCGGCAACT ACGAGCCCAAGGGGGACAGCCAGAACACCGCCCCCAGCAGAAACAGCTGA |
| 3 | PHK09T Vector Sequence | TGAGCTGATACCGCTCGCCGCAGCCGAACGACCGAGCGCAGCGAGTCAGTGAGCG AGGAAGCGGAAGAGCGCCCAATACGCAAACCGCCTCTCCCCGCGCGTTGGCCGATT CATTAATGCAGCTGGCACGACAGGTTTCCCGACTGGAAAGCGGGCAGTGAGCGCAA CGCAATTAATGTGAGTTAGCTCACTCATTAGGCACCCCAGGCTTTACACTTTATGCTT CCGGCTCGTATGTTGTGTGGAATTGTGAGCGGATAACAATTTCACACAGGAAACAG CTATGACCATGATTACGCCAAGCGCGCAATTAACCCTCACTAAAGGGAACAAAAGCT GGAGCTGCAAGCTTAATGTAGTCTTATGCAATACTCTTGTAGTCTTGCAACATGGTA ACGATGAGTTAGCAACATGCCTTACAAGGAGAGAAAAAGCACCGTGCATGCCGATT GGTGGAAGTAAGGTGGTACGATCGTGCCTTATTAGGAAGGCAACAGACGGGTCTG ACATGGATTGGACGAACCACTGAATTGCCGCATTGCAGAGATATTGTATTTAAGTGC CTAGCTCGATACATAAACGGGTCTCTCTGGTTAGACCAGATCTGAGCCTGGGAGCTC TCTGGCTAACTAGGGAACCCACTGCTTAAGCCTCAATAAAGCTTGCCTTGAGTGCTT CAAGTAGTGTGTGCCCGTCTGTTGTGTGACTCTGGTAACTAGAGATCCCTCAGACCC TTTTAGTCAGTGTGGAAAATCTCTAGCAGTGGCGCCCGAACAGGGACTTGAAAGCG AAAGGGAAACCAGAGGAGCTCTCTCGACGCAGGACTCGGCTTGCTGAAGCGCGCA CGGCAAGAGGCGAGGGGCGGCGACTGGTGAGTACGCCAAAAATTTTGACTAGCGG AGGCTAGAAGGAGAGAGATGGGTGCGAGAGCGTCAGTATTAAGCGGGGGAGAAT TAGATCGCGATGGGAAAAAATTCGGTTAAGGCCAGGGGGAAAGAAAAAATATAAA TTAAAACATATAGTATGGGCAAGCAGGGAGCTAGAACGATTCGCAGTTAATCCTGG CCTGTTAGAAACATCAGAAGGCTGTAGACAAATACTGGGACAGCTACAACCATCCCT TCAGACAGGATCAGAAGAACTTAGATCATTATATAATACAGTAGCAACCCTCTATTG TGTGCATCAAAGGATAGAGATAAAAGACACCAAGGAAGCTTTAGACAAGATAGAG GAAGAGCAAAACAAAAGTAAGACCACCGCACAGCAAGCGGCCGCTGATCTTCAGAC CTGGAGGAGGAGATATGAGGGACAATTGGAGAAGTGAATTATATAAATATAAAGT AGTAAAAATTGAACCATTAGGAGTAGCACCCACCAAGGCAAAGAGAAGAGTGGTG CAGAGAGAAAAAAGAGCAGTGGGAATAGGAGCTTTGTTCCTTGGGTTCTTGGGAGC AGCAGGAAGCACTATGGGCGCAGCGTCAATGACGCTGACGGTACAGGCCAGACAA TTATTGTCTGGTATAGTGCAGCAGCAGAACAATTTGCTGAGGGCTATTGAGGCGCA ACAGCATCTGTTGCAACTCACAGTCTGGGGCATCAAGCAGCTCCAGGCAAGAATCCT GGCTGTGGAAAGATACCTAAAGGATCAACAGCTCCTGGGGATTTGGGGTTGCTCTG GAAAACTCATTTGCACCACTGCTGTGCCTTGGAATGCTAGTTGGAGTAATAAATCTC |
| | | TGGAACAGATTTGGAATCACACGACCTGGATGGAGTGGGACAGAGAAATTAACAAT TACACAAGCTTAATACACTCCTTAATTGAAGAATCGCAAAACCAGCAAGAAAAGAAT GAACAAGAATTATTGGAATTAGATAAATGGGCAAGTTTGTGGAATTGGTTTAACATA ACAAATTGGCTGTGGTATATAAAATTATTCATAATGATAGTAGGAGGCTTGGTAGGT TTAAGAATAGTTTTTGCTGTACTTTCTATAGTGAATAGAGTTAGGCAGGGATATTCAC CATTATCGTTTCAGACCCACCTCCCAACCCCGAGGGGACCCAGAGAGGGCCTATTTC CCATGATTCCTTCATATTTGCATATACGATACAAGGCTGTTAGAGAGATAATTAGAAT TAATTTGACTGTAAACACAAAGATATTAGTACAAAATACGTGACGTAGAAAGTAATA ATTTCTTGGGTAGTTTGCAGTTTTAAAATTATGTTTTAAAATGGACTATCATATGCTT ACCGTAACTTGAAAGTATTTCGATTTCTTGGCTTTATATATCTTGTGGAAAGGACGAA ACACCGGAGACGGACGTCTCTTTTTTTAAGCTTGGCGTAACTAGATCTTGAGACAAA TGGCAGTATTCATCCACAATTTTAAAAGAAAAGGGGGGATTGGGGGGTACAGTGCA GGGGAAAGAATAGTAGACATAATAGCAACAGACATACAAACTAAAGAATTACAAAA ACAAATTACAAAAATTCAAAATTTTCGGGTTTATTACAGGGACAGCAGAGATCCACT TTGGCGCCGGCTCGAGGGGGCCCGGGTGCAAAGATGGATAAAGTTTTAAACAGAG AGGAATCTTTGCAGCTAATGGACCTTCTAGGTCTTGAAAGGAGTGGGAATTGGCTCC GGTGCCCGTCAGTGGGCAGAGCGCACATCGCCCACAGTCCCCGAGAAGTTGGGGG GAGGGGTCGGCAATTGATCCGGTGCCTAGAGAAGGTGGCGCGGGGTAAACTGGGA AAGTGATGTCGTGTACTGGCTCCGCCTTTTTCCCGAGGGTGGGGGAGAACCGTATA TAAGTGCAGTAGTCGCCGTGAACGTTCTTTTTCGCAACGGGTTTGCCGCCAGAACAC AGGTAAGTGCCGTGTGTGGTTCCCGCGGGCCTGGCCTCTTTACGGGTTATGGCCCTT GCGTGCCTTGAATTACTTCCACCTGGCTGCAGTACGTGATTCTTGATCCCGAGCTTCG GGTTGGAAGTGGGTGGGAGAGTTCGAGGCCTTGCGCTTAAGGAGCCCCTTCGCCTC GTGCTTGAGTTGAGGCCTGGCCTGGGCGCTGGGGCCGCCGCGTGCGAATCTGGTG GCACCTTCGCGCCTGTCTCGCTGCTTTCGATAAGTCTCTAGCCATTTAAAATTTTTGAT GACCTGCTGCGACGCTTTTTTTCTGGCAAGATAGTCTTGTAAATGCGGGCCAAGATC TGCACACTGGTATTTCGGTTTTTGGGGCCGCGGGCGGCGACGGGGCCCGTGCGTCC CAGCGCACATGTTCGGCGAGGCGGGGCCTGCGAGCGCGGCCACCGAGAATCGGAC GGGGGTAGTCTCAAGCTGGCCGGCCTGCTCTGGTGCCTGGCCTCGCGCCGCCGTGT ATCGCCCCGCCCTGGGCGGCAAGGCTGGCCCGGTCGGCACCAGTTGCGTGAGCGG AAAGATGGCCGCTTCCCGGCCCTGCTGCAGGGAGCTCAAAATGGAGGACGCGGCG CTCGGGAGAGCGGGCGGGTGAGTCACCCACACAAAGGAAAAGGGCCTTTCCGTCCT CAGCCGTCGCTTCATGTGACTCCACGGAGTACCGGGCGCCGTCCAGGCACCTCGATT AGTTCTCGAGCTTTTGGAGTACGTCGTCTTTAGGTTGGGGGGAGGGGTTTTATGCG |
| | | ATGGAGTTTCCCCACACTGAGTGGGTGGAGACTGAAGTTAGGCCAGCTTGGCACTT GATGTAATTCTCCTTGGAATTTGCCCTTTTTGAGTTTGGATCTTGGTTCATTCTCAAGC CTCAGACAGTGGTTCAAAGTTTTTTTCTTCCATTTCAGGTGTCGTGACGTACGGCCAC CATGACCGAGTACAAGCCCACGGTGCGCCTCGCCACCCGCGACGACGTCCCCAGGG CCGTACGCACCCTCGCCGCCGCGTTCGCCGACTACCCCGCCACGCGCCACACCGTCG ATCCGGACCGCCACATCGAGCGGGTCACCGAGCTGCAAGAACTCTTCCTCACGCGC GTCGGGCTCGACATCGGCAAGGTGTGGGTCGCGGACGACGGCGCCGCCGTGGCGG TCTGGACCACGCCGGAGAGCGTCGAAGCGGGGGCGGTGTTCGCCGAGATCGGCCC GCGCATGGCCGAGTTGAGCGGTTCCCGGCTGGCCGCGCAGCAACAGATGGAAGGC CTCCTGGCGCCGCACCGGCCCAAGGAGCCCGCGTGGTTCCTGGCCACCGTCGGAGT CTCGCCCGACCACCAGGGCAAGGGTCTGGGCAGCGCCGTCGTGCTCCCCGGAGTGG AGGCGGCCGAGCGCGCCGGGGTGCCCGCCTTCCTGGAGACCTCCGCGCCCCGCAAC CTCCCCTTCTACGAGCGGCTCGGCTTCACCGTCACCGCCGACGTCGAGGTGCCCGAA GGACCGCGCACCTGGTGCATGACCCGCAAGCCCGGTGCCTGAACGCGTTAAGTCGA CAATCAACCTCTGGATTACAAAATTTGTGAAAGATTGACTGGTATTCTTAACTATGTT GCTCCTTTTACGCTATGTGGATACGCTGCTTTAATGCCTTTGTATCATGCTATTGCTTC CCGTATGGCTTTCATTTTCTCCTCCTTGTATAAATCCTGGTTGCTGTCTCTTTATGAGG AGTTGTGGCCCGTTGTCAGGCAACGTGGCGTGGTGTGCACTGTGTTTGCTGACGCA ACCCCCACTGGTTGGGGCATTGCCACCACCTGTCAGCTCCTTTCCGGGACTTTCGCTT TCCCCCTCCCTATTGCCACGGCGGAACTCATCGCCGCCTGCCTTGCCCGCTGCTGGAC AGGGGCTCGGCTGTTGGGCACTGACAATTCCGTGGTGTTGTCGGGGAAATCATCGT CCTTTCCTTGGCTGCTCGCCTGTGTTGCCACCTGGATTCTGCGCGGGACGTCCTTCTG CTACGTCCCTTCGGCCCTCAATCCAGCGGACCTTCCTTCCCGCGGCCTGCTGCCGGCT CTGCGGCCTCTTCCGCGTCTTCGCCTTCGCCCTCAGACGAGTCGGATCTCCCTTTGGG CCGCCTCCCCGCGTCGACTTTAAGACCAATGACTTACAAGGCAGCTGTAGATCTTAG CCACTTTTTAAAAGAAAAGGGGGGACTGGAAGGGCTAATTCACTCCCAACGAAGAC AAGATCTGCTTTTTGCTTGTACTGGGTCTCTCTGGTTAGACCAGATCTGAGCCTGGG AGCTCTCTGGCTAACTAGGGAACCCACTGCTTAAGCCTCAATAAAGCTTGCCTTGAG TGCTTCAAGTAGTGTGTGCCCGTCTGTTGTGTGACTCTGGTAACTAGAGATCCCTCA GACCCTTTTAGTCAGTGTGGAAAATCTCTAGCAGTACGTATAGTAGTTCATGTCATCT TATTATTCAGTATTTATAACTTGCAAAGAAATGAATATCAGAGAGTGAGAGGAACTT GTTTATTGCAGCTTATAATGGTTACAAATAAAGCAATAGCATCACAAATTTCACAAAT AAAGCATTTTTTTCACTGCATTCTAGTTGTGGTTTGTCCAAACTCATCAATGTATCTTA TCATGTCTGGCTCTAGCTATCCCGCCCCTAACTCCGCCCATCCCGCCCCTAACTCCGC |
| | | CCAGTTCCGCCCATTCTCCGCCCCATGGCTGACTAATTTTTTTTATTTATGCAGAGGC CGAGGCCGCCTCGGCCTCTGAGCTATTCCAGAAGTAGTGAGGAGGCTTTTTTGGAG GCCTAGGGACGTACCCAATTCGCCCTATAGTGAGTCGTATTACGCGCGCTCACTGGC CGTCGTTTTACAACGTCGTGACTGGGAAAACCCTGGCGTTACCCAACTTAATCGCCTT GCAGCACATCCCCCTTTCGCCAGCTGGCGTAATAGCGAAGAGGCCCGCACCGATCG CCCTTCCCAACAGTTGCGCAGCCTGAATGGCGAATGGGACGCGCCCTGTAGCGGCG CATTAAGCGCGGCGGGTGTGGTGGTTACGCGCAGCGTGACCGCTACACTTGCCAGC GCCCTAGCGCCCGCTCCTTTCGCTTTCTTCCCTTCCTTTCTCGCCACGTTCGCCGGCTT TCCCCGTCAAGCTCTAAATCGGGGGCTCCCTTTAGGGTTCCGATTTAGTGCTTTACG GCACCTCGACCCCAAAAAACTTGATTAGGGTGATGGTTCACGTAGTGGGCCATCGCC CTGATAGACGGTTTTTCGCCCTTTGACGTTGGAGTCCACGTTCTTTAATAGTGGACTC TTGTTCCAAACTGGAACAACACTCAACCCTATCTCGGTCTATTCTTTTGATTTATAAG GGATTTTGCCGATTTCGGCCTATTGGTTAAAAAATGAGCTGATTTAACAAAAATTTA ACGCGAATTTTAACAAAATATTAACGCTTACAATTTAGGTGGCACTTTTCGGGGAAA TGTGCGCGGAACCCCTATTTGTTTATTTTTCTAAATACATTCAAATATGTATCCGCTCA TGAGACAATAACCCTGATAAATGCTTCAATAATATTGAAAAAGGAAGAGTATGAGT ATTCAACATTTCCGTGTCGCCCTTATTCCCTTTTTTGCGGCATTTTGCCTTCCTGTTTTT GCTCACCCAGAAACGCTGGTGAAAGTAAAAGATGCTGAAGATCAGTTGGGTGCACG AGTGGGTTACATCGAACTGGATCTCAACAGCGGTAAGATCCTTGAGAGTTTTCGCCC CGAAGAACGTTTTCCAATGATGAGCACTTTTAAAGTTCTGCTATGTGGCGCGGTATT ATCCCGTATTGACGCCGGGCAAGAGCAACTCGGTCGCCGCATACACTATTCTCAGAA TGACTTGGTTGAGTACTCACCAGTCACAGAAAAGCATCTTACGGATGGCATGACAGT AAGAGAATTATGCAGTGCTGCCATAACCATGAGTGATAACACTGCGGCCAACTTACT TCTGACAACGATCGGAGGACCGAAGGAGCTAACCGCTTTTTTGCACAACATGGGGG ATCATGTAACTCGCCTTGATCGTTGGGAACCGGAGCTGAATGAAGCCATACCAAAC GACGAGCGTGACACCACGATGCCTGTAGCAATGGCAACAACGTTGCGCAAACTATT AACTGGCGAACTACTTACTCTAGCTTCCCGGCAACAATTAATAGACTGGATGGAGGC GGATAAAGTTGCAGGACCACTTCTGCGCTCGGCCCTTCCGGCTGGCTGGTTTATTGC TGATAAATCTGGAGCCGGTGAGCGTGGGTCTCGCGGTATCATTGCAGCACTGGGGC CAGATGGTAAGCCCTCCCGTATCGTAGTTATCTACACGACGGGGAGTCAGGCAACT ATGGATGAACGAAATAGACAGATCGCTGAGATAGGTGCCTCACTGATTAAGCATTG GTAACTGTCAGACCAAGTTTACTCATATATACTTTAGATTGATTTAAAACTTCATTTTT AATTTAAAAGGATCTAGGTGAAGATCCTTTTTGATAATCTCATGACCAAAATCCCTTA ACGTGAGTTTTCGTTCCACTGAGCGTCAGACCCCGTAGAAAAGATCAAAGGATCTTC |
| | | TTGAGATCCTTTTTTTCTGCGCGTAATCTGCTGCTTGCAAACAAAAAAACCACCGCTA CCAGCGGTGGTTTGTTTGCCGGATCAAGAGCTACCAACTCTTTTTCCGAAGGTAACT GGCTTCAGCAGAGCGCAGATACCAAATACTGTTCTTCTAGTGTAGCCGTAGTTAGGC CACCACTTCAAGAACTCTGTAGCACCGCCTACATACCTCGCTCTGCTAATCCTGTTAC CAGTGGCTGCTGCCAGTGGCGATAAGTCGTGTCTTACCGGGTTGGACTCAAGACGA TAGTTACCGGATAAGGCGCAGCGGTCGGGCTGAACGGGGGGTTCGTGCACACAGC CCAGCTTGGAGCGAACGACCTACACCGAACTGAGATACCTACAGCGTGAGCTATGA GAAAGCGCCACGCTTCCCGAAGGGAGAAAGGCGGACAGGTATCCGGTAAGCGGCA GGGTCGGAACAGGAGAGCGCACGAGGGAGCTTCCAGGGGGAAACGCCTGGTATCT TTATAGTCCTGTCGGGTTTCGCCACCTCTGACTTGAGCGTCGATTTTTGTGATGCTCG TCAGGGGGGCGGAGCCTATGGAAAAACGCCAGCAACGCGGCCTTTTTACGGTTCCT GGCCTTTTGCTGGCCTTTTGCTCACATGTTCTTTCCTGCGTTATCCCCTGATTCTGTGG ATAACCGTATTACCGCCTTTGAG |
| 4 | Target plasmid | acaccatcgaatggtgcaaaacctttcgcggtatggcatgatagcgcccggaagagagtcaattcagggtggt gaatgtgaaaccagtaacgttatacgatgtcgcagagtatgccggtgtctcttatcagaccgtttcccgcgtggt gaaccaggccagccacgtttctgcgaaaacgcgggaaaaagtggaagcggcgatggcggagctgaattacat tcccaaccgcgtggcacaacaactggcgggcaaacagtcgttgctgattggcgttgccacctccagtctggccc tgcacgcgccgtcgcaaattgtcgcggcgattaaatctcgcgccgatcaactgggtgccagcgtggtggtgtcg atggtagaacgaagcggcgtcgaagcctgtaaagcggcggtgcacaatcttctcgcgcaacgcgtcagtggg ctgatcattaactatccgctggatgaccaggatgccattgctgtggaagctgcctgcactaatgttccggcgtta tttcttgatgtctctgaccagacacccatcaacagtattattttctcccatgaagacggtacgcgactgggcgtg gagcatctggtcgcattgggtcaccagcaaatcgcgctgttagcgggcccattaagttctgtctcggcgcgtctg cgtctggctggctggcataaatatctcactcgcaatcaaattcagccgatagcggaacgggaaggcgactgga gtgccatgtccggttttcaacaaaccatgcaaatgctgaatgagggcatcgttcccactgcgatgctggttgcca acgatcagatggcgctgggcgcaatgcgcgccattaccgagtccgggctgcgcgttggtgcggatatctcggta gtgggatacgacgataccgaagacagctcatgttatatcccgccgttaaccaccatcaaacaggattttcgcct gctggggcaaaccagcgtggaccgcttgctgcaactctctcagggccaggcggtgaagggcaatcagctgttg cccgtctcactggtgaaaagaaaaaccaccctggcgcccaatacgcaaaccgcctctccccgcgcgttggccg attcattaatgcagctggcacgacaggtttcccgactggaaagcgggcagtgagcgcaacgcaattaatgtaa gttagctcaaatattctgaaatgagctgttgacaattaatcatcggctcgtataatgtgtggaattgtgagcgga taacaatttcacacaggaaacagaattcTTTGCATCTCCCCATTCCATGAGcATATGCgatcttccc catcggtgatgtcggcgatataggcgccagcaaccgcacctgtggcgccggtgatgccggccacgatgcgtcc ggcgtagaggatctgctcatgtttgacagcttatcatcgatgcataatgtgcctgtcaaatggacgaagcaggg attctgcaaaccctatgctactccgtcaagccgtcaattgtctgattcgttaccaattatgacaacttgacggcta |
| | | catcattcactttttcttcacaaccggcacggaactcgctcgggctggccccggtgcattttttaaatacccgcga gaaatagagttgatcgtcaaaaccaacattgcgaccgacggtggcgataggcatccgggtggtgctcaaaag cagcttcgcctggctgatacgttggtcctcgcgccagcttaagacgctaatccctaactgctggcggaaaagat gtgacagacgcgacggcgacaagcaaacatgctgtgcgacgctggcgatatcaaaattgctgtctgccaggtg atcgctgatgtactgacaagcctcgcgtacccgattatccatcggtggatggagcgactcgttaatcgcttccat gcgccgcagtaacaattgctcaagcagatttatcgccagcagctccgaatagcgcccttccccttgcccggcgtt aatgatttgcccaaacaggtcgctgaaatgcggctggtgcgcttcatccgggcgaaagaaccccgtattggca aatattgacggccagttaagccattcatgccagtaggcgcgcggacgaaagtaaacccactggtgataccatt cgcgagcctccggatgacgaccgtagtgatgaatctctcctggcgggaacagcaaaatatcacccggtcggca aacaaattctcgtccctgatttttcaccaccccctgaccgcgaatggtgagattgagaatataacctttcattccc agcggtcggtcgataaaaaaatcgagataaccgttggcctcaatcggcgttaaacccgccaccagatgggcat taaacgagtatcccggcagcaggggatcattttgcgcttcagccatacttttcatactcccgccattcagagaag aaaccaattgtccatattgcatcagacattgccgtcactgcgtcttttactggctcttctcgctaaccaaaccggt aaccccgcttattaaaagcattctgtaacaaagcgggaccaaagccatgacaaaaacgcgtaacaaaagtgt ctataatcacggcagaaaagtccacattgattatttgcacggcgtcacactttgctatgccatagcatttttatcc ataagattagcggatcctacctgacgctttttatcgcaactctctactgtttctccatacccgtttttttgggctagc gaattcgagctcatgcagtttaaggtttacacctataaaagagagagccgttatcgtctgtttgtggatgtacag agtgatattattgacacgcccgggcgacggatggtgatccccctggccagtgcacgtctgctgtcagataaagt ctcccgtgaactttacccggtggtgcatatcggggatgaaagctggcgcatgatgaccaccgatatggccagtg tgccggtatccgttatcggggaagaagtggctgatctcagccaccgcgaaaatgacatcaaaaacgccattaa cctgatgttctggggaatataatctagagtcgacctgcaggcatgcaagcttggctgttttggcggatgagaga agattttcagcctgatacagattaaatcagaacgcagaagcggtctgataaaacagaatttgcctggcggcag tagcgcggtggtcccacctgaccccatgccgaactcagaagtgaaacgccgtagcgccgatggtagtgtgggg tctccccatgcgagagtagggaactgccaggcatcaaataaaacgaaaggctcagtcgaaagactgggccttt cgttttatctgttgtttgtcggtgaacgctctcctgagtaggacaaatccgccgggagcggatttgaacgttgcg aagcaacggcccggagggtggcgggcaggacgcccgccataaactgccaggcatcaaattaagcagaaggc catcctgacggatggcctttttgcgtttctacaaactctggatcctccggcgttcagcctgtgccacagccgaca ggatggtgaccaccatttgccccatatcaccgtcggtactgatcccgtcgtcaataaaccgaaccgctacaccc tgagcatcaaactcttttatcagttggatcatgtcggcggtgtcgcggccaagacggtcgagcttcttcaccaga atgacatcaccttcctccaccttcatcctcagcaaatccagcccttcccgatctgttgaactgccggatgccttgt cggtaaagatgcggttagcttttacccctgcatctttgagcgctgaggtctgcctcgtgaagaaggtgttgctga ctcataccaggcctgaatcgccccatcatccagccagaaagtgagggagccacggttgatgagagctttgttgt aggtggaccagttggtgattttgaacttttgctttgccacggaacggtctgcgttgtcgggaagatgcgtgatct gatccttcaactcagcaaaagttcgatttattcaacaaagccgccgtcccgtcaagtcagcgtaatgctctgcca |
| | | gtgttacaaccaattaaccaattctgattagaaaaactcatcgagcatcaaatgaaactgcaatttattcatatc aggattatcaataccatatttttgaaaaagccgtttctgtaatgaaggagaaaactcaccgaggcagttccata ggatggcaagatcctggtatcggtctgcgattccgactcgtccaacatcaatacaacctattaatttcccctcgt caaaaataaggttatcaagtgagaaatcaccatgagtgacgactgaatccggtgagaatggcaaaagcttat gcatttctttccagacttgttcaacaggccagccattacgctcgtcatcaaaatcactcgcatcaaccaaaccgt tattcattcgtgattgcgcctgagcgagacgaaatacgcgatcgctgttaaaaggacaattacaaacaggaat cgaatgcaaccggcgcaggaacactgccagcgcatcaacaatattttcacctgaatcaggatattcttctaata cctggaatgctgttttcccggggatcgcagtggtgagtaaccatgcatcatcaggagtacggataaaatgcttg atggtcggaagaggcataaattccgtcagccagtttagtctgaccatctcatctgtaacatcattggcaacgcta cctttgccatgtttcagaaacaactctggcgcatcgggcttcccatacaatcgatagattgtcgcacctgattgcc cgacattatcgcgagcccatttatacccatataaatcagcatccatgttggaatttaatcgcggcctcgagcaag acgtttcccgttgaatatggctcataacaccccttgtattactgtttatgtaagcagacagttttattgttcatgat gatatatttttatcttgtgcaatgtaacatcagagattttgagacacaacgtggctttgttgaataaatcgaactt ttgctgagttgaaggatcagatcacgcatcttcccgacaacgcagaccgttccgtggcaaagcaaaagttcaa aatcaccaactggtccacctacaacaaagctctcatcaaccgtggctccctcactttctggctggatgatggggc gattcaggcctggtatgagtcagcaacaccttcttcacgaggcagacctcagcgctagcggagtgtatactggc ttactatgttggcactgatgagggtgtcagtgaagtgcttcatgtggcaggagaaaaaaggctgcaccggtgc gtcagcagaatatgtgatacaggatatattccgcttcctcgctcactgactcgctacgctcggtcgttcgactgc ggcgagcggaaatggcttacgaacggggcggagatttcctggaagatgccaggaagatacttaacagggaa gtgagagggccgcggcaaagccgtttttccataggctccgcccccctgacaagcatcacgaaatctgacgctc aaatcagtggtggcgaaacccgacaggactataaagataccaggcgtttccccctggcggctccctcgtgcgc tctcctgttcctgcctttcggtttaccggtgtcattccgctgttatggccgcgtttgtctcattccacgcctgacact cagttccgggtaggcagttcgctccaagctggactgtatgcacgaaccccccgttcagtccgaccgctgcgcctt atccggtaactatcgtcttgagtccaacccggaaagacatgcaaaagcaccactggcagcagccactggtaat tgatttagaggagttagtcttgaagtcatgcgccggttaaggctaaactgaaaggacaagttttggtgactgcg ctcctccaagccagttacctcggttcaaagagttggtagctcagagaaccttcgaaaaaccgccctgcaaggc ggttttttcgttttcagagcaagagattacgcgcagaccaaaacgatctcaagaagatcatcttattaaggggt ctgacgctcagtggaacgaaaactcacgttaagggattttggtcatgagattatcaaaaaggatcttcacctag atccttttggtcg |
| 5 | Direct Repeat | CAAGTTGAATCCGTCTATAACTGACGG |
| 6 | Target-TTR-spacer1 | gcatctccccattccatgag |
| 7 | CasY7-TTR-sgRNA1 sequence | CAAGTTGAATCCGTCTATAACTGACGGgcatctccccattccatgag |
| 8 | LbCpf1-TTR-sgRNA1 sequence | TAATTTCTACTAAGTGTAGATGCATCTCCCCATTCCATGAG |
| 9 | CasY7-sgRNA1 expression fragment sequence | CAATTGTAATACGACTCACTATAGGCACCGCAAGTTGAATCCGTCTATAACTGACGG gcatctccccattccatgagTTTTTTAAGCTTGGCGTagaaggccatcctgacggatggccttttACGC GT |
| 10 | LbCpf1-sgRNA1 expression fragment sequence | CAATTGTAATACGACTCACTATAGGCACCGTAATTTCTACTAAGTGTAGATGCATCTC CCCATTCCATGAGTTTTTTAAGCTTGGCGTagaaggccatcctgacggatggcctttt ACGCGT |
| 11 | Target-TTR-spacer2 | tagaagggatatacaaagtg |
| 12 | CasY7-TTR-sgRNA2 | CAAGTTGAATCCGTCTATAACTGACGG tagaagggatatacaaagtg |
| 13 | LbCpf1-TTR-sgRNA2 | TAATTTCTACTAAGTGTAGATtagaagggatatacaaagtg |
| 14 | CasY7-TTR-sgRNA2-Oligo-F | CACCCAAGTTGAATCCGTCTATAACTGACGGtagaagggatatacaaagtg |
| 15 | CasY7-TTR-sgRNA-Oligo-R | AAAACACTTTGTATATCCCTTCTACCGTCAGTTATAGACGGATTCAACTTG |
| 16 | LbCpf1-TTR-sgRNA2-Oligo-F | CACCTAATTTCTACTAAGTGTAGAT tagaagggatatacaaagtg |
| 17 | LbCpf1-TTR-sgRNA2-Oligo-R | AAAACACTTTGTATATCCCTTCTAATCTACACTTAGTAGAAATTA |
| 18 | araC-pBAD-ccdb | TTTGCATCTCCCCATTCCATGAGcATATGCgatcttccccatcggtgatgtcggcgatataggcgcc agcaaccgcacctgtggcgccggtgatgccggccacgatgcgtccggcgtagaggatctgctcatgtttgaca gcttatcatcgatgcataatgtgcctgtcaaatggacgaagcagggattctgcaaaccctatgctactccgtca agccgtcaattgtctgattcgttaccaattatgacaacttgacggctacatcattcactttttcttcacaaccggc acggaactcgctcgggctggccccggtgcattttttaaatacccgcgagaaatagagttgatcgtcaaaaccaa cattgcgaccgacggtggcgataggcatccgggtggtgctcaaaagcagcttcgcctggctgatacgttggtcc tcgcgccagcttaagacgctaatccctaactgctggcggaaaagatgtgacagacgcgacggcgacaagcaa acatgctgtgcgacgctggcgatatcaaaattgctgtctgccaggtgatcgctgatgtactgacaagcctcgcgt acccgattatccatcggtggatggagcgactcgttaatcgcttccatgcgccgcagtaacaattgctcaagcag atttatcgccagcagctccgaatagcgcccttccccttgcccggcgttaatgatttgcccaaacaggtcgctgaa atgcggctggtgcgcttcatccgggcgaaagaaccccgtattggcaaatattgacggccagttaagccattcat gccagtaggcgcgcggacgaaagtaaacccactggtgataccattcgcgagcctccggatgacgaccgtagt gatgaatctctcctggcgggaacagcaaaatatcacccggtcggcaaacaaattctcgtccctgatttttcacc accccctgaccgcgaatggtgagattgagaatataacctttcattcccagcggtcggtcgataaaaaaatcga gataaccgttggcctcaatcggcgttaaacccgccaccagatgggcattaaacgagtatcccggcagcagggg atcattttgcgcttcagccatacttttcatactcccgccattcagagaagaaaccaattgtccatattgcatcaga cattgccgtcactgcgtcttttactggctcttctcgctaaccaaaccggtaaccccgcttattaaaagcattctgt aacaaagcgggaccaaagccatgacaaaaacgcgtaacaaaagtgtctataatcacggcagaaaagtccac attgattatttgcacggcgtcacactttgctatgccatagcatttttatccataagattagcggatcctacctgac gctttttatcgcaactctctactgtttctccatacccgtttttttgggctagcgaattcgagctcatgcagtttaagg tttacacctataaaagagagagccgttatcgtctgtttgtggatgtacagagtgatattattgacacgcccgggc gacggatggtgatccccctggccagtgcacgtctgctgtcagataaagtctcccgtgaactttacccggtggtgc atatcggggatgaaagctggcgcatgatgaccaccgatatggccagtgtgccggtatccgttatcggggaaga agtggctgatctcagccaccgcgaaaatgacatcaaaaacgccattaacctgatgttctggggaatataatcta gagtcgacctgcaggcatgcaagcttggctgttttggcggatgagagaagattttcagcctgatacagattaaa tcagaacgcagaagcggtctgataaaacagaatttgcctggcggcagtagcgcggtggtcccacctgacccca tgccgaactcagaagtgaaacgccgtagcgccgatggtagtgtggggtctccccatgcgagagtagggaactg ccaggcatcaaataaaacgaaaggctcagtcgaaagactgggcctttcgttttatctgttgtttgtcggtgaacg ctctcctgagtaggacaaatccgccgggagcggatttgaacgttgcgaagcaacggcccggagggtggcggg caggacgcccgccataaactgccaggcatcaaattaagcagaaggccatcctgacggatggcctttttgcgttt ctacaaactct |
| 19 | LbCpf1 amino acid sequence | MSKLEKFTNCYSLSKTLRFKAIPVGKTQENIDNKRLLVEDEKRAEDYKGVKKLLDRYYLSFI NDVLHSIKLKNLNNYISLFRKKTRTEKENKELENLEINLRKEIAKAFKGNEGYKSLFKKDIIET ILPEFLDDKDEIALVNSFNGFTTAFTGFFDNRENMFSEEAKSTSIAFRCINENLTRYISNMD |
| | | IFEKVDAIFDKHEVQEIKEKILNSDYDVEDFFEGEFFNFVLTQEGIDVYNAIIGGFVTESGEK IKGLNEYINLYNQKTKQKLPKFKPLYKQVLSDRESLSFYGEGYTSDEEVLEVFRNTLNKNSE IFSSIKKLEKLFKNFDEYSSAGIFVKNGPAISTISKDIFGEWNVIRDKWNAEYDDIHLKKKA VVTEKYEDDRRKSFKKIGSFSLEQLQEYADADLSVVEKLKEIIIQKVDEIYKVYGSSEKLFDA DFVLEKSLKKNDAVVAIMKDLLDSVKSFENYIKAFFGEGKETNRDESFYGDFVLAYDILLK VDHIYDAIRNYVTQKPYSKDKFKLYFQNPQFMGGWDKDKETDYRATILRYGSKYYLAIM DKKYAKCLQKIDKDDVNGNYEKINYKLLPGPNKMLPKVFFSKKWMAYYNPSEDIQKIYK NGTFKKGDMFNLNDCHKLIDFFKDSISRYPKWSNAYDFNFSETEKYKDIAGFYREVEEQ GYKVSFESASKKEVDKLVEEGKLYMFQIYNKDFSDKSHGTPNLHTMYFKLLFDENNHGQ IRLSGGAELFMRRASLKKEELVVHPANSPIANKNPDNPKKTTTLSYDVYKDKRFSEDQYEL HIPIAINKCPKNIFKINTEVRVLLKHDDNPYVIGIDRGERNLLYIVVVDGKGNIVEQYSLNEI INNFNGIRIKTDYHSLLDKKEKERFEARQNWTSIENIKELKAGYISQVVHKICELVEKYDAV IALEDLNSGFKNSRVKVEKQVYQKFEKMLIDKLNYMVDKKSNPCATGGALKGYQITNKF ESFKSMSTQNGFIFYIPAWLTSKIDPSTGFVNLLKTKYTSIADSKKFISSFDRIMYVPEEDLF EFALDYKNFSRTDADYIKKWKLYSYGNRIRIFRNPKKNNVFDWEEVCLTSAYKELFNKYGI NYQQGDIRALLCEQSDKAFYSSFMALMSLMLQMRNSITGRTDVDFLISPVKNSDGIFYD SRNYEAQENAILPKNADANGAYNIARKVLWAIGQFKKAEDEKLDKVKIAISNKEWLEYA QTSVKHGS |
| 20 | LbCpf1 nucleotide sequence | atgtcaaagctggagaaattcaccaactgttatagcctgtctaagaccctgcgcttcaaggcaatcccagtggg caagacacaagagaacattgacaacaaacggctcctggtggaggatgagaagagggctgaagattacaagg gcgttaagaagctgctggataggtactatctgtcattcatcaacgatgtcctccacagtatcaagctgaagaatc tgaacaattacatttctctgttccggaagaagacacggaccgagaaggagaacaaagagctggagaatctgg agatcaacctgaggaaagaaatagctaaggctttcaaagggaacgagggttacaagtccctgttcaagaaag acattatcgagactattctgcctgagttcctggacgataaagatgagatcgccctcgtcaattccttcaatgggtt taccacagcctttaccggcttcttcgacaatagagagaatatgttctctgaagaggccaaatccactagcatcg cctttcgctgcataaacgagaacctgactaggtacatcagcaatatggacatctttgagaaagtcgatgccata ttcgacaaacatgaggtgcaggagattaaggagaagatcctgaactcagattacgatgtcgaagatttcttcg agggagagttcttcaacttcgtgctcacacaagagggcattgatgtgtacaatgcaatcattggagggttcgtg acagagagtggcgagaagataaagggcctgaacgagtatatcaacctctacaaccagaaaaccaagcagaa actgcctaagttcaagccactgtacaaacaagtgctctcagatagggaaagcctgagcttctacggtgaaggg tatacatcagatgaagaagtgctcgaagtgttccgcaacaccctcaataagaacagtgaaatcttctcttcaat caagaagctggagaaactgttcaagaatttcgatgagtactcctctgccggaatctttgtgaagaatggccctg caatatccactattagcaaagacatctttggcgagtggaacgttatcagggataagtggaatgccgagtacga tgatattcatctcaagaagaaagccgtggttacagagaaatacgaggatgatagacgcaagagctttaagaa |
| | | gattggtagcttctctctcgaacagctgcaggagtacgccgacgctgacctgtcagtcgtggagaaactcaagg agatcataatccagaaggtggatgaaatctacaaagtgtatggaagctctgagaaactcttcgatgcagacttt gttctggagaagagtctgaagaagaacgacgcagtggttgctatcatgaaggacctgctggattctgttaagtc tttcgagaattacattaaggcattctttggtgaagggaaggagacaaatagggacgagagcttctatggcgac tttgttctggcctacgacatcctcctcaaggttgaccacatctatgacgctatacggaattacgttacccagaag ccctatagcaaagacaagttcaagctgtatttccagaatccacagtttatgggtgggtgggataaagacaaag aaacagattacagggccactatcctgcggtacggcagcaaatactatctggctatcatggataagaagtacgc caaatgcctccagaagatcgacaaggacgacgtgaacggtaactacgagaagatcaattacaagctcctgcc aggacctaacaagatgctgcccaaggtgttcttctccaagaaatggatggcctactataacccaagcgaggac attcagaagatatacaagaatgggacattcaagaagggcgatatgttcaacctcaacgactgccacaagctg attgatttcttcaaggatagcatttctcgctatcccaagtggtctaatgcatacgatttcaacttcagcgagactg agaagtacaaagacatcgctggcttctaccgggaggtggaagagcaaggctataaggtgtcattcgaatccgc ttctaagaaggaagtggataagctcgtggaagagggtaagctgtacatgttccagatatacaacaaagacttc agcgataagagccacggcactccaaacctccatactatgtatttcaagctgctgtttgacgagaacaaccacg gacagattaggctgtcaggaggcgcagaactcttcatgcgcagagcttcactgaagaaggaggaactcgttgt ccacccagccaatagccctatagccaataagaatccagacaatcctaagaaaaccactactctgtcttacgat gtgtataaggataagagattctctgaagatcagtacgaactgcacatacccattgccattaacaagtgccctaa gaacatcttcaagattaacacagaggttagagtgctcctgaaacacgacgataacccttatgttataggcattg atcgcggagagagaaacctgctgtacatcgtcgtggtggacggcaaaggcaacatcgtggaacagtacagtc tcaatgaaatcattaacaatttcaacggaatccgcattaagaccgactaccattctctcctcgacaagaaggag aaagaaaggttcgaagcaagacagaattggacaagtatagagaatatcaaagaactgaaggctgggtacat ctctcaggttgtgcacaagatatgtgagctggtggagaagtacgacgctgttatcgccctcgaggacctgaata gcggcttcaagaactccagggtgaaggtggagaagcaggtgtatcagaagttcgagaagatgctgatcgaca agctcaactatatggtggacaagaaatccaatccttgcgctactggtggagccctgaagggctatcaaatcacc aataagttcgaatctttcaagtctatgagcacccagaatggcttcatcttctacatacccgcatggctgacatcc aagattgatccctctaccggatttgttaatctgctcaagactaagtacacctctattgctgactcaaagaagttc atatcatcatttgaccgcatcatgtacgtgccagaagaggacctgttcgagtttgccctggattacaagaatttc tctcggactgacgccgactacatcaagaagtggaagctctactcttatggtaatcggattcgcatattccgcaat cccaagaagaataacgtgttcgattgggaggaagtttgcctcaccagcgcttacaaggagctgttcaataagt atgggattaactaccagcagggcgacataagagccctgctgtgcgaacaatctgataaggcattctattcctct ttcatggcactgatgtcactgatgctgcaaatgcgcaattccatcaccggaagaacagacgtggactttctgat ctctcctgtcaagaactcagatggcatcttctacgattcccgcaactatgaagcacaggagaatgctatcctgcc taagaatgccgatgcaaatggagcctataacatcgccagaaaggtcctctgggccataggacaattcaagaa |
| | | agctgaagatgagaagctggacaaggtgaagatcgccatttcaaacaaagagtggctcgaatatgctcagac ctcagtgaagcatggatca |
| 21 | TTR-F | TCGTCGGCAGCGTCAGATGTGTATAAGAGACAGatccagactttcacacctta |
| 22 | TTR-R | GTCTCGTGGGCTCGGAGATGTGTATAAGAGACAGtcatggaatggggagatgcc |
| 23 | CasY7-TTR-sgRNA3 | CAAGTTGAATCCGTCTATAACTGACGGtatatcccttctacaaattc |
| 24 | CasY7-TTR-sgRNA4 | CAAGTTGAATCCGTCTATAACTGACGGgtgtctatttccactttgta |
| 25 | CasY7-TTR-sgRNA3-Oligo-F | CACCCAAGTTGAATCCGTCTATAACTGACGGtatatcccttctacaaattc |
| 26 | CasY7-TTR-sgRNA3-Oligo-R | AAAAgaatttgtagaagggatataCCGTCAGTTATAGACGGATTCAACTTG |
| 27 | CasY7-TTR-sgRNA4-Oligo-F | CACCCAAGTTGAATCCGTCTATAACTGACGGgtgtctatttccactttgta |
| 28 | CasY7-TTR-sgRNA4-Oligo-R | AAAAtacaaagtggaaatagacacCCGTCAGTTATAGACGGATTCAACTTG |
| 29 | TTR-F' | Gggtgtattactttgccatg |
| 30 | TTR-R' | Aa cctttggtcattcatca ccttc |
| 31 | 005V1 amino acid sequence | MSELNDAYWMKQALALAQKAREQGEVPVGAILVLDDEVIGQGWNRAITLHDPTAHA EIMALQQGGQIVQNYRLLNATLYVTFEPCVMCAGAMVHSRIKRLVYGVSNSKRGAAGS LLNVLNYPGMNHQIEITAGVMANECSEMLCQFYQQPREVFNAEREARRLNQPDRAD |
| 32 | 005V1-10-3 amino acid sequence | MSELNDAYWMKQALALAQKAREQGEVPVGAILVLDDEVIGQGWNRAITLHDPTAHA EIMALQQGGQIVQNYRLLNATLYVTFEPCVMCAGAMVHSRIKRLVYGVSNSKRGAAGS LLNVLNYPGMNHQIEITAGVMANECSEMLCQFYGMRREVFNAEREARRLNQPDRAD |
| 33 | 005V1-10-3 nucleotide sequence | ATGAGTGAGCTGAATGATGCTTACTGGATGAAACAGGCACTCGCTTTAGCTCAGAA GGCCcggGAACAGGGAGAAGTTCCAGTGGGCGCTATTCTGGTGctgGATGATGAAGT GATAGGACAGGGATGGAATAGAgccATCACCctgCACGACCCCACCGCCCACGCCGA GATCATGGCCCTGCAGCAGGGCGGCCAGATCGTGCAGAACTACCGGCTGCTGAACG CCACCCTGTACGTGACCttcGAGCCCTGCGTGATGTGCGCCGGCGCCATGGTGCACA |
| | | GCAGGATCAAGAGACTGGTCTACGGCgtgAGCaactcaAAAagaGGCGCCGCCGGCAG CCTGCTGaacGTGCTGaactacCCCGGCATGAACCACCAGATCGAGATCACCGCCGGC GTGATGGCCAACGAGTGCAGCGAGATGCTGtgcCAGTTCtatGGTATGCGCAGAGAA gtgttcaatGCTGAGCGTGAGGCTAGGCGGCTGAACCAACCTGATAGAGCTGAC |
| 34 | nucleotide sequence of 005V1-10-3 with homologous arm and linker | ccaaagaagaagcggaaagtcATGAGTGAGCTGAATGATGCTTACTGGATGAAACAGGC ACTCGCTTTAGCTCAGAAGGCCcggGAACAGGGAGAAGTTCCAGTGGGCGCTATTCT GGTGctgGATGATGAAGTGATAGGACAGGGATGGAATAGAgccATCACCctgCACGA CCCCACCGCCCACGCCGAGATCATGGCCCTGCAGCAGGGCGGCCAGATCGTGCAGA ACTACCGGCTGCTGAACGCCACCCTGTACGTGACCttcGAGCCCTGCGTGATGTGCGC CGGCGCCATGGTGCACAGCAGGATCAAGAGACTGGTCTACGGCgtgAGCaactcaAAA agaGGCGCCGCCGGCAGCCTGCTGaacGTGCTGaactacCCCGGCATGAACCACCAGA TCGAGATCACCGCCGGCGTGATGGCCAACGAGTGCAGCGAGATGCTGtgcCAGTTCt atGGTATGCGCAGAGAAgtgttcaatGCTGAGCGTGAGGCTAGGCGGCTGAACCAACC TGATAGAGCTGACtccggaggatctagcggaggctcctctggctctgagacacctggcacaagcgagag cgcaacacctgaaagcagcgggggcagcagcggggggtcaGCCACGAAGACCATCGTGAG |
| 35 | D992A-dCasY7-F | GCCACGAAGACCATCGTGAG |
| 36 | D992A-dCasY7-R | Gactttccgcttcttctttgg |
| 37 | 005V1-10-3-D992A-dCasY7 nucleotide sequence | atgaaacggacagccgacggaagcgagttcgagtcaccaaagaagaagcggaaagtcATGAGTGAGC TGAATGATGCTTACTGGATGAAACAGGCACTCGCTTTAGCTCAGAAGGCCcggGAAC AGGGAGAAGTTCCAGTGGGCGCTATTCTGGTGctgGATGATGAAGTGATAGGACAG GGATGGAATAGAgccATCACCctgCACGACCCCACCGCCCACGCCGAGATCATGGCCC TGCAGCAGGGCGGCCAGATCGTGCAGAACTACCGGCTGCTGAACGCCACCCTGTAC GTGACCttcGAGCCCTGCGTGATGTGCGCCGGCGCCATGGTGCACAGCAGGATCAAG AGACTGGTCTACGGCgtgAGCaactcaAAAagaGGCGCCGCCGGCAGCCTGCTGaacGT GCTGaactacCCCGGCATGAACCACCAGATCGAGATCACCGCCGGCGTGATGGCCAA CGAGTGCAGCGAGATGCTGtgcCAGTTCtatGGTATGCGCAGAGAAgtgttcaatGCTGA GCGTGAGGCTAGGCGGCTGAACCAACCTGATAGAGCTGACtccggaggatctagcggagg ctcctctggctctgagacacctggcacaagcgagagcgcaacacctgaaagcagcgggggcagcagcgggg ggtcaGCCACGAAGACCATCGTGAGGCCATACACCAGCAACCTGTCCCCTAACGCCGC CAAGAAGGCCATGCTGGACGAGAGCTTCAAGTTCTTCGACCACGCCTACACCGTGTT CTTCAGCGTGTTCATCAAGCTGTGGGGCGGCGTGAAGCCCACCCAGGTGGCCCTGG TGGAGAACGACACCAACAAGATCGACGCCATCTGCAGCATCCTGTGGTTCAGGCTG |
| | | CAGACCAAGACCGACAGCACCAACATCACCCTCCAGTCCGCCGAGGAGAGGATCCG CCGCTTCAAGGAGTACGCCCAGCACGACCCTAGCCCCCTGGCCCTCAGCTACCTGAC GGGCAACCTGGACCCCGAGAAGCACGAGTGGGTCGACTGCCGCGAGCTGTACCAG AACTGGTGCGCCGAGCTGAAGTGCGACCTCGCCACCGACATCGAAACCATGATCAA CCACAACCTGCTGCCTATCAGCGCGAAGCAGGAGTACAACTGCTACAGCTCGTTCAG CAACCTGTTCGGCGAGGCCGAGAAGGAGAACAAGGCCGAGAAGGTGGAGTGGTAC AGAAGCGTGGTGGCCGCGCTGAAGTCCAAGAGCGCCTGGAACTGGGACGAGTACA GGAAGATGCTGTTCGAGAAGATCGGCTGCACCGCCCCCAAGGGCGGCGGCAGACT GAGCCTCCTGTTCCTGGACATGAGGTCCGACACCACCGGCCCCATCAGCTCCCAGGA GATCACCAAGCGGATCACCAGCTTCGAGAAGGTGGCCGAGGAGAAGAGCAAGACC TACGAGCTCCCCCACAGGCTGACCGTGAAGCAGTACATCTGCAACCAGCTGGCCCAC CCCTACGACCTGGCCATCTGGAGCGCCGTGGCCAACTACGCCATCTCCAGCATCAGG AGCAAGAACAGCATCAATATGCTGTACAGCGACGAGAAGCTCAACAGGGAAACCAT CATCGCCAAGATCAAGAGCCAGAACCCCAACATCGAGCAGGTGCAGGAGATCCTGT CCCAGTTCAGGGCCGGCGAGAACAACGCGTTCGTGATCGAGCCCAGACACCTGTCC AACCTGAAGAGCCTGTTCAGGTTCTGGTCCAACAGCAGCATGGACGAGGGCATCAC CACCCTGCTGCAGATCCCTCAGAGCGAGTACGAGCGCAGCCCCATCGTGGACCTGCT CCGGCACATCCACCCCCACAGGAACAAGTTCACCGCCGAGCAGTTCATCGTGGCCGC CGAGCTGAACGGCATCGAGTTCGACAACGCGATGAAGAAGATCCACCCCACCGTGT ACGGCAAGACCACCGTGGACTACGGCCCCAAGAGCACCATCTACGGCAGCATCACC CCACCAAGCAAGCTGATCCGCGGCGTGCACGCCGGCAGCCACGGCATGATGTGGGT GACCATGACCCTGCTGGACAACGGCAAGTGGATCAAGCACCACATCCCCTTCCACAA CAGCAGGTACTACGAGGAGATGTACGCCTACAGAGAGGGCCTGCCGGTCGGCGAG AAGCCCAGGAGGCCGGTGCTGGGCAACCGGGTGGGCAACCACATCGACAACACCG GCCTGATCAATAAGAAGTGCAGGAAGGCCAGCAAGGCCTACCTGAGGACCATCCAG AACATGAATCACAACGTGTGGTTCGACCCCGACACCCACTTCCTGGTGCAGAGGAAC GGCGACAACTTCGCCATCCAGATCTCCTCCAGGATCAAGGAGAGCAAGCCCAAGTC CAACGTGGAGGTGGGCGACCGCATCCTGGGCATGGACCAGAACCAGACGGCCAGC AACACCTACAGCGTGGTGGAGGTGGTGCGCGAGGGCACCGAGAATAGCCACCCCT ACAACAACTACTTTGTCAAGGTGATCGAGGACGGCAACGTGACCAGCTGCACCACC AAGAATGACAGGGGCGAGTTCGACCAGCTGAGCTACGAGGGCCTGGCCTACAGCG AGTTCGAGATCTGGCGGCAGGCCAGGATCGCCTTCCTGAGCGCCCACGACCCCGAG CTGGCCCAGAAGATGATCGACAAGACCAACGAGAGCCTGTACAAGTGGAACAACAA CTACGCCTACTACCTGAAGGTGTGCATGAGGAACAAGATGAACGGCGACAACCACG |
| | | CCCTGTTCCGGAACGAGATCAAGGAGTTCATCGAGGGCATGCCCGTGGCGGGCAAG AACGAGAAGCACAAGTTCTTCGGCTCCATCAGGGGCAGCCTGTCCCTGGAGAGCCT GGAGGGCCTGAGCAAGTCCCGCAGCCTGATCAGCTGCTACTTCTACCTGCTCGAGA AGAAGGAGATCGAGCAGCAGAAGGAGTTCGACAGCGATCTGTTCAAGCTGGGCGA GTGCCTGAGCGAGAAGAGAGTGAACAAGCGGGAGGAGAGAGCCAACCGGATCGT GAGCTCGGTGCTGCAGATCTGCAGCCGGCTGAACGTGAGCAGGATCGTGATCGAG AACAAGCTGCCCACCGCCAACCACGAGAACAAGAGCAGCGCCAACCGCAGAGCCAC CGACTGGTGCCCCAGGAAGGTGCAGCAGAAGCTGCTGGACGCGGTGAAGATGGTG GGGATCAAGGTGCTGGCCGTGAAGCCCTACAACACCAGCCACATCGACCCCTTCGT GAACGGCGAGAGCAACAGACAGGCCCTGGAGGCCAGGTTCATGGACGTGGACGTG AAGGACATCACCGACAGAAACATCAAGCAGTTCAAGAAGATGCACAGAAACCAGGT GGGGGTGCTGAACTCCATCTACCACAACGCCCTGCGGTCCTTCGCCGCCAACTACGG CCTGAATTGGAACGAGCTGCCGAACATGAATCTGGAGCAGATCAAGAACGCCCTGA AGGACCACGTGAGGGTGATGTTCCCTCAGTGGGGCGGCCGGAGCTTCATGAGCACC CACAACGTGACCCGGAACAGCGTGAGAGTGAGCTACAACAACAGGACCCGCTGGCT GAACTTCAGCgcaGTGATCGCCGCCCTCAACATCGCCCTCAGGGGCAGCGGCAACTA CGAGCCCAAGGGGGACAGCCAGAACACCGCCCCCAGCAGAAACAGCtctggcggctca aaaagaaccgccgacggcagcgaattcgagcccaagaagaagaggaaagtctaa |
| 38 | NLS | KRTADGSEFESPKKKRKV |
| 39 | NLS | AVKRPAATKKAGQAKKKKLD |
| 40 | NLS | KRPAATKKAGQAKKKK |
| 41 | NLS | KKTELQTTNAENKTKKL |
| 42 | NLS | KRGINDRNFWRGENGRKTR |
| 43 | NLS | RKSGKIAAIVVKRPRK |
| 44 | NLS | PKKKRKV |
| 45 | NLS | MDSLLMNRRKFLYQFKNVRWAKGRRETYLC |
| 46 | 005V1-10-3-dCasY7 fusion protein | MKRTADGSEFESPKKKRKVMSELNDAYWMKQALALAQKAREQGEVPVGAILVLDDEV IGQGWNRAITLHDPTAHAEIMALQQGGQIVQNYRLLNATLYVTFEPCVMCAGAMVHS RIKRLVYGVSNSKRGAAGSLLNVLNYPGMNHQIEITAGVMANECSEMLCQFYGMRREV FNAEREARRLNQPDRADSGGSSGGSSGSETPGTSESATPESSGGSSGGSATKTIVRPYTS NLSPNAAKKAMLDESFKFFDHAYTVFFSVFIKLWGGVKPTQVALVENDTNKIDAICSILW FRLQTKTDSTNITLQSAEERIRRFKEYAQHDPSPLALSYLTGNLDPEKHEWVDCRELYQN WCAELKCDLATDIETMINHNLLPISAKQEYNCYSSFSNLFGEAEKENKAEKVEWYRSVVA ALKSKSAWNWDEYRKMLFEKIGCTAPKGGGRLSLLFLDMRSDTTGPISSQEITKRITSFEK |
| | | VAEEKSKTYELPHRLTVKQYICNQLAHPYDLAIWSAVANYAISSIRSKNSINMLYSDEKLN RETIIAKIKSQNPNIEQVQEILSQFRAGENNAFVIEPRHLSNLKSLFRFWSNSSMDEGITTL LQIPQSEYERSPIVDLLRHIHPHRNKFTAEQFIVAAELNGIEFDNAMKKIHPTVYGKTTVD YGPKSTIYGSITPPSKLIRGVHAGSHGMMWVTMTLLDNGKWIKHHIPFHNSRYYEEMY AYREGLPVGEKPRRPVLGNRVGNHIDNTGLINKKCRKASKAYLRTIQNMNHNVWFDPD THFLVQRNGDNFAIQISSRIKESKPKSNVEVGDRILGMDQNQTASNTYSVVEVVREGTE NSHPYNNYFVKVIEDGNVTSCTTKNDRGEFDQLSYEGLAYSEFEIWRQARIAFLSAHDPE LAQKMIDKTNESLYKWNNNYAYYLKVCMRNKMNGDNHALFRNEIKEFIEGMPVAGK NEKHKFFGSIRGSLSLESLEGLSKSRSLISCYFYLLEKKEIEQQKEFDSDLFKLGECLSEKRVN KREERANRIVSSVLQICSRLNVSRIVIENKLPTANHENKSSANRRATDWCPRKVQQKLLD AVKMVGIKVLAVKPYNTSHIDPFVNGESNRQALEARFMDVDVKDITDRNIKQFKKMHR NQVGVLNSIYHNALRSFAANYGLNWNELPNMNLEQIKNALKDHVRVMFPQWGGRSF MSTHNVTRNSVRVSYNNRTRWLNFSAVIAALNIALRGSGNYEPKGDSQNTAPSRNSSG GSKRTADGSEFEPKKKRKV |
| 47 | D592A-dCasY7 | MATKTIVRPYTSNLSPNAAKKAMLDESFKFFDHAYTVFFSVFIKLWGGVKPTQVALVEN DTNKIDAICSILWFRLQTKTDSTNITLQSAEERIRRFKEYAQHDPSPLALSYLTGNLDPEKH EWVDCRELYQNWCAELKCDLATDIETMINHNLLPISAKQEYNCYSSFSNLFGEAEKENK AEKVEWYRSVVAALKSKSAWNWDEYRKMLFEKIGCTAPKGGGRLSLLFLDMRSDTTGP ISSQEITKRITSFEKVAEEKSKTYELPHRLTVKQYICNQLAHPYDLAIWSAVANYAISSIRSK NSINMLYSDEKLNRETIIAKIKSQNPNIEQVQEILSQFRAGENNAFVIEPRHLSNLKSLFRF WSNSSMDEGITTLLQIPQSEYERSPIVDLLRHIHPHRNKFTAEQFIVAAELNGIEFDNAM KKIHPTVYGKTTVDYGPKSTIYGSITPPSKLIRGVHAGSHGMMWVTMTLLDNGKWIKH HIPFHNSRYYEEMYAYREGLPVGEKPRRPVLGNRVGNHIDNTGLINKKCRKASKAYLRTI QNMNHNVWFDPDTHFLVQRNGDNFAIQISSRIKESKPKSNVEVGDRILGMAQNQTAS NTYSVVEVVREGTENSHPYNNYFVKVIEDGNVTSCTTKNDRGEFDQLSYEGLAYSEFEIW RQARIAFLSAHDPELAQKMIDKTNESLYKWNNNYAYYLKVCMRNKMNGDNHALFRNE IKEFIEGMPVAGKNEKHKFFGSIRGSLSLESLEGLSKSRSLISCYFYLLEKKEIEQQKEFDSDL FKLGECLSEKRVNKREERANRIVSSVLQICSRLNVSRIVIENKLPTANHENKSSANRRATD WCPRKVQQKLLDAVKMVGIKVLAVKPYNTSHIDPFVNGESNRQALEARFMDVDVKDIT DRNIKQFKKMHRNQVGVLNSIYHNALRSFAANYGLNWNELPNMNLEQIKNALKDHVR VMFPQWGGRSFMSTHNVTRNSVRVSYNNRTRWLNFSDVIAALNIALRGSGNYEPKGD SQNTAPSRNS |
| 48 | D643A-dCasY7 | MATKTIVRPYTSNLSPNAAKKAMLDESFKFFDHAYTVFFSVFIKLWGGVKPTQVALVEN DTNKIDAICSILWFRLQTKTDSTNITLQSAEERIRRFKEYAQHDPSPLALSYLTGNLDPEKH |
| | | EWVDCRELYQNWCAELKCDLATDIETMINHNLLPISAKQEYNCYSSFSNLFGEAEKENK AEKVEWYRSVVAALKSKSAWNWDEYRKMLFEKIGCTAPKGGGRLSLLFLDMRSDTTGP ISSQEITKRITSFEKVAEEKSKTYELPHRLTVKQYICNQLAHPYDLAIWSAVANYAISSIRSK NSINMLYSDEKLNRETIIAKIKSQNPNIEQVQEILSQFRAGENNAFVIEPRHLSNLKSLFRF WSNSSMDEGITTLLQIPQSEYERSPIVDLLRHIHPHRNKFTAEQFIVAAELNGIEFDNAM KKIHPTVYGKTTVDYGPKSTIYGSITPPSKLIRGVHAGSHGMMWVTMTLLDNGKWIKH HIPFHNSRYYEEMYAYREGLPVGEKPRRPVLGNRVGNHIDNTGLINKKCRKASKAYLRTI QNMNHNVWFDPDTHFLVQRNGDNFAIQISSRIKESKPKSNVEVGDRILGMDQNQTAS NTYSVVEVVREGTENSHPYNNYFVKVIEDGNVTSCTTKNDRGEFAQLSYEGLAYSEFEIW RQARIAFLSAHDPELAQKMIDKTNESLYKWNNNYAYYLKVCMRNKMNGDNHALFRNE IKEFIEGMPVAGKNEKHKFFGSIRGSLSLESLEGLSKSRSLISCYFYLLEKKEIEQQKEFDSDL FKLGECLSEKRVNKREERANRIVSSVLQICSRLNVSRIVIENKLPTANHENKSSANRRATD WCPRKVQQKLLDAVKMVGIKVLAVKPYNTSHIDPFVNGESNRQALEARFMDVDVKDIT DRNIKQFKKMHRNQVGVLNSIYHNALRSFAANYGLNWNELPNMNLEQIKNALKDHVR VMFPQWGGRSFMSTHNVTRNSVRVSYNNRTRWLNFSDVIAALNIALRGSGNYEPKGD SQNTAPSRNS |
| 49 | E820A-dCasY7 | MATKTIVRPYTSNLSPNAAKKAMLDESFKFFDHAYTVFFSVFIKLWGGVKPTQVALVEN DTNKIDAICSILWFRLQTKTDSTNITLQSAEERIRRFKEYAQHDPSPLALSYLTGNLDPEKH EWVDCRELYQNWCAELKCDLATDIETMINHNLLPISAKQEYNCYSSFSNLFGEAEKENK AEKVEWYRSVVAALKSKSAWNWDEYRKMLFEKIGCTAPKGGGRLSLLFLDMRSDTTGP ISSQEITKRITSFEKVAEEKSKTYELPHRLTVKQYICNQLAHPYDLAIWSAVANYAISSIRSK NSINMLYSDEKLNRETIIAKIKSQNPNIEQVQEILSQFRAGENNAFVIEPRHLSNLKSLFRF WSNSSMDEGITTLLQIPQSEYERSPIVDLLRHIHPHRNKFTAEQFIVAAELNGIEFDNAM KKIHPTVYGKTTVDYGPKSTIYGSITPPSKLIRGVHAGSHGMMWVTMTLLDNGKWIKH HIPFHNSRYYEEMYAYREGLPVGEKPRRPVLGNRVGNHIDNTGLINKKCRKASKAYLRTI QNMNHNVWFDPDTHFLVQRNGDNFAIQISSRIKESKPKSNVEVGDRILGMDQNQTAS NTYSVVEVVREGTENSHPYNNYFVKVIEDGNVTSCTTKNDRGEFDQLSYEGLAYSEFEIW RQARIAFLSAHDPELAQKMIDKTNESLYKWNNNYAYYLKVCMRNKMNGDNHALFRNE IKEFIEGMPVAGKNEKHKFFGSIRGSLSLESLEGLSKSRSLISCYFYLLEKKEIEQQKEFDSDL FKLGECLSEKRVNKREERANRIVSSVLQICSRLNVSRIVIANKLPTANHENKSSANRRATD WCPRKVQQKLLDAVKMVGIKVLAVKPYNTSHIDPFVNGESNRQALEARFMDVDVKDIT DRNIKQFKKMHRNQVGVLNSIYHNALRSFAANYGLNWNELPNMNLEQIKNALKDHVR VMFPQWGGRSFMSTHNVTRNSVRVSYNNRTRWLNFSDVIAALNIALRGSGNYEPKGD SQNTAPSRNS |
| 50 | D992A-dCasY7 | MATKTIVRPYTSNLSPNAAKKAMLDESFKFFDHAYTVFFSVFIKLWGGVKPTQVALVEN DTNKIDAICSILWFRLQTKTDSTNITLQSAEERIRRFKEYAQHDPSPLALSYLTGNLDPEKH EWVDCRELYQNWCAELKCDLATDIETMINHNLLPISAKQEYNCYSSFSNLFGEAEKENK AEKVEWYRSVVAALKSKSAWNWDEYRKMLFEKIGCTAPKGGGRLSLLFLDMRSDTTGP ISSQEITKRITSFEKVAEEKSKTYELPHRLTVKQYICNQLAHPYDLAIWSAVANYAISSIRSK NSINMLYSDEKLNRETIIAKIKSQNPNIEQVQEILSQFRAGENNAFVIEPRHLSNLKSLFRF WSNSSMDEGITTLLQIPQSEYERSPIVDLLRHIHPHRNKFTAEQFIVAAELNGIEFDNAM KKIHPTVYGKTTVDYGPKSTIYGSITPPSKLIRGVHAGSHGMMWVTMTLLDNGKWIKH HIPFHNSRYYEEMYAYREGLPVGEKPRRPVLGNRVGNHIDNTGLINKKCRKASKAYLRTI QNMNHNVWFDPDTHFLVQRNGDNFAIQISSRIKESKPKSNVEVGDRILGMDQNQTAS NTYSVVEVVREGTENSHPYNNYFVKVIEDGNVTSCTTKNDRGEFDQLSYEGLAYSEFEIW RQARIAFLSAHDPELAQKMIDKTNESLYKWNNNYAYYLKVCMRNKMNGDNHALFRNE IKEFIEGMPVAGKNEKHKFFGSIRGSLSLESLEGLSKSRSLISCYFYLLEKKEIEQQKEFDSDL FKLGECLSEKRVNKREERANRIVSSVLQICSRLNVSRIVIENKLPTANHENKSSANRRATD WCPRKVQQKLLDAVKMVGIKVLAVKPYNTSHIDPFVNGESNRQALEARFMDVDVKDIT DRNIKQFKKMHRNQVGVLNSIYHNALRSFAANYGLNWNELPNMNLEQIKNALKDHVR VMFPQWGGRSFMSTHNVTRNSVRVSYNNRTRWLNFSAVIAALNIALRGSGNYEPKGD SQNTAPSRNS |
| 51 | Trac-R2 | TGAAGCAAGGAAACAGCCTG |
| 52 | NT-sgRNA targeting sequence | TTCCGCGGCCACGGTAATGA |
| 53 | Target-PD-1-spacer | tagcaccgcccagacgactggc |
| 54 | CasY7-PD-1-sgRNA sequence | CAAGTTGAATCCGTCTATAACTGACGGtagcaccgcccagacgactggc |
| 55 | Target-Trac-spacer1 | gagtctctcagctggtacac |
| 56 | CasY7-Trac-sgRNA1 sequence | CAAGTTGAATCCGTCTATAACTGACGGgagtctctcagctggtacac |
| 57 | Target-Trac-spacer2 | catgtgcaaacgccttcaac |
| 58 | CasY7-Trac-sgRNA2 sequence | CAAGTTGAATCCGTCTATAACTGACGGcatgtgcaaacgccttcaac |
| 59 | Target-Trac-spacer3 | ttgctccaggccacagcact |
| 60 | CasY7-Trac-sgRNA3 sequence | CAAGTTGAATCCGTCTATAACTGACGGttgctccaggccacagcact |
| 61 | Target-Trac-spacer4 | tctgtgatatacacatcaga |
| 62 | CasY7-Trac-sgRNA4 sequence | CAAGTTGAATCCGTCTATAACTGACGGtctgtgatatacacatcaga |
| 63 | TTR-F1 | atccagactttcacacctta |
| 64 | TTR-R1 | caaaagcaaaaaccaaaacc |
| 65 | PD-1-F | acagtttcccttccgctcac |
| 66 | PD-1-R | agggactgagggtggaaggt |
| 67 | Trac-F1 | CCCTGATCCTCTTGTCCCAC |
| 68 | Trac-R1 | CAGATTTGTTGCTCCAGGCC |
| 69 | Trac-F2 | GTCACAAAGTAAGGATTCTG |
| 70 | Deaminase 004V1 amino acid sequence | MYNAPRFFCRSAAVSDHEFNDEYWMRHALTLAKRAREEGEVPVGAVLVLNNQVIGEG WNRAIGLHDPTAHAEIMALRQGGLVLQNYRLIDATLYVTFEPCVMCAGAMVHSRISRL VFGVRNSKRGAAGSLINVLNYPGMNHRVEITEGILAESCSAMLCDFYRWPREVFNALKK ARQEEG |
| 71 | DR-1 sequence | AGAAATCCGTCCAAAGCTGACGG |
| 72 | DR-1-TTR-sgRNA | AGAAATCCGTCCAAAGCTGACGGtagaagggatatacaaagtg |
| 73 | TTR-LUxUC sequence | ATGGGATCCCAGTCCGGCgtcttcacactcgaagatttcgttggggactggcgacagacagccggcta caacctggaccaagtccttgaacagggaggtgtgtccagtttgtttcagaatcttttgtagaagggatatacaa agtgcGGgtgacggctacaacctggaccaagtccttgaacagggaggtgtgtccagtttgtttcagaatctcg gggtgtccgtaactccgatccaaaggattgtcctgagcggtgaaaatgggctgaagatcgacatccatgtcatc atcccgtatgaaggtctgagcggcgaccaaatgggccagatcgaaaaaatttttaaggtggtgtaccctgtgg atgatcatcactttaaggtgatcctgcactatggcacactggtaatcgacggggttacgccgaacatgatcgac |
| | | tatttcggacggccgtatgaaggcatcgccgtgttcgacggcaaaaagatcactgtaacagggaccctgtgga acggcaacaaaattatcgacgagcgcctgatcaaccccgacggctccctgctgttccgagtaaccatcaacgg agtgaccggctggcggctgtgcgaacgcattctggcgtaa |
| 74 | PD-1'-LUxUC sequence | tatttcggacggccgtatgaaggcatcgccgtgttcgacggcaaaaagatcactgtaacagggaccctgtgga acggcaacaaaattatcgacgagcgcctgatcaaccccgacggctccctgctgttccgagtaaccatcaacgg agtgaccggctggcggctgtgcgaacgcattctggcgtaa ATGGGATCCCAGTCCGGCgtcttcacactcgaagatttcgttggggactggcgacagacagccggcta caacctggaccaagtccttgaacagggaggtgtgtccagtttgtttcagaatcttttagcacgaagctctccgat gtgcGGgtgacggctacaacctggaccaagtccttgaacagggaggtgtgtccagtttgtttcagaatctcgg ggtgtccgtaactccgatccaaaggattgtcctgagcggtgaaaatgggctgaagatcgacatccatgtcatca tcccgtatgaaggtctgagcggcgaccaaatgggccagatcgaaaaaatttttaaggtggtgtaccctgtggat gatcatcactttaaggtgatcctgcactatggcacactggtaatcgacggggttacgccgaacatgatcgacta tttcggacggccgtatgaaggcatcgccgtgttcgacggcaaaaagatcactgtaacagggaccctgtggaac ggcaacaaaattatcgacgagcgcctgatcaaccccgacggctccctgctgttccgagtaaccatcaacggag tgaccggctggcggctgtgcgaacgcattctggcgtaa |
| 75 | PD-1' target sequence | gcacgaagctctccgatgtg |
| 76 | TTR insertion sequence 1 | tttgtagaagggatatacaaagtgcGGgtga |
| 77 | PD-1' inserstion sequence 2 | tttagcacgaagctctccgatgtgcGGgtga |
| 78 | Non-target sequence | ggagacggacgtctct |
| 79 | TadA8e amino acid sequence | MSEVEFSHEYWMRHALTLAKRARDEREVPVGAVLVLNNRVIGEGWNRAIGLHDPTAH AEIMALRQGGLVMQNYRLIDATLYVTFEPCVMCAGAMIHSRIGRVVFGVRNSKRGAA GSLMNVLNYPGMNHRVEITEGILADECAALLCDFYRMPRQVFNAQKKAQSSIN |
| 80 | Stem-loop structure (N is any base of A, T, G, or C) | CCGTCNNNNNNNGACGG |
| 81 | T5 exonuclease amino acid sequence | SKSWGKFIEEEEAEMASRRNLMIVDGTNLGFRFKHNNSKKPFASSYVSTIQSLAKSYSAR TTIVLGDKGKSVFRLEHLPEYKGNRDEKYAQRTEEEKALDEQFFEYLKDAFELCKTTFPTFT IRGVEADDMAAYIVKLIGHLYDHVWLISTDGDWDTLLTDKVSRFSFTTRREYHLRDMYE HHNVDDVEQFISLKAIMGDLGDNIRGVEGIGAKRGYNIIREFGNVLDIIDQLPLPGKQKYI QNLNASEELLFRNLILVDLPTYCVDAIAAVGQDVLDKFTKDILEIAEQ |
| 82 | C05440 amino acid sequence | ATKTIVRPYTSNLSPNAAKKAMLDESFKFFDHAYTVFFSVFIKLWGGVKPTQVALVENDT NKIDAICSILWFRLQTKTDSTNITLQSAEERIRRFKEYAQHDPSPLALSYLTGNLDPEKHE WVDCRELYQNWCAELKCDLATDIETMINHNLLPISAKQEYNCYSSFSNLFGEAEKENKA EKVEWYRSVVAALKSKSAWNWDEYRKMLFEKIGCTAPKGGGRLSLLFLDMRSDTTGPI SSQEITKRITSFEKVAEEKSKTYELPHRLTVKQYICNQLAHPFQLTIWSAVANYAISSIRSKN SINMLYSDEKLNRETIIAKIKSQNPNIEQVQEILSQFRAGENNAFVIEPRHLSNLKSLFRFW SNSSMDEGITTLLQIPQSEYERSPIVDLLRHIHPHRNKFTAEQFIVAAELNSHQTVNAMK KIHPTVYGKTTVDYGPKSTIYGSITPPSKLIRGVHAGSHGMMWVTMTLLDNGKWIKHHI PFHNSRYYEEMYAYREGLPVGEKPRRPVLGNRVGNHIDNTGLINKKCRKASKAYLRTIQ NMNHNVWFDPDTHFLVQRNGDNFAIQISSRIKESKPKSNVEVGDRILGMDQNQTASN TYSVVEVVREGTENSHPYNNYFVKVIEDGNVTSCTTKNDRGEFDQLSYEGLAYSEFEIWR QARIAFLSAHDPELAQKMIDKTNESLYKWNNNYAYYLKVCMRNKMNGDNHALFRNEI KEFIEGMPVAGKNEKHKFFGSIRGSLSLESLEGLSKSRSLISCYFYLLEKKEIEQQKEFDSDLF KLGECLSEKRVNKREERANRIVSSVLQICSRLNVSRIVIENKLPTANHENKSSANRRATDW CPRKVQQKLLDAVKMVGIKVLAVKPYNTSHIDPFVNGESNRQALEARFMDVDVKDITD RNIKQFKKMHRNQVGVLNSIYHNALRSFAANYGLNWNELPNMNLEQIKNALKDHVRV MFPQWGGRSFMSTHNVTRNSVRVSYNNRTRWLNFSDVIAALNIALRGSGNYEPKGDS QNTAPSRNS |
| 83 | C05440 nucleotide coding sequence | GCCACGAAGACCATCGTGAGGCCATACACCAGCAACCTGTCCCCTAACGCCGCCAA GAAGGCCATGCTGGACGAGAGCTTCAAGTTCTTCGACCACGCCTACACCGTGTTCTT CAGCGTGTTCATCAAGCTGTGGGGCGGCGTGAAGCCCACCCAGGTGGCCCTGGTGG AGAACGACACCAACAAGATCGACGCCATCTGCAGCATCCTGTGGTTCAGGCTGCAG ACCAAGACCGACAGCACCAACATCACCCTCCAGTCCGCCGAGGAGAGGATCCGCCG CTTCAAGGAGTACGCCCAGCACGACCCTAGCCCCCTGGCCCTCAGCTACCTGACGGG CAACCTGGACCCCGAGAAGCACGAGTGGGTCGACTGCCGCGAGCTGTACCAGAACT GGTGCGCCGAGCTGAAGTGCGACCTCGCCACCGACATCGAAACCATGATCAACCAC AACCTGCTGCCTATCAGCGCGAAGCAGGAGTACAACTGCTACAGCTCGTTCAGCAAC CTGTTCGGCGAGGCCGAGAAGGAGAACAAGGCCGAGAAGGTGGAGTGGTACAGA AGCGTGGTGGCCGCGCTGAAGTCCAAGAGCGCCTGGAACTGGGACGAGTACAGGA AGATGCTGTTCGAGAAGATCGGCTGCACCGCCCCCAAGGGCGGCGGCAGACTGAG CCTCCTGTTCCTGGACATGAGGTCCGACACCACCGGCCCCATCAGCTCCCAGGAGAT CACCAAGCGGATCACCAGCTTCGAGAAGGTGGCCGAGGAGAAGAGTAAGACCTAC GAGCTCCCCCACAGGCTGACCGTGAAGCAGTACATCTGCAACCAGCTGGCCCACCCC TTTCAACTGACTATTTGGTCTGCGGTTGCAAACTACGCCATCTCCAGCATCAGGAGC |
| | | AAGAACAGCATCAATATGCTGTACAGCGACGAGAAGCTCAACAGGGAAACCATCAT CGCCAAGATCAAGAGCCAGAACCCCAACATCGAGCAGGTGCAGGAGATCCTGTCCC AGTTCAGGGCCGGCGAGAACAACGCGTTCGTGATCGAGCCCAGACACCTGTCCAAC CTGAAGAGTCTGTTCAGGTTCTGGTCCAACAGCAGCATGGACGAGGGCATCACCAC CCTGCTGCAGATCCCTCAGAGCGAGTACGAGCGCAGCCCCATCGTGGACCTGCTCC GGCACATCCACCCCCACAGGAACAAGTTCACCGCCGAGCAGTTCATCGTGGCCGCC GAGCTGAACTCCCATCAGACTGTCAACGCGATGAAGAAGATCCACCCCACCGTGTAC GGCAAGACCACCGTGGACTACGGCCCCAAGAGCACCATCTACGGCAGCATCACCCC ACCAAGCAAGCTGATCCGCGGCGTGCACGCCGGCAGCCACGGCATGATGTGGGTG ACCATGACCCTGCTGGACAACGGCAAGTGGATCAAGCACCACATCCCCTTCCACAAC AGCAGGTACTACGAGGAGATGTACGCCTACAGAGAGGGCCTGCCGGTCGGCGAGA AGCCCAGGAGGCCGGTGCTGGGCAACCGGGTGGGCAACCACATCGACAACACCGG CCTGATCAATAAGAAGTGCAGGAAGGCCAGCAAGGCCTACCTGAGGACCATCCAGA ACATGAATCACAACGTGTGGTTCGACCCCGACACCCACTTCCTGGTGCAGAGGAAC GGCGACAACTTCGCCATCCAGATCTCCTCCAGGATCAAGGAGAGCAAGCCCAAGTC CAACGTGGAGGTGGGCGACCGCATCCTGGGCATGGACCAGAACCAGACGGCCAGC AACACCTACAGCGTGGTGGAGGTGGTGCGCGAGGGCACCGAGAATAGCCACCCCT ACAACAACTACTTTGTCAAGGTGATCGAGGACGGCAACGTGACCAGCTGCACCACC AAGAATGACAGGGGCGAGTTCGACCAGCTGAGCTACGAGGGCCTGGCCTACAGCG AGTTCGAGATCTGGCGGCAGGCCAGGATCGCCTTCCTGAGCGCCCACGACCCCGAG CTGGCCCAGAAGATGATCGACAAGACCAACGAGAGCCTGTACAAGTGGAACAACAA CTACGCCTACTACCTGAAGGTGTGCATGAGGAACAAGATGAACGGCGACAACCACG CCCTGTTCCGGAACGAGATCAAGGAGTTCATCGAGGGCATGCCCGTGGCGGGCAAG AACGAGAAGCACAAGTTCTTCGGCTCCATCAGGGGCAGCCTGTCCCTGGAGAGCCT GGAGGGCCTGAGCAAGTCCCGCAGCCTGATCAGCTGCTACTTCTACCTGCTCGAGA AGAAGGAGATCGAGCAGCAGAAGGAGTTCGACAGCGATCTGTTCAAGCTGGGCGA GTGCCTGAGCGAGAAGAGAGTGAACAAGCGGGAGGAGAGAGCCAACCGGATCGT GAGCTCGGTGCTGCAGATCTGCAGCCGGCTGAACGTGAGCAGGATCGTGATCGAG AACAAGCTGCCCACCGCCAACCACGAGAACAAGAGCAGCGCCAACCGCAGAGCCAC CGACTGGTGCCCCAGGAAGGTGCAGCAGAAGCTGCTGGACGCGGTGAAGATGGTG GGGATCAAGGTGCTGGCCGTGAAGCCCTACAACACCAGCCACATCGACCCCTTCGT GAACGGCGAGAGCAACAGACAGGCCCTGGAGGCCAGGTTCATGGACGTGGACGTG AAGGACATCACCGACAGAAACATCAAGCAGTTCAAGAAGATGCACAGAAACCAGGT GGGGGTGCTGAACTCCATCTACCACAACGCCCTGCGGTCCTTCGCCGCCAACTACGG |
| | | CCTGAATTGGAACGAGCTGCCGAACATGAATCTGGAGCAGATCAAGAACGCCCTGA AGGACCACGTGAGGGTGATGTTCCCTCAGTGGGGCGGCCGGAGCTTCATGAGCACC CACAACGTGACCCGGAACAGCGTGAGAGTGAGCTACAACAACAGGACCCGCTGGCT GAACTTCAGCGACGTGATCGCCGCCCTCAACATCGCCCTCAGGGGCAGCGGCAACT ACGAGCCCAAGGGGGACAGCCAGAACACCGCCCCCAGCAGAAACAGC |
| 84 | C05440-T5 amino acid sequence | MGPAAKRVKLDATKTIVRPYTSNLSPNAAKKAMLDESFKFFDHAYTVFFSVFIKLWGGV KPTQVALVENDTNKIDAICSILWFRLQTKTDSTNITLQSAEERIRRFKEYAQHDPSPLALSY LTGNLDPEKHEWVDCRELYQNWCAELKCDLATDIETMINHNLLPISAKQEYNCYSSFSN LFGEAEKENKAEKVEWYRSVVAALKSKSAWNWDEYRKMLFEKIGCTAPKGGGRLSLLFL DMRSDTTGPISSQEITKRITSFEKVAEEKSKTYELPHRLTVKQYICNQLAHPFQLTIWSAVA NYAISSIRSKNSINMLYSDEKLNRETIIAKIKSQNPNIEQVQEILSQFRAGENNAFVIEPRHL SNLKSLFRFWSNSSMDEGITTLLQIPQSEYERSPIVDLLRHIHPHRNKFTAEQFIVAAELNS HQTVNAMKKIHPTVYGKTTVDYGPKSTIYGSITPPSKLIRGVHAGSHGMMWVTMTLLD NGKWIKHHIPFHNSRYYEEMYAYREGLPVGEKPRRPVLGNRVGNHIDNTGLINKKCRKA SKAYLRTIQNMNHNVWFDPDTHFLVQRNGDNFAIQISSRIKESKPKSNVEVGDRILGM DQNQTASNTYSVVEVVREGTENSHPYNNYFVKVIEDGNVTSCTTKNDRGEFDQLSYEG LAYSEFEIWRQARIAFLSAHDPELAQKMIDKTNESLYKWNNNYAYYLKVCMRNKMNG DNHALFRNEIKEFIEGMPVAGKNEKHKFFGSIRGSLSLESLEGLSKSRSLISCYFYLLEKKEIE QQKEFDSDLFKLGECLSEKRVNKREERANRIVSSVLQICSRLNVSRIVIENKLPTANHENKS SANRRATDWCPRKVQQKLLDAVKMVGIKVLAVKPYNTSHIDPFVNGESNRQALEARF MDVDVKDITDRNIKQFKKMHRNQVGVLNSIYHNALRSFAANYGLNWNELPNMNLEQI KNALKDHVRVMFPQWGGRSFMSTHNVTRNSVRVSYNNRTRWLNFSDVIAALNIALRG SGNYEPKGDSQNTAPSRNSSGGSSGGSSGSETPGTSESATPESSGGSSGGSSKSWGKFIE EEEAEMASRRNLMIVDGTNLGFRFKHNNSKKPFASSYVSTIQSLAKSYSARTTIVLGDKG KSVFRLEHLPEYKGNRDEKYAQRTEEEKALDEQFFEYLKDAFELCKTTFPTFTIRGVEADD MAAYIVKLIGHLYDHVWLISTDGDWDTLLTDKVSRFSFTTRREYHLRDMYEHHNVDDV EQFISLKAIMGDLGDNIRGVEGIGAKRGYNIIREFGNVLDIIDQLPLPGKQKYIQNLNASEE LLFRNLILVDLPTYCVDAIAAVGQDVLDKFTKDILEIAEQSGGSKRTADGSEFEPKKKRKVV DGSGSKRPAATKKAGQAKKKK* |
| 85 | C05440-T5 nucleotide coding sequence | ATGGGACCGGCAGCTAAGAGAGTGAAACTGGATGCCACGAAGACCATCGTGAGGC CATACACCAGCAACCTGTCCCCTAACGCCGCCAAGAAGGCCATGCTGGACGAGAGC TTCAAGTTCTTCGACCACGCCTACACCGTGTTCTTCAGCGTGTTCATCAAGCTGTGGG GCGGCGTGAAGCCCACCCAGGTGGCCCTGGTGGAGAACGACACCAACAAGATCGA CGCCATCTGCAGCATCCTGTGGTTCAGGCTGCAGACCAAGACCGACAGCACCAACAT |
| | | CACCCTCCAGTCCGCCGAGGAGAGGATCCGCCGCTTCAAGGAGTACGCCCAGCACG ACCCTAGCCCCCTGGCCCTCAGCTACCTGACGGGCAACCTGGACCCCGAGAAGCAC GAGTGGGTCGACTGCCGCGAGCTGTACCAGAACTGGTGCGCCGAGCTGAAGTGCG ACCTCGCCACCGACATCGAAACCATGATCAACCACAACCTGCTGCCTATCAGCGCGA AGCAGGAGTACAACTGCTACAGCTCGTTCAGCAACCTGTTCGGCGAGGCCGAGAAG GAGAACAAGGCCGAGAAGGTGGAGTGGTACAGAAGCGTGGTGGCCGCGCTGAAG TCCAAGAGCGCCTGGAACTGGGACGAGTACAGGAAGATGCTGTTCGAGAAGATCG GCTGCACCGCCCCCAAGGGCGGCGGCAGACTGAGCCTCCTGTTCCTGGACATGAGG TCCGACACCACCGGCCCCATCAGCTCCCAGGAGATCACCAAGCGGATCACCAGCTTC GAGAAGGTGGCCGAGGAGAAGAGTAAGACCTACGAGCTCCCCCACAGGCTGACCG TGAAGCAGTACATCTGCAACCAGCTGGCCCACCCCTTTCAACTGACTATTTGGTCTGC GGTTGCAAACTACGCCATCTCCAGCATCAGGAGCAAGAACAGCATCAATATGCTGTA CAGCGACGAGAAGCTCAACAGGGAAACCATCATCGCCAAGATCAAGAGCCAGAACC CCAACATCGAGCAGGTGCAGGAGATCCTGTCCCAGTTCAGGGCCGGCGAGAACAAC GCGTTCGTGATCGAGCCCAGACACCTGTCCAACCTGAAGAGTCTGTTCAGGTTCTGG TCCAACAGCAGCATGGACGAGGGCATCACCACCCTGCTGCAGATCCCTCAGAGCGA GTACGAGCGCAGCCCCATCGTGGACCTGCTCCGGCACATCCACCCCCACAGGAACA AGTTCACCGCCGAGCAGTTCATCGTGGCCGCCGAGCTGAACTCCCATCAGACTGTCA ACGCGATGAAGAAGATCCACCCCACCGTGTACGGCAAGACCACCGTGGACTACGGC CCCAAGAGCACCATCTACGGCAGCATCACCCCACCAAGCAAGCTGATCCGCGGCGT GCACGCCGGCAGCCACGGCATGATGTGGGTGACCATGACCCTGCTGGACAACGGCA AGTGGATCAAGCACCACATCCCCTTCCACAACAGCAGGTACTACGAGGAGATGTAC GCCTACAGAGAGGGCCTGCCGGTCGGCGAGAAGCCCAGGAGGCCGGTGCTGGGCA ACCGGGTGGGCAACCACATCGACAACACCGGCCTGATCAATAAGAAGTGCAGGAA GGCCAGCAAGGCCTACCTGAGGACCATCCAGAACATGAATCACAACGTGTGGTTCG ACCCCGACACCCACTTCCTGGTGCAGAGGAACGGCGACAACTTCGCCATCCAGATCT CCTCCAGGATCAAGGAGAGCAAGCCCAAGTCCAACGTGGAGGTGGGCGACCGCAT CCTGGGCATGGACCAGAACCAGACGGCCAGCAACACCTACAGCGTGGTGGAGGTG GTGCGCGAGGGCACCGAGAATAGCCACCCCTACAACAACTACTTTGTCAAGGTGAT CGAGGACGGCAACGTGACCAGCTGCACCACCAAGAATGACAGGGGCGAGTTCGAC CAGCTGAGCTACGAGGGCCTGGCCTACAGCGAGTTCGAGATCTGGCGGCAGGCCA GGATCGCCTTCCTGAGCGCCCACGACCCCGAGCTGGCCCAGAAGATGATCGACAAG ACCAACGAGAGCCTGTACAAGTGGAACAACAACTACGCCTACTACCTGAAGGTGTG CATGAGGAACAAGATGAACGGCGACAACCACGCCCTGTTCCGGAACGAGATCAAG |
| | | GAGTTCATCGAGGGCATGCCCGTGGCGGGCAAGAACGAGAAGCACAAGTTCTTCG GCTCCATCAGGGGCAGCCTGTCCCTGGAGAGCCTGGAGGGCCTGAGCAAGTCCCGC AGCCTGATCAGCTGCTACTTCTACCTGCTCGAGAAGAAGGAGATCGAGCAGCAGAA GGAGTTCGACAGCGATCTGTTCAAGCTGGGCGAGTGCCTGAGCGAGAAGAGAGTG AACAAGCGGGAGGAGAGAGCCAACCGGATCGTGAGCTCGGTGCTGCAGATCTGCA GCCGGCTGAACGTGAGCAGGATCGTGATCGAGAACAAGCTGCCCACCGCCAACCAC GAGAACAAGAGCAGCGCCAACCGCAGAGCCACCGACTGGTGCCCCAGGAAGGTGC AGCAGAAGCTGCTGGACGCGGTGAAGATGGTGGGGATCAAGGTGCTGGCCGTGAA GCCCTACAACACCAGCCACATCGACCCCTTCGTGAACGGCGAGAGCAACAGACAGG CCCTGGAGGCCAGGTTCATGGACGTGGACGTGAAGGACATCACCGACAGAAACATC AAGCAGTTCAAGAAGATGCACAGAAACCAGGTGGGGGTGCTGAACTCCATCTACCA CAACGCCCTGCGGTCCTTCGCCGCCAACTACGGCCTGAATTGGAACGAGCTGCCGA ACATGAATCTGGAGCAGATCAAGAACGCCCTGAAGGACCACGTGAGGGTGATGTTC CCTCAGTGGGGCGGCCGGAGCTTCATGAGCACCCACAACGTGACCCGGAACAGCGT GAGAGTGAGCTACAACAACAGGACCCGCTGGCTGAACTTCAGCGACGTGATCGCCG CCCTCAACATCGCCCTCAGGGGCAGCGGCAACTACGAGCCCAAGGGGGACAGCCA GAACACCGCCCCCAGCAGAAACAGCTCTGGGGGAAGTAGCGGTGGAAGCAGTGGC AGCGAAACCCCTGGAACAAGCGAAAGCGCTACCCCCGAATCTAGCGGCGGTTCATC AGGTGGATCTTCAAAGAGTTGGGGCAAGTTCATTGAGGAGGAGGAAGCCGAAATG GCCAGTCGGCGCAATCTCATGATTGTTGACGGCACCAATCTCGGCTTCAGATTCAAG CACAACAACTCCAAGAAACCCTTTGCTTCCAGTTATGTGAGCACGATTCAAAGCCTG GCCAAGTCATATTCTGCTCGCACTACGATTGTGCTGGGGGACAAAGGGAAAAGCGT GTTCAGACTTGAGCACCTGCCAGAGTACAAAGGAAATCGAGATGAGAAGTACGCCC AAAGAACCGAAGAGGAGAAGGCCCTGGATGAGCAGTTTTTCGAGTACCTGAAAGA TGCTTTCGAGCTTTGCAAAACGACCTTCCCAACATTCACAATTCGCGGGGTTGAGGC CGATGACATGGCAGCCTACATTGTCAAGTTGATCGGACACCTGTATGACCATGTGTG GCTTATCAGCACTGATGGAGACTGGGATACTCTTCTGACAGACAAAGTATCTCGGTT CAGCTTCACCACGAGGCGAGAATATCACCTGAGGGATATGTACGAGCACCATAATG TGGACGATGTTGAGCAGTTCATTTCTCTGAAGGCGATTATGGGCGACCTGGGGGAC AATATAAGAGGAGTTGAGGGAATCGGGGCCAAGAGAGGGTACAACATCATACGAG AGTTTGGGAACGTACTTGATATCATCGACCAGCTGCCATTGCCTGGGAAGCAAAAAT ATATACAGAACCTGAACGCTTCCGAAGAGTTGCTGTTTAGGAACCTGATCCTGGTGG ATTTGCCCACCTATTGCGTCGACGCAATAGCAGCAGTAGGTCAGGACGTGCTCGATA AATTTACCAAGGATATACTTGAGATCGCTGAGCAATCTGGCGGCTCAAAAAGAACC |
| | | GCCGACGGCAGCGAATTCGAGCCCAAGAAGAAGAGGAAAGTCGTCGACGGATCTG GCAGCAAGAGACCTGCTGCCACCAAGAAGGCCGGCCAGGCCAAGAAAAAAAAGTA A |
| 86 | T5-C05440 amino acid sequence | MGPAAKRVKLDSKSWGKFIEEEEAEMASRRNLMIVDGTNLGFRFKHNNSKKPFASSYV STIQSLAKSYSARTTIVLGDKGKSVFRLEHLPEYKGNRDEKYAQRTEEEKALDEQFFEYLKD AFELCKTTFPTFTIRGVEADDMAAYIVKLIGHLYDHVWLISTDGDWDTLLTDKVSRFSFTT RREYHLRDMYEHHNVDDVEQFISLKAIMGDLGDNIRGVEGIGAKRGYNIIREFGNVLDII DQLPLPGKQKYIQNLNASEELLFRNLILVDLPTYCVDAIAAVGQDVLDKFTKDILEIAEQSG GSSGGSSGSETPGTSESATPESSGGSSGGSATKTIVRPYTSNLSPNAAKKAMLDESFKFFD HAYTVFFSVFIKLWGGVKPTQVALVENDTNKIDAICSILWFRLQTKTDSTNITLQSAEERI RRFKEYAQHDPSPLALSYLTGNLDPEKHEWVDCRELYQNWCAELKCDLATDIETMINH NLLPISAKQEYNCYSSFSNLFGEAEKENKAEKVEWYRSVVAALKSKSAWNWDEYRKMLF EKIGCTAPKGGGRLSLLFLDMRSDTTGPISSQEITKRITSFEKVAEEKSKTYELPHRLTVKQY ICNQLAHPFQLTIWSAVANYAISSIRSKNSINMLYSDEKLNRETIIAKIKSQNPNIEQVQEIL SQFRAGENNAFVIEPRHLSNLKSLFRFWSNSSMDEGITTLLQIPQSEYERSPIVDLLRHIHP HRNKFTAEQFIVAAELNSHQTVNAMKKIHPTVYGKTTVDYGPKSTIYGSITPPSKLIRGVH AGSHGMMWVTMTLLDNGKWIKHHIPFHNSRYYEEMYAYREGLPVGEKPRRPVLGNR VGNHIDNTGLINKKCRKASKAYLRTIQNMNHNVWFDPDTHFLVQRNGDNFAIQISSRIK ESKPKSNVEVGDRILGMDQNQTASNTYSVVEVVREGTENSHPYNNYFVKVIEDGNVTS CTTKNDRGEFDQLSYEGLAYSEFEIWRQARIAFLSAHDPELAQKMIDKTNESLYKWNNN YAYYLKVCMRNKMNGDNHALFRNEIKEFIEGMPVAGKNEKHKFFGSIRGSLSLESLEGLS KSRSLISCYFYLLEKKEIEQQKEFDSDLFKLGECLSEKRVNKREERANRIVSSVLQICSRLNVS RIVIENKLPTANHENKSSANRRATDWCPRKVQQKLLDAVKMVGIKVLAVKPYNTSHIDP FVNGESNRQALEARFMDVDVKDITDRNIKQFKKMHRNQVGVLNSIYHNALRSFAANY GLNWNELPNMNLEQIKNALKDHVRVMFPQWGGRSFMSTHNVTRNSVRVSYNNRTR WLNFSDVIAALNIALRGSGNYEPKGDSQNTAPSRNSSGGSKRTADGSEFEPKKKRKVVD GSGSKRPAATKKAGQAKKKK* |
| 87 | T5-C05440 nucleotide coding sequence | ATGGGACCGGCAGCTAAGAGAGTGAAACTGGATTCAAAGAGTTGGGGCAAGTTCA TTGAGGAGGAGGAAGCCGAAATGGCCAGTCGGCGCAATCTCATGATTGTTGACGGC ACCAATCTCGGCTTCAGATTCAAGCACAACAACTCCAAGAAACCCTTTGCTTCCAGTT ATGTGAGCACGATTCAAAGCCTGGCCAAGTCATATTCTGCTCGCACTACGATTGTGC TGGGGGACAAAGGGAAAAGCGTGTTCAGACTTGAGCACCTGCCAGAGTACAAAGG AAATCGAGATGAGAAGTACGCCCAAAGAACCGAAGAGGAGAAGGCCCTGGATGAG CAGTTTTTCGAGTACCTGAAAGATGCTTTCGAGCTTTGCAAAACGACCTTCCCAACAT |
| | | TCACAATTCGCGGGGTTGAGGCCGATGACATGGCAGCCTACATTGTCAAGTTGATC GGACACCTGTATGACCATGTGTGGCTTATCAGCACTGATGGAGACTGGGATACTCTT CTGACAGACAAAGTATCTCGGTTCAGCTTCACCACGAGGCGAGAATATCACCTGAG GGATATGTACGAGCACCATAATGTGGACGATGTTGAGCAGTTCATTTCTCTGAAGGC GATTATGGGCGACCTGGGGGACAATATAAGAGGAGTTGAGGGAATCGGGGCCAAG AGAGGGTACAACATCATACGAGAGTTTGGGAACGTACTTGATATCATCGACCAGCT GCCATTGCCTGGGAAGCAAAAATATATACAGAACCTGAACGCTTCCGAAGAGTTGC TGTTTAGGAACCTGATCCTGGTGGATTTGCCCACCTATTGCGTCGACGCAATAGCAG CAGTAGGTCAGGACGTGCTCGATAAATTTACCAAGGATATACTTGAGATCGCTGAG CAATCAGGTGGAAGTTCCGGAGGCAGTTCCGGCTCCGAAACTCCTGGCACCTCAGA ATCTGCTACACCCGAATCTTCCGGGGGTTCCTCAGGCGGGTCAGCCACGAAGACCAT CGTGAGGCCATACACCAGCAACCTGTCCCCTAACGCCGCCAAGAAGGCCATGCTGG ACGAGAGCTTCAAGTTCTTCGACCACGCCTACACCGTGTTCTTCAGCGTGTTCATCAA GCTGTGGGGCGGCGTGAAGCCCACCCAGGTGGCCCTGGTGGAGAACGACACCAAC AAGATCGACGCCATCTGCAGCATCCTGTGGTTCAGGCTGCAGACCAAGACCGACAG CACCAACATCACCCTCCAGTCCGCCGAGGAGAGGATCCGCCGCTTCAAGGAGTACG CCCAGCACGACCCTAGCCCCCTGGCCCTCAGCTACCTGACGGGCAACCTGGACCCCG AGAAGCACGAGTGGGTCGACTGCCGCGAGCTGTACCAGAACTGGTGCGCCGAGCT GAAGTGCGACCTCGCCACCGACATCGAAACCATGATCAACCACAACCTGCTGCCTAT CAGCGCGAAGCAGGAGTACAACTGCTACAGCTCGTTCAGCAACCTGTTCGGCGAGG CCGAGAAGGAGAACAAGGCCGAGAAGGTGGAGTGGTACAGAAGCGTGGTGGCCG CGCTGAAGTCCAAGAGCGCCTGGAACTGGGACGAGTACAGGAAGATGCTGTTCGA GAAGATCGGCTGCACCGCCCCCAAGGGCGGCGGCAGACTGAGCCTCCTGTTCCTGG ACATGAGGTCCGACACCACCGGCCCCATCAGCTCCCAGGAGATCACCAAGCGGATC ACCAGCTTCGAGAAGGTGGCCGAGGAGAAGAGTAAGACCTACGAGCTCCCCCACA GGCTGACCGTGAAGCAGTACATCTGCAACCAGCTGGCCCACCCCTTTCAACTGACTA TTTGGTCTGCGGTTGCAAACTACGCCATCTCCAGCATCAGGAGCAAGAACAGCATCA ATATGCTGTACAGCGACGAGAAGCTCAACAGGGAAACCATCATCGCCAAGATCAAG AGCCAGAACCCCAACATCGAGCAGGTGCAGGAGATCCTGTCCCAGTTCAGGGCCGG CGAGAACAACGCGTTCGTGATCGAGCCCAGACACCTGTCCAACCTGAAGAGTCTGTT CAGGTTCTGGTCCAACAGCAGCATGGACGAGGGCATCACCACCCTGCTGCAGATCC CTCAGAGCGAGTACGAGCGCAGCCCCATCGTGGACCTGCTCCGGCACATCCACCCCC ACAGGAACAAGTTCACCGCCGAGCAGTTCATCGTGGCCGCCGAGCTGAACTCCCAT CAGACTGTCAACGCGATGAAGAAGATCCACCCCACCGTGTACGGCAAGACCACCGT |
| | | GGACTACGGCCCCAAGAGCACCATCTACGGCAGCATCACCCCACCAAGCAAGCTGA TCCGCGGCGTGCACGCCGGCAGCCACGGCATGATGTGGGTGACCATGACCCTGCTG GACAACGGCAAGTGGATCAAGCACCACATCCCCTTCCACAACAGCAGGTACTACGA GGAGATGTACGCCTACAGAGAGGGCCTGCCGGTCGGCGAGAAGCCCAGGAGGCCG GTGCTGGGCAACCGGGTGGGCAACCACATCGACAACACCGGCCTGATCAATAAGAA GTGCAGGAAGGCCAGCAAGGCCTACCTGAGGACCATCCAGAACATGAATCACAACG TGTGGTTCGACCCCGACACCCACTTCCTGGTGCAGAGGAACGGCGACAACTTCGCCA TCCAGATCTCCTCCAGGATCAAGGAGAGCAAGCCCAAGTCCAACGTGGAGGTGGGC GACCGCATCCTGGGCATGGACCAGAACCAGACGGCCAGCAACACCTACAGCGTGGT GGAGGTGGTGCGCGAGGGCACCGAGAATAGCCACCCCTACAACAACTACTTTGTCA AGGTGATCGAGGACGGCAACGTGACCAGCTGCACCACCAAGAATGACAGGGGCGA GTTCGACCAGCTGAGCTACGAGGGCCTGGCCTACAGCGAGTTCGAGATCTGGCGGC AGGCCAGGATCGCCTTCCTGAGCGCCCACGACCCCGAGCTGGCCCAGAAGATGATC GACAAGACCAACGAGAGCCTGTACAAGTGGAACAACAACTACGCCTACTACCTGAA GGTGTGCATGAGGAACAAGATGAACGGCGACAACCACGCCCTGTTCCGGAACGAG ATCAAGGAGTTCATCGAGGGCATGCCCGTGGCGGGCAAGAACGAGAAGCACAAGT TCTTCGGCTCCATCAGGGGCAGCCTGTCCCTGGAGAGCCTGGAGGGCCTGAGCAAG TCCCGCAGCCTGATCAGCTGCTACTTCTACCTGCTCGAGAAGAAGGAGATCGAGCA GCAGAAGGAGTTCGACAGCGATCTGTTCAAGCTGGGCGAGTGCCTGAGCGAGAAG AGAGTGAACAAGCGGGAGGAGAGAGCCAACCGGATCGTGAGCTCGGTGCTGCAGA TCTGCAGCCGGCTGAACGTGAGCAGGATCGTGATCGAGAACAAGCTGCCCACCGCC AACCACGAGAACAAGAGCAGCGCCAACCGCAGAGCCACCGACTGGTGCCCCAGGA AGGTGCAGCAGAAGCTGCTGGACGCGGTGAAGATGGTGGGGATCAAGGTGCTGGC CGTGAAGCCCTACAACACCAGCCACATCGACCCCTTCGTGAACGGCGAGAGCAACA GACAGGCCCTGGAGGCCAGGTTCATGGACGTGGACGTGAAGGACATCACCGACAG AAACATCAAGCAGTTCAAGAAGATGCACAGAAACCAGGTGGGGGTGCTGAACTCCA TCTACCACAACGCCCTGCGGTCCTTCGCCGCCAACTACGGCCTGAATTGGAACGAGC TGCCGAACATGAATCTGGAGCAGATCAAGAACGCCCTGAAGGACCACGTGAGGGT GATGTTCCCTCAGTGGGGCGGCCGGAGCTTCATGAGCACCCACAACGTGACCCGGA ACAGCGTGAGAGTGAGCTACAACAACAGGACCCGCTGGCTGAACTTCAGCGACGTG ATCGCCGCCCTCAACATCGCCCTCAGGGGCAGCGGCAACTACGAGCCCAAGGGGGA CAGCCAGAACACCGCCCCCAGCAGAAACAGCTCTGGCGGCTCAAAAAGAACCGCCG ACGGCAGCGAATTCGAGCCCAAGAAGAAGAGGAAAGTCGTCGACGGATCTGGCAG CAAGAGACCTGCTGCCACCAAGAAGGCCGGCCAGGCCAAGAAAAAAAAGTAA |
| 88 | C05440-T5 mRNA transcription template | TAATACGACTCACTATAAGGGGAAATAAGAGAGAAAAGAAGAGTAAGAAGAAATA TAAGAGCCACCGCCACCATGGGACCGGCAGCTAAGAGAGTGAAACTGGATGCCAC GAAGACCATCGTGAGGCCATACACCAGCAACCTGTCCCCTAACGCCGCCAAGAAGG CCATGCTGGACGAGAGCTTCAAGTTCTTCGACCACGCCTACACCGTGTTCTTCAGCG TGTTCATCAAGCTGTGGGGCGGCGTGAAGCCCACCCAGGTGGCCCTGGTGGAGAAC GACACCAACAAGATCGACGCCATCTGCAGCATCCTGTGGTTCAGGCTGCAGACCAA GACCGACAGCACCAACATCACCCTCCAGTCCGCCGAGGAGAGGATCCGCCGCTTCA AGGAGTACGCCCAGCACGACCCTAGCCCCCTGGCCCTCAGCTACCTGACGGGCAAC CTGGACCCCGAGAAGCACGAGTGGGTCGACTGCCGCGAGCTGTACCAGAACTGGT GCGCCGAGCTGAAGTGCGACCTCGCCACCGACATCGAAACCATGATCAACCACAAC CTGCTGCCTATCAGCGCGAAGCAGGAGTACAACTGCTACAGCTCGTTCAGCAACCTG TTCGGCGAGGCCGAGAAGGAGAACAAGGCCGAGAAGGTGGAGTGGTACAGAAGC GTGGTGGCCGCGCTGAAGTCCAAGAGCGCCTGGAACTGGGACGAGTACAGGAAGA TGCTGTTCGAGAAGATCGGCTGCACCGCCCCCAAGGGCGGCGGCAGACTGAGCCTC CTGTTCCTGGACATGAGGTCCGACACCACCGGCCCCATCAGCTCCCAGGAGATCACC AAGCGGATCACCAGCTTCGAGAAGGTGGCCGAGGAGAAGAGTAAGACCTACGAGC TCCCCCACAGGCTGACCGTGAAGCAGTACATCTGCAACCAGCTGGCCCACCCCTTTC AACTGACTATTTGGTCTGCGGTTGCAAACTACGCCATCTCCAGCATCAGGAGCAAGA ACAGCATCAATATGCTGTACAGCGACGAGAAGCTCAACAGGGAAACCATCATCGCC AAGATCAAGAGCCAGAACCCCAACATCGAGCAGGTGCAGGAGATCCTGTCCCAGTT CAGGGCCGGCGAGAACAACGCGTTCGTGATCGAGCCCAGACACCTGTCCAACCTGA AGAGTCTGTTCAGGTTCTGGTCCAACAGCAGCATGGACGAGGGCATCACCACCCTG CTGCAGATCCCTCAGAGCGAGTACGAGCGCAGCCCCATCGTGGACCTGCTCCGGCA CATCCACCCCCACAGGAACAAGTTCACCGCCGAGCAGTTCATCGTGGCCGCCGAGCT GAACTCCCATCAGACTGTCAACGCGATGAAGAAGATCCACCCCACCGTGTACGGCA AGACCACCGTGGACTACGGCCCCAAGAGCACCATCTACGGCAGCATCACCCCACCA AGCAAGCTGATCCGCGGCGTGCACGCCGGCAGCCACGGCATGATGTGGGTGACCAT GACCCTGCTGGACAACGGCAAGTGGATCAAGCACCACATCCCCTTCCACAACAGCA GGTACTACGAGGAGATGTACGCCTACAGAGAGGGCCTGCCGGTCGGCGAGAAGCC CAGGAGGCCGGTGCTGGGCAACCGGGTGGGCAACCACATCGACAACACCGGCCTG ATCAATAAGAAGTGCAGGAAGGCCAGCAAGGCCTACCTGAGGACCATCCAGAACAT GAATCACAACGTGTGGTTCGACCCCGACACCCACTTCCTGGTGCAGAGGAACGGCG ACAACTTCGCCATCCAGATCTCCTCCAGGATCAAGGAGAGCAAGCCCAAGTCCAACG TGGAGGTGGGCGACCGCATCCTGGGCATGGACCAGAACCAGACGGCCAGCAACAC |
| | | CTACAGCGTGGTGGAGGTGGTGCGCGAGGGCACCGAGAATAGCCACCCCTACAAC AACTACTTTGTCAAGGTGATCGAGGACGGCAACGTGACCAGCTGCACCACCAAGAA TGACAGGGGCGAGTTCGACCAGCTGAGCTACGAGGGCCTGGCCTACAGCGAGTTC GAGATCTGGCGGCAGGCCAGGATCGCCTTCCTGAGCGCCCACGACCCCGAGCTGGC CCAGAAGATGATCGACAAGACCAACGAGAGCCTGTACAAGTGGAACAACAACTACG CCTACTACCTGAAGGTGTGCATGAGGAACAAGATGAACGGCGACAACCACGCCCTG TTCCGGAACGAGATCAAGGAGTTCATCGAGGGCATGCCCGTGGCGGGCAAGAACG AGAAGCACAAGTTCTTCGGCTCCATCAGGGGCAGCCTGTCCCTGGAGAGCCTGGAG GGCCTGAGCAAGTCCCGCAGCCTGATCAGCTGCTACTTCTACCTGCTCGAGAAGAAG GAGATCGAGCAGCAGAAGGAGTTCGACAGCGATCTGTTCAAGCTGGGCGAGTGCC TGAGCGAGAAGAGAGTGAACAAGCGGGAGGAGAGAGCCAACCGGATCGTGAGCT CGGTGCTGCAGATCTGCAGCCGGCTGAACGTGAGCAGGATCGTGATCGAGAACAA GCTGCCCACCGCCAACCACGAGAACAAGAGCAGCGCCAACCGCAGAGCCACCGACT GGTGCCCCAGGAAGGTGCAGCAGAAGCTGCTGGACGCGGTGAAGATGGTGGGGAT CAAGGTGCTGGCCGTGAAGCCCTACAACACCAGCCACATCGACCCCTTCGTGAACG GCGAGAGCAACAGACAGGCCCTGGAGGCCAGGTTCATGGACGTGGACGTGAAGGA CATCACCGACAGAAACATCAAGCAGTTCAAGAAGATGCACAGAAACCAGGTGGGG GTGCTGAACTCCATCTACCACAACGCCCTGCGGTCCTTCGCCGCCAACTACGGCCTG AATTGGAACGAGCTGCCGAACATGAATCTGGAGCAGATCAAGAACGCCCTGAAGGA CCACGTGAGGGTGATGTTCCCTCAGTGGGGCGGCCGGAGCTTCATGAGCACCCACA ACGTGACCCGGAACAGCGTGAGAGTGAGCTACAACAACAGGACCCGCTGGCTGAA CTTCAGCGACGTGATCGCCGCCCTCAACATCGCCCTCAGGGGCAGCGGCAACTACG AGCCCAAGGGGGACAGCCAGAACACCGCCCCCAGCAGAAACAGCTCTGGGGGAAG TAGCGGTGGAAGCAGTGGCAGCGAAACCCCTGGAACAAGCGAAAGCGCTACCCCC GAATCTAGCGGCGGTTCATCAGGTGGATCTTCAAAGAGTTGGGGCAAGTTCATTGA GGAGGAGGAAGCCGAAATGGCCAGTCGGCGCAATCTCATGATTGTTGACGGCACC AATCTCGGCTTCAGATTCAAGCACAACAACTCCAAGAAACCCTTTGCTTCCAGTTATG TGAGCACGATTCAAAGCCTGGCCAAGTCATATTCTGCTCGCACTACGATTGTGCTGG GGGACAAAGGGAAAAGCGTGTTCAGACTTGAGCACCTGCCAGAGTACAAAGGAAA TCGAGATGAGAAGTACGCCCAAAGAACCGAAGAGGAGAAGGCCCTGGATGAGCAG TTTTTCGAGTACCTGAAAGATGCTTTCGAGCTTTGCAAAACGACCTTCCCAACATTCA CAATTCGCGGGGTTGAGGCCGATGACATGGCAGCCTACATTGTCAAGTTGATCGGA CACCTGTATGACCATGTGTGGCTTATCAGCACTGATGGAGACTGGGATACTCTTCTG ACAGACAAAGTATCTCGGTTCAGCTTCACCACGAGGCGAGAATATCACCTGAGGGA |
| | | TATGTACGAGCACCATAATGTGGACGATGTTGAGCAGTTCATTTCTCTGAAGGCGAT TATGGGCGACCTGGGGGACAATATAAGAGGAGTTGAGGGAATCGGGGCCAAGAG AGGGTACAACATCATACGAGAGTTTGGGAACGTACTTGATATCATCGACCAGCTGCC ATTGCCTGGGAAGCAAAAATATATACAGAACCTGAACGCTTCCGAAGAGTTGCTGTT TAGGAACCTGATCCTGGTGGATTTGCCCACCTATTGCGTCGACGCAATAGCAGCAGT AGGTCAGGACGTGCTCGATAAATTTACCAAGGATATACTTGAGATCGCTGAGCAATC TGGCGGCTCAAAAAGAACCGCCGACGGCAGCGAATTCGAGCCCAAGAAGAAGAGG AAAGTCGTCGACGGATCTGGCAGCAAGAGACCTGCTGCCACCAAGAAGGCCGGCC AGGCCAAGAAAAAAAAGTAATGATAATAGGCTGGAGCCTCGGTGGCCATGCTTCTT GCCCCTTGGGCCTCCCCCCAGCCCCTCCTCCCCTTCCTGCACCCGTACCCCCGTGGTC TTTGAATAAAGTCTGAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAAAAAAAAAA AAAAAAAAAAAAAAAAAAAAAAAAAAAAAA |
| 89 | T5-C05440 mRNA transcription template | TAATACGACTCACTATAAGGGGAAATAAGAGAGAAAAGAAGAGTAAGAAGAAATA TAAGAGCCACCGCCACCATGGGACCGGCAGCTAAGAGAGTGAAACTGGATTCAAAG AGTTGGGGCAAGTTCATTGAGGAGGAGGAAGCCGAAATGGCCAGTCGGCGCAATC TCATGATTGTTGACGGCACCAATCTCGGCTTCAGATTCAAGCACAACAACTCCAAGA AACCCTTTGCTTCCAGTTATGTGAGCACGATTCAAAGCCTGGCCAAGTCATATTCTGC TCGCACTACGATTGTGCTGGGGGACAAAGGGAAAAGCGTGTTCAGACTTGAGCACC TGCCAGAGTACAAAGGAAATCGAGATGAGAAGTACGCCCAAAGAACCGAAGAGGA GAAGGCCCTGGATGAGCAGTTTTTCGAGTACCTGAAAGATGCTTTCGAGCTTTGCAA AACGACCTTCCCAACATTCACAATTCGCGGGGTTGAGGCCGATGACATGGCAGCCTA CATTGTCAAGTTGATCGGACACCTGTATGACCATGTGTGGCTTATCAGCACTGATGG AGACTGGGATACTCTTCTGACAGACAAAGTATCTCGGTTCAGCTTCACCACGAGGCG AGAATATCACCTGAGGGATATGTACGAGCACCATAATGTGGACGATGTTGAGCAGT TCATTTCTCTGAAGGCGATTATGGGCGACCTGGGGGACAATATAAGAGGAGTTGAG GGAATCGGGGCCAAGAGAGGGTACAACATCATACGAGAGTTTGGGAACGTACTTG ATATCATCGACCAGCTGCCATTGCCTGGGAAGCAAAAATATATACAGAACCTGAACG CTTCCGAAGAGTTGCTGTTTAGGAACCTGATCCTGGTGGATTTGCCCACCTATTGCG TCGACGCAATAGCAGCAGTAGGTCAGGACGTGCTCGATAAATTTACCAAGGATATA CTTGAGATCGCTGAGCAATCAGGTGGAAGTTCCGGAGGCAGTTCCGGCTCCGAAAC TCCTGGCACCTCAGAATCTGCTACACCCGAATCTTCCGGGGGTTCCTCAGGCGGGTC AGCCACGAAGACCATCGTGAGGCCATACACCAGCAACCTGTCCCCTAACGCCGCCAA GAAGGCCATGCTGGACGAGAGCTTCAAGTTCTTCGACCACGCCTACACCGTGTTCTT |
| | | CAGCGTGTTCATCAAGCTGTGGGGCGGCGTGAAGCCCACCCAGGTGGCCCTGGTGG AGAACGACACCAACAAGATCGACGCCATCTGCAGCATCCTGTGGTTCAGGCTGCAG ACCAAGACCGACAGCACCAACATCACCCTCCAGTCCGCCGAGGAGAGGATCCGCCG CTTCAAGGAGTACGCCCAGCACGACCCTAGCCCCCTGGCCCTCAGCTACCTGACGGG CAACCTGGACCCCGAGAAGCACGAGTGGGTCGACTGCCGCGAGCTGTACCAGAACT GGTGCGCCGAGCTGAAGTGCGACCTCGCCACCGACATCGAAACCATGATCAACCAC AACCTGCTGCCTATCAGCGCGAAGCAGGAGTACAACTGCTACAGCTCGTTCAGCAAC CTGTTCGGCGAGGCCGAGAAGGAGAACAAGGCCGAGAAGGTGGAGTGGTACAGA AGCGTGGTGGCCGCGCTGAAGTCCAAGAGCGCCTGGAACTGGGACGAGTACAGGA AGATGCTGTTCGAGAAGATCGGCTGCACCGCCCCCAAGGGCGGCGGCAGACTGAG CCTCCTGTTCCTGGACATGAGGTCCGACACCACCGGCCCCATCAGCTCCCAGGAGAT CACCAAGCGGATCACCAGCTTCGAGAAGGTGGCCGAGGAGAAGAGTAAGACCTAC GAGCTCCCCCACAGGCTGACCGTGAAGCAGTACATCTGCAACCAGCTGGCCCACCCC TTTCAACTGACTATTTGGTCTGCGGTTGCAAACTACGCCATCTCCAGCATCAGGAGC AAGAACAGCATCAATATGCTGTACAGCGACGAGAAGCTCAACAGGGAAACCATCAT CGCCAAGATCAAGAGCCAGAACCCCAACATCGAGCAGGTGCAGGAGATCCTGTCCC AGTTCAGGGCCGGCGAGAACAACGCGTTCGTGATCGAGCCCAGACACCTGTCCAAC CTGAAGAGTCTGTTCAGGTTCTGGTCCAACAGCAGCATGGACGAGGGCATCACCAC CCTGCTGCAGATCCCTCAGAGCGAGTACGAGCGCAGCCCCATCGTGGACCTGCTCC GGCACATCCACCCCCACAGGAACAAGTTCACCGCCGAGCAGTTCATCGTGGCCGCC GAGCTGAACTCCCATCAGACTGTCAACGCGATGAAGAAGATCCACCCCACCGTGTAC GGCAAGACCACCGTGGACTACGGCCCCAAGAGCACCATCTACGGCAGCATCACCCC ACCAAGCAAGCTGATCCGCGGCGTGCACGCCGGCAGCCACGGCATGATGTGGGTG ACCATGACCCTGCTGGACAACGGCAAGTGGATCAAGCACCACATCCCCTTCCACAAC AGCAGGTACTACGAGGAGATGTACGCCTACAGAGAGGGCCTGCCGGTCGGCGAGA AGCCCAGGAGGCCGGTGCTGGGCAACCGGGTGGGCAACCACATCGACAACACCGG CCTGATCAATAAGAAGTGCAGGAAGGCCAGCAAGGCCTACCTGAGGACCATCCAGA ACATGAATCACAACGTGTGGTTCGACCCCGACACCCACTTCCTGGTGCAGAGGAAC GGCGACAACTTCGCCATCCAGATCTCCTCCAGGATCAAGGAGAGCAAGCCCAAGTC CAACGTGGAGGTGGGCGACCGCATCCTGGGCATGGACCAGAACCAGACGGCCAGC AACACCTACAGCGTGGTGGAGGTGGTGCGCGAGGGCACCGAGAATAGCCACCCCT ACAACAACTACTTTGTCAAGGTGATCGAGGACGGCAACGTGACCAGCTGCACCACC AAGAATGACAGGGGCGAGTTCGACCAGCTGAGCTACGAGGGCCTGGCCTACAGCG AGTTCGAGATCTGGCGGCAGGCCAGGATCGCCTTCCTGAGCGCCCACGACCCCGAG |
| | | CTGGCCCAGAAGATGATCGACAAGACCAACGAGAGCCTGTACAAGTGGAACAACAA CTACGCCTACTACCTGAAGGTGTGCATGAGGAACAAGATGAACGGCGACAACCACG CCCTGTTCCGGAACGAGATCAAGGAGTTCATCGAGGGCATGCCCGTGGCGGGCAAG AACGAGAAGCACAAGTTCTTCGGCTCCATCAGGGGCAGCCTGTCCCTGGAGAGCCT GGAGGGCCTGAGCAAGTCCCGCAGCCTGATCAGCTGCTACTTCTACCTGCTCGAGA AGAAGGAGATCGAGCAGCAGAAGGAGTTCGACAGCGATCTGTTCAAGCTGGGCGA GTGCCTGAGCGAGAAGAGAGTGAACAAGCGGGAGGAGAGAGCCAACCGGATCGT GAGCTCGGTGCTGCAGATCTGCAGCCGGCTGAACGTGAGCAGGATCGTGATCGAG AACAAGCTGCCCACCGCCAACCACGAGAACAAGAGCAGCGCCAACCGCAGAGCCAC CGACTGGTGCCCCAGGAAGGTGCAGCAGAAGCTGCTGGACGCGGTGAAGATGGTG GGGATCAAGGTGCTGGCCGTGAAGCCCTACAACACCAGCCACATCGACCCCTTCGT GAACGGCGAGAGCAACAGACAGGCCCTGGAGGCCAGGTTCATGGACGTGGACGTG AAGGACATCACCGACAGAAACATCAAGCAGTTCAAGAAGATGCACAGAAACCAGGT GGGGGTGCTGAACTCCATCTACCACAACGCCCTGCGGTCCTTCGCCGCCAACTACGG CCTGAATTGGAACGAGCTGCCGAACATGAATCTGGAGCAGATCAAGAACGCCCTGA AGGACCACGTGAGGGTGATGTTCCCTCAGTGGGGCGGCCGGAGCTTCATGAGCACC CACAACGTGACCCGGAACAGCGTGAGAGTGAGCTACAACAACAGGACCCGCTGGCT GAACTTCAGCGACGTGATCGCCGCCCTCAACATCGCCCTCAGGGGCAGCGGCAACT ACGAGCCCAAGGGGGACAGCCAGAACACCGCCCCCAGCAGAAACAGCTCTGGCGG CTCAAAAAGAACCGCCGACGGCAGCGAATTCGAGCCCAAGAAGAAGAGGAAAGTC GTCGACGGATCTGGCAGCAAGAGACCTGCTGCCACCAAGAAGGCCGGCCAGGCCA AGAAAAAAAAGTAATGATAATAGGCTGGAGCCTCGGTGGCCATGCTTCTTGCCCCTT GGGCCTCCCCCCAGCCCCTCCTCCCCTTCCTGCACCCGTACCCCCGTGGTCTTTGAAT AAAGTCTGAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAAAAAAAAA AAAAAAAAAAAAAAAAAAAAAA |
| 90 | hHAO1-crRNA | AGAAATCCGTCCAAAGCTGACGGggacagagggtcagcatgccaa |
| 91 | hHAO1-F | gatgctccggaatgttgctg |
| 92 | hHAO1-R | ccttctgtccctgtggtgac |
| 93 | DR-1-Trac-crRNA | AGAAATCCGTCCAAAGCTGACGGttgctccaggccacagcact |
| 94 | OsBEL-crRNA | AGAAATCCGTCCAAAGCTGACGGATCTCCTTCTAGAAGCACAA |
| 95 | hAPOBEC3-W104A amino acid sequence | MEASPASGPRHLMDPHIFTSNFNNGIGRHKTYLCYEVERLDNGTSVKMDQHRGFLHN QAKNLLCGFYGRHAELRFLDLVPSLQLDPAQIYRVTWFISYSPCFSAGCAGEVRAFLQEN THVRLRIFAARIFDYDPLYKEALQMLRDAGAQVSIMTYDEFKHCWDTFVDHQGCPFQP WDGLDEHSQALSGRLRAILQNQGN |
| 96 | Human UGI domain amino acid sequence | TNLSDIIEKETGKQLVIQESILMLPEEVEEVIGNKPESDILVHTAYDESTDENVMLLTSDAP EYKPWALVIQDSNGENKIKML |
| 97 | TTR dual-luciferase reporter system vector | taacgcaggaaagaacatgtgagcaaaaggccagcaaaaggccaggaaccgtaaaaaggccgcgttgctg gcgtttttccataggctccgcccccctgacgagcatcacaaaaatcgacgctcaagtcagaggtggcgaaacc cgacaggactataaagataccaggcgtttccccctggaagctccctcgtgcgctctcctgttccgaccctgccgc ttaccggatacctgtccgcctttctcccttcgggaagcgtggcgctttctcatagctcacgctgtaggtatctcagt tcggtgtaggtcgttcgctccaagctgggctgtgtgcacgaaccccccgttcagcccgaccgctgcgccttatcc ggtaactatcgtcttgagtccaacccggtaagacacgacttatcgccactggcagcagccactggtaacaggat tagcagagcgaggtatgtaggcggtgctacagagttcttgaagtggtggcctaactacggctacactagaagg acagtatttggtatctgcgctctgctgaagccagttaccttcggaaaaagagttggtagctcttgatccggcaaa caaaccaccgctggtagcggtggtttttttgtttgcaagcagcagattacgcgcagaaaaaaaggatctcaag aagatcctttgatcttttctacggggtctgacgctcagtggaacgaaaactcacgttaagggattttggtcatga gattatcaaaaaggatcttcacctagatccttttaaattaaaaatgaagttttaaatcaatctaaagtatatatg agtaacctgaggctatggcagggcctgccgccccgacgttggctgcgagccctgggccttcacccgaacttggg gggtggggtggggaaaaggaagaaacgcgggcgtattggccccaatggggtctcggtggggtatcgacaga gtgccagccctgggaccgaaccccgcgtttatgaacaaacgacccaacaccgtgcgttttattctgtctttttatt gccgtcatagcgcgggttccttccggtattgtctccttccgtgtttcagttagcctccccctagggtgggcgaaga actccagcatgagatccccgcgctggaggatcatccagccggcgtcccggaaaacgattccgaagcccaacct ttcatagaaggcggcggtggaatcgaaatctcgtgatggcaggttgggcgtcgcttggtcggtcatttcgaacc ccagagtcccgctcagaagaactcgtcaagaaggcgatagaaggcgatgcgctgcgaatcgggagcggcga taccgtaaagcacgaggaagcggtcagcccattcgccgccaagctcttcagcaatatcacgggtagccaacgc tatgtcctgatagcggtccgccacacccagccggccacagtcgatgaatccagaaaagcggccattttccacc atgatattcggcaagcaggcatcgccatgggtcacgacgagatcctcgccgtcgggcatgctcgccttgagcct ggcgaacagttcggctggcgcgagcccctgatgctcttcgtccagatcatcctgatcgacaagaccggcttcca tccgagtacgtgctcgctcgatgcgatgtttcgcttggtggtcgaatgggcaggtagccggatcaagcgtatgc agccgccgcattgcatcagccatgatggatactttctcggcaggagcaaggtgagatgacaggagatcctgcc ccggcacttcgcccaatagcagccagtcccttcccgcttcagtgacaacgtcgagcacagctgcgcaaggaac gcccgtcgtggccagccacgatagccgcgctgcctcgtcttgcagttcattcagggcaccggacaggtcggtct tgacaaaaagaaccgggcgcccctgcgctgacagccggaacacggcggcatcagagcagccgattgtctgtt |
| | | gtgcccagtcatagccgaatagcctctccacccaagcggccggagaacctgcgtgcaatccatcttgttcaatc atgcgaaacgatcctcatcctgtctcttgatcgatctttgcaaaagcctaggcctccaaaaaagcctcctcacta cttctggaatagctcagaggccgaggcggcctcggcctctgcataaataaaaaaaattagtcagccatggggc ggagaatgggcggaactgggcggagttaggggcgggatgggcggagttaggggcgggactatggttgctgac taattgagatgcatgctttgcatacttctgcctgctggggagcctggggactttccacacctggttgctgactaat tgagatgcatgctttgcatacttctgcctgctggggagcctggggactttccacaccctaactgacacacattcc acagctggttctttccgcctcaggactcttcctttttcaatattattgaagcatttatcagggttattgtctcatgag cggatacatatttgaatgtatttagaaaaataaacaaataggggttccgcgcacatttccccgaaaagtgccac ctgacgcgccctgtagcggcgcattaagcgcggcgggtgtggtggttacgcgcagcgtgaccgctacacttgcc agcgccctagcgcccgctcctttcgctttcttcccttcctttctcgccacgttcgccggctttccccgtcaagctcta aatcgggggctccctttagggttccgatttagtgctttacggcacctcgaccccaaaaaacttgattagggtgat ggttcacgtagtgggccatcgccctgatagacggtttttcgccctttgacgttggagtccacgttctttaatagtg gactcttgttccaaactggaacaacactcaaccctatctcggtctattcttttgatttataagggattttgccgatt tcggcctattggttaaaaaatgagctgatttaacaaaaatttaacgcgaattttaacaaaatattaacgcttaca atttacgcgttaagatacattgatgagtttggacaaaccacaactagaatgcagtgaaaaaaatgctttatttgt gaaatttgtgatgctattgctttatttgtaaccattataagctgcaataaacaagttaacaacaacaattgcattc attttatgtttcaggttcagggggaggtgtgggaggttttttaaagcaagtaaaacctctacaaatgtggtatgg ctgattatgatcagttatctagatccggtggatcccgggttacaatttggactttccgcccttcttggcctttatga ggatctctctgatttttcttgcgtcgagttttccggtaagacctttcggtacttcgtccacaaacacaactcctccg cgcaactttttcgcggttgttacttgactggcgacgtaatccacgatctctttttccgtcatcgtctttccgtgctcc aaaacaacaacggcggcgggaagttcaccggcgtcatcgtcgggaagacctgccacgcccgcgtcgaagatg ttggggtgttgtaacaatatcgattccaattcagcgggggccacctgatatcctttgtatttaattaaagacttca agcggtcaactatgaagaagtgttcgtcttcgtcccagtaagctatgtctccagaatgtagccatccatccttgt caatcaaggcgttggtcgcttccggattgtttacataaccggacataatcataggtcctctgacacataattcgc ctctctgattaacgcccagcgttttcccggtatccagatccacaaccttcgcttcaaaaaatggaacaactttac cgaccgcgcccggtttatcatccccctcgggtgtaatcagaatagctgatgtagtctcagtgagcccatatcctt gtcgtatccctggaagatggaagcgttttgcaaccgcttccccgacttctttcgaaagaggtgcgcccccagaa gcaatttcgtgtaaattagataaatcgtatttgtcaatcagagtgcttttggcgaagaatgaaaatagggttggt actagcaacgcactttgaattttgtaatcctgaagggatcgtaaaaacagctcttcttcaaatctatacattaag acgactcgaaatccacatatcaaatatccgagtgtagtaaacattccaaaaccgtgatggaatggaacaacac ttaaaatcgcagtatccggaatgatttgattgccaaaaataggatctctggcatgcgagaatctgacgcaggca gttctatgcggaagggccacacccttaggtaacccagtagatccagaggaattcattatcagtgcaattgttttg tcacgatcaaaggactctggtacaaaatcgtattcattaaaaccgggaggtagatgagatgtgacgaacgtgt acatcgactgaaatccctggtaatccgttttagaatccatgataataattttctggattattggtaattttttttgc |
| | | acgttcaaaattttttgcaacccctttttggaaacaaacactacggtaggctgcgaaatgttcatactgttgagc aattcacgttcattataaatgtcgttcgcgggcgcaactgcaactccgataaataacgcgcccaacaccggcat aaagaattgaagagagttttcactgcatacgacgattctgtgatttgtattcagcccatatcgtttcatagcttct gccaaccgaacggacatttcgaagtattccgcgtacgtgatgttcacctcgatatgtgcatctgtaaaagcaatt gttccaggaaccagggcgtatctcttcatagccttatgcagttgctctccagcggttccatcctctagaggatag aatggcgccgggcctttctttatgtttttggcgtcttccatgctagcggatctgacggttcactaaaccagctctgc ttatatagacctcccaccgtacacgcctaccgcccatttgcgtcaatggggcggagttgttacgacattttggaa agtcccgttgattttggtgccaaaacaaactcccattgacgtcaatggggtggagacttggaaatccccgtgag tcaaaccgctatccacgcccattgatgtactgccaaaaccgcatcaccatggtaatagcgatgactaatacgta gatgtactgccaagtaggaaagtcccataaggtcatgtactgggcataatgccaggcgggccatttaccgtcat tgacgtcaatagggggcgtacttggcatatgatacacttgatgtactgccaagtgggcagtttaccgtaaatact ccacccattgacgtcaatggaaagtccctattggcgttactatgggaacatacgtcattattgacgtcaatgggc gggggtcgttgggcggtcagccaggcgggccatttaccgtaagttatgtaacgcggaactccatatatgggcta tgaactaatgaccccgtaattgattactatTAGGcacgtgggctccggtgcccgtcagtgggcagagcgcac atcgcccacagtccccgagaagttggggggaggggtcggcaattgaaccggtgcctagagaaggtggcgcgg ggtaaactgggaaagtgatgtcgtgtactggctccgcctttttcccgagggtgggggagaaccgtatataagtg cagtagtcgccgtgaacgttctttttcgcaacgggtttgccgccagaacacaggtaagtgccgtgtgtggttccc gcgggcctggcctctttacgggttatggcccttgcgtgccttgaattacttccacctggctgcagtacgtgattctt gatcccgagcttcgggttggaagtgggtgggagagttcgaggccttgcgcttaaggagccccttcgcctcgtgc ttgagttgaggcctggcctgggcgctggggccgccgcgtgcgaatctggtggcaccttcgcgcctgtctcgctgc tttcgataagtctctagccatttaaaatttttgatgacctgctgcgacgctttttttctggcaagatagtcttgtaa atgcgggccaagatctgcacactggtatttcggtttttggggccgcgggcggcgacggggcccgtgcgtcccag cgcacatgttcggcgaggcggggcctgcgagcgcggccaccgagaatcggacgggggtagtctcaagctggc cggcctgctctggtgcctggcctcgcgccgccgtgtatcgccccgccctgggcggcaaggctggcccggtcggc accagttgcgtgagcggaaagatggccgcttcccggccctgctgcagggagctcaaaatggaggacgcggcg ctcgggagagcgggcgggtgagtcacccacacaaaggaaaagggcctttccgtcctcagccgtcgcttcatgt gactccactgagtaccgggcgccgtccaggcacctcgattagttctcgagcttttggagtacgtcgtctttaggtt ggggggaggggttttatgcgatggagtttccccacactgagtgggtggagactgaagttaggccagcttggca cttgatgtaattctccttggaatttgccctttttgagtttggatcttggttcattctcaagcctcagacagtggttca aagtttttttcttccatttcaggtgtcgtgatctagagACTAGTCCCCTAGCCATGGGATCCCAGTCC GGCgtcttcacactcgaagatttcgttggggactggcgacagacagccggctacaacctggaccaagtccttg aacagggaggtgtgtccagtttgtttcagaatcttttgtagaagggatatacaaagtgcGGgtgacggctaca acctggaccaagtccttgaacagggaggtgtgtccagtttgtttcagaatctcggggtgtccgtaactccgatcc aaaggattgtcctgagcggtgaaaatgggctgaagatcgacatccatgtcatcatcccgtatgaaggtctgag |
| | | cggcgaccaaatgggccagatcgaaaaaatttttaaggtggtgtaccctgtggatgatcatcactttaaggtga tcctgcactatggcacactggtaatcgacggggttacgccgaacatgatcgactatttcggacggccgtatgaa ggcatcgccgtgttcgacggcaaaaagatcactgtaacagggaccctgtggaacggcaacaaaattatcgac gagcgcctgatcaaccccgacggctccctgctgttccgagtaaccatcaacggagtgaccggctggcggctgt gcgaacgcattctggcgtaaGTCGACTGTGCCTTCTAGTTGCCAGCCATCTGTTGTTTGCCC CTCCCCCGTGCCTTCCTTGACCCTGGAAGGTGCCACTCCCACTGTCCTTTCCTAATAA AATGAGGAAATTGCATCGCATTGTCTGAGTAGGTGTCATTCTATTCTGGGGGGTGG GGTGGGGCAGGACAGCAAGGGGGAGGATTGGGAAGACAATAGCAGGCATGCTGG GGATGCGGTGGGCTCTATGGctcaagcttataactaatgcatggcggtaatacggttatccacagaa tcaggga |
| 98 | hHao1-Insertion sequence | TTTAGGACAGAGGGTCAGCATGCCcgggtga |
| 99 | hHao1-LUxUC sequence | gtcttcacactcgaagatttcgttggggactggcgacagacagccggctacaacctggaccaagtccttgaaca gggaggtgtgtccagtttgtttcagaa tctcttgggacagagggtcagcatgcccGGgtgacggctacaacctggaccaagtccttgaacagggaggtg tgtccagtttgtttcagaatctcggggtgtccgtaactccgatccaaaggattgtcctgagcggtgaaaatgggc tgaagatcgacatccatgtcatcatcccgtatgaaggtctgagcggcgaccaaatgggccagatcgaaaaaat ttttaaggtggtgtaccctgtggatgatcatcactttaaggtgatcctgcactatggcacactggtaatcgacgg ggttacgccgaacatgatcgactatttcggacggccgtatgaaggcatcgccgtgttcgacggcaaaaagatc actgtaacagggaccctgtggaacggcaacaaaattatcgacgagcgcctgatcaaccccgacggctccctgc tgttccgagtaaccatcaacggagtgaccggctggcggctgtgcgaacgcattctggcgtaa |
| 100 | T5 exonuclease nucleotide coding sequence | ATGTCAAAGAGTTGGGGCAAGTTCATTGAGGAGGAGGAAGCCGAAATGGCCAGTC GGCGCAATCTCATGATTGTTGACGGCACCAATCTCGGCTTCAGATTCAAGCACAACA ACTCCAAGAAACCCTTTGCTTCCAGTTATGTGAGCACGATTCAAAGCCTGGCCAAGT CATATTCTGCTCGCACTACGATTGTGCTGGGGGACAAAGGGAAAAGCGTGTTCAGA CTTGAGCACCTGCCAGAGTACAAAGGAAATCGAGATGAGAAGTACGCCCAAAGAAC CGAAGAGGAGAAGGCCCTGGATGAGCAGTTTTTCGAGTACCTGAAAGATGCTTTCG AGCTTTGCAAAACGACCTTCCCAACATTCACAATTCGCGGGGTTGAGGCCGATGACA TGGCAGCCTACATTGTCAAGTTGATCGGACACCTGTATGACCATGTGTGGCTTATCA GCACTGATGGAGACTGGGATACTCTTCTGACAGACAAAGTATCTCGGTTCAGCTTCA CCACGAGGCGAGAATATCACCTGAGGGATATGTACGAGCACCATAATGTGGACGAT GTTGAGCAGTTCATTTCTCTGAAGGCGATTATGGGCGACCTGGGGGACAATATAAG AGGAGTTGAGGGAATCGGGGCCAAGAGAGGGTACAACATCATACGAGAGTTTGGG |
| | | AACGTACTTGATATCATCGACCAGCTGCCATTGCCTGGGAAGCAAAAATATATACAG AACCTGAACGCTTCCGAAGAGTTGCTGTTTAGGAACCTGATCCTGGTGGATTTGCCC ACCTATTGCGTCGACGCAATAGCAGCAGTAGGTCAGGACGTGCTCGATAAATTTACC AAGGATATACTTGAGATCGCTGAGCAATAA |
| 101 | Target-hHao1-spacer | ggacagagggtcagcatgcc |

All the documents mentioned in the disclosure are incorporated herein by reference, as if each document was cited individually. In addition, it should be understood that, after reading the above-mentioned teachings of the disclosure, those skilled in the art can make various changes or modifications to the disclosure, and these equivalent forms also fall within the scope defined by the appended claims of the present application.

## Claims

1. A Cas protein, selected from the group consisting of:
(a) a polypeptide having the amino acid sequence represented by SEQ ID NO:1;
(b) a polypeptide having at least about 60%, e.g. 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5% identity to the amino acid sequence represented by SEQ ID NO:1; and
(c) a polypeptide obtained by substituting, deleting, or adding one or more amino acid residues, optionally about 1-50, preferably 1-10, about 1-40, about 1-30, about 2-25, preferably 2-20 amino acid residues, in the amino acid sequence represented by SEQ ID NO:1.

2. The Cas protein according to claim 1, containing mutations at one or more sites relative to the polypeptide having the amino acid sequence represented by SEQ ID NO:1, such that the cleavage activity or preference is altered, for example, such that the double-strand cleavage activity is increased or decreased, the single-strand cleavage activity is increased or decreased, or there is no cleavage activity.

3. The Cas protein according to claim 1 or 2, comprising any amino acid substitution(s) at sites corresponding to one or more of the following sites in SEQ ID NO: 1: V7, R8, T11, S12, S110, I149, N150, H151, N152, L153, Q160, E161, Y162, N163, C164, Y165, S166, S167, F168, K195, S196, K197, S198, A199, C215, T216, A217, K219, S225, L227, L229, M231, D234, S240, S241, Q242, E243, I244, S250, F251, E252, K253, V254, K260, T261, E263, N276, Y282, D283, A285, N292, S303, I304, L307, Y308, S309, R315, E316, T317, 1318, 1319, V348, I349, E350, P351, L354, S355, N356, L357, K358, I373, G416, I417, E418, F419, D420, I455, R456, V458, H486, N487, S488, L501, R509, P510, V511, L512, G513, N514, R515, V516, L525, I526, N527, K528, K529, C633, T634, T635, K636, N637, D638, R639, G640, E641, F642, E648, L650, A651, Y652, A661, T681, N682, E683, 5684, G727, K728, N729, E730, S743, E748, G749, S751, K752, K781, L782, G783, E784, C785, S787, K865, P866, Y867, N868, I872, D896, N936, M957, F958, Q960, W961, P1018, S1019, R1020, N1021, and S1022;
optionally, wherein the Cas protein contains one of the following mutation patterns relative to SEQ ID NO:1:
(a) M231+D234; preferably M231K+D234V;
(b) S240+S241+Q242+E243; preferably selected from S240A+S241R+Q242P+E243L, and S240L+S241A+Q242S+E243H+1244L+L307R+Y308F+S309A;
(c) L227+L229; preferably selected from L227I+L229R, and S225P+L227A+L229R;
(d) L307+Y308+5309; preferably selected from S240L+S241A+Q242S+E243H+I244L+L307R+Y308F+S309A, D283S+A285P+L307R+Y308F+S309A, L307R+Y308F+S309A+E648R+L650I+A651P+Y652F, D283S+A285P+L307R+Y308F+S309A+I373V+E748G+S751G+K752R+S787F, S303T+I304M+L307R+Y308A+S309I, S196N+K197F+S198N+A199T+I304A+L307R+Y308A+S309A, and L307R+Y308F+S309A;
(e) 5250+F251+E252+K253+V254; preferably S250W+F251A+E252A+K253R+V254R;
(f) K260+T261+E263; preferably K260R+T261P+E263H;
(g) R315+E316+T317+I318+I319; preferably selected from R315L+E316R+T317R+I318V+I319A, Y282F+D283Q+A285T+R315A+E316Q+T317R+I318L+I319Q, and Y282F+D283Q+A285T+R315S+E316R+T317K+I318K+I319W+E648S+A651L;
(h) L354+5355+N356+L357+K358; preferably L354F+S355G+N356D+L357I+K358R;
(i) E648+A651; preferably selected from E6485+A651L, E648S+A651T+Y652W, Y282F+D283Q+A285T+E648T+A651S+Y652F, Y282F+D283Q+A285T+E648L+A651G, N2765+D283N+E6485+A651L, and Y282F+D283Q+A285T+R315S+E316R+T317K+I318K+I319W+E648S+A651L;
(j) T681+N682+E683; preferably selected from
T681V+N682G+E683R+S684G,
T681P+N682T+E683G+S684R,
T681S+N682P+E683R+S684G,
T681A+N682H+E683Y+S684R,
Y282F+D283Q+A285T+T681P+N682P+E683Q+S684C, Y282F+D283Q+A285T+G416S+I417H+E418Q+F419T+D420V+L501P+T681N+N682P+E6 83G+S684A, Y165W+S166R+G416R+I417V+E418Q+F419V+D420T+T681D+N682A+E683R+S684D, Y165W+S166R+S198N+G416R+I417V+E418Q+F419V+D420T+T681S+N682R+E683R, Y165W+S166R+G416R+I417V+E418Q+F419V+D420T+R509N+P510G+V511G+L512S+T 681S+N682R+E683R, G416R+I417V+E418Q+F419V+D420T+T681S+N682R+E683R+G727V+K728C+N729G+E 730G, Y282F+D283Q+A285T+G416A+I417R+E418V+F419C+D420Q+R509V+P510R+V511L+L5 12A+T681S+N682R+E683R, Y165W+5166R+G416R+I417V+E418Q+F419V+D420T+R509Q+P510A+V511A+L512R+T 681S+N682R+E683R+A661V, S110G+Y165W+S166R+G416R+I417V+E418Q+F419V+D420T+R509A+P510A+V511R+L 512Q+T681S+N682R+E683R, Y165W+S166R+G416R+I417V+E418Q+F419V+D420T+R509L+P510V+V511S+L512F+T6 81S+N682R+E683R, Y282F+D283Q+A285T+G416A+I417R+E418V+F419C+D420Q+R509G+P510K+L512C+T6 81S+N682R+E683R, and Y165W+S166R+G416R+I417V+E418Q+F419V+D420T+H486A+S488W+T634V+T635Y+K 636S+N637R+T681S+N682R+E683R;
(k) Y165+5166; preferably selected from
Y165W+S166R,
Y165W+S166V+S167I+F168I, Y165W+S166R+G416R+I417V+E418Q+F419V+D420T, Y165W+S166R+G416R+I417E+E418Q+F419V+D420A, Y165W+S166R+G416R+I417V+E418Q+F419V+D420T+T681D+N682A+E683R+S684D, Y165W+S166R+S198N+G416R+I417V+E418Q+F419V+D420T+T681S+N682R+E683R, Y165W+S166R+G416R+I417V+E418Q+F419V+D420T+N682G+E683A+D896N, Y165W+S166R+G416R+I417V+E418Q+F419V+D420T+R509N+P510G+V511G+L512S+T 681S+N682R+E683R, Y165W+S166R+G416R+I417V+E418Q+F419V+D420T+G513L+N514A+R515V+V516M+ N682G+E683A+S684R+D896N, Y165W+S166R+G416R+I417V+E418Q+F419V+D420T+G513C+R515Y+V516M+N682G+ E683A+S684R+D896N, Y165W+S166R+G416A+I417R+E418V+F419C+D420Q+H486S+N487N+S488W+N682G+ E683A+S684R+D896N, Y165W+S166R+G416R+I417V+E418Q+F419V+D420T+R509Q+P510A+V511A+L512R+T 681S+N682R+E683R+A661V,
S110G+Y165W+S166R+G416R+I417V+E418Q+F419V+D420T+R509A+P510A+V511R+L 512Q+T681S+N682R+E683R, Y165W+S166R+G416R+I417V+E418Q+F419V+D420T+R509L+P510V+V511S+L512F+T6 81S+N682R+E683R, Y165W+S166R+G416R+I417V+E418Q+F419V+D420T+G513C+R515Y+V516M+T635S+K 636G+N637L+N682G+E683A+S684R+D896N, Y165W+S166R+G416R+I417V+E418Q+F419V+D420T+H486A+S488W+T634V+T635Y+K 636S+N637R+T681S+N682R+E683R, Y165W+S166R+G416A+I417R+E418V+F419C+D420Q+H486S+S488W+K781T+G783S+E 784F+C785L+D896N, Y165W+S166R+G416A+I417R+E418V+F419C+D420Q+H486S+S488W+D638F+R639P+ G640N+E641Y+F642L, and Y165W+S166R+G416R+I417V+E418Q+F419V+D420T+H486A+S488W+N682G+E683A+ S684R+P1018L+S1019R+R1020P+N1021R+S1022V;
(l) Q160+E161+Y162+N163+C164; preferably selected from Q160V+E161I+Y162T+N163S+C164V, Q160S+E161S+Y162L+N163C+C164V, Q160P+E161V+Y162H+N163S+C164T, and Q160L+E161S+Y162F+N163S+C164A;
(m) V7+T11+S12; preferably selected from V7H+T11R+S12M, V7F+R8L+T11R+S12V, and V7I+T11V+S12M+Y282F+D283Q+A285T;
(n) I149+N150+H151+N152+L153; preferably I149M+N150S+H151C+N152T+L153Y;
(o) K195+K197+5198+A199; preferably K195R+K197A+S198A+A199G;
(p) T216+A217+K219; preferably selected from T216L+A217T+K219R, and C215V+T216S+A217S+K219R;
(q) D283+A285; preferably selected from Y282F+D283Q+A285T, D283I+A285R+N292L, Y282F+D283Q+A285T+K865S+P866S+Y867H+N868F+I872L, Y282F+D283Q+A285T+M957V+F958L+Q960V+W961V, Y282F+D283Q+A285T+V348I+I349V+E350L+P351T, Y282F+D283Q+A285T+T681P+N682P+E683Q+S684C, Y282F+D283Q+A285T+E748A+G749S+S751G, Y282F+D283Q+A285T+G416S+I417H+E418Q+F419T+D420V, Y282F+D283Q+A285T+G416L+I417Q+E418M+F419R+D420A, Y282F+D283Q+A285T+G416A+I417R+E418V+F419C+D420Q, Y282F+D283Q+A285T+G416S+I417H+E418Q+F419T+D420V+L501P+N936S, Y282F+D283Q+A285T+G416S+I417H+E418Q+F419T+D420V+L501P+T681N+N682P+E6 83G+S684A, Y282F+D283Q+A285T+G416A+I417R+E418V+F419C+D420Q, Y282F+D283Q+A285T+G416L+I417Q+E418M+F419R+D420A+I455G+R456E+V458E, Y282F+D283Q+A285T+G416A+I417R+E418V+F419C+D420Q+R509C+P510S+L512F+L5 25R+I526V+N527D+K528P+K529G+N682G+E683A+S684R+D896N, Y282F+D283Q+A285T+G416A+I417R+E418V+F419C+D420Q+R509V+P510R+V511L+L5 12A+T681S+N682R+E683R, Y282F+D283Q+A285T+G416A+I417R+E418V+F419C+D420Q+P510D+V511H+L512V, Y282F+D283Q+A285T+G416A+I417R+E418V+F419C+D420Q+R509G+P510K+L512C+T6 81S+N682R+E683R, Y282F+D283Q+A285T+G416A+I417R+E418V+F419C+D420Q+R509C+P510S+L512F+N6 82G+E683A+S684R+G727E+K728H+N729Q+E730R, Y282F+D283Q+A285T+G416A+I417R+E418V+F419C+D420Q+R509C+P510S+L512F+N6 82G+E683A+S684R+S743T, Y282F+D283Q+A285T+G416A+I417R+E418V+F419C+D420Q+P510D+V511H+L512V+K 781H+L782I+G783F+E784R+C785S, Y282F+D283Q+A285T+G416A+I417R+E418V+F419C+D420Q+P510D+V511H+L512V+D 638P+E641M+F642V, Y282F+D283Q+A285T+G416A+I417R+E418V+F419C+D420Q+P510D+V511H+L512V+P 1018S+S1019R+R1020V+N1021M+S1022P, Y282F+D283Q+A285T+G416A+1417R+E418V+F419C+D420Q+R509C+P5105+L512F+C6 33N+T634E+T635E+K636G+N637L+N682G+E683A+S684R+S743T, Y282F+D283Q+A285T+G416A+1417R+E418V+F419C+D420Q+R509C+P5105+L512F+C6 33F+T634P+T635F+K636P+N637C+N682G+E683A+S684R+S743T, Y282F+D283Q+A285T+G416A+1417R+E418V+F419C+D420Q+R509C+P5105+L512F+C6 33S+T634C+T635G+K636H+N637F+N682G+E683A+S684R+S743T, and Y282F+D283Q+A285T+G416A+I417R+E418V+F419C+D420Q+R509C+P510S+L512F+C6 33R+T634Y+T635L+K636V+N637D+N682G+E683A+S684R+S743T; and
(r) G416+I417+E418+F419+D420; preferably selected from G416R+I417W+E418T+F419R+D420V, Y282F+D283Q+A285T+G416S+I417H+E418Q+F419T+D420V, Y165W+S166R+G416R+I417V+E418Q+F419V+D420T, Y282F+D283Q+A285T+G416L+I417Q+E418M+F419R+D420A, Y282F+D283Q+A285T+G416A+I417R+E418V+F419C+D420Q, Y165W+S166R+G416R+I417E+E418Q+F419V+D420A, Y282F+D283Q+A285T+G416S+I417H+E418Q+F419T+D420V+L501P+N936S, Y282F+D283Q+A285T+G416S+I417H+E418Q+F419T+D420V+L501P+T681N+N682P+E6 83G+S684A, Y165W+S166R+G416R+I417V+E418Q+F419V+D420T+T681D+N682A+E683R+S684D, Y165W+S166R+S198N+G416R+I417V+E418Q+F419V+D420T+T681S+N682R+E683R, Y165W+S166R+G416R+I417V+E418Q+F419V+D420T+N682G+E683A+D896N, Y282F+D283Q+A285T+G416A+I417R+E418V+F419C+D420Q, R509C+P510S+L512F+G416R+I417V+E418Q+F419V+D420T+H486A+S488W+N682G+E 683A+S684R+D896N, Y165W+S166R+G416R+I417V+E418Q+F419V+D420T+R509N+P510G+V511G+L512S+T 681S+N682R+E683R, Y165W+S166R+G416R+I417V+E418Q+F419V+D420T+G513L+N514A+R515V+V516M+ N682G+E683A+S684R+D896N, Y165W+S166R+G416R+I417V+E418Q+F419V+D420T+G513C+R515Y+V516M+N682G+ E683A+S684R+D896N, G416R+I417V+E418Q+F419V+D420T+T681S+N682R+E683R+G727V+K728C+N729G+E 730G, Y282F+D283Q+A285T+G416L+I417Q+E418M+F419R+D420A+I455G+R456E+V458E, Y165W+S166R+G416A+I417R+E418V+F419C+D420Q+H486S+N487N+S488W+N682G+ E683A+S684R+D896N, Y282F+D283Q+A285T+G416A+I417R+E418V+F419C+D420Q+R509C+P510S+L512F+L5 25R+I526V+N527D+K528P+K529G+N682G+E683A+S684R+D896N, Y282F+D283Q+A285T+G416A+I417R+E418V+F419C+D420Q+R509V+P510R+V511L+L5 12A+T681S+N682R+E683R, Y165W+S166R+G416R+I417V+E418Q+F419V+D420T+R509Q+P510A+V511A+L512R+T 681S+N682R+E683R+A661V, Y282F+D283Q+A285T+G416A+I417R+E418V+F419C+D420Q+P510D+V511H+L512V, S110G+Y165W+S166R+G416R+I417V+E418Q+F419V+D420T+R509A+P510A+V511R+L 512Q+T681S+N682R+E683R, Y165W+S166R+G416R+I417V+E418Q+F419V+D420T+R509L+P510V+V511S+L512F+T6 81S+N682R+E683R, Y282F+D283Q+A285T+G416A+I417R+E418V+F419C+D420Q+R509G+P510K+L512C+T6 81S+N682R+E683R, Y282F+D283Q+A285T+G416A+I417R+E418V+F419C+D420Q+R509C+P510S+L512F+N6 82G+E683A+S684R+G727E+K728H+N729Q+E730R, Y165W+S166R+G416R+I417V+E418Q+F419V+D420T+G513C+R515Y+V516M+T635S+K 636G+N637L+N682G+E683A+S684R+D896N, Y165W+S166R+G416R+I417V+E418Q+F419V+D420T+H486A+S488W+T634V+T635Y+K 636S+N637R+T681S+N682R+E683R, Y282F+D283Q+A285T+G416A+I417R+E418V+F419C+D420Q+R509C+P510S+L512F+N6 82G+E683A+S684R+S743T, Y282F+D283Q+A285T+G416A+I417R+E418V+F419C+D420Q+P510D+V511H+L512V+K 781H+L782I+G783F+E784R+C785S, Y165W+S166R+G416A+I417R+E418V+F419C+D420Q+H486S+S488W+K781T+G783S+E 784F+C785L+D896N, Y282F+D283Q+A285T+G416A+I417R+E418V+F419C+D420Q+P510D+V511H+L512V+D 638P+E641M+F642V, Y165W+S166R+G416A+I417R+E418V+F419C+D420Q+H486S+S488W+D638F+R639P+ G640N+E641Y+F642L, Y165W+S166R+G416R+I417V+E418Q+F419V+D420T+H486A+S488W+N682G+E683A+ 5684R+P1018L+S1019R+R1020P+N1021R+S1022V, Y282F+D283Q+A285T+G416A+I417R+E418V+F419C+D420Q+P510D+V511H+L512V+P 1018S+S1019R+R1020V+N1021M+S1022P, Y282F+D283Q+A285T+G416A+1417R+E418V+F419C+D420Q+R509C+P5105+L512F+C6 33N+T634E+T635E+K636G+N637L+N682G+E683A+S684R+S743T, Y282F+D283Q+A285T+G416A+1417R+E418V+F419C+D420Q+R509C+P5105+L512F+C6 33F+T634P+T635F+K636P+N637C+N682G+E683A+S684R+S743T, Y282F+D283Q+A285T+G416A+1417R+E418V+F419C+D420Q+R509C+P5105+L512F+C6 33S+T634C+T635G+K636H+N637F+N682G+E683A+S684R+S743T, and Y282F+D283Q+A285T+G416A+I417R+E418V+F419C+D420Q+R509C+P510S+L512F+C6 33R+T634Y+T635L+K636V+N637D+N682G+E683A+S684R+S743T;
and/or
(2) wherein the Cas protein contains amino acid substitutions at sites corresponding to one or more of the following sites in SEQ ID NO: 1: D592, D643, E820, and D992;
optionally, relative to SEQ ID NO: 1, the Cas protein contains mutation(s) selected from D592A, D643A, E820A and/or D992A;
preferably, the Cas protein comprises an amino acid sequence represented by any one of SEQ ID NO: 47-50, or an amino acid sequence having at least about 80%, e.g., at least about 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% or 100% sequence identity to an amino acid sequence represented by any one of SEQ ID NO: 47-50.

4. A fusion protein, comprising the Cas protein according to any one of claims 1-3; and one or more functional domains;
optionally, wherein the functional domain is selected from a nuclear localization signal (NLS), a nuclear export signal (NES), a reporter protein (such as fluorescent protein), a Cas protein targeting moiety, a DNA-binding domain (such as Lex A DBD, Gal4 DBD, and Sp1 DBD), an epitope tag (such as His, myc, V5, FLAG, HA, VSV-G, etc.), a transcriptional activation domain (such as VP64, VPR, p65, and Rta), a transcriptional repression domain (such as KRAB domain, SID domain, NuE domain, NcoR domain or SID4X domain), a nuclease, a deaminase (such as adenosine deaminase or cytidine deaminase), a methylase (such as DNA methylase DNMT), a demethylase, a transcription release factor, HDAC, a cleavage-active polypeptide, a ligase, an integrase, a transposase, a recombinase, a polymerase, an exonuclease (such as T5E) and a base-excision repair inhibitor (such as uracil-DNA glycosylase inhibitor (UGI));
optionally, wherein the functional domain includes one or more of the following enzymatic activities on a target sequence: methylase activity, demethylase activity, acetyltransferase activity, deacetylase activity, kinase activity, phosphatase activity, ubiquitin ligase activity, deubiquitination activity, adenylation activity, deadenylation activity, SUMOylation activity, deSUMOylation activity, ribosylation activity, deribosylation activity, myristoylation activity, demyristoylation activity, glycosylation activity (e.g., from O-GlcNAc transferase) and deglycosylation activity;
optionally, wherein the functional domain is selected from an adenosine deaminase catalytic domain or a cytidine deaminase catalytic domain;
optionally, wherein the adenosine deaminase catalytic domain or cytidine deaminase catalytic domain includes one or more of ADAR1, ADAR2, APOBEC, AID or TAD;
optionally, wherein the adenosine deaminase catalytic domain comprises an amino acid sequence having at least 80%, 82%, 85%, 87%, 90%, 92%, 95%, 96%, 97%, 98% or 99% identity to the amino acid sequence represented by SEQ ID NO: 31 (the deaminase 005V1 from CN114634923A, the amino acid sequence SEQ ID NO: 2 in that application) or SEQ ID NO: 70 (the deaminase 004V1 from CN114634923A, the amino acid sequence SEQ ID NO: 1 in that application), and retains the deamination activity of the amino acid sequence represented by SEQ ID NO: 31;
optionally, wherein the amino acid sequence of the adenosine deaminase catalytic domain has amino acid addition, insertion, deletion, or substitution relative to the amino acid sequence represented by SEQ ID NO: 31 or 70;
optionally, wherein the adenosine deaminase catalytic domain includes a mutant of the amino acid sequence represented by SEQ ID NO: 31 or 70;
optionally, wherein the functional domain is the full-length TadA8e or a functional fragment of TadA8e;
optionally, wherein the localization signal includes nuclear localization signal (NLS) and/or nuclear export signal (NES);
optionally, wherein the sequence of the nuclear localization signal is represented by any one of SEQ ID NO: 38-45;
optionally, wherein the sequence of the nuclear localization signal is located at, close to, or near an end, e.g. N-terminus or C-terminus, of the Cas protein according to claim 1;
optionally, wherein the nuclear export signal includes protein tyrosine kinase 2 (such as human protein tyrosine kinase 2);
optionally, wherein the reporter protein includes glutathione-S-transferase (GST), horseradish peroxidase (HRP), chloramphenicol acetyltransferase (CAT), β-galactosidase, β-glucuronidase, or autofluorescent protein;
optionally, wherein the autofluorescent protein includes green fluorescent protein (e.g., GFP, GFP-2, tagGFP, turboGFP, eGFP, CopGFP, AceGFP), HcRed, DsRed, cyan fluorescent protein (e.g., eCFP, Cerulean, CyPet, AmCyanl), yellow fluorescent protein (e.g., YFP, eYFP, Citrine, Venus, YPet, PhiYFP), or blue fluorescent protein (e.g., eBFP, eBFP2, Azurite, mKalamal, GFPuv, Sapphire, T-sapphire);
optionally, wherein the DNA-binding domain includes a methyl-binding protein, LexA DBD, or Gal4 DBD;
optionally, wherein the epitope tag includes a histidine tag, a V5 tag, a FLAG tag, an influenza virus hemagglutinin tag, a Myc tag, a VSV-G tag, a thioredoxin tag, or a streptavidin tag;
optionally, wherein the transcriptional activation domain includes VP64 and/or VPR;
optionally, wherein the transcriptional repression domain includes KRAB and/or SID;
optionally, wherein the nuclease includes Fokl;
optionally, wherein the cleavage-active polypeptide includes a polypeptide with single-strand RNA cleavage activity, a polypeptide with double-strand RNA cleavage activity, a polypeptide with single-strand DNA cleavage activity, or a polypeptide with double-strand DNA cleavage activity;
optionally, wherein the ligase includes DNA ligase and/or RNA ligase;
optionally, wherein the exonuclease is selected from TREX2 protein, TREX1 protein, APE1 protein, Artemis protein, CtIP protein, Exo1 protein, Mre11 protein, RAD1 protein, RAD9 protein, Tp53 protein, WRN protein, exonuclease V, T5 exonuclease or T7 exonuclease, or variants thereof;
optionally, wherein the exonuclease is T5 exonuclease, and optionally, the T5 exonuclease comprises the amino acid sequence represented by SEQ ID NO: 81;
optionally, wherein the functional domain is linked to the N-terminus, and/or C-terminus of the Cas protein variant;
optionally, wherein the functional domain is inserted between the N-terminus and C-terminus of the Cas protein variant;
optionally, wherein the one or more functional domains are linked to the N-terminus and/or C-terminus of the Cas protein variant through a linker;
optionally, wherein the fusion protein comprises the amino acid sequence represented by any one of SEQ ID NO: 46, 84, and 86.

5. An isolated polynucleotide, which encodes the Cas protein according to any one of claims 1-3 or the fusion protein according to claim 4; preferably, wherein the polynucleotide has been codon-optimized for expression in a eukaryotic cell;
optionally, wherein the polynucleotide comprises a nucleotide sequence having at least about 80%, e.g., at least about 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% or 100% sequence identity to the nucleotide sequence represented by any one of SEQ ID NO: 2, 37, 83, 85, and 87;
optionally, wherein the polynucleotide comprises the nucleotide sequence represented by any one of SEQ ID NO: 2, 37, 83, 85, and 87.

6. A guide RNA (gRNA), comprising:
(1) a direct repeat (DR) sequence, which can form a complex with the Cas protein according to any one of claims 1-3 or with the fusion protein according to claim 4;
preferably, wherein the direct repeat (DR) sequence comprises a nucleotide sequence represented by any one of SEQ ID NO. 5 and 71, or comprises a nucleotide sequence having at least about 50%, e.g., at least 55%, 60%, 65%, 70%, 75%, 80%, 85%, 90%, 91%, 92%, 93%, 94%, 95%, 96%, 97%, 98%, 99%, or 100% identity to the nucleotide sequence represented by any one of SEQ ID NO. 5 and 71;
more preferably, wherein the DR sequence comprises a stem-loop structure near the 3' end of the DR sequence, and the stem-loop structure comprises 5'-X₁X₂X₃X₄X₅NNNNNNNX₆X₇X₈X₉X₁₀-3'; wherein X₁, X₂, X₃, X₄, X₅, X₆, X₇, X₈, X₉, and X₁₀ are any bases of A, T, C, or G, and N is any base of A, T, C, or G; wherein X₁, X₂, X₃, X₄, X₅ can hybridize to X₆, X₇, X₈, X₉, X₁₀ to form a stem and cause the NNNNNNN to form a loop;
more preferably, wherein the DR sequence contains, near the 3' end of the DR sequence, a stem-loop structure of 5'-CCGTCNNNNNNNGACGG-3' (SEQ ID NO:80), wherein N is any base of A, T, C, or G;
(2) a spacer sequence, capable of hybridizing to a target sequence of a target DNA, thereby guiding the complex to the target DNA;
optionally, wherein the length of the spacer sequence is at least 15 nt, for example, the length of the spacer sequence is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70 nt;
optionally, the length of the spacer sequence is 15-50 nt;
optionally, the length of the spacer sequence is 18-41 nt;
optionally, the length of the spacer sequence is 18-27 nt;
optionally, the length of the spacer sequence is 18-24 nt;
optionally, the length of the spacer sequence is 18-22 nt;
optionally, wherein the 5' end of the direct repeat sequence is linked to the spacer sequence;
optionally, wherein the spacer sequence comprises at least 15 consecutive nucleotides in the nucleotide sequence represented by any one of SEQ ID NO: 6, 11, 53, 55, 57, 59, 61, and 101.

7. An isolated nucleic acid molecule, comprising or consisting of a sequence selected from the group consisting of:
(i) the sequence represented by SEQ ID NO: 5 or 71;
(ii) a sequence obtained by substitution, deletion, or addition of one or more bases, e.g., substitution, deletion, or addition of 1, 2, 3, 4, 5, 6, 7, 8, 9, or 10 bases, in the sequence represented by SEQ ID NO: 5 or 71;
(iii) a sequence having at least 20%, at least 25%, at least 30%, at least 35%, at least 40%, at least 50%, at least 60%, at least 70%, at least 80%, at least 90%, at least 95%, or 100% sequence identity to the sequence represented by SEQ ID NO: 5 or 71;
(iv) a sequence that hybridizes to the sequence according to any one of (i)-(iii) under a stringent condition;
(v) a sequence complementary to the sequence according to any one of (i)-(iii); or
(vi) a coding nucleotide sequence of the sequence according to any one of (i)-(v);
and wherein the sequence according to any one of (ii)-(vi) substantially retains the biological function of the sequence from which it is derived;
for example, wherein the isolated nucleic acid molecule is RNA;
for example, wherein the isolated nucleic acid molecule comprises a direct repeat (DR) sequence in a CRISPR/Cas system.

8. A vector, comprising the polynucleotide according to claim 5, and/or a polynucleotide encoding the guide RNA according to claim 6;
optionally, wherein the vector includes a plasmid or a viral vector;
optionally, wherein the viral vector is selected from the group consisting of adeno-associated virus (AAV), adenovirus, lentivirus, retrovirus, herpesvirus, SV40, poxvirus, or combinations thereof;
optionally, wherein the vector includes a cloning vector, a transformation vector, an expression vector, a shuttle vector, an integration vector, or a multifunctional vector;
optionally, wherein the vector comprises:
(1) a first regulatory element, which is operably linked to a nucleotide sequence encoding the Cas protein according to any one of claims 1-3 or a nucleotide sequence encoding the fusion protein according to claim 4; and
(2) a second regulatory element, which is operably linked to a nucleotide sequence encoding a guide RNA, wherein the guide RNA comprises:
(a) a direct repeat (DR) sequence capable of forming a complex with the Cas protein or fusion protein, and
(b) a spacer sequence capable of hybridizing to a target sequence of a target DNA, thereby guiding the complex to the target DNA;
optionally, wherein the first regulatory element and the second regulatory element are located in the same vector or in different vectors;
optionally, wherein the first regulatory element and/or the second regulatory element is a promoter, such as an inducible promoter;
optionally, wherein the vector contains one or more promoters, and the promoter is operably linked to the nucleic acid sequence, an enhancer, a transcription termination signal, a polyadenylation sequence, an origin of replication, a selectable marker, a nucleic acid restriction site, and/or a homologous recombination site.

9. A complex, comprising
(i) a protein component selected from the group consisting of the Cas protein according to any one of claims 1-3, the fusion protein according to claim 4, or a combination thereof; and
(ii) the guide RNA according to claim 6.

10. A CRISPR-Cas system, comprising:
(i) the Cas protein according to any one of claims 1-3 or the fusion protein according to claim 4, or a polynucleotide encoding the Cas protein or the fusion protein; and
(ii) the guide RNA according to claim 6, or a polynucleotide encoding the guide RNA; wherein the guide RNA comprises:
(a) a direct repeat (DR) sequence capable of forming a complex with the Cas protein or fusion protein, and
(b) a spacer sequence capable of hybridizing to a target sequence of a target DNA, thereby guiding the complex to the target DNA.

11. A CRISPR-Cas composition, comprising:
(i) a first component selected from the group consisting of the Cas protein according to any one of claims 1-3, the fusion protein according to claim 4, a nucleotide sequence encoding the Cas protein according to any one of claims 1-3 or the fusion protein according to claim 4, and any combination thereof; and
(ii) a second component, which comprises one or more guide RNAs according to claim 6, or a nucleotide sequence encoding one or more guide RNAs according to claim 6;
wherein the guide RNA comprises:
(a) a direct repeat (DR) sequence capable of forming a complex with the Cas protein or fusion protein, and
(b) a spacer sequence capable of hybridizing to a target sequence of a target DNA, thereby guiding the complex to the target DNA.

12. A CRISPR-Cas system comprising one or more vectors, wherein the one or more vectors comprise:
(i) a first nucleic acid, which is a nucleotide sequence encoding the Cas protein according to any one of claims 1-3 or the fusion protein according to claim 4; optionally, the first nucleic acid is operably linked to a first regulatory element; and
(ii) a second nucleic acid, which encodes a nucleotide sequence comprising the guide RNA according to claim 6; optionally, the second nucleic acid is operably linked to a second regulatory element;
wherein:
the first nucleic acid and the second nucleic acid are present in the same vector or in different vectors;
the guide RNA comprises:
(a) a direct repeat (DR) sequence capable of forming a complex with the Cas protein or fusion protein, and
(b) a spacer sequence capable of hybridizing to a target sequence of a target DNA, thereby guiding the complex to the target DNA.

13. A kit, comprising one or more components selected from the group consisting of: the Cas protein according to any one of claims 1-3, the fusion protein according to claim 4, the polynucleotide according to claim 5, the vector according to claim 8, the complex according to claim 9, the CRISPR-Cas system according to claim 10, the CRISPR-Cas composition according to claim 11, or the CRISPR-Cas system according to claim 12.

14. A delivery composition, comprising: a delivery vehicle, and one or more selected from the group consisting of: the Cas protein according to any one of claims 1-3, the fusion protein according to claim 4, the polynucleotide according to claim 5, the vector according to claim 8, the complex according to claim 9, the CRISPR-Cas system according to claim 10, the CRISPR-Cas composition according to claim 11, or the system according to claim 12.

15. A host cell, comprising the Cas protein according to any one of claims 1-3, the fusion protein according to claim 4, the polynucleotide according to claim 5, the vector according to claim 8, the complex according to claim 9, the CRISPR-Cas system according to claim 10, the CRISPR-Cas composition according to claim 11, the system according to claim 12, or the delivery composition according to claim 14.

16. An enzyme preparation, comprising the Cas protein according to any one of claims 1-3, the fusion protein according to claim 4, the complex according to claim 9, the CRISPR-Cas system according to claim 10, the CRISPR-Cas composition according to claim 11, the system according to claim 12, or the delivery composition according to claim 14.

17. A pharmaceutical kit, comprising:
a first container, and in the first container, the complex according to claim 9, the CRISPR-Cas system according to claim 10, the CRISPR-Cas composition according to claim 11, or the system according to claim 12, or a drug containing the complex according to claim 9, the CRISPR-Cas system according to claim 10, the CRISPR-Cas composition according to claim 11, or the system according to claim 12.

18. A pharmaceutical kit, comprising:
(a1) a first container, and in the first container, the Cas protein according to any one of claims 1-3, or the fusion protein according to claim 4, or an encoding gene or expression vector thereof, or a drug containing the Cas protein variant according to any one of claims 1-3, or the fusion protein according to claim 4, or an encoding gene or expression vector thereof;
(b1) optionally a second container, and in the second container, the guide RNA according to claim 6 or its expression vector, or a drug containing the guide RNA according to claim 6 or its expression vector.

19. A method for targeting and editing a target gene or cleaving a target gene, comprising: bringing the Cas protein according to any one of claims 1-3, or the fusion protein according to claim 4, or the complex according to claim 9, or the CRISPR-Cas system according to claim 10, the composition according to claim 11, or the system according to claim 12, or the delivery composition according to claim 14, or the enzyme preparation according to claim 16, or the pharmaceutical kit according to claim 17 or 18 into contact with the target gene; or delivering it into a cell containing the target gene, wherein the target sequence is present in the target gene.

20. A method for inducing a change in the state of a cell, comprising: contacting a target gene in the cell with the Cas protein according to any one of claims 1-3, or the fusion protein according to claim 4, or the complex according to claim 9, or the CRISPR-Cas system according to claim 10, the composition according to claim 11, or the system according to claim 12, or the delivery composition according to claim 14, or the enzyme preparation according to claim 16, or the pharmaceutical kit according to claim 17 or 18.

21. A method for altering the expression of a gene product, comprising: bringing the Cas protein according to any one of claims 1-3, or the fusion protein according to claim 4, or the complex according to claim 9, or the CRISPR-Cas system according to claim 10, the composition according to claim 11, or the system according to claim 12, or the delivery composition according to claim 14, or the enzyme preparation according to claim 16, or the pharmaceutical kit according to claim 17 or 18 into contact with a nucleic acid molecule encoding the gene product; or delivering it into a cell containing the nucleic acid molecule, wherein the target sequence is present in the nucleic acid molecule.

22. Use of the Cas protein according to any one of claims 1-3, the fusion protein according to claim 4, the polynucleotide according to claim 5, the vector according to claim 8, the complex according to claim 9, the CRISPR-Cas system according to claim 10, the CRISPR-Cas composition according to claim 11, or the system according to claim 12, or the kit according to claim 13, or the delivery composition according to claim 14, or the enzyme preparation according to claim 16, or the pharmaceutical kit according to claim 17 or 18, in the manufacture of a medicament or formulation for nucleic acid editing, e.g. gene editing or genome editing.

23. Use of the Cas protein according to any one of claims 1-3, the fusion protein according to claim 4, the polynucleotide according to claim 5, the vector according to claim 8, the complex according to claim 9, the CRISPR-Cas system according to claim 10, the CRISPR-Cas composition according to claim 11, or the system according to claim 12, or the kit according to claim 13, or the delivery composition according to claim 14, or the enzyme preparation according to claim 16, or the pharmaceutical kit according to claim 17 or 18, in the manufacture of a medicament or formulation for one or more selected from the group consisting of:
*(i) ex-vivo* gene editing or genome editing;
(ii) *ex-vivo* detection of a single-stranded DNA;
(iii) modifying an organism or a non-human organism by editing a target sequence in a target locus;
(iv) treating a disorder caused by a defect in a target sequence in a target locus; and
(v) treating a disorder or disease of a subject in need.

24. A method for detecting the presence of a target nucleic acid molecule in a sample, comprising:
contacting the sample with the Cas protein according to any one of claims 1-3, the fusion protein according to claim 4, or the complex according to claim 9, the CRISPR-Cas system according to claim 10, the CRISPR-Cas composition according to claim 11, or the system according to claim 12, the kit according to claim 13, or the delivery composition according to claim 14, or the enzyme preparation according to claim 16, and a non-target sequence; and detecting a detectable signal generated by the cleavage of the non-target sequence, thereby detecting the target nucleic acid molecule, wherein the non-target sequence does not hybridize to the guide RNA.

25. A method for diagnosing, preventing, or treating a disease in a subject in need, comprising: administering to the subject the vector according to claim 8, the complex according to claim 9, the CRISPR-Cas system according to claim 10, the CRISPR-Cas composition according to claim 11, or the system according to claim 12, or the kit according to claim 13, or the delivery composition according to claim 14, or the enzyme preparation according to claim 16, or the pharmaceutical kit according to claim 17 or 18, wherein the disease is associated with a target DNA, the spacer sequence is capable of hybridizing to a target sequence of the target DNA, and the target DNA is modified, thereby diagnosing, preventing, or treating the disease.

26. A pharmaceutical composition, comprising: the vector according to claim 8, the complex according to claim 9, the CRISPR-Cas system according to claim 10, the CRISPR-Cas composition according to claim 11, or the system according to claim 12, or the kit according to claim 13, or the delivery composition according to claim 14, or the host cell according to claim 15, or the enzyme preparation according to claim 16; and a pharmaceutically acceptable carrier or excipient.
